# EUROPEAN PATENT APPLICATION

(11) **EP 4 813 993 A1**
(43) Date of publication of application: **30.09.2026**
(21) Application number: 24893391.3
(22) Date of filing: 18.11.2024
(51) Int. Cl.: C07F 5/02, A61P 29/00, A61K 31/69, A61K 31/381

(54) **COMPOUND, PREPARATION METHOD THEREFOR, AND USE THEREOF**

(30) Priority: 20.11.2023 CN 202311551257
(71) Applicant: Sichuan Kelun Pharmaceutical Research Institute Co., Ltd., Chengdu, Sichuan 611138 (CN); Suzhou Kelun Pharmaceutical Research Co., Ltd., Suzhou, Jiangsu 215000 (CN)
(72) Inventor: CAO, Gang, Chengdu, Sichuan 611138 (CN); PAN, Junzhu, Chengdu, Sichuan 611138 (CN); ZHAO, Dong, Chengdu, Sichuan 611138 (CN); CHEN, Cheng, Chengdu, Sichuan 611138 (CN); SONG, Zhuang, Chengdu, Sichuan 611138 (CN); ZHANG, Shenglie, Chengdu, Sichuan 611138 (CN); LUO, Wei, Chengdu, Sichuan 611138 (CN); WU, Lingjing, Chengdu, Sichuan 611138 (CN)
(74) Representative: Ipsilon
(86) International application number: PCT/CN2024/132708
(87) International publication number: WO 2025/108234

(57) **Abstract**

The present invention relates to the field of biomedicine, in particular to a compound, a preparation method therefor, and a use thereof. In particular, the present application relates to a compound represented by formula (I) or a pharmaceutically acceptable salt, stereoisomer, solvate, isotope-labeled compound, or polymorph thereof, a preparation method therefor, a pharmaceutical composition comprising same, and a use thereof. The compound can be used as a sodium ion channel inhibitor and has an analgesic effect. The compound of the present application also has a plurality of excellent properties.

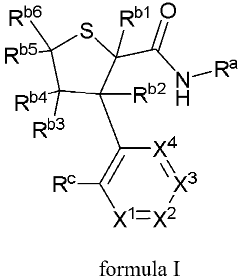

## Description

### TECHNICAL FIELD

The present disclosure relates to the field of biomedicine, and in particular relates to a class of compounds, or a pharmaceutically acceptable salt, a stereoisomer, a solvate, an isotopically labeled compound, or a polymorph thereof, a preparation method therefor, a pharmaceutical composition comprising same, and use thereof.

### BACKGROUND

Pain is one of the most common clinical symptoms and the fifth vital sign following respiration, pulse, blood pressure, and body temperature, and seriously affects the quality of life of a patient. According to statistics, the global analgesic market was valued at about $36 billion in 2018, and is expected to reach $56 billion in 2023. Acute moderate-to-severe pain relies primarily on opioid drugs, which account for about two-thirds of the market of analgesics. The market share is expected to grow steadily at a compound annual growth rate of 2.5% in the future. The number of patients with chronic pain, which mainly includes neuropathic pain and arthritic pain, is increasing year by year, and the market is expected to show a compound annual growth rate of about 18%, which may be a major driving force for the continuous growth of the global pain market in the next decade.

Neuropathic pain is a chronic pain resulting from injury or diseases of the peripheral somatosensory nervous system, whose symptoms include spontaneous pain and hyperalgesia in response to normally innocuous stimuli. Common causes of neuropathic pain include: diabetes, herpes zoster, spinal cord injury, stroke, multiple sclerosis, cancer, HIV infection, lumbar or cervical radiculopathy, traumatic or post-operative nerve damage, and the like. Osteoarthritis, also known as degenerative arthritis, is characterized by the degeneration of articular cartilage caused by multiple factors, which can cause unevenness of the articular bone surface and possibly the formation of bone spurs, and the clinical manifestations are mainly joint pain and joint stiffness. Long-term pain not only affects the sleep, work, and living ability of patients, but also increases the incidence of emotional disorders such as depression or anxiety, thereby bringing a heavy economic burden to patients' families and society.

Opioid receptors (µ, δ, and κ) are widely present in the central nervous system and the peripheral nervous system. Traditional opioid receptor agonists (e.g., morphine and its derivatives) are the most effective drugs for the treatment of moderate-to-severe pain caused by chronic arthritis, inflammatory neuralgia, post-operative pain, and various cancers.

CN 101627049 B discloses a class of synthetic peptide amides with polypeptide chains, which can be used as ligands for κ-opioid receptors. Further research is required to evaluate the feasibility of developing these compounds into drugs.

Inward currents of voltage-gated sodium channels (Nav) are an important part of the generation and transmission of action potentials of central and peripheral neurons, and increased neuronal excitability or increased responsiveness to stimuli are important mechanisms for the generation and development of various types of pain. Therefore, the role of voltage-gated sodium channels in pain signaling pathways, particularly in peripheral sensory neurons, has been a hot spot in pain research.

There are 9 human sodium ion channels: Nav1.1 to Nav1.9. Nav1.5, Nav1.8, and Nav1.9 are tetrodotoxin (TTX)-resistant sodium channels. Nav1.8 is mainly expressed on afferent neurons including sensory neurons, and plays an important role in maintaining the excitability of nociceptive sensory neurons, initiating and sustaining action potentials, regulating pain sensitivity, and the like by controlling sodium ions entering and exiting cells. Patients with NaV1.8-activating mutations may suffer from paroxysmal pain caused by small fiber neuropathy (damage to an Aδ fiber and an unmyelinated C fiber mainly responsible for pain transmission). Diseases such as chronic inflammation, diabetes, and the like may cause increased expression or altered properties of NaV1.8 to sensitize the nociceptive sensory neurons and cause various pains. NaV1.8 gene-knockout mice are insensitive to pains.

With the established role of Nav1.8 in chronic pain, drug research targeting this channel has been increasingly active. Currently, one small-molecule inhibitor is undergoing Phase III clinical trial recruitment globally, multiple other small-molecule inhibitors and antibodies are in preclinical development, and domestically, one small-molecule inhibitor developed by Jemincare is in Phase I clinical trials. Leading the research and development pipeline is VX-548, a small-molecule NaV1.8 blocker developed by Vertex Pharmaceuticals Inc. of the United States. To date, it has completed Phase II clinical trials in patients with acute pain following bunionectomy and abdominoplasty, yielding positive results, and Vertex Pharmaceuticals Inc. is now preparing for Phase III clinical trials. In addition, the mechanism of action and Phase II clinical trial data of NaV1.8 blockers have demonstrated their broad applicability, covering multiple pain types including neuropathic pain, osteoarthritis pain, and acute injury pain. They also exhibit favorable safety profiles, with no risk of addiction, and none of the gastrointestinal or cardio-cerebrovascular side effects associated with non-steroidal anti-inflammatory drugs. Furthermore, they can be combined with other analgesics to enhance efficacy and reduce side effects.

### SUMMARY

In one aspect, the present application provides a compound of formula I, or a pharmaceutically acceptable salt, a stereoisomer, a solvate, an isotopically labeled compound, or a polymorph thereof. Such compounds can be used as sodium ion channel inhibitors and have an analgesic effect. The compound of the present application also has various excellent properties. For example, the compound not only has stronger analgesic activity *in vitro* and *in vivo,* but also has superior pharmacokinetic profiles. The compound has the following structure: 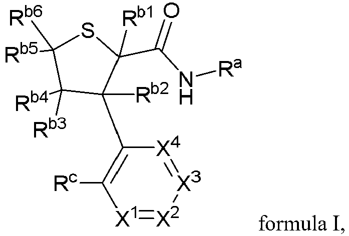 wherein:
R^{a} is selected from 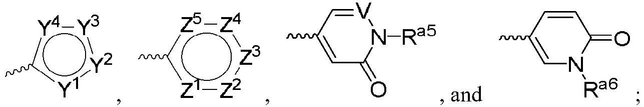
Y¹, Y², Y³, and Y⁴ are each independently selected from O, S, N, N-R^{a1}, and C-R^{a2};
each R^{a1} is independently selected from H, C₁₋₆ alkyl, C₁₋₆ haloalkyl, and -S(O)₂R¹;
each R^{a2} is independently selected from H, halogen, hydroxy, -CN, C₁₋₆ alkyl, C₁₋₆ haloalkyl, C₁₋₆ alkoxy, C₁₋₆ haloalkoxy, C₂₋₆ alkenyl, C₁₋₆ cycloalkyl, -NR²R³, -NHC(O)R⁴, -C(O)OR⁵, -C(O)NR⁶R⁷, SR⁸, -S(O)R⁹, -S(O)₂R¹⁰, - S(O)₂NR¹¹R¹², -S(O)(NR¹³)R¹⁴, -P(O)R¹⁵R¹⁶, and 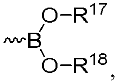 wherein the C₁₋₆ alkyl, C₁₋₆ haloalkyl, C₁₋₆ alkoxy, and C₁₋₆ haloalkoxy are optionally substituted with one or more substituents selected from hydroxy and -NR¹⁹R²⁰; or adjacent R^{a1} and R^{a2}, or two R^{a2}, together with the carbon atoms to which they are attached, form a 5-6 membered heteroaromatic ring;
R¹, R⁵, R⁶, R⁷, R⁸, R⁹, R¹⁰, R¹¹, R¹², R¹³, R¹⁴ , R¹⁵, R¹⁶, R¹⁹, and R²⁰ are each independently selected from H and C₁₋₆ alkyl;
R² and R³ are each independently selected from H, C₁₋₆ alkyl, and C₁₋₆ alkyl substituted with carbonyl;
each R⁴ is independently selected from C₁₋₆ alkyl, C₁₋₆ haloalkyl, and C₂₋₆ alkenyl;
R¹⁷ and R¹⁸ are each independently selected from H and C₁₋₆ alkyl, or R¹⁷ and R¹⁸, together with the boron and oxygen atoms to which they are attached, form 5-10 membered heterocyclyl, wherein the 5-10 membered heterocyclyl is optionally substituted with one or more substituents selected from H, halogen, and C₁₋₆ alkyl;
Z¹, Z², Z³, Z⁴, and Z⁵ are each independently selected from N, N⁺-O⁻, and C-R^{a3};
each R^{a3} is independently selected from H, halogen, hydroxy, -CN, C₁₋₆ alkyl, C₁₋₆ haloalkyl, C₁₋₆ alkoxy, C₁₋₆ haloalkoxy, C₂₋₆ alkenyl, C₃₋₆ cycloalkyl, -NR²¹R²², -NHC(O)R²³, -C(O)OR²⁴, -C(O)NR²⁵R²⁶, -SR²⁷, -S(O)R²⁸, - S(O)₂R²⁹, -S(O)₂NR³⁰R³¹, -S(O)(NR³²)R³³, -P(O)R³⁴R³⁵, and 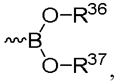 wherein the C₁₋₆ alkyl, C₁₋₆ haloalkyl, C₁₋₆ alkoxy, and C₁₋₆ haloalkoxy are optionally substituted with one or more substituents selected from hydroxy and - NR³⁸R³⁹;
or two adjacent R^{a3}, together with the carbon atoms to which they are attached, form 5-6 membered heterocyclyl or a 5-6 membered heteroaromatic ring, wherein the 5-6 membered heterocyclyl or 5-6 membered heteroaromatic ring is optionally substituted with one or more OH and C₁₋₆ alkyl;
R²⁴, R²⁵, R²⁶, R²⁷, R²⁸, R²⁹, R³⁰, R³¹, R³², R³³, R³⁴, R³⁵, R³⁸, and R³⁹ are each independently selected from H and C₁₋₆ alkyl;
R²¹ and R²² are each independently selected from H, C₁₋₆ alkyl, C₁₋₆ alkyl substituted with carbonyl, and -C(O)OC₁₋₆ alkyl;
each R²³ is independently selected from C₁₋₆ alkyl, C₁₋₆ haloalkyl, and C₂₋₆ alkenyl;
R³⁶ and R³⁷ are each independently selected from H and C₁₋₆ alkyl, or R³⁶ and R³⁷, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₆ alkyl;
V is selected from N, N⁺-O⁻, and C-R^{a4};
R^{a4} is selected from H and C₁₋₆ alkyl;
R^{a5} is selected from H and C₁₋₆ alkyl;
R^{a6} is selected from H and C₁₋₆ alkyl;
R^{b1} and R^{b2} are each independently selected from H and deuterium;
R^{b3} and R^{b4} are each independently selected from H, deuterium, C₁₋₆ alkyl, C₁₋₆ haloalkyl, and C₃₋₆ cycloalkyl;
R^{b5} and R^{b6} are each independently selected from H, C₁₋₆ alkyl, C₁₋₆ haloalkyl, and C₃₋₆ cycloalkyl, or R^{b5} and R^{b6}, together with the carbon atom to which they are attached, form C₃₋₅ cycloalkyl and 4-6 membered heterocyclyl;
R^{c} is selected from H, hydroxy, halogen, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl, and -O-C₃₋₆ cycloalkyl, wherein the C₁₋₆ alkyl, C₁₋₆ haloalkyl, C₁₋₆ alkoxy, C₁₋₆ haloalkoxy, and C₃₋₆ cycloalkyl are optionally substituted with one or more substituents selected from hydroxy, carboxyl, C₁₋₆ alkoxy, C₁₋₆ haloalkoxy, C₂₋₆ alkenyl, -NR⁴⁰R⁴¹, C₃₋₆ cycloalkyl, 3-6 membered heterocyclyl, and 5-6 membered heteroaryl, wherein the 3-6 membered heterocyclyl and 5-6 membered heteroaryl are optionally substituted with one or more halogen and C₁₋₆ alkyl;
R⁴⁰ and R⁴¹ are each independently selected from H and C₁₋₆ alkyl;
X¹, X², X³, and X⁴ are each independently selected from N and C-R^{c1};
each R^{c1} is independently selected from H, halogen, C₁₋₆ alkyl, C₁₋₆ haloalkyl, C₁₋₆ alkoxy, and C₁₋₆ haloalkoxy. The present disclosure further provides a compound of formula II, or a pharmaceutically acceptable salt, a stereoisomer, a solvate, an isotopically labeled compound, or a polymorph thereof, wherein the compound has the following structure: 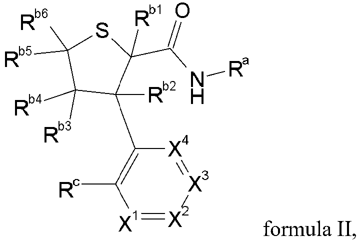
wherein, R^{a} is selected from 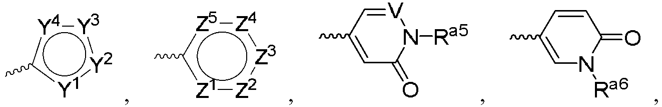 and 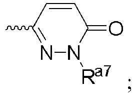
Y¹, Y², Y³, and Y⁴ are each independently selected from O, S, N, N-R^{a1}, and C-R^{a2};
each R^{a1} is independently selected from -S(O)₂-R⁴²;
each R^{a2} is independently selected from H and C₁₋₆ alkyl;
each R⁴² is independently selected from C₁₋₆ haloalkyl and C₃₋₆ cycloalkyl; Z¹, Z², Z³, Z⁴, and Z⁵ are each independently selected from N, N⁺-O⁻, and C-R^{a3};
each R^{a3} is independently selected from
H, halogen, -N₃, -NO₂, -SCN, -S(F)₅, -CH(O), C₂₋₆ alkenyl, C₂₋₆ alkynyl, C₃₋₆ cycloalkyl, 3-6 membered heterocyclyl, 5-6 membered heteroaryl, -C(O)NR⁴³R⁴⁴, -C(O)NR⁴⁵S(O)₂NR⁴⁶R⁴⁷, -C(S)NR⁴⁸R⁴⁹, -C(=NR⁵⁰)NR⁵¹R⁵², - C(=NR⁵³)R⁵⁴, -NR⁵⁵R⁵⁶, -NR⁵⁷S(O)₂R⁵⁸, -NR⁵⁹S(O)₂NR⁶⁰R⁶¹, -N=S(O)R⁶²R⁶³, -OS(O)₂R⁶⁴, -S(O)₂R⁶⁵, - S(O)₂NR⁶⁶R⁶⁷, -Si(OR⁶⁸)₃, and 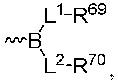 wherein the C₂₋₆ alkenyl, C₂₋₆ alkynyl, C₃₋₆ cycloalkyl, 3-6 membered heterocyclyl, and 5-6 membered heteroaryl are optionally substituted with one or more substituents selected from halogen, hydroxy, -CN, amino, C₁₋₆ alkyl, C₁₋₆ haloalkyl, C₁₋₆ alkoxy, C₁₋₆ haloalkoxy, -C(O)OR⁷¹, and -C(O)NR⁷²R⁷³; or two adjacent R^{a3}, together with the carbon atoms to which they are attached, form 5-6 membered heterocyclyl or a 5-10 membered heteroaromatic ring, wherein the 5-6 membered heterocyclyl or 5-10 membered heteroaromatic ring is optionally substituted with one or more substituents selected from halogen, hydroxy, amino, -CN, =O, C₁₋₆ alkyl, and -C₁₋₆ alkyl NR⁷⁴R⁷⁵;
R⁴³, R⁴⁴, R⁵⁰, and R⁵³ are each independently selected from H, hydroxy, C₁₋₆ alkyl, and C₁₋₆ alkoxy;
R⁴⁵, R⁴⁶, R⁴⁷, R⁴⁸, R⁴⁹, R⁵¹, R⁵², R⁵⁴, R⁵⁷, R⁵⁹, R⁶², R⁶³, R⁶⁸, R⁷¹, R⁷², R⁷³, R⁷⁴, and R⁷⁵ are each independently selected from H, C₁₋₆ alkyl, and C₁₋₆ haloalkyl;
R⁵⁵, R⁵⁶, R⁶⁶, and R⁶⁷ are each independently selected from H, hydroxy, -CN, C₁₋₆ alkyl, -C(O)C₁₋₆ alkyl, -C(O)C₂₋₆ alkenyl, -C(O)C₂₋₆ alkynyl, C₃₋₆ membered cycloalkyl, and 3-6 membered heterocyclyl, wherein the C₁₋₆ alkyl, C₂₋₆ alkenyl, C₂₋₆ alkynyl, C₃₋₆ membered cycloalkyl, and 3-6 membered heterocyclyl are optionally substituted with halogen, hydroxy, amino, -CN, N₃, C₁₋₆ alkyl, C₁₋₆ alkoxy, and C₁₋₆ alkylamino;
R⁵⁸ is independently selected from halogen and C₁₋₆ alkyl, wherein the C₁₋₆ alkyl is optionally substituted with one or more substituents selected from halogen, hydroxy, amino, C₁₋₆ alkoxy, C₁₋₆ alkylamino, and N₃;
R⁶⁰ and R⁶¹ are each independently selected from C₁₋₆ alkyl, wherein the C₁₋₆ alkyl is optionally substituted with one or more substituents selected from halogen, hydroxy, amino, C₁₋₆ alkoxy, C₁₋₆ alkylamino, and N₃;
R⁶⁴ is independently selected from halogen, amino, and C₁₋₆ alkylamino;
R⁶⁵ is independently selected from halogen, N₃, C₁₋₆ alkyl, and C₁₋₆ haloalkyl;
R⁶⁹ and R⁷⁰ are each independently selected from H, halogen, -CN, C₁₋₆ alkyl, C₁₋₆ haloalkyl, -C(O)OC₁₋₆ alkyl, - C(O)NH₂, and -C(O)NHC₁₋₆ alkyl, wherein the C₁₋₆ alkyl and C₁₋₆ haloalkyl are optionally substituted with hydroxy and amino; or R⁶⁹ and R⁷⁰, together with the boron and oxygen atoms to which they are attached, form 5-10 membered heterocyclyl (e.g., 5-6 membered heterocyclyl), wherein the heterocyclyl is optionally substituted with one or more substituents selected from hydrogen, halogen, hydroxy, C₁₋₄ alkyl, and =O;
L¹ and L² are each independently selected from O and N;
V is selected from N, N⁺-O⁻, and C-R^{a4};
R^{a4} is selected from H and C₁₋₆ alkyl;
R^{a5} is selected from H and C₁₋₆ alkyl;
R^{a6} is selected from H and C₁₋₆ alkyl;
R^{a7} is selected from H and C₁₋₆ alkyl;
R^{b1} and R^{b2} are each independently selected from H and deuterium;
R^{b3} and R^{b4} are each independently selected from H, deuterium, C₁₋₆ alkyl, C₁₋₆ haloalkyl, and C₃₋₆ cycloalkyl;
R^{b5} and R^{b6} are each independently selected from H, C₁₋₆ alkyl, C₁₋₆ haloalkyl, and C₃₋₆ cycloalkyl, or R^{b5} and R^{b6}, together with the carbon atom to which they are attached, form C₃₋₅ cycloalkyl and 4-6 membered heterocyclyl;
R^{c} is selected from H, hydroxy, halogen, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl, and -O-C₃₋₆ cycloalkyl, wherein the C₁₋₆ alkyl, C₁₋₆ haloalkyl, C₁₋₆ alkoxy, C₁₋₆ haloalkoxy, and C₃₋₆ cycloalkyl are optionally substituted with one or more substituents selected from hydroxy, carboxyl, C₁₋₆ alkoxy, C₁₋₆ haloalkoxy, C₂₋₆ alkenyl, -NR⁴⁰R⁴¹, C₃₋₆ cycloalkyl, 3-6 membered heterocyclyl, and 5-6 membered heteroaryl, wherein the 3-6 membered heterocyclyl and 5-6 membered heteroaryl are optionally substituted with one or more halogen and C₁₋₆ alkyl;
R⁴⁰ and R⁴¹ are each independently selected from H and C₁₋₆ alkyl;
X¹, X², X³, and X⁴ are each independently selected from N and C-R^{c1};
each R^{c1} is independently selected from H, halogen, C₁₋₆ alkyl, C₁₋₆ haloalkyl, C₁₋₆ alkoxy, and C₁₋₆ haloalkoxy.

In another aspect, the present application further provides a pharmaceutical composition comprising a prophylactically and/or therapeutically effective amount of the compound represented by formula I or formula II, or the pharmaceutically acceptable salt, the stereoisomer, the solvate, the isotopically labeled compound, or the polymorph thereof of the present application, and one or more pharmaceutically acceptable carriers.

In another aspect, the present application further provides a kit comprising the compound represented by formula I or formula II, or the pharmaceutically acceptable salt, the stereoisomer, the solvate, the isotopically labeled compound, or the polymorph thereof of the present application, or the pharmaceutical composition of the present application.

In another aspect, the present application further provides use of the compound represented by formula I or formula II, or the pharmaceutically acceptable salt, the stereoisomer, the solvate, the isotopically labeled compound, or the polymorph thereof of the present application, or the pharmaceutical composition of the present application, or the kit of the present application in the preparation of a medicament for preventing and/or treating a NaV1.8-associated disease, wherein preferably, the NaV1.8-associated disease is pain.

In another aspect, the present application further provides the compound represented by formula I or formula II, or the pharmaceutically acceptable salt, the stereoisomer, the solvate, the isotopically labeled compound, or the polymorph thereof of the present application, or the pharmaceutical composition of the present application, or the kit of the present application, for use in the prevention and/or treatment of a NaV1.8-associated disease, wherein preferably, the NaV1.8-associated disease is pain.

In another aspect, the present application further provides a method for preventing and/or treating a NaV1.8-associated disease, which comprises administering to an individual a therapeutically effective amount of the compound represented by formula I or formula II, or the pharmaceutically acceptable salt, the stereoisomer, the solvate, the isotopically labeled compound, or the polymorph thereof of the present application, or the pharmaceutical composition of the present application, or the kit of the present application, wherein preferably, the NaV1.8-associated disease is pain.

In another aspect, the present application further provides a method for preparing the compound represented by formula I or formula II of the present application.

### Definitions and Description

Unless otherwise defined below, all technical and scientific terms used herein are intended to have the same meaning as commonly understood by those skilled in the art. Reference to the techniques used herein is intended to refer to techniques commonly understood in the art, including those variations of or equivalent alternatives to the techniques that are apparent to those skilled in the art. Although the following terms are believed to be well understood by those skilled in the art, the following definitions are set forth to better explain the present disclosure.

As used herein, the term "include", "comprise", "have", "contain", or "involve", as well as other variant forms thereof herein, is inclusive or open-ended and does not exclude other unrecited elements or method steps.

Unless otherwise stated, the following definitions as used herein should be applied. For purposes of the present disclosure, the chemical elements are defined in accordance with the Periodic Table of Elements, CAS version, and Handbook of Chemistry and Physics, 75th edition, 1994. In addition, for general principles of organic chemistry, reference can be made to the descriptions in "Organic Chemistry", Thomas Sorrell, University Science Books, Sausalito: 1999, and "March's Advanced Organic Chemistry" by Michael B. Smith and Jerry March, John Wiley & Sons, New York: 2007, which are incorporated herein by reference in their entireties.

The term "alkyl" herein is a saturated linear or branched aliphatic hydrocarbon group with 1-20 carbon atoms, wherein the alkyl may be independently and optionally substituted with one or more substituents described in the present disclosure. As used herein, the term "C₁₋₆ alkyl" refers to a linear or branched group having 1 to 6 carbon atoms. The term "C₁₋₄ alkyl" refers to a linear or branched group having 1 to 4 carbon atoms, and it is optionally substituted with one or more (such as 1 to 4) suitable substituents, such as halogen. Examples of the alkyl group further include, but are not limited to, methyl (Me, -CH₃), ethyl (Et, -CH₂CH₃), n-propyl (n-Pr, -CH₂CH₂CH₃), isopropyl (i-Pr, -CH(CH₃)₂), n-butyl (-Bu, -CH₂CH₂CH₂CH₃), 2-methylpropyl or isobutyl (i-Bu, -CH₂CH(CH₃)₂), 1-methylpropyl or sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, and the like. The term "alkyl" and its prefix "alk-", as used herein, include both linear and branched saturated carbon chains. The term "alkoxy" herein refers to a group wherein an alkyl group is attached to the parent carbon chain via an oxygen atom. The "alkyl" is as defined above. For example, the term "C₁₋₁₂ alkoxy" refers to "C₁₋₁₂ alkyl-O-", and the alkoxy group contains 1-12 carbon atoms. In one embodiment, the alkoxy group contains 1-6 carbon atoms. In another embodiment, the alkoxy group contains 1-4 carbon atoms. In yet another embodiment, the alkoxy group contains 1-3 carbon atoms. Such embodiments include, but are not limited to, methoxy, ethoxy, n-propoxy, isopropoxy, n-butoxy, isobutoxy, sec-butoxy, tert-butoxy, n-pentoxy, n-hexoxy, or the like.

The term "cycloalkyl" herein refers to a saturated or partially unsaturated non-aromatic monocyclic or polycyclic (such as bicyclic) hydrocarbon ring (e.g., a monocyclic ring, such as cyclopropyl, cyclobutyl, cyclopentyl, cyclohexyl, cycloheptyl, cyclooctyl, or cyclononyl, or a bicyclic ring, including spiro-ring, fused, or bridged systems, such as bicyclo[1.1.1]pentyl, bicyclo[2.2.1]heptyl, bicyclo[3.2.1]octyl, bicyclo[5.2.0]nonyl, or decahydronaphthyl), and it is optionally substituted with one or more (such as 1 to 3) suitable substituents. For example, the term "C₃₋₁₀ cycloalkyl" refers to a saturated or partially unsaturated non-aromatic monocyclic or polycyclic (including fused, bridged, or spiro ring structures) hydrocarbon ring having 3 to 10 ring-forming carbon atoms (e.g., cyclopropyl, cyclobutyl, cyclopentyl, or cyclohexyl), and it is optionally substituted with one or more (such as 1 to 3) suitable substituents, wherein the substituents may be, but are not limited to, oxo (=O), fluorine, chlorine, bromine, iodine, hydroxy, amino, -C(=O)-NH₂, carboxyl, -S(=O)tO-H, -OS(=O)ₜ-H, -S(=O)ₜNH₂, triazolyl, tetrazolyl, -(CR^{3b}R^{3c})ₙ-NH₂, alkyl, alkylS(=O)t-, haloalkyl, hydroxyalkyl, alkoxy, alkylamino, alkylthio, haloalkoxy, amino, aryl, heteroaryl, alkenyl, alkynyl, heterocyclyl, sulfydryl, nitro, aryloxy, hydroxyalkoxy, alkanoyl, benzyl, cyclopropyl, phenyl, alkyl-C(=O)-, alkyl-C(=O)-NH-, carboxamido, alkoxyalkyl, or the like, and t is selected from 1, 2, 3, 4, 5, 6, 7, 8, 9, and 10. For example, the C₃₋₁₀ cycloalkyl is C3-8 cycloalkyl or C3-6 cycloalkyl.

Examples of the cycloalkyl further include, but are not limited to, cyclopropyl, cyclobutyl, cyclopentyl, 1-cyclopentyl-1-enyl, 1-cyclopentyl-2-enyl, 1-cyclopentyl-3-enyl, cyclohexyl, 1-cyclohexyl-1-enyl, 1-cyclohexyl-2-enyl, 1-cyclohexyl-3-enyl, cyclohexadienyl, cycloheptyl, cyclooctyl, cyclononyl, cyclodecyl, cycloundecyl, cyclododecyl, adamantyl, and the like.

As used herein, the term "halogen" group is defined to include fluorine, chlorine, bromine, or iodine.

As used herein, the term "halo" refers to substitution with one or more (such as 1 to 3) identical or different halogen atoms.

As used herein, the term "haloalkyl" refers to an alkyl group substituted with one or more (such as 1 to 3) identical or different halogen atoms. For example, the term "C₁₋₆ haloalkyl" refers to a haloalkyl group having 1 to 6 carbon atoms, e.g., -CF₃, -C₂F₅, -CHF₂, -CH₂F, -CH₂CF₃, -CH₂Cl, or -CH₂CH₂CF₃.

As used herein, the term "haloalkoxy" refers to an alkoxy group substituted with one or more (such as 1 to 3) identical or different halogen atoms. For example, the term "C₁₋₆ haloalkoxy" refers to a haloalkoxy group having 1 to 6 carbon atoms, e.g., -O-CF₃, -O-C₂F₅, -O-CHF₂, -O-CH₂F, -O-CH₂CF₃, -O-CH₂Cl, or -O-CH₂CH₂CF₃.

As used herein, the term "alkenyl" refers to an aliphatic hydrocarbon group containing at least one carbon-carbon double bond and having one of its hydrogen atoms replaced by a bond. The alkenyl may be a linear or branched alkenyl group and contains about 2 to about 15 carbon atoms. In one embodiment, the alkenyl contains about 2 to about 12 carbon atoms. In another embodiment, the alkenyl contains about 2 to about 6 carbon atoms. Non-limiting examples of the alkenyl include ethenyl, propenyl, n-butenyl, 3-methylbut-2-enyl, n-pentenyl, octenyl, and decenyl. The alkenyl may be unsubstituted alkenyl, or substituted with one or more identical or different substituents, each substituent being independently selected from halogen, alkenyl, alkynyl, aryl, cycloalkyl, cyano, hydroxy, -O-alkyl, -O-aryl, -alkylene-O-alkyl, alkylthio, -NH₂, -NH(alkyl), -N(alkyl)₂, -NH(cycloalkyl), -O-C(O)-alkyl, -O-C(O)-aryl, -O-C(O)-cycloalkyl, -C(O)OH, and -C(O)O-alkyl. The term "C₂₋₆ alkenyl" refers to an alkenyl group containing 2 to 6 carbon atoms.

As used herein, the term "heterocycle" or "heterocyclyl" refers to a saturated or partially unsaturated non-aromatic monocyclic or polycyclic group, for example, having 2, 3, 4, 5, 6, 7, 8, or 9 carbon atoms and one or more (e.g., 1, 2, 3, or 4) heteroatoms independently selected from N, O, and S(O)t (wherein t is 0, 1, or 2) in the ring(s), e.g., 3-12 membered heterocyclyl, 3-10 membered heterocyclyl, 3-9 membered heterocyclyl, 3-8 membered heterocyclyl, 3-7 membered heterocyclyl, 3-6 membered heterocyclyl, and 5-6 membered heterocyclyl. Representative examples of the heterocyclyl include, but are not limited to, oxiranyl, aziridinyl, azetidinyl, oxetanyl, tetrahydrofuranyl, tetrahydropyrrolidinyl, hexahydro-1H-pyrroline, pyrrolidinonyl, imidazolidinyl, pyrazolidinyl, tetrahydropyranyl, tetrahydropyridinyl, piperidinyl, morpholinyl, dithianyl, thiomorpholinyl, piperazinyl, and the like.

As used herein, the term "aryl" or "aromatic ring" refers to an all-carbon monocyclic or fused polycyclic aromatic group having a conjugated π-electron system. For example, the term "C₆₋₁₀ aryl" or "C₆₋₁₀ aromatic ring" refers to an aromatic group containing 6 to 10 carbon atoms, such as phenyl (ring) or naphthyl (ring). The aryl or aromatic ring is optionally substituted with one or more (such as 1 to 3) suitable substituents (e.g., halogen, -OH, -CN, -NO₂, or C₁₋₆ alkyl).

The term "heteroaryl" herein refers to an aromatic cyclic group, wherein at least one ring atom is a heteroatom, such as a nitrogen atom, an oxygen atom, a boron atom, or a sulfur atom. Optionally, a ring atom (e.g., a carbon atom, a nitrogen atom, or a sulfur atom) in the cyclic structure may be substituted with oxo. Specific examples include, but are not limited to, 5-10 membered heteroaryl, 6-10 membered heteroaryl, 5-10 membered nitrogen-containing heteroaryl, 6-10 membered oxygen-containing heteroaryl, 6-8 membered nitrogen-containing heteroaryl, 5-8 membered oxygen-containing heteroaryl, and the like, such as furanyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridinyl, 2-pyridonyl, 4-pyridonyl, pyrimidinyl, 1,4-dioxacyclohexadienyl, 2H-1,2-oxazinyl, 4H-1,2-oxazinyl, 6H-1,2-oxazinyl, 4H-1,3-oxazinyl, 6H-1,3-oxazinyl, 4H-1,4-oxazinyl, pyridazinyl, pyrazinyl, 1,2,3-triazinyl, 1,3,5-triazinyl, 1,2,4,5-tetrazinyl, azepinyl, 1,3-diazepinyl, and azocinyl.

The hydrogen in the groups involved in the present disclosure may be substituted with isotopes such as protium, deuterium, and tritium.

The term "substituted" means that one or more (e.g., 1, 2, 3, or 4) hydrogen atoms on a specified atom are replaced with a selection from the indicated group, provided that the normal valency of the specified atom under the present circumstances is not exceeded and that the replacement results in a stable compound. A combination of substituents and/or variables is permissible only if the combination results in a stable compound.

If a substituent is described as being "optionally substituted with...", the substituent may be (1) unsubstituted or (2) substituted. If a carbon atom of a substituent is described as being optionally substituted with one or more of a list of substituents, then one or more hydrogen atoms on the carbon atom (to the extent of any hydrogen atom present) may be replaced individually and/or together with an independently selected substituent or not replaced. If a nitrogen atom of a substituent is described as being optionally substituted with one or more of a list of substituents, then one or more hydrogen atoms on the nitrogen atom (to the extent of any hydrogen atom present) may each be replaced with an independently selected substituent or not replaced.

If substituents are described as being "independently selected from" one set of groups, each substituent is selected independently of another. Thus, each substituent may be identical to or different from another (other) substituent. As used herein, the term "one or more" means one or more than one, such as 2, 3, 4, 5, 6, 7, 8, 9, or 10, under reasonable conditions.

Unless otherwise specified, as used herein, the point of attachment of a substituent may be from any suitable position of the substituent.

When a bond of a substituent is shown to pass through a bond connecting two atoms in a ring, the substituent may be bonded to any ring-forming atom in the substitutable ring.

The present disclosure also includes all pharmaceutically acceptable isotopically labeled compounds, which are identical to the compounds of the present disclosure, except that one or more atoms are replaced by an atom having the same atomic number, but an atomic mass or mass number different from the atomic mass or mass number predominant in nature. Examples of the isotopes suitable for incorporation into the compounds of the present disclosure include, but are not limited to, isotopes of hydrogen (e.g., ²H, ³H, deuterium D, and tritium T); isotopes of carbon (e.g., ¹¹C, ¹³C, and ¹⁴C); isotopes of chlorine (e.g., ³⁷Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O, and ¹⁸O); isotopes of phosphorus (e.g., ³²P); and isotopes of sulfur (e.g., ³⁵S). Certain isotopically labeled compounds of the present disclosure (e.g., those into which a radioactive isotope is incorporated) can be used in drug and/or substrate tissue distribution studies (e.g., analysis). The radioactive isotopes tritium (i.e., ³H) and carbon-14 (i.e., ¹⁴C) are particularly useful for this purpose because they can be easily incorporated and easily monitored. Substitution with positron emitting isotopes (e.g., ¹¹C, ¹⁸F, ¹⁵O, and ¹³N) can be used to examine substrate receptor occupancy in positron emission tomography (PET) studies. The isotopically labeled compounds of the present disclosure can be prepared by processes analogous to those described in the accompanying routes and/or in the examples and preparations by using an appropriate isotopically labeled reagent in place of the non-labeled reagent previously employed. The pharmaceutically acceptable solvates of the present disclosure include those in which the crystallization solvent can be isotopically substituted, e.g., D₂O, acetone-d₆, or DMSO-d₆.

The term "stereoisomer" refers to an isomer formed due to at least one asymmetric center. In compounds having one or more (e.g., 1, 2, 3, or 4) asymmetric centers, racemic mixtures, single enantiomers, mixtures of diastereoisomers, and individual diastereoisomers may be produced. Particular individual molecules may also present as geometric isomers (cis/trans). Similarly, the compounds of the present disclosure may present as mixtures of two or more structurally different forms in rapid equilibrium (commonly referred to as tautomers). Representative examples of tautomers include keto-enol tautomers, phenol-keto tautomers, nitroso-oxime tautomers, imine-enamine tautomers, and the like. It will be appreciated that the scope of the present application encompasses all such isomers or mixtures thereof in any proportion (e.g., 60%, 65%, 70%, 75%, 80%, 85%, 90%, 95%, 96%, 97%, 98%, or 99%).

The present disclosure encompasses all possible crystalline forms or polymorphs of the compounds of the present disclosure, which may be single polymorphs or mixtures of more than one polymorph in any ratio.

It will also be appreciated that certain compounds of the present disclosure can be present in free form for treatment or, where appropriate, in the form of a pharmaceutically acceptable derivative thereof. In the present disclosure, pharmaceutically acceptable derivatives include, but are not limited to, pharmaceutically acceptable salts, solvates, metabolites, or prodrugs; upon administration to a patient in need thereof, they are capable of providing, directly or indirectly, the compounds of the present disclosure or metabolites or residues thereof. Thus, when reference is made herein to "the compounds of the present disclosure", it is also intended to encompass the various derivative forms of the compounds described above.

The pharmaceutically acceptable salts of the compounds of the present disclosure include acid addition salts and base addition salts thereof. Suitable acid addition salts are formed from acids that form pharmaceutically acceptable salts. Suitable base addition salts are formed from bases that form pharmaceutically acceptable salts. A review of suitable salts is found in Stahl and Wermuth, "Handbook of Pharmaceutical Salts: Properties, Selection, and Use" (Wiley-VCH, 2002). Methods for preparing the pharmaceutically acceptable salts of the compounds of the present disclosure are known to those skilled in the art.

The compounds of the present disclosure may be present in the form of a solvate (preferably a hydrate), and the compounds of the present disclosure contain polar solvents as a structural element of the crystal lattice of the compounds. The amount of polar solvents, particularly water, may be present in a stoichiometric or non-stoichiometric ratio.

Those skilled in the art will appreciate that not all nitrogen-containing heterocycles are capable of forming N-oxides, as nitrogen requires an available lone pair of electrons for oxidation to the oxides; those skilled in the art will recognize nitrogen-containing heterocycles capable of forming N-oxides. Those skilled in the art will also recognize that tertiary amines are capable of forming N-oxides. Synthesis methods for preparing N-oxides of heterocycles and tertiary amines are well known to those skilled in the art and include the oxidation of heterocycles and tertiary amines with peroxy acids such as peroxyacetic acid and m-chloroperoxybenzoic acid (MCPBA), hydrogen peroxide, alkyl hydroperoxides such as tert-butyl hydroperoxide, sodium perborate, and dioxiranes such as dimethyldioxirane. These methods for preparing N-oxides have been extensively described and reviewed in the literature. See, e.g., T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

Also included within the scope of the present disclosure are metabolites of the compounds of the present disclosure, i.e., substances formed *in vivo* upon administration of the compounds of the present disclosure. Such products may result, for example, from the oxidation, reduction, hydrolysis, amidation, deamidation, esterification, or enzymolysis of the administered compound. Thus, the present disclosure includes metabolites of the compounds of the present disclosure, including compounds prepared by the process of contacting the compounds of the present disclosure with a mammal for a time sufficient to produce metabolites thereof.

Further included within the scope of the present disclosure are prodrugs of the compounds of the present disclosure, which are certain derivatives of the compounds of the present disclosure that may themselves have little or no pharmacological activity and, when administered into or onto the body, can be converted to the compounds of the present disclosure having the desired activity, for example, by hydrolysis. Generally, such prodrugs are functional group derivatives of the compounds, which are readily converted into desired therapeutically active compounds *in vivo.* Additional information on the use of prodrugs can be found in "Pro-drugs as Novel Delivery Systems", volume 14, ACS Symposium Series (T. Higuchi and V. Stella) and "Bioreversible Carriers in Drug Design", Pergamon Press, 1987 (E. B. Roche ed., American Pharmaceutical Association). The prodrugs of the present disclosure may be prepared by, for example, replacing appropriate functional groups present in the compounds of the present disclosure with certain moieties known to those skilled in the art as "pro-moieties", for example, as described in "Design of Prodrugs", H. Bundgaard (Elsevier, 1985).

The present disclosure further encompasses the compounds of the present disclosure containing a protective group. In any process of preparing the compounds of the present disclosure, it may be necessary and/or desirable to protect sensitive or reactive groups on any of the molecules concerned, thereby forming a chemically protected form of the compounds of the present disclosure. This can be achieved by conventional protective groups, as described, for example, in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which are incorporated herein by reference. Protective groups can be removed at an appropriate subsequent stage using methods known in the art. The term "about" means being within +10%, preferably within +5%, and more preferably within +2%, of the stated numerical value.

The present application is in no way limited to the methods and materials described herein. In the event that one or more of the incorporated literature, patents, and similar materials differ from or contradict the present application (including but not limited to defined terms, term usage, described techniques, and the like), the description of the present application and the accompanying structural formulas shall prevail.

### Compound

One objective of the present application is to provide a compound of formula I, or a pharmaceutically acceptable salt, a stereoisomer, a solvate, an isotopically labeled compound, or a polymorph thereof: 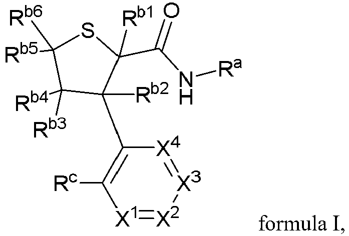 wherein:
R^{a} is selected from 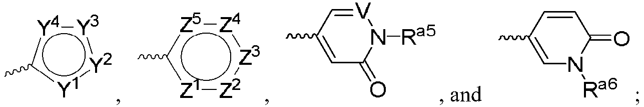
Y¹, Y², Y³, and Y⁴ are each independently selected from O, S, N, N-R^{a1}, and C-R^{a2};
each R^{a1} is independently selected from H, C₁₋₆ alkyl, C₁₋₆ haloalkyl, and -S(O)₂R¹;
each R^{a2} is independently selected from H, halogen, hydroxy, -CN, C₁₋₆ alkyl, C₁₋₆ haloalkyl, C₁₋₆ alkoxy, C₁₋₆ haloalkoxy, C₂₋₆ alkenyl, C₁₋₆ cycloalkyl, -NR²R³, -NHC(O)R⁴, -C(O)OR⁵, -C(O)NR⁶R⁷, SR⁸, -S(O)R⁹, -S(O)₂R¹⁰, - S(O)₂NR¹¹R¹², -S(O)(NR¹³)R¹⁴, -P(O)R¹⁵R¹⁶, and 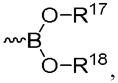 wherein the C₁₋₆ alkyl, C₁₋₆ haloalkyl, C₁₋₆ alkoxy, and C₁₋₆ haloalkoxy are optionally substituted with one or more substituents selected from hydroxy and -NR¹⁹R²⁰; or adjacent R^{a1} and R^{a2}, or two R^{a2}, together with the carbon atoms to which they are attached, form a 5-6 membered heteroaromatic ring;
R¹, R⁵, R⁶, R⁷, R⁸, R⁹, R¹⁰, R¹¹, R¹², R¹³, R¹⁴, R¹⁵, R¹⁶, R¹⁹, and R²⁰ are each independently selected from H and C₁₋₆ alkyl;
R² and R³ are each independently selected from H, C₁₋₆ alkyl, and C₁₋₆ alkyl substituted with carbonyl;
each R⁴ is independently selected from C₁₋₆ alkyl, C₁₋₆ haloalkyl, and C₂₋₆ alkenyl;
R¹⁷ and R¹⁸ are each independently selected from H and C₁₋₆ alkyl, or R¹⁷ and R¹⁸, together with the boron and oxygen atoms to which they are attached, form 5-10 membered heterocyclyl, wherein the 5-6 membered heterocyclyl is optionally substituted with one or more substituents selected from H, halogen, and C₁₋₆ alkyl;
Z¹, Z², Z³, Z⁴, and Z⁵ are each independently selected from N, N⁺-O⁻, and C-R^{a3};
each R^{a3} is independently selected from H, halogen, hydroxy, -CN, C₁₋₆ alkyl, C₁₋₆ haloalkyl, C₁₋₆ alkoxy, C₁₋₆ haloalkoxy, C₂₋₆ alkenyl, C₃₋₆ cycloalkyl, -NR²¹R²², -NHC(O)R²³, -C(O)OR²⁴, -C(O)NR²⁵R²⁶, -SR²⁷, -S(O)R²⁸, - S(O)₂R²⁹, -S(O)₂NR³⁰R³¹, -S(O)(NR³²)R³³, -P(O)R³⁴R³⁵, and 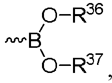 wherein the C₁₋₆ alkyl, C₁₋₆ haloalkyl, C₁₋₆ alkoxy, and C₁₋₆ haloalkoxy are optionally substituted with one or more substituents selected from hydroxy and - NR³⁸R³⁹;
or two adjacent R^{a3}, together with the carbon atoms to which they are attached, form 5-6 membered heterocyclyl or a 5-6 membered heteroaromatic ring, wherein the 5-6 membered heterocyclyl or 5-6 membered heteroaromatic ring is optionally substituted with one or more OH and C₁₋₆ alkyl;
R²⁴, R²⁵, R²⁶, R²⁷, R²⁸, R²⁹, R³⁰, R³¹, R³², R³³, R³⁴, R³⁵, R³⁸, and R³⁹ are each independently selected from H and C₁₋₆ alkyl;
R²¹ and R²² are each independently selected from H, C₁₋₆ alkyl, C₁₋₆ alkyl substituted with carbonyl, and -C(O)OC₁₋₆ alkyl;
each R²³ is independently selected from C₁₋₆ alkyl, C₁₋₆ haloalkyl, and C₂₋₆ alkenyl;
R³⁶ and R³⁷ are each independently selected from H and C₁₋₆ alkyl, or R³⁶ and R³⁷, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₆ alkyl;
V is selected from N, N⁺-O⁻, and C-R^{a4};
R^{a4} is selected from H and C₁₋₆ alkyl;
R^{a5} is selected from H and C₁₋₆ alkyl;
R^{a6} is selected from H and C₁₋₆ alkyl;
R^{b1} and R^{b2} are each independently selected from H and deuterium;
R^{b3} and R^{b4} are each independently selected from H, deuterium, C₁₋₆ alkyl, C₁₋₆ haloalkyl, and C₃₋₆ cycloalkyl;
R^{b5} and R^{b6} are each independently selected from H, C₁₋₆ alkyl, C₁₋₆ haloalkyl, and C₃₋₆ cycloalkyl, or R^{b5} and R^{b6}, together with the carbon atom to which they are attached, form C₃₋₅ cycloalkyl and 4-6 membered heterocyclyl;
R^{c} is selected from H, hydroxy, halogen, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl, and -O-C₃₋₆ cycloalkyl, wherein the C₁₋₆ alkyl, C₁₋₆ haloalkyl, C₁₋₆ alkoxy, C₁₋₆ haloalkoxy, and C₃₋₆ cycloalkyl are optionally substituted with one or more substituents selected from hydroxy, carboxyl, C₁₋₆ alkoxy, C₁₋₆ haloalkoxy, C₂₋₆ alkenyl, -NR⁴⁰R⁴¹, C₃₋₆ cycloalkyl, 3-6 membered heterocyclyl, and 5-6 membered heteroaryl, wherein the 3-6 membered heterocyclyl and 5-6 membered heteroaryl are optionally substituted with one or more halogen and C₁₋₆ alkyl;
R⁴⁰ and R⁴¹ are each independently selected from H and C₁₋₆ alkyl;
X¹, X², X³, and X⁴ are each independently selected from N and C-R^{c1};
each R^{c1} is independently selected from H, halogen, C₁₋₆ alkyl, C₁₋₆ haloalkyl, C₁₋₆ alkoxy, and C₁₋₆ haloalkoxy. In some embodiments, in the compound of formula I of the present application,
R^{a} is selected from 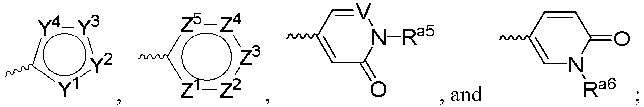
Y¹, Y², Y³, and Y⁴ are each independently selected from O, S, N, N-R^{a1}, and C-R^{a2};
each R^{a1} is independently selected from H, C₁₋₄ alkyl (e.g., methyl, ethyl, propyl, and butyl), C₁₋₄ haloalkyl (e.g., CF₃, CHF₂, and CH₂F), and -S(O)₂R¹ (e.g., -S(O)₂CH₃);
each R^{a2} is independently selected from H, halogen (e.g., fluorine, chlorine, bromine, and iodine), hydroxy, -CN, C₁₋₄ alkyl (e.g., methyl, ethyl, propyl, and butyl), C₁₋₄ haloalkyl (e.g., CF₃, CHF₂, and CH₂F), C₁₋₄ alkoxy, C₁₋₄ haloalkoxy, C₂₋₆ alkenyl, C₁₋₄ cycloalkyl, -NR²R³, -NHC(O)R⁴, -C(O)OR⁵, -C(O)NR⁶R⁷, SR⁸, -S(O)R⁹, -S(O)₂R¹⁰, -S(O)₂NR¹¹R¹², -S(O)(NR¹³)R¹⁴, -P(O)R¹⁵R¹⁶, and 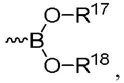 wherein the C₁₋₄ alkyl, C₁₋₄ haloalkyl, C₁₋₄ alkoxy, and C₁₋₄ haloalkoxy are optionally substituted with one or more substituents selected from hydroxy and -NR¹⁹R²⁰;
or adjacent R^{a1} and R^{a2}, or two R^{a2}, together with the carbon atoms to which they are attached, form a 5-6 membered heteroaromatic ring;
R¹, R⁵, R⁶, R⁷, R⁸, R⁹, R¹⁰, R¹¹, R¹², R¹³, R¹⁴, R¹⁵, R¹⁶, R¹⁹, and R²⁰ are each independently selected from H and C₁₋₄ alkyl;
R² and R³ are each independently selected from H, C₁₋₄ alkyl, and C₁₋₄ alkyl substituted with carbonyl;
each R⁴ is independently selected from C₁₋₄ alkyl, C₁₋₄ haloalkyl, and C₂₋₆ alkenyl;
R¹⁷ and R¹⁸ are each independently selected from H and C₁₋₄ alkyl, or R¹⁷ and R¹⁸, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₄ alkyl;
Z¹, Z², Z³, Z⁴, and Z⁵ are each independently selected from N, N⁺-O⁻, and C-R^{a3};
each R^{a3} is independently selected from H, halogen (e.g., fluorine, chlorine, bromine, and iodine), hydroxy, -CN, C₁₋₄ alkyl (e.g., methyl, ethyl, propyl, and butyl), C₁₋₄ haloalkyl (e.g., CF₃, CHF₂, and CH₂F), C₁₋₄ alkoxy, C₁₋₄ haloalkoxy, C₂₋₆ alkenyl, C₁₋₄ cycloalkyl, -NR²¹R²², -NHC(O)R²³, -C(O)OR²⁴, -C(O)NR²⁵R²⁶, -SR²⁷, -S(O)R²⁸, -S(O)₂R²⁹, - S(O)₂NR³⁰R³¹, -S(O)(NR³²)R³³, -P(O)R³⁴R³⁵, and 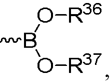 wherein the C₁₋₄ alkyl, C₁₋₄ haloalkyl, C₁₋₄ alkoxy, and C₁₋₄ haloalkoxy are optionally substituted with one or more substituents selected from hydroxy and -NR³⁸R³⁹; or two adjacent R^{a3}, together with the carbon atoms to which they are attached, form 5-6 membered heterocyclyl or a 5-6 membered heteroaromatic ring, wherein the 5-6 membered heterocyclyl or 5-6 membered heteroaromatic ring is optionally substituted with one or more OH and C₁₋₆ alkyl;
R²⁴, R²⁵, R²⁶, R²⁷, R²⁸, R²⁹, R³⁰, R³¹, R³², R³³, R³⁴, R³⁵, R³⁸, and R³⁹ are each independently selected from H and C₁₋₄ alkyl;
R²¹ and R²² are each independently selected from H, C₁₋₄ alkyl, C₁₋₄ alkyl substituted with carbonyl, and -C(O)OC₁₋₄ alkyl;
each R²³ is independently selected from C₁₋₄ alkyl, C₁₋₄ haloalkyl, and C₂₋₆ alkenyl;
R³⁶ and R³⁷ are each independently selected from H and C₁₋₄ alkyl, or R³⁶ and R³⁷, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₄ alkyl;
V is selected from N, N⁺-O⁻, and C-R^{a4};
R^{a4} is selected from H and C₁₋₄ alkyl (e.g., methyl, ethyl, propyl, and butyl);
R^{a5} is selected from H and C₁₋₄ alkyl (e.g., methyl, ethyl, propyl, and butyl);
R^{a6} is selected from H and C₁₋₄ alkyl (e.g., methyl, ethyl, propyl, and butyl).

In some embodiments, in the compound of formula I of the present application,
R^{a} is selected from 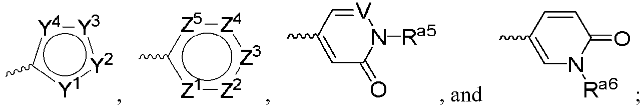
Y¹, Y², Y³, and Y⁴ are each independently selected from O, S, N, N-R^{a1}, and C-R^{a2};
each R^{a1} is independently selected from H, methyl, CF₃, CHF₂, and -S(O)₂CH₃;
each R^{a2} is independently selected from H, fluorine, chlorine, methyl, CF₃, CHF₂, -C(O)NH₂, -NH₂, and 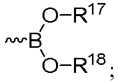 or adjacent R^{a1} and R^{a2}, or two R^{a2}, together with the carbon atoms to which they are attached, form a 5-6 membered heteroaromatic ring;
R¹⁷ and R¹⁸ are each independently selected from H and C₁₋₄ alkyl, or R¹⁷ and R¹⁸, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₄ alkyl;
Z¹, Z², Z³, Z⁴, and Z⁵ are each independently selected from N, N⁺-O⁻, and C-R^{a3};
each R^{a3} is independently selected from H, fluorine, chlorine, bromine, hydroxy, -CN, methyl, CF₃, CHF₂, methoxy, trifluoromethoxy, ethenyl, cyclopropyl, -CH₂OH, -C(CH₃)₂OH, -CH(OH)CH₂(OH), -CH(OH)CH₂F, - CH(NH₂)CH₂(OH), -NR²¹R²², -NC(O)R²³, -C(O)OR²⁴, -C(O)NR²⁵R²⁶, -SR²⁷, -S(O)R²⁸, -S(O)₂R²⁹, -S(O)₂NR³⁰R³¹, -S(O)(NR³²)R³³, -P(O)R³⁴R³⁵, and 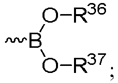
or two adjacent R^{a3}, together with the carbon atoms to which they are attached, form 5-6 membered heterocyclyl or a 5-6 membered heteroaromatic ring, wherein the heterocyclyl is optionally substituted with one or more hydroxy or methyl;
R²⁴, R²⁵, R²⁶, R²⁷, R²⁸, R²⁹, R³⁰, R³¹, R³², R³³, R³⁴, and R³⁵ are each independently selected from H and C₁₋₄ alkyl; R²¹ and R²² are each independently selected from H, C₁₋₄ alkyl, and C₁₋₄ alkyl substituted with carbonyl;
each R²³ is independently selected from C₁₋₄ alkyl, C₁₋₄ haloalkyl, and C₂₋₆ alkenyl;
R³⁶ and R³⁷ are each independently selected from H and C₁₋₄ alkyl, or R³⁶ and R³⁷, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₄ alkyl;
V is selected from N, N⁺-O⁻, and C-R^{a4};
R^{a4} is selected from H and methyl;
R^{a5} is selected from H and methyl;
R^{a6} is selected from H and methyl.

In some embodiments, in the compound of formula I of the present application,
each R^{a3} is independently selected from H, fluorine, chlorine, bromine, hydroxy, -CN, methyl, CF₃, CHF₂, methoxy, trifluoromethoxy, ethenyl, cyclopropyl, -CH₂OH, -C(CH₃)₂OH, -CH(OH)CH₂(OH), -CH(OH)CH₂F, - CH(NH₂)CH₂(OH), -NR²¹R²², -NC(O)R²³, -C(O)OR²⁴, -C(O)NR²⁵R²⁶, -SR²⁷, -S(O)R²⁸, -S(O)₂R²⁹, -S(O)₂NR³⁰R³¹, -S(O)(NR³²)R³³, -P(O)R³⁴R³⁵, and 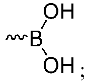
or two adjacent R^{a3}, together with the carbon atoms to which they are attached, form 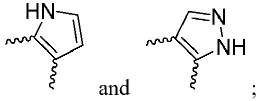 R²⁴, R²⁵, R²⁶, R²⁷, R²⁸, R²⁹, R³⁰, R³¹, R³², R³³, R³⁴, and R³⁵ are each independently selected from H and C₁₋₄ alkyl;
R²¹ and R²² are each independently selected from H, C₁₋₄ alkyl, and C₁₋₄ alkyl substituted with carbonyl;
each R²³ is independently selected from C₁₋₄ alkyl, C₁₋₄ haloalkyl, and C₂₋₆ alkenyl.

In some embodiments, in the compound of formula I of the present application, each R^{a3} is independently selected from H, fluorine, chlorine, bromine, hydroxy, -CN, methyl, CF₃, CHF₂, methoxy, trifluoromethoxy, ethenyl, cyclopropyl, -CH₂OH, -C(CH₃)₂OH, -CH(OH)CH₂(OH), -CH(OH)CH₂F, -CH(NH₂)CH₂(OH), -N(CH₃)-Boc, - NH(CH₃), -N(CH₃)₂, -C(O)OCH₃, -C(O)NH₂, -SH, -S(O)CH₃, -S(O)₂CH₃, -S(O)₂NH₂, -S(O)(NH)CH₃, - S(O)(NCH₃)CH₃, -P(O)(CH₃)₂, and 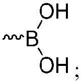 or two adjacent R^{a3}, together with the carbon atoms to which they are attached, form 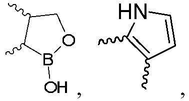 and

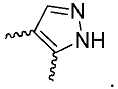

In some embodiments, in the compound of formula I of the present application, R^{b3} and R^{b4} are each independently selected from H, deuterium, C₁₋₄ alkyl (e.g., methyl, ethyl, propyl, and butyl), C₁₋₄ haloalkyl, and C₃₋₆ cycloalkyl. In some embodiments, in the compound of formula I of the present application, R^{b3} and R^{b4} are each independently selected from H, deuterium, and methyl.

In some embodiments, in the compound of formula I of the present application, R^{b5} and R^{b6} are each independently selected from H, C₁₋₄ alkyl, C₁₋₄ haloalkyl, and C₃₋₆ cycloalkyl, or R^{b5} and R^{b6}, together with the carbon atom to which they are attached, form C₃₋₅ cycloalkyl and 4-6 membered heterocyclyl (e.g., 4-6 membered oxygen-containing heterocyclyl).

In some embodiments, in the compound of formula I of the present application, R^{b5} and R^{b6} are each independently selected from H, methyl, cyclopropyl, and trifluoromethyl, or R^{b5} and R^{b6}, together with the carbon atom to which they are attached, form cyclobutyl, cyclopentyl, and 4-6 membered oxygen-containing heterocyclyl.

In some embodiments, in the compound of formula I of the present application, R^{b5} and R^{b6} are each independently selected from H, methyl, cyclopropyl, and trifluoromethyl, or R^{b5} and R^{b6}, together with the carbon atom to which they are attached, form cyclobutyl and

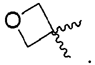

In some embodiments, in the compound of formula I of the present application, R^{b5} and R^{b6} are each independently selected from H, methyl, cyclopropyl, and trifluoromethyl.

In some embodiments, in the compound of formula I of the present application, R^{b5} is selected from methyl, and R^{b6} is selected from trifluoromethyl.

In some embodiments, in the compound of formula I of the present application, R^{c} is selected from H, hydroxy, halogen, C₁₋₄ alkyl, C₁₋₄ deuterated alkyl, C₁₋₄ haloalkyl, C₁₋₄ alkoxy, C₁₋₄ deuterated alkoxy, C₁₋₄ haloalkoxy, C₂₋₆ alkenyl, and -O-C₃₋₆ cycloalkyl, wherein the C₁₋₄ alkyl, C₁₋₄ haloalkyl, C₁₋₄ alkoxy, C₁₋₄ haloalkoxy, and C₃₋₆ cycloalkyl are optionally substituted with one or more substituents selected from hydroxy, carboxyl, C₁₋₄ alkoxy, C₁₋₄ haloalkoxy, C₂₋₆ alkenyl, -NR⁴⁰R⁴¹, C₃₋₆ cycloalkyl, 3-6 membered heterocyclyl, and 5-6 membered heteroaryl, wherein the 3-6 membered heterocyclyl and 5-6 membered heteroaryl are optionally substituted with one or more halogen and C₁₋₄ alkyl; R⁴⁰ and R⁴¹ are each independently selected from H and C₁₋₄ alkyl.

In some embodiments, in the compound of formula I of the present application, X¹, X², X³, and X⁴ are each independently selected from C-R^{c1}.

In some embodiments, in the compound of formula I of the present application, each R^{c1} is independently selected from H, halogen, C₁₋₄ alkyl, C₁₋₄ haloalkyl, C₁₋₄ alkoxy, and C₁₋₄ haloalkoxy.

In some embodiments, in the compound of formula I of the present application, each R^{c1} is independently selected from H, F, Cl, methyl, ethyl, propyl, butyl, fluoromethyl, fluoroethyl, fluoropropyl, methoxy, ethoxy, propoxy, butoxy, fluoromethoxy, fluoroethoxy, and fluoropropoxy.

In some embodiments, in the compound of formula I of the present application, each R^{c1} is independently selected from H, F, methyl, difluoromethyl, trifluoromethyl, difluoromethoxy, methoxy, and difluoromethoxy.

In some embodiments, in the compound of formula I of the present application, R^{a} is selected from:

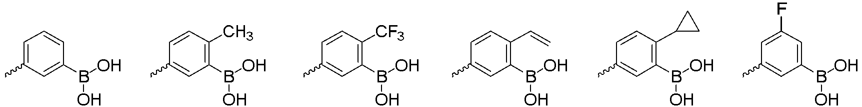

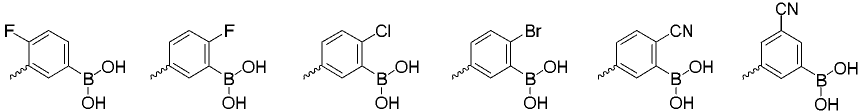

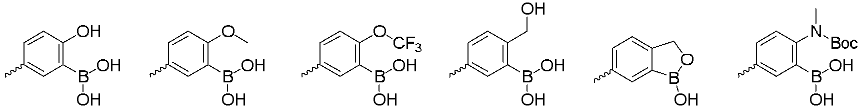

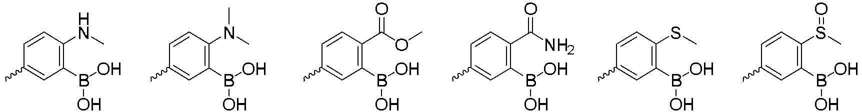

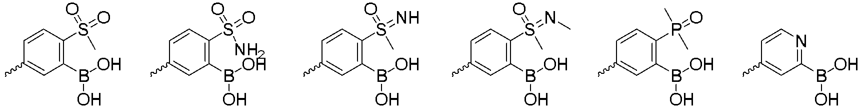

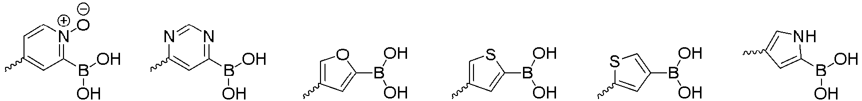

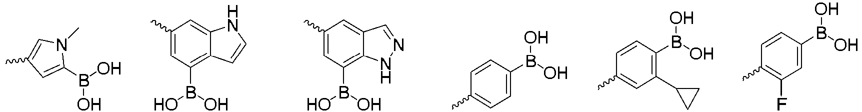

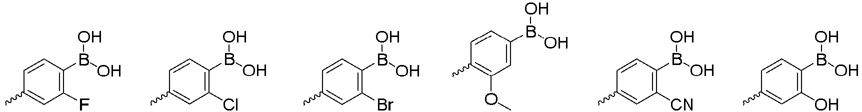

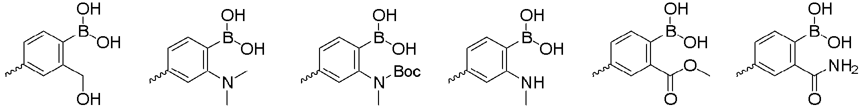

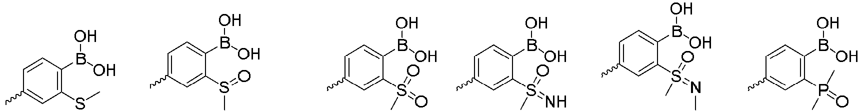

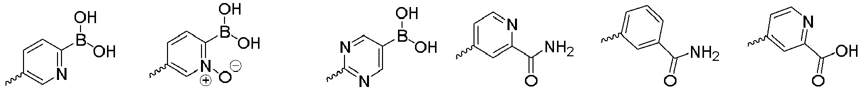

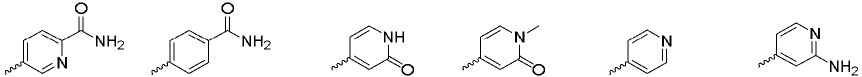

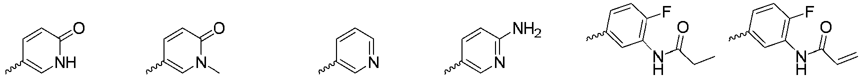

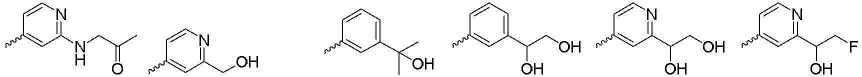

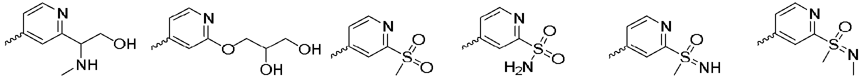

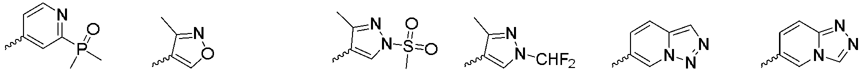

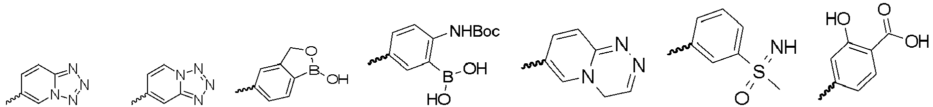

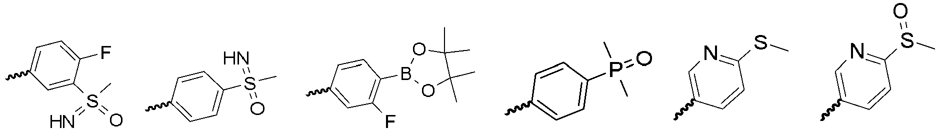

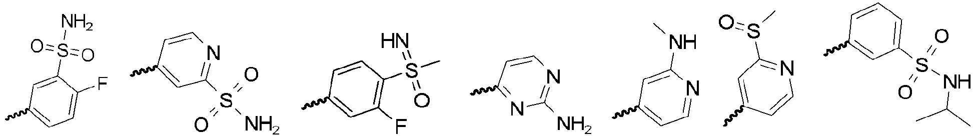

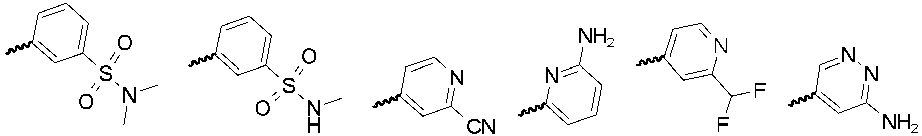

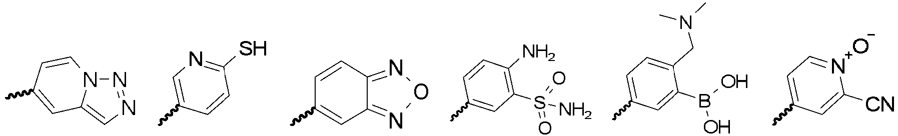

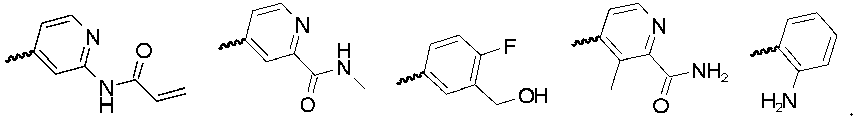

In some embodiments, in the compound of formula I of the present application, R^{a} is selected from:

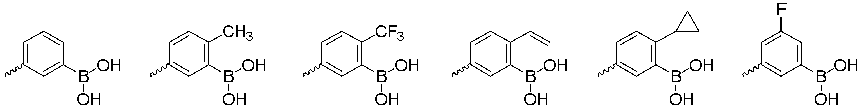

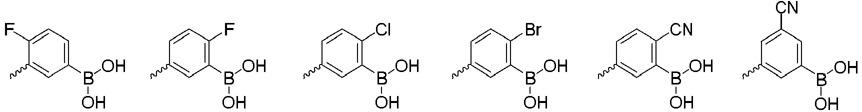

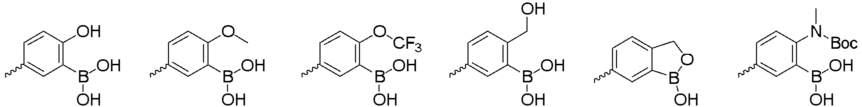

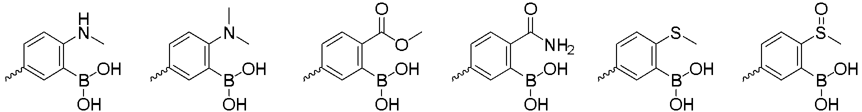

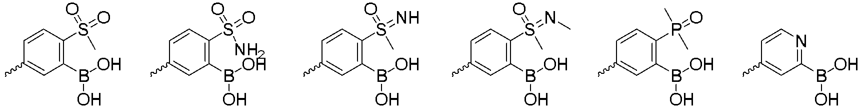

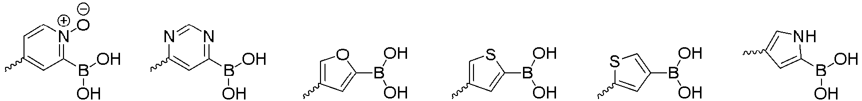

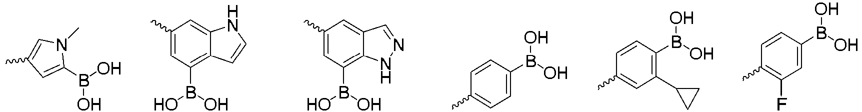

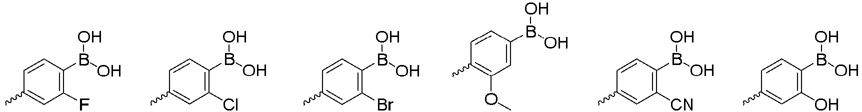

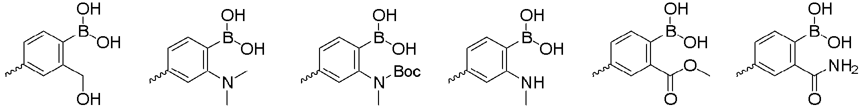

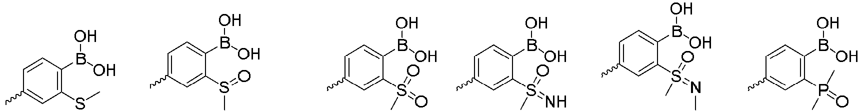

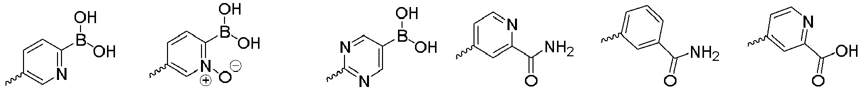

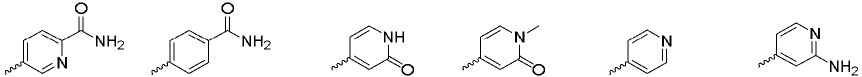

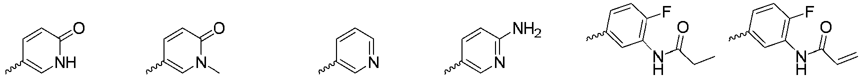

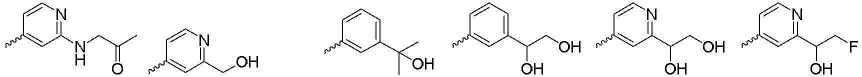

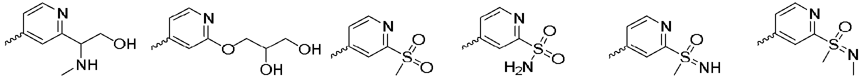

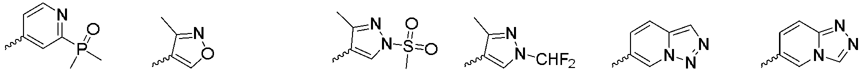

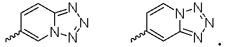

In some embodiments, in the compound of formula I of the present application, R^{c} is selected from H, -OCH₃, -OCD₃, -OCH₂CH₃, -OCH(CH₃)₂, -OCH₂CF₃, -OCH₂CF₂CH₃, -OCH₂CHF₂, 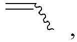 -OCHF₂, 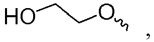 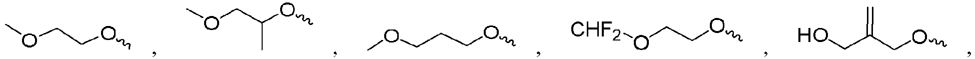 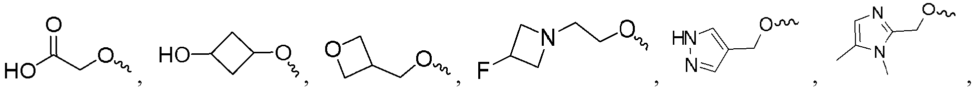 and

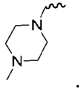

In some embodiments, the compound of the present disclosure is selected from:

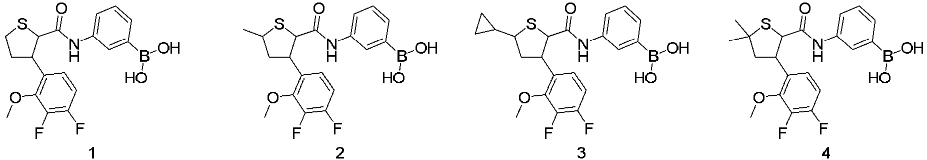

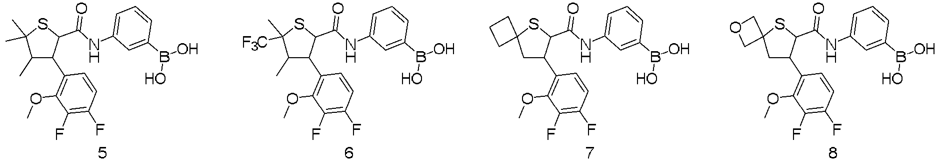

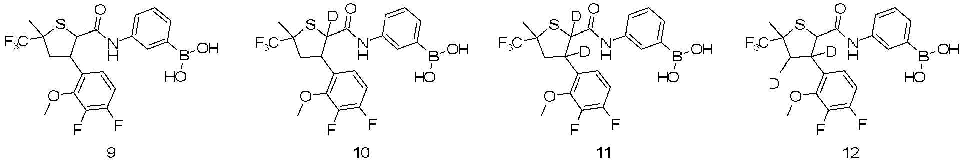

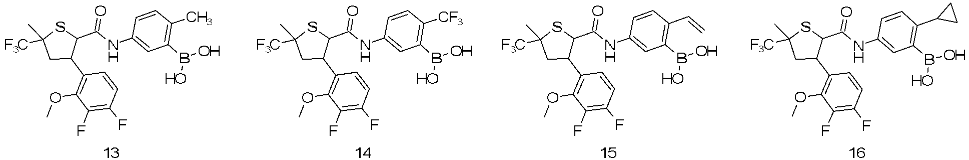

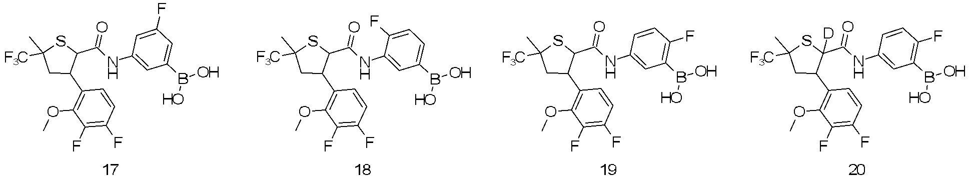

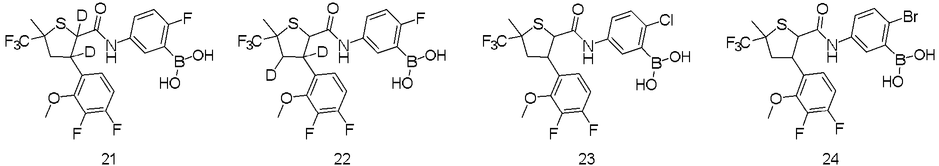

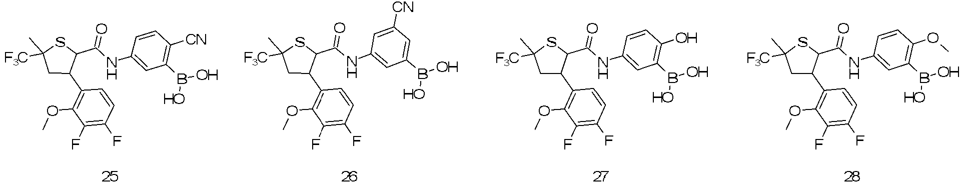

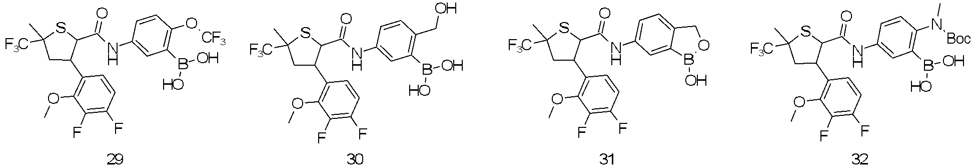

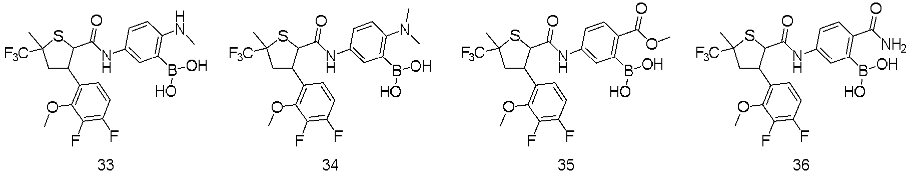

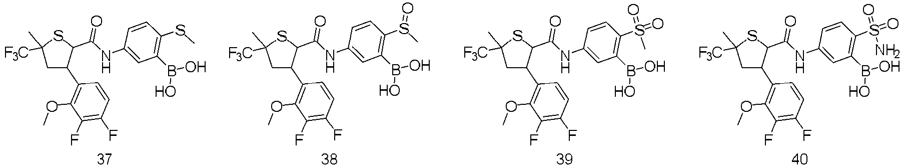

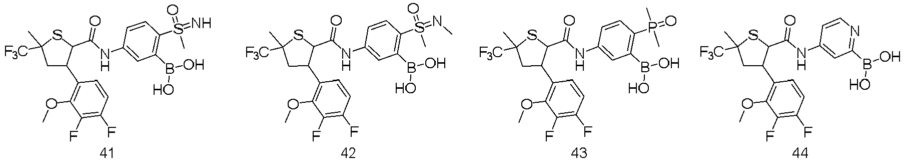

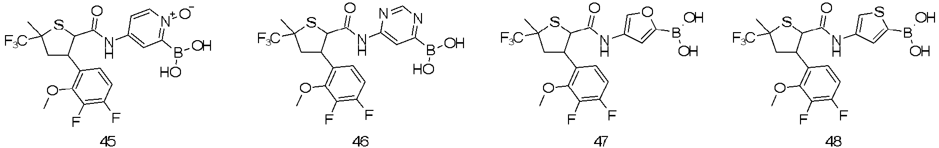

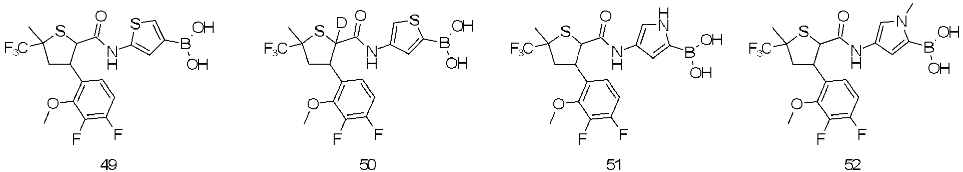

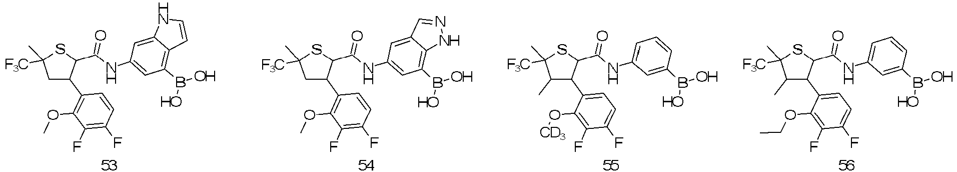

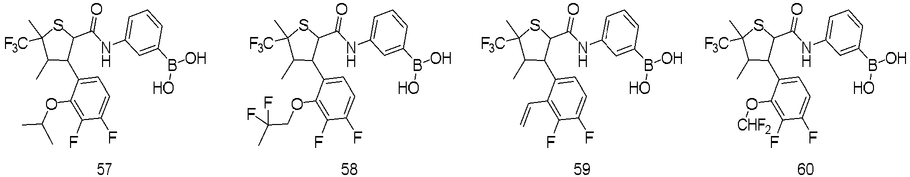

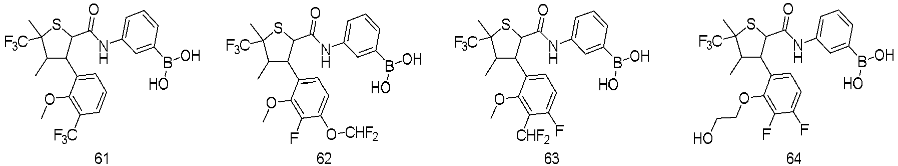

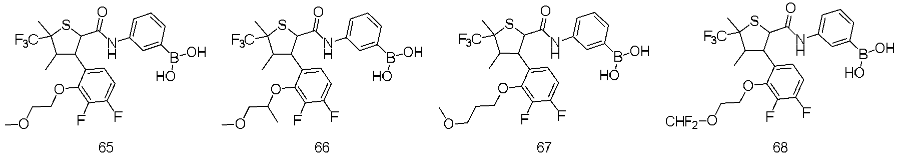

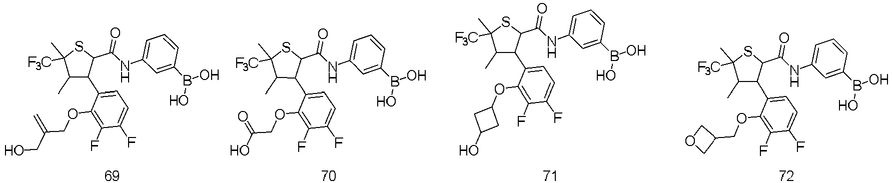

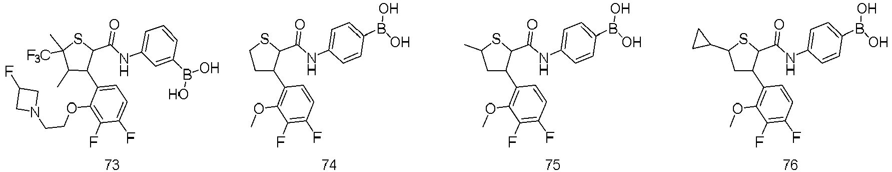

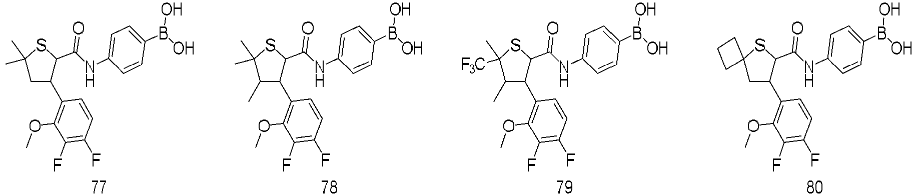

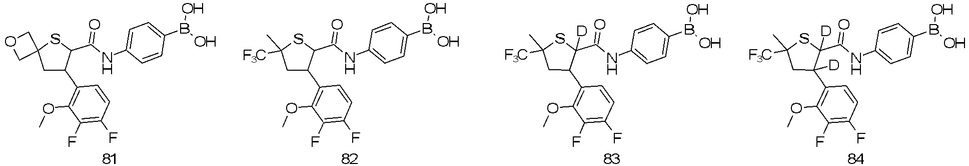

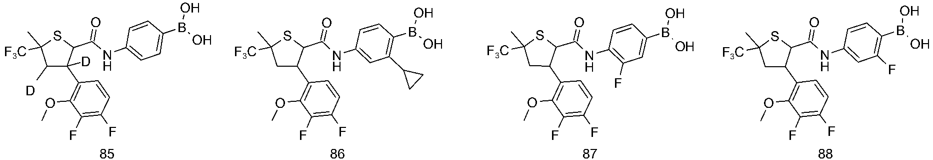

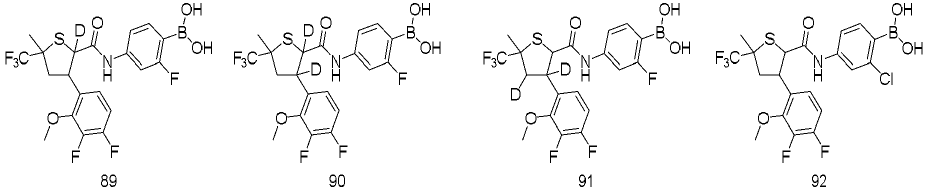

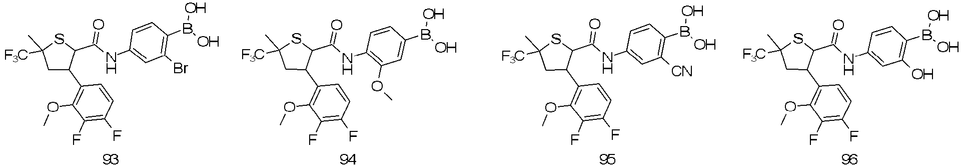

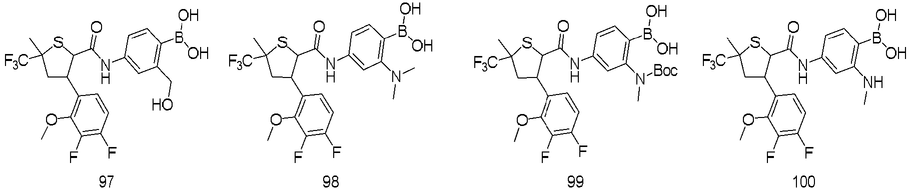

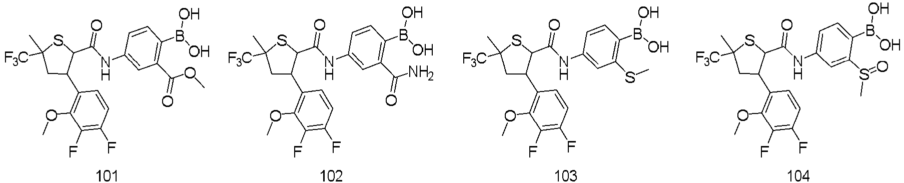

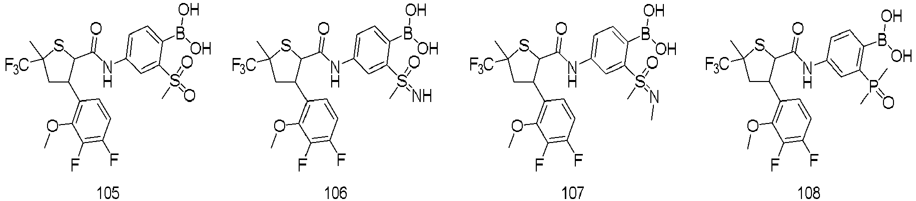

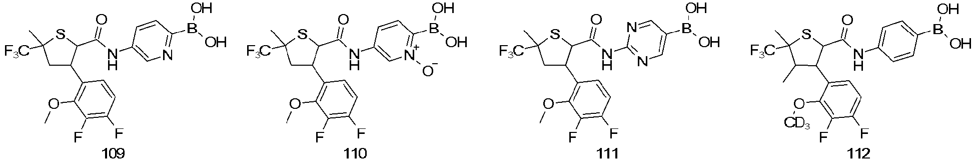

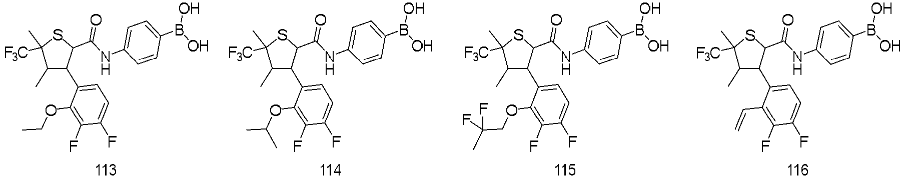

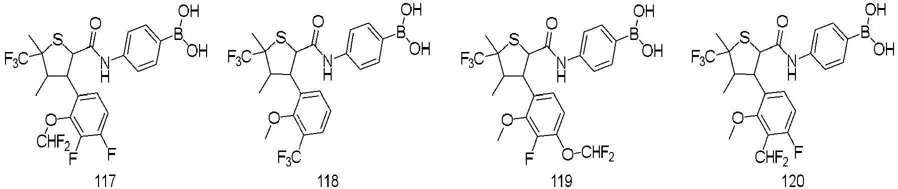

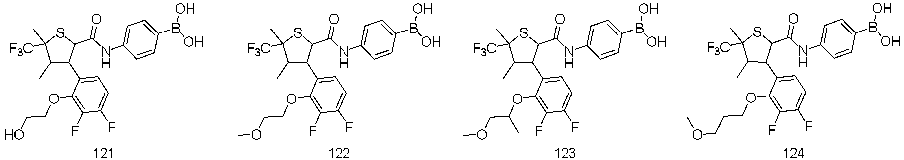

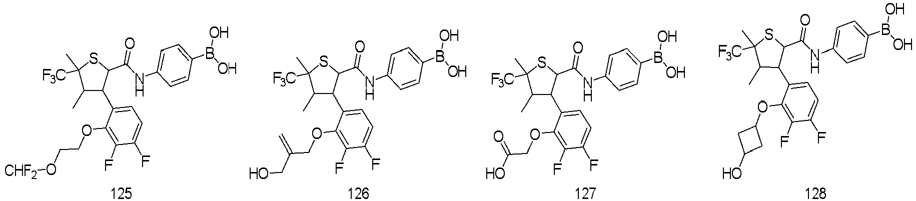

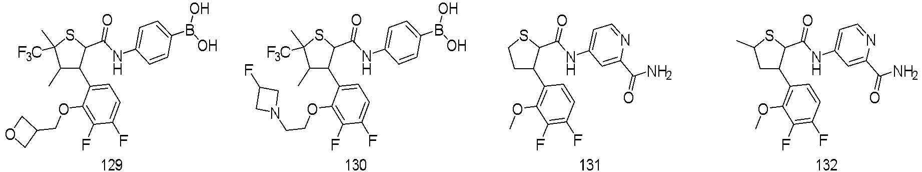

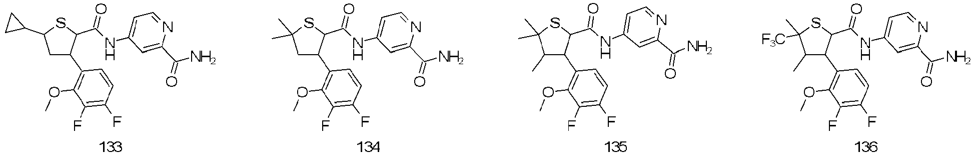

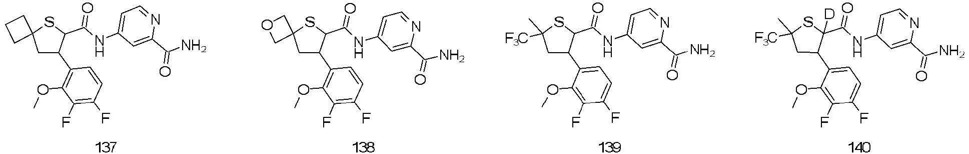

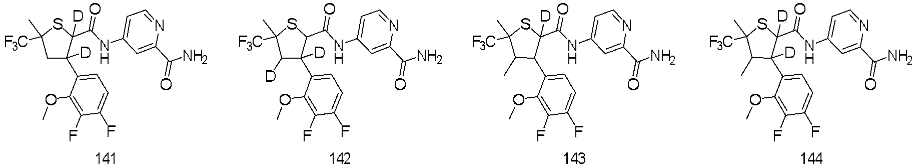

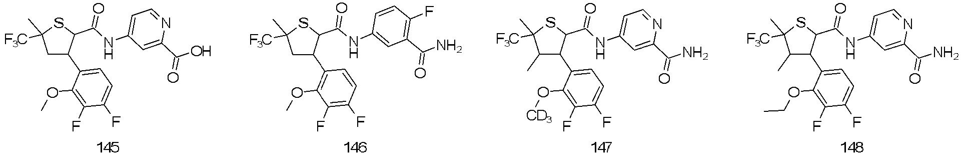

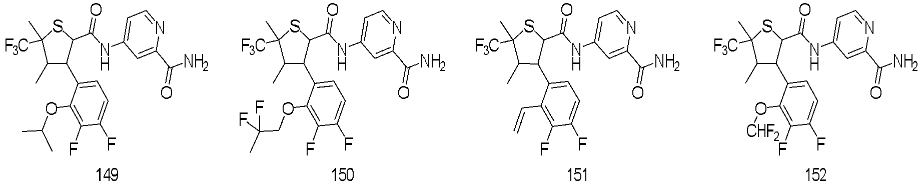

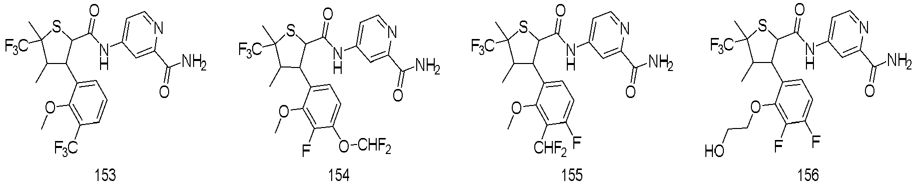

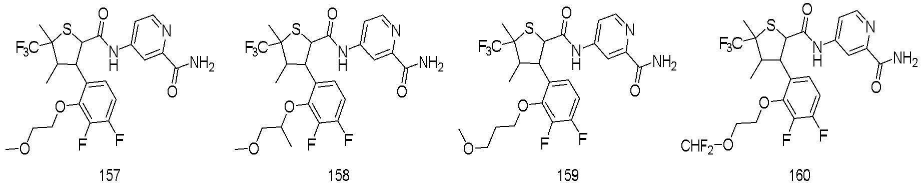

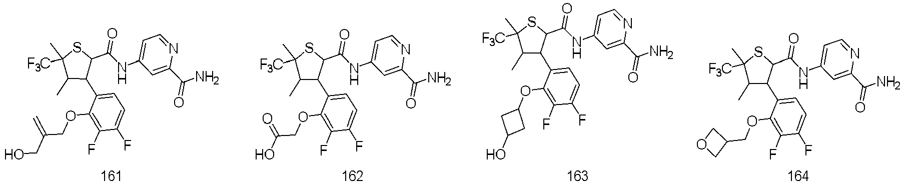

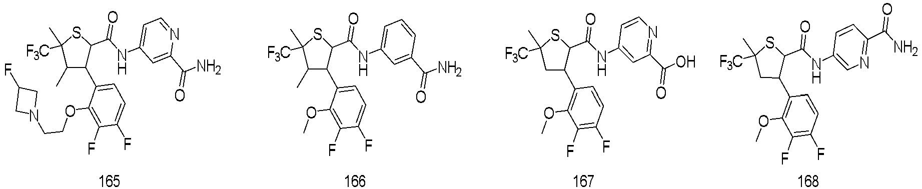

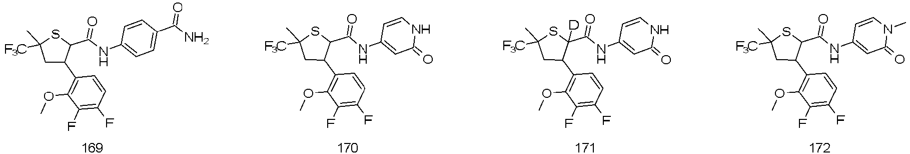

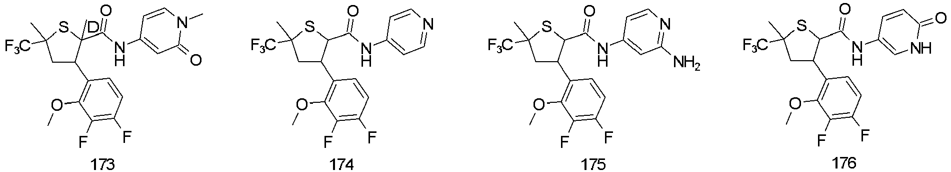

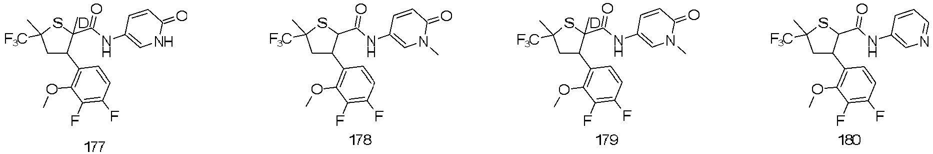

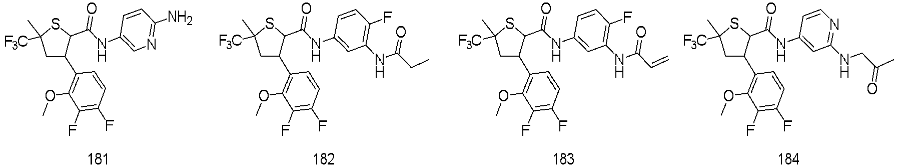

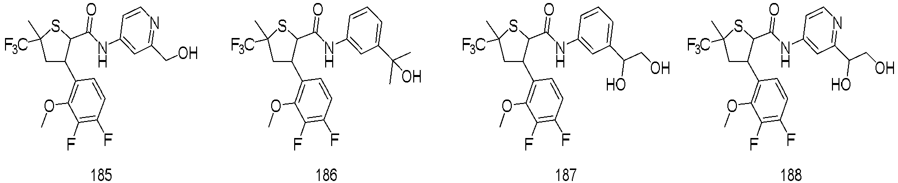

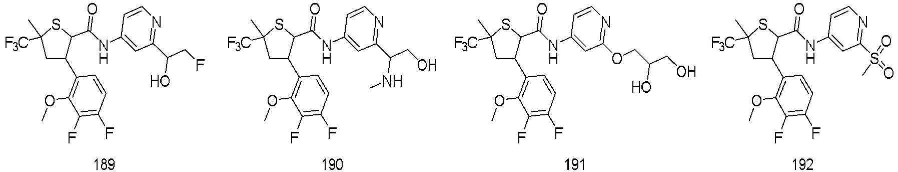

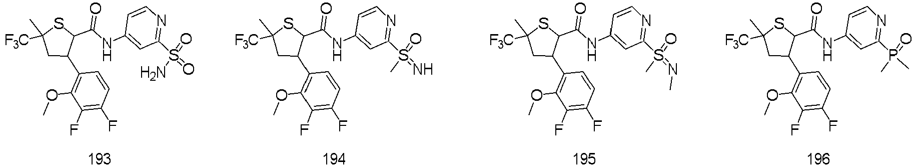

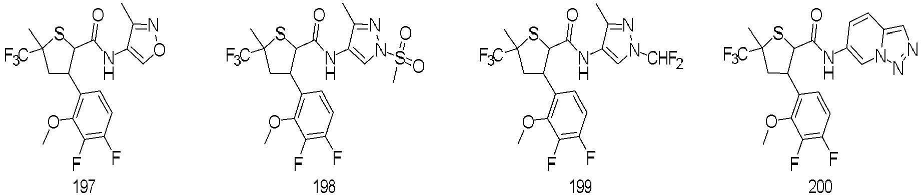

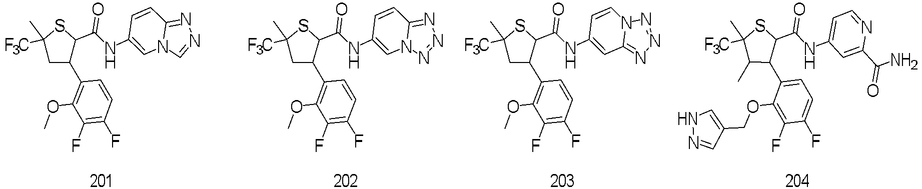

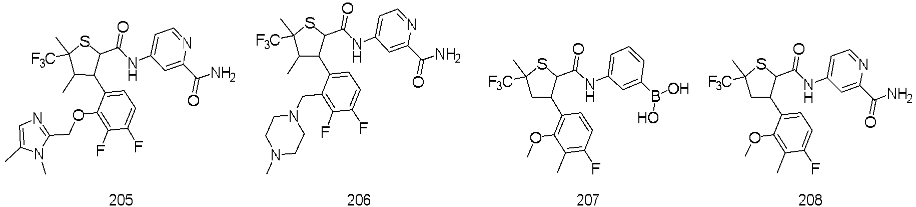

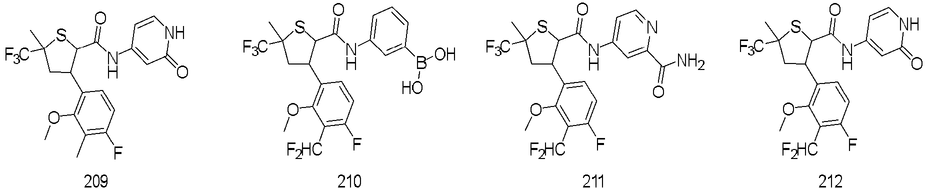

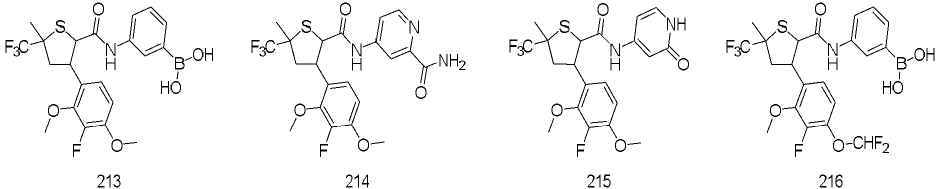

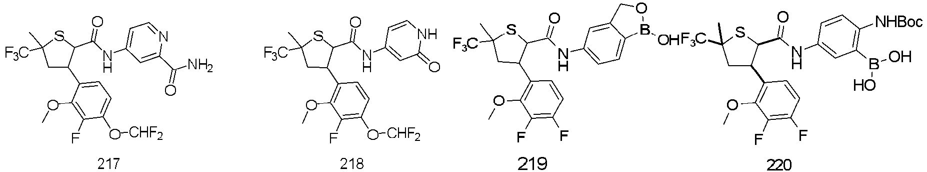

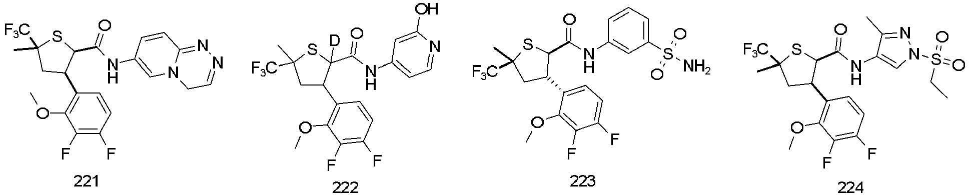

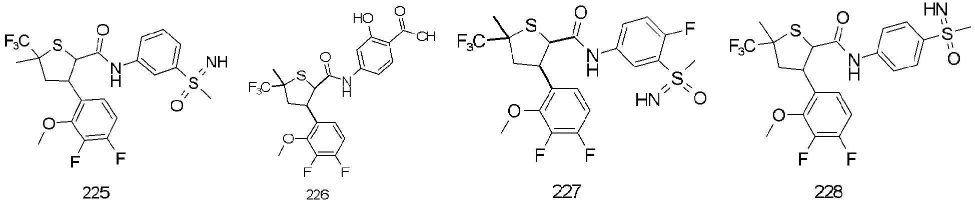

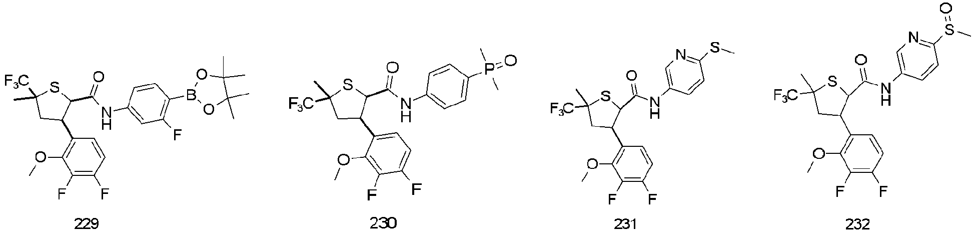

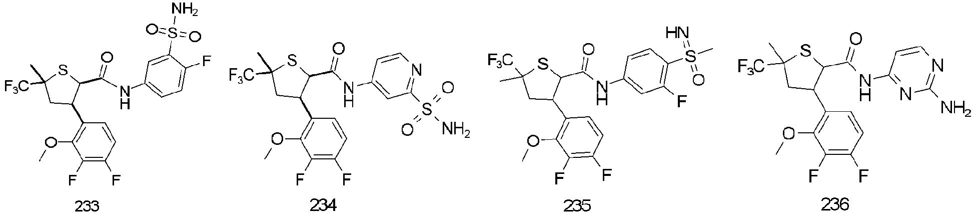

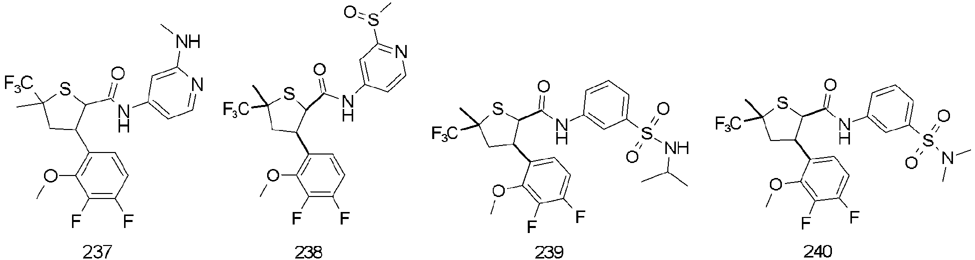

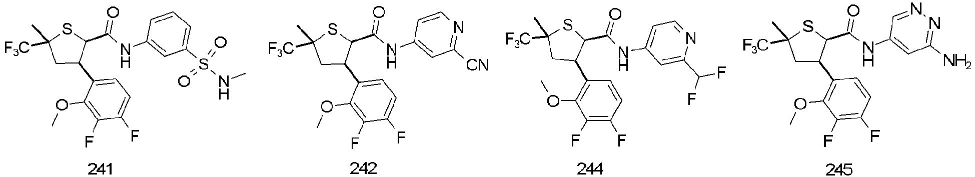

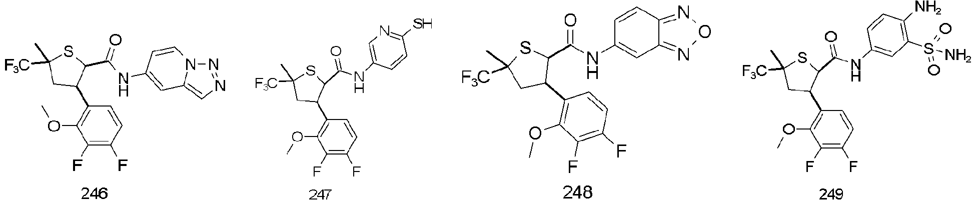

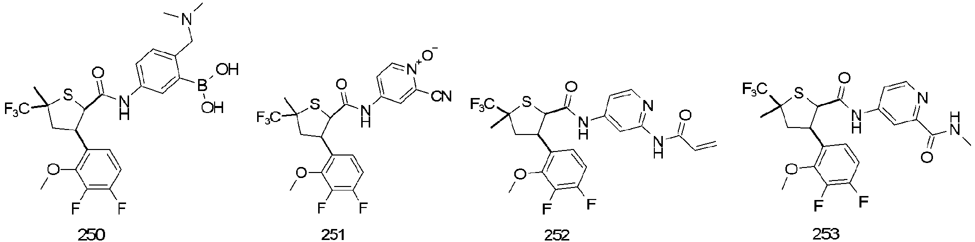

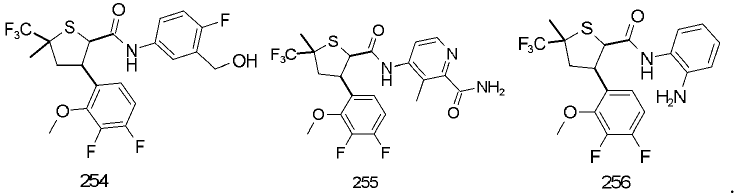

In some embodiments, the compound of the present disclosure is selected from:

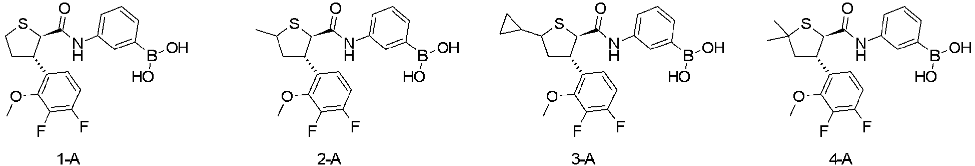

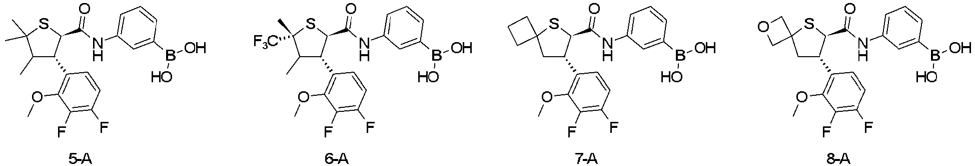

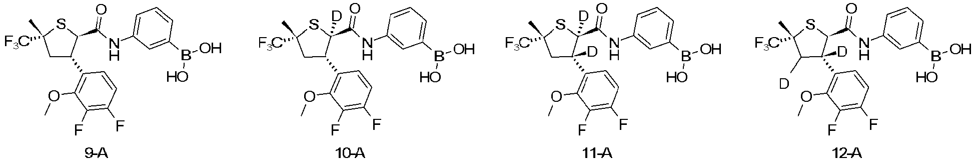

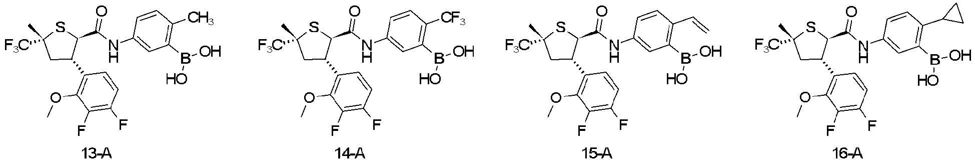

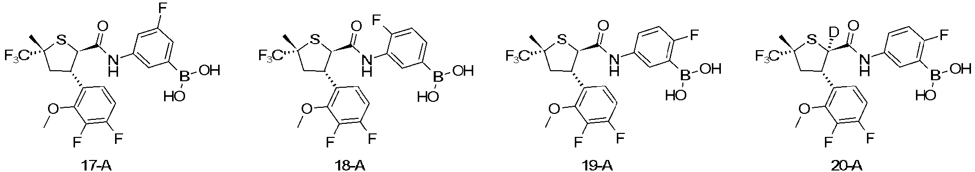

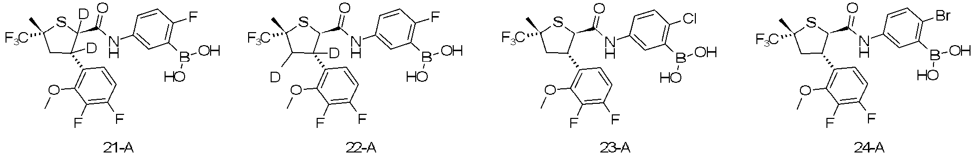

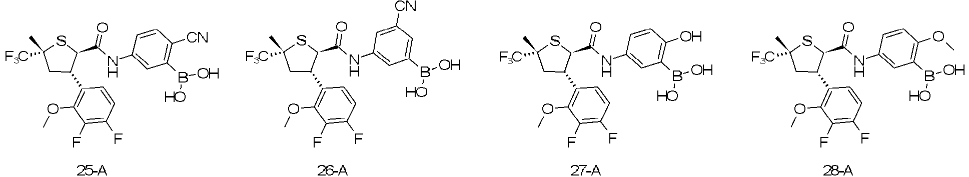

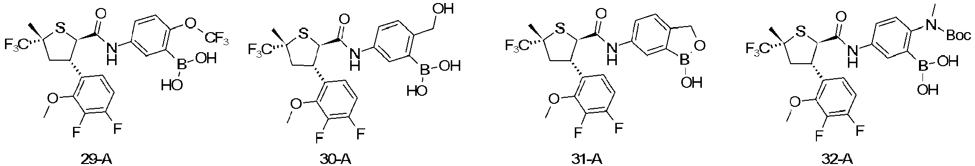

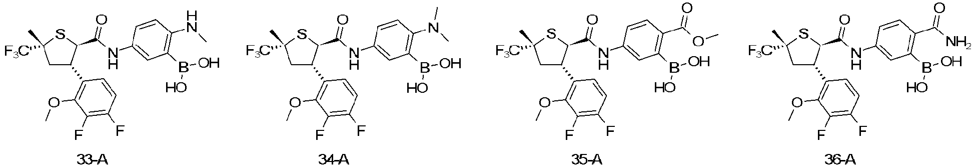

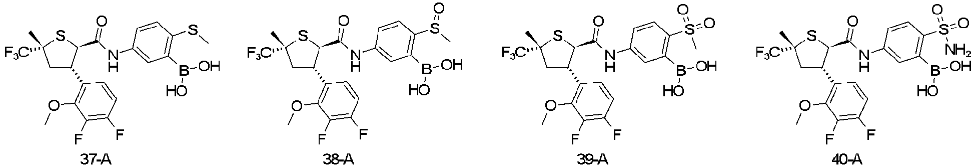

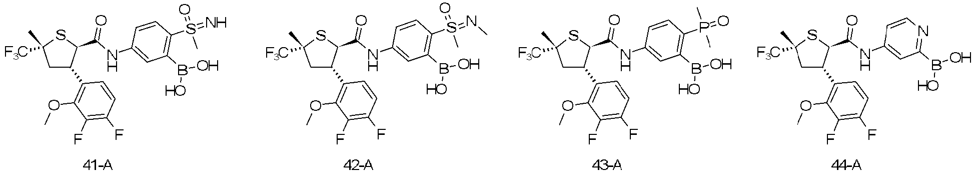

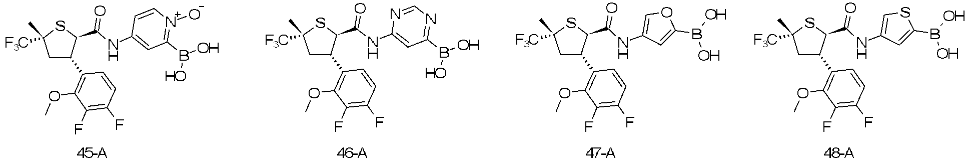

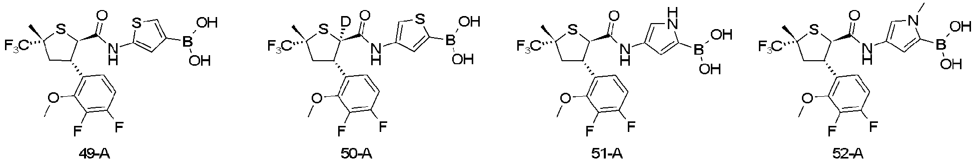

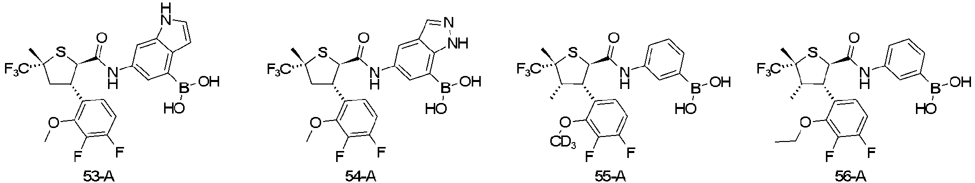

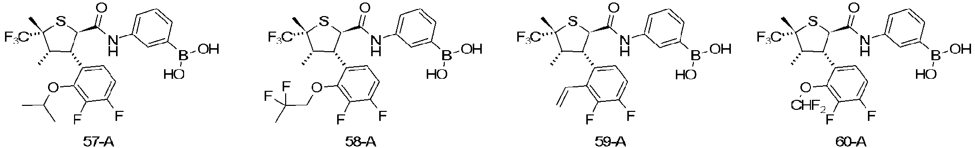

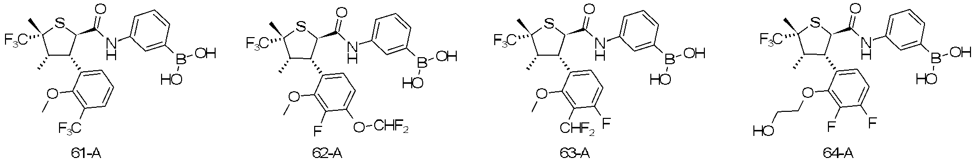

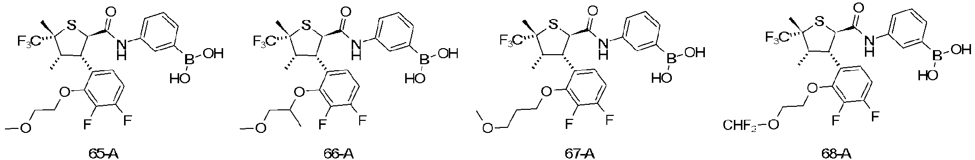

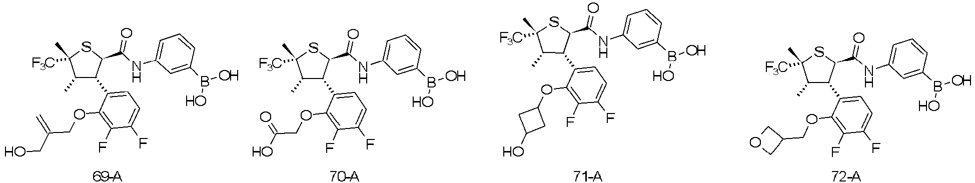

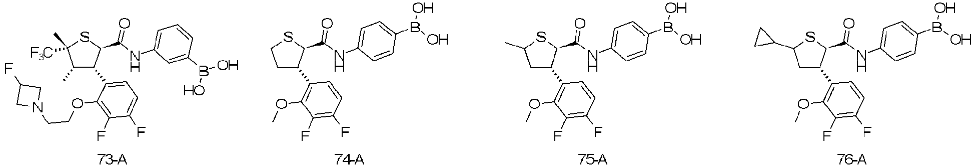

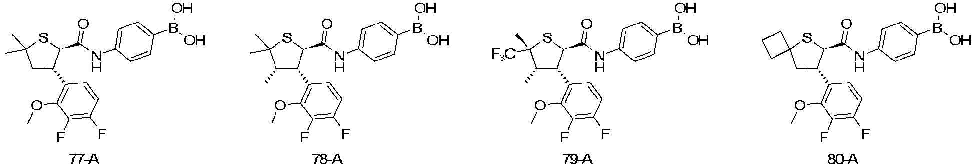

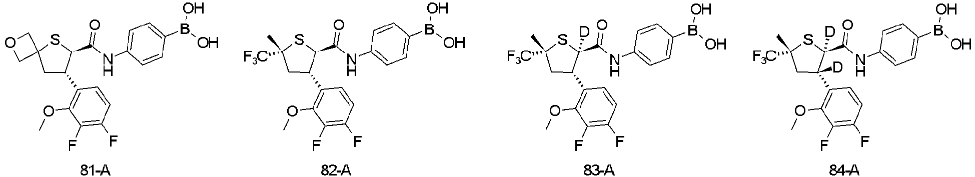

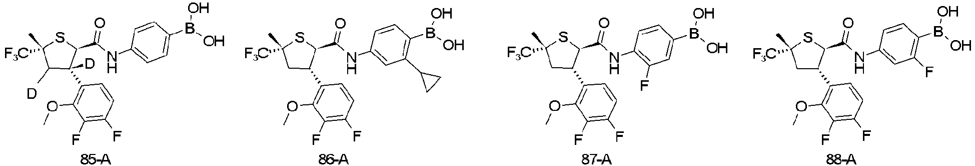

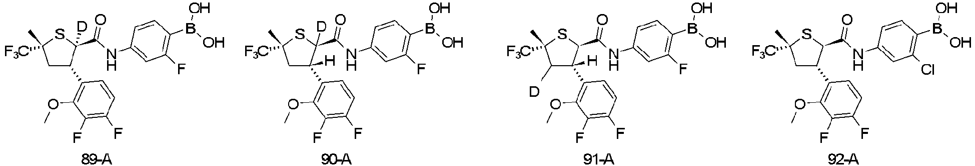

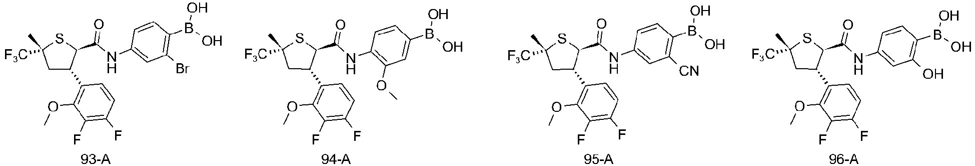

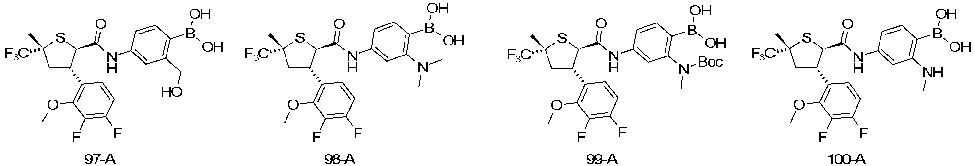

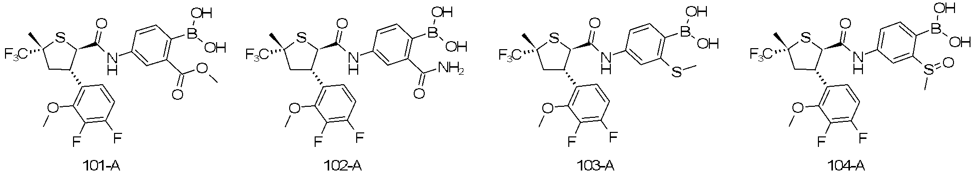

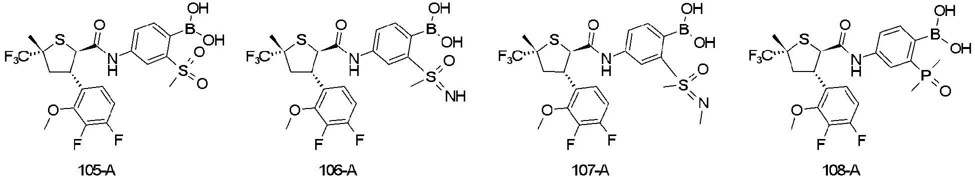

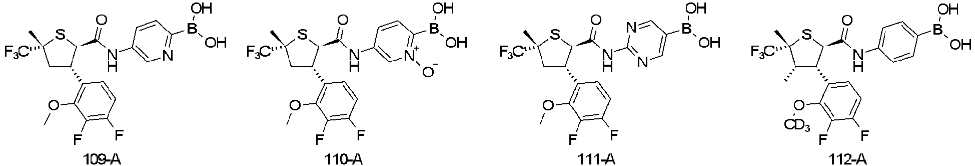

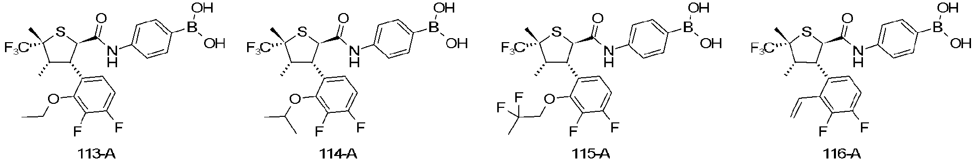

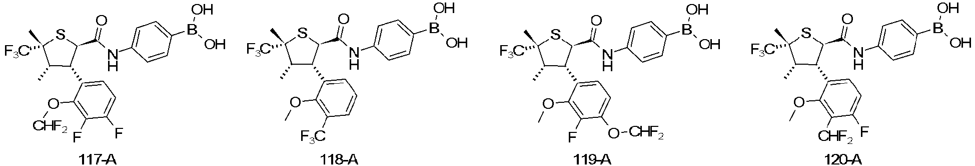

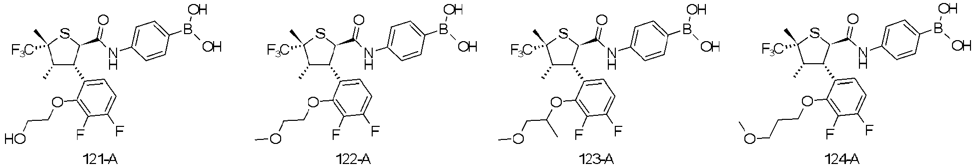

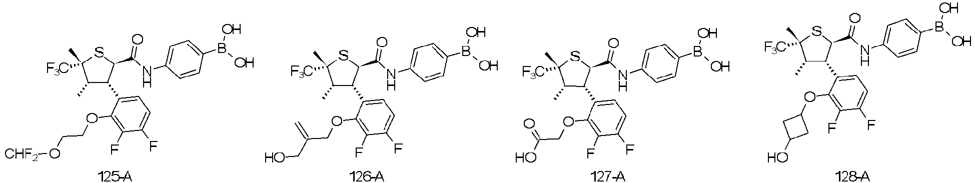

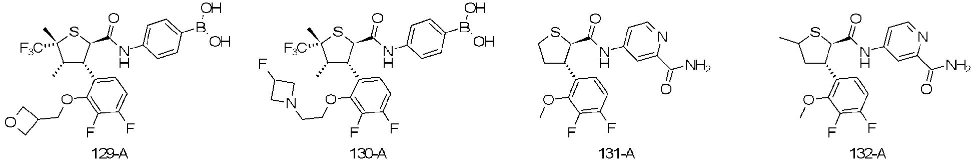

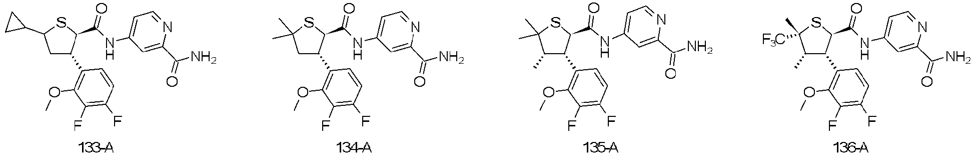

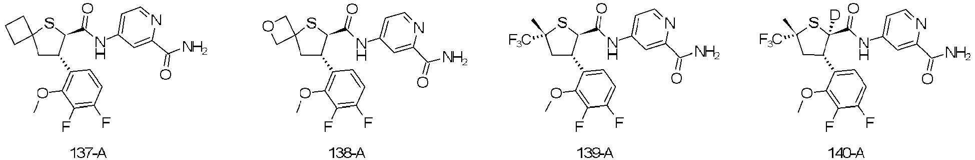

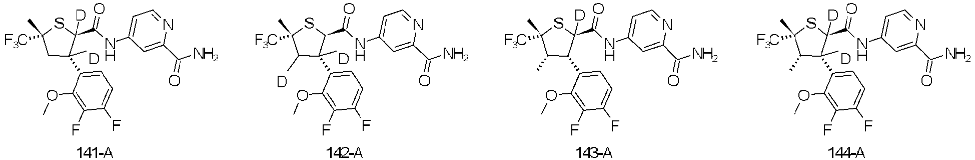

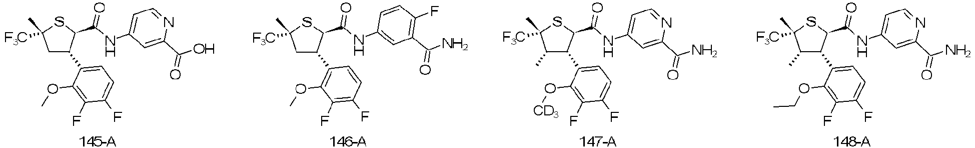

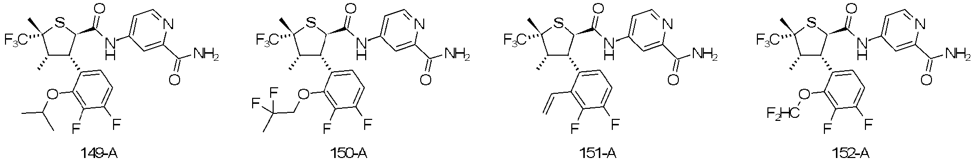

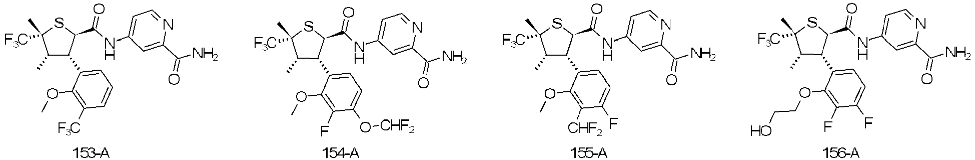

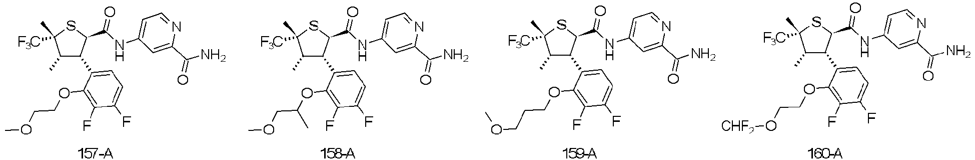

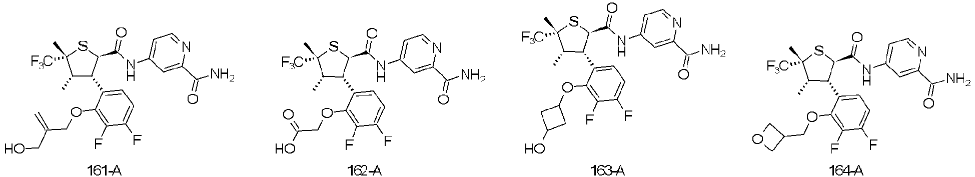

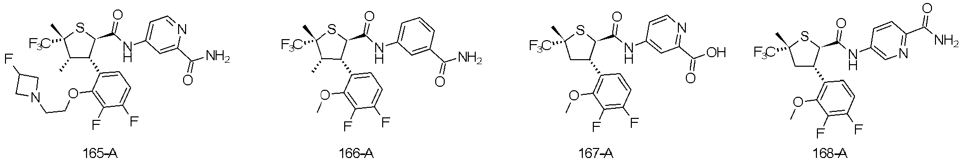

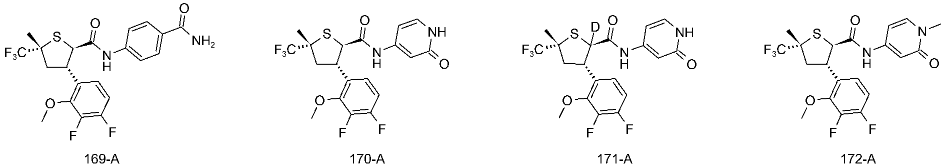

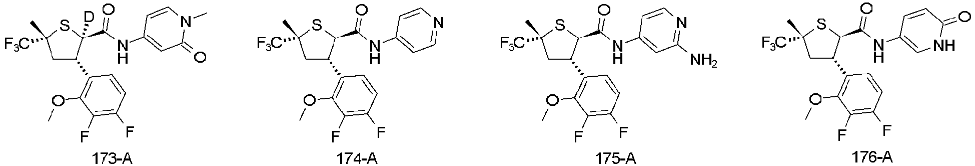

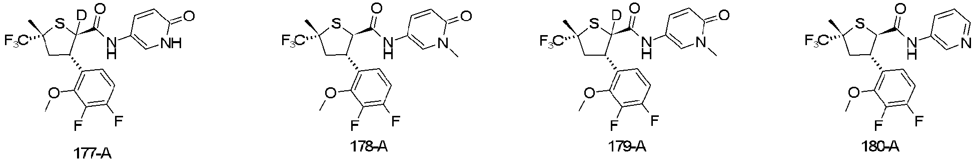

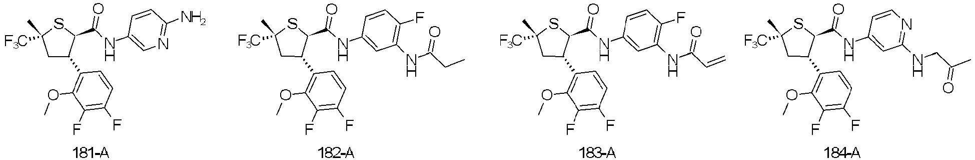

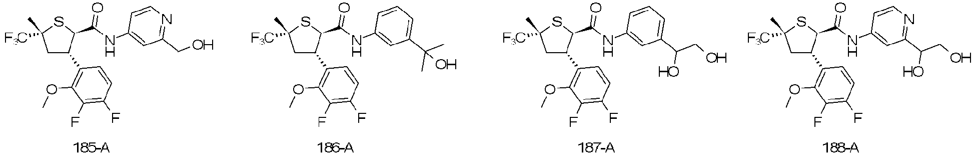

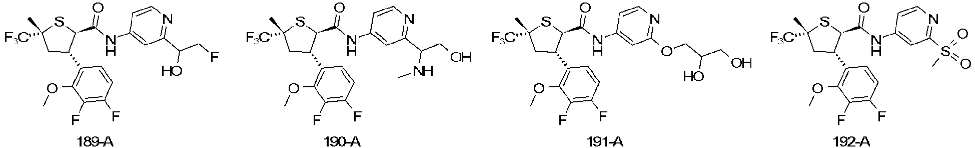

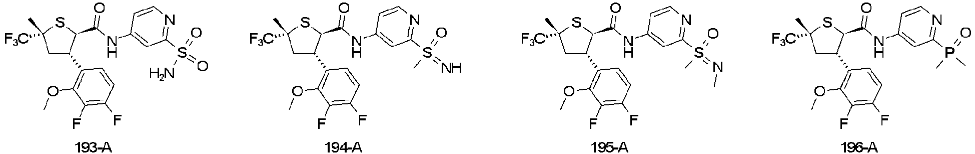

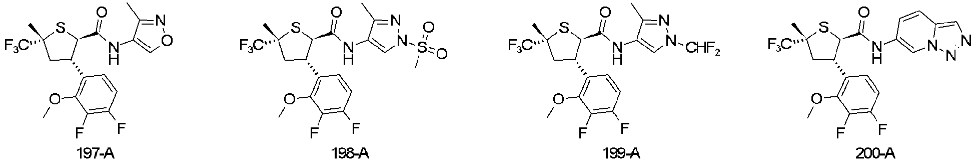

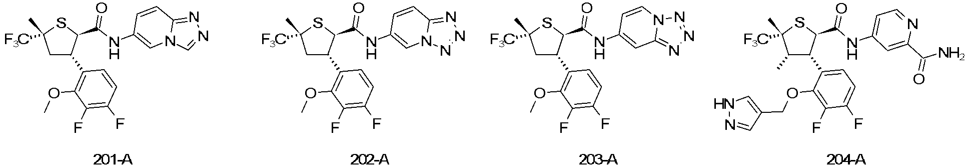

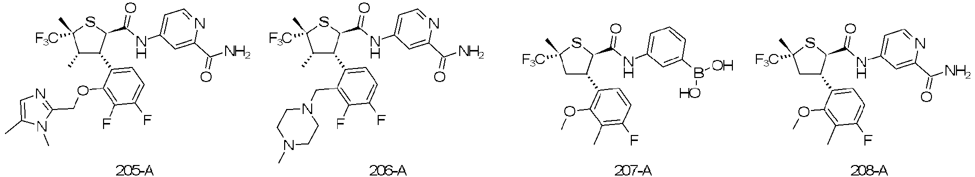

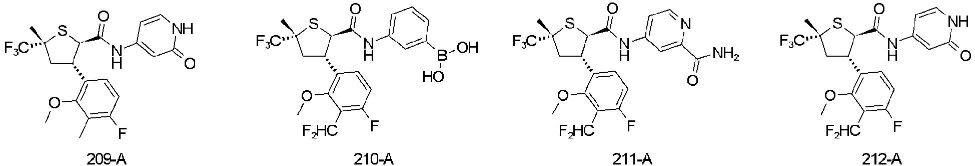

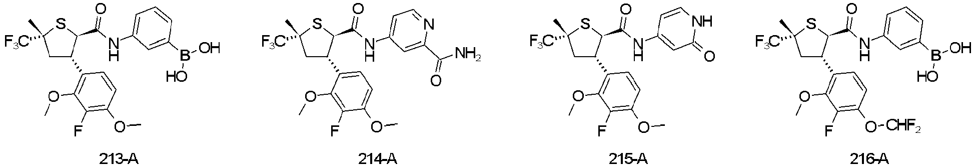

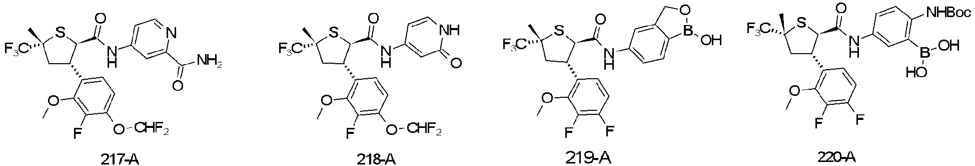

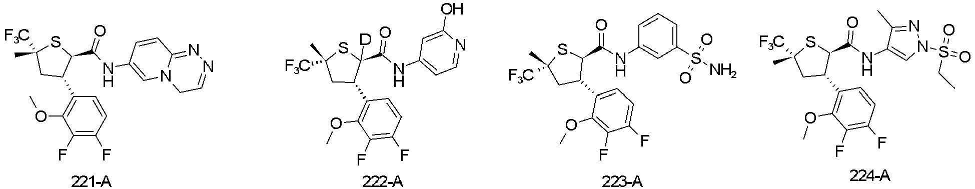

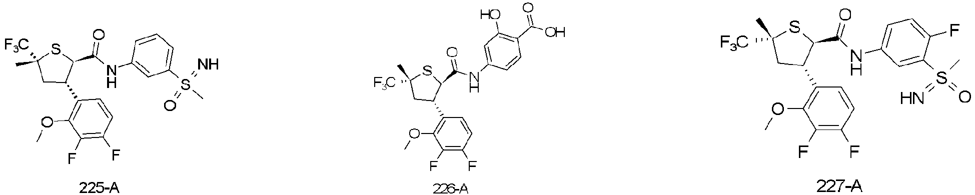

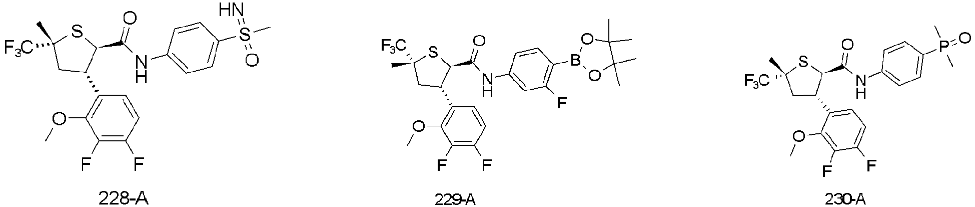

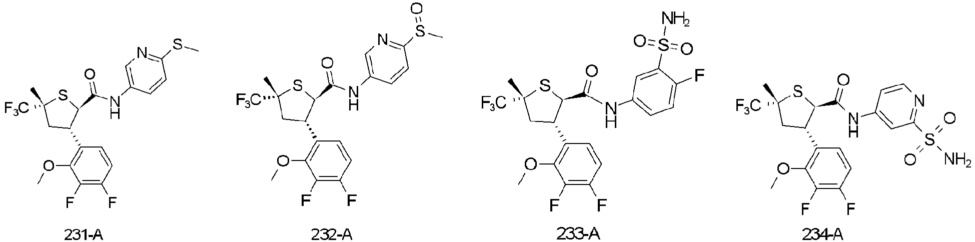

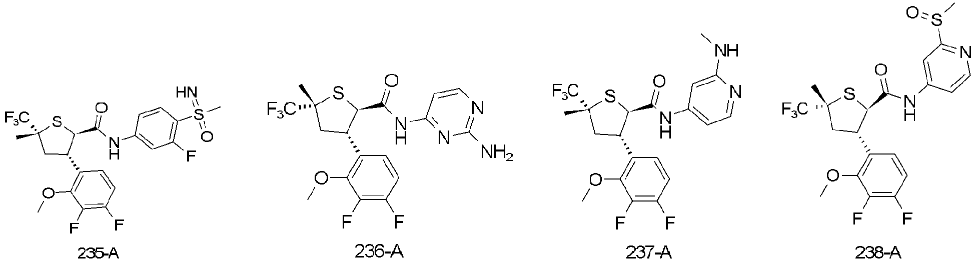

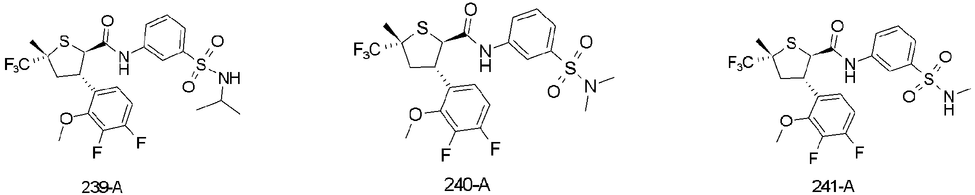

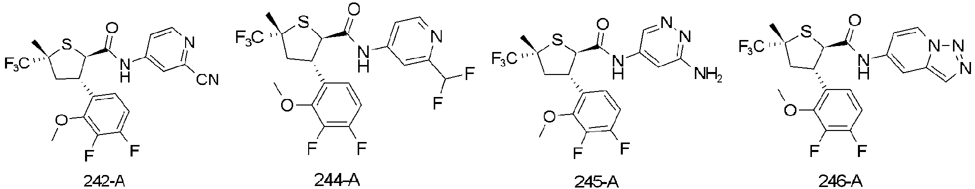

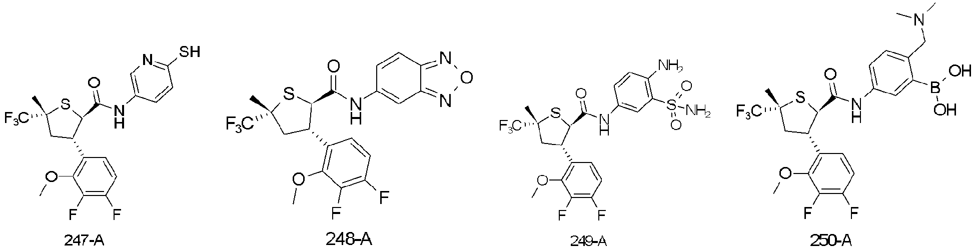

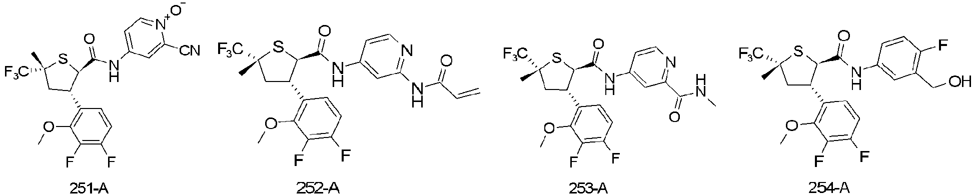

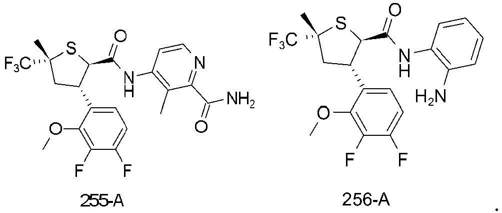

In another aspect of the present disclosure, a compound of formula II, or a pharmaceutically acceptable salt, a stereoisomer, a solvate, an isotopically labeled compound, or a polymorph thereof is provided, wherein the compound has the following structure: 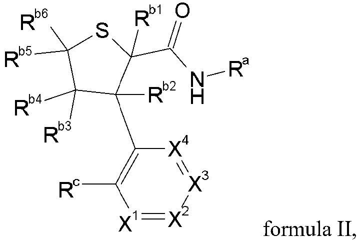
wherein, R^{a} is selected from 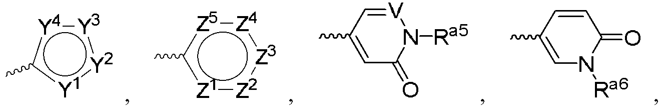 and 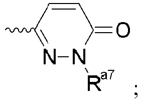
Y¹, Y², Y³, and Y⁴ are each independently selected from O, S, N, N-R^{a1}, and C-R^{a2};
each R^{a1} is independently selected from -S(O)₂-R⁴²;
each R^{a2} is independently selected from H and C₁₋₆ alkyl;
each R⁴² is independently selected from C₁₋₆ haloalkyl and C₃₋₆ cycloalkyl;
Z¹, Z², Z³, Z⁴, and Z⁵ are each independently selected from N, N⁺-O⁻, and C-R^{a3};
each R^{a3} is independently selected from H, halogen, -N₃, -NO₂, -SCN, -S(F)₅, -CH(O), C₂₋₆ alkenyl, C₂₋₆ alkynyl, C₃₋₆ cycloalkyl, 3-6 membered heterocyclyl, 5-6 membered heteroaryl, -C(O)NR⁴³R⁴⁴, -C(0)NR⁴⁵S(O)₂NR⁴⁶R⁴⁷, - C(S)NR⁴⁸R⁴⁹, -C(=NR⁵⁰)NR⁵¹R⁵², -C(=NR⁵³)R⁵⁴, -NR⁵⁵R⁵⁶, -NR⁵⁷S(O)₂R⁵⁸, -NR⁵⁹S(O)₂NR⁶⁰R⁶¹, -N=S(O)R⁶²R⁶³, - OS(O)₂R⁶⁴, -S(O)₂R⁶⁵, -S(O)₂NR⁶⁶R⁶⁷, -Si(OR⁶⁸)₃, and 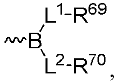 wherein the C₂₋₆ alkenyl, C₂₋₆ alkynyl, C₃₋₆ cycloalkyl, 3-6 membered heterocyclyl, and 5-6 membered heteroaryl are optionally substituted with one or more substituents selected from halogen, hydroxy, -CN, amino, C₁₋₆ alkyl, C₁₋₆ haloalkyl, C₁₋₆ alkoxy, C₁₋₆ haloalkoxy, - C(O)OR⁷¹, and -C(O)NR⁷²R⁷³;
or two adjacent R^{a3}, together with the carbon atoms to which they are attached, form 5-6 membered heterocyclyl or a 5-10 membered heteroaromatic ring, wherein the 5-6 membered heterocyclyl or 5-10 membered heteroaromatic ring is optionally substituted with one or more substituents selected from halogen, hydroxy, amino, -CN, =O, C₁₋₆ alkyl, and -C₁₋₆ alkyl NR⁷⁴R⁷⁵;
R⁴³, R⁴⁴, R⁵⁰, and R⁵³ are each independently selected from H, hydroxy, C₁₋₆ alkyl, and C₁₋₆ alkoxy;
R⁴⁵, R⁴⁶, R⁴⁷, R⁴⁸, R⁴⁹, R⁵¹, R⁵², R⁵⁴, R⁵⁷, R⁵⁹, R⁶², R⁶³, R⁶⁸, R⁷¹, R⁷², R⁷³, R⁷⁴, and R⁷⁵ are each independently selected from H, C₁₋₆ alkyl, and C₁₋₆ haloalkyl;
R⁵⁵, R⁵⁶, R⁶⁶, and R⁶⁷ are each independently selected from H, hydroxy, -CN, C₁₋₆ alkyl, -C(O)C₁₋₆ alkyl, -C(O)C₂₋₆ alkenyl, -C(O)C₂₋₆ alkynyl, C₃₋₆ membered cycloalkyl, and 3-6 membered heterocyclyl, wherein the C₁₋₆ alkyl, C₂₋₆ alkenyl, C₂₋₆ alkynyl, C₃₋₆ membered cycloalkyl, and 3-6 membered heterocyclyl are optionally substituted with halogen, hydroxy, amino, -CN, N₃, C₁₋₆ alkyl, C₁₋₆ alkoxy, and C₁₋₆ alkylamino;
R⁵⁸ is independently selected from halogen and C₁₋₆ alkyl, wherein the C₁₋₆ alkyl is optionally substituted with one or more substituents selected from halogen, hydroxy, amino, C₁₋₆ alkoxy, C₁₋₆ alkylamino, and N₃;
R⁶⁰ and R⁶¹ are each independently selected from C₁₋₆ alkyl, wherein the C₁₋₆ alkyl is optionally substituted with one or more substituents selected from halogen, hydroxy, amino, C₁₋₆ alkoxy, C₁₋₆ alkylamino, and N₃;
R⁶⁴ is independently selected from halogen, amino, and C₁₋₆ alkylamino;
R⁶⁵ is independently selected from halogen, N₃, C₁₋₆ alkyl, and C₁₋₆ haloalkyl;
R⁶⁹ and R⁷⁰ are each independently selected from H, halogen, -CN, C₁₋₆ alkyl, C₁₋₆ haloalkyl, -C(O)OC₁₋₆ alkyl, - C(O)NH₂, and -C(O)NHC₁₋₆ alkyl, wherein the C₁₋₆ alkyl and C₁₋₆ haloalkyl are optionally substituted with hydroxy and amino; or R⁶⁹ and R⁷⁰, together with the boron and oxygen atoms to which they are attached, form 5-10 membered heterocyclyl (e.g., 5-6 membered heterocyclyl), wherein the heterocyclyl is optionally substituted with one or more substituents selected from hydrogen, halogen, hydroxy, C₁₋₄ alkyl, and =O;
L¹ and L² are each independently selected from O and N;
V is selected from N, N⁺-O⁻, and C-R^{a4};
R^{a4} is selected from H and C₁₋₆ alkyl;
R^{a5} is selected from H and C₁₋₆ alkyl;
R^{a6} is selected from H and C₁₋₆ alkyl;
R^{a7} is selected from H and C₁₋₆ alkyl;
R^{b1} and R^{b2} are each independently selected from H and deuterium;
R^{b3} and R^{b4} are each independently selected from H, deuterium, C₁₋₆ alkyl, C₁₋₆ haloalkyl, and C₃₋₆ cycloalkyl;
R^{b5} and R^{b6} are each independently selected from H, C₁₋₆ alkyl, C₁₋₆ haloalkyl, and C₃₋₆ cycloalkyl, or R^{b5} and R^{b6}, together with the carbon atom to which they are attached, form C₃₋₅ cycloalkyl and 4-6 membered heterocyclyl;
R^{c} is selected from H, hydroxy, halogen, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl, and -O-C₃₋₆ cycloalkyl, wherein the C₁₋₆ alkyl, C₁₋₆ haloalkyl, C₁₋₆ alkoxy, C₁₋₆ haloalkoxy, and C₃₋₆ cycloalkyl are optionally substituted with one or more substituents selected from hydroxy, carboxyl, C₁₋₆ alkoxy, C₁₋₆ haloalkoxy, C₂₋₆ alkenyl, -NR⁴⁰R⁴¹, C₃₋₆ cycloalkyl, 3-6 membered heterocyclyl, and 5-6 membered heteroaryl, wherein the 3-6 membered heterocyclyl and 5-6 membered heteroaryl are optionally substituted with one or more halogen and C₁₋₆ alkyl;
R⁴⁰ and R⁴¹ are each independently selected from H and C₁₋₆ alkyl;
X¹, X², X³, and X⁴ are each independently selected from N and C-R^{c1};
each R^{c1} is independently selected from H, halogen, C₁₋₆ alkyl, C₁₋₆ haloalkyl, C₁₋₆ alkoxy, and C₁₋₆ haloalkoxy.

In some embodiments, in the compound of formula II of the present application, R^{b1} and R^{b2} are each independently selected from H.

In some embodiments, in the compound of formula II of the present application, R^{b1} and R^{b2} are each independently selected from deuterium.

In some embodiments, in the compound of formula II of the present application, R^{b3} and R^{b4} are each independently selected from H, deuterium, and methyl.

In some embodiments, in the compound of formula II of the present application, R^{b3} and R^{b4} are both selected from H. In some embodiments, in the compound of formula II of the present application, R^{b5} and R^{b6} are each independently selected from H, methyl, cyclopropyl, and trifluoromethyl, or R^{b5} and R^{b6}, together with the carbon atom to which they are attached, form cyclobutyl, cyclopentyl, and 4-6 membered oxygen-containing heterocyclyl.

In some embodiments, in the compound of formula II of the present application, R^{b5} and R^{b6} are each independently selected from H, methyl, cyclopropyl, and trifluoromethyl, or R^{b5} and R^{b6}, together with the carbon atom to which they are attached, form cyclobutyl and

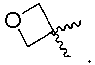

In some embodiments, in the compound of formula II of the present application, R^{b5} and R^{b6} are each independently selected from H, methyl, cyclopropyl, and trifluoromethyl.

In some embodiments, in the compound of formula II of the present application, R^{b5} is selected from methyl, and R^{b6} is selected from trifluoromethyl.

In some embodiments, in the compound of formula II of the present application, R^{c} is selected from H, hydroxy, halogen, C₁₋₄ alkyl, C₁₋₄ deuterated alkyl, C₁₋₄ haloalkyl, C₁₋₄ alkoxy, C₁₋₄ deuterated alkoxy, C₁₋₄ haloalkoxy, C₂₋₆ alkenyl, and -O-C₃₋₆ cycloalkyl, wherein the C₁₋₄ alkyl, C₁₋₄ haloalkyl, C₁₋₄ alkoxy, C₁₋₄ haloalkoxy, and C₃₋₆ cycloalkyl are optionally substituted with one or more substituents selected from hydroxy, carboxyl, C₁₋₄ alkoxy, C₁₋₄ haloalkoxy, C₂₋₆ alkenyl, -NR⁴⁰R⁴¹, C₃₋₆ cycloalkyl, 3-6 membered heterocyclyl, and 5-6 membered heteroaryl, wherein the 3-6 membered heterocyclyl and 5-6 membered heteroaryl are optionally substituted with one or more halogen and C₁₋₄ alkyl; R⁴⁰ and R⁴¹ are each independently selected from H and C₁₋₄ alkyl.

In some embodiments, in the compound of formula II of the present application, R^{c} is selected from H, hydroxy, halogen, C₁₋₄ alkyl, C₁₋₄ deuterated alkyl, C₁₋₄ haloalkyl, and C₁₋₄ alkoxy.

In some embodiments, in the compound of formula II of the present application, R^{c} is selected from C₁₋₄ alkoxy.

In some embodiments, in the compound of formula II of the present application, R^{c} is selected from methoxy.

In some embodiments, in the compound of formula II of the present application, X¹, X², X³, and X⁴ are each independently selected from C-R^{c1}.

In some embodiments, in the compound of formula II of the present application, each R^{c1} is independently selected from H, halogen, C₁₋₄ alkyl, C₁₋₄ haloalkyl, C₁₋₄ alkoxy, and C₁₋₄ haloalkoxy.

In some embodiments, in the compound of formula II of the present application, each R^{c1} is independently selected from H, F, Cl, methyl, ethyl, propyl, butyl, fluoromethyl, fluoroethyl, fluoropropyl, methoxy, ethoxy, propoxy, butoxy, fluoromethoxy, fluoroethoxy, and fluoropropoxy.

In some embodiments, in the compound of formula II of the present application, each R^{c1} is independently selected from H, F, methyl, difluoromethyl, trifluoromethyl, difluoromethoxy, methoxy, and difluoromethoxy.

In some embodiments, in the compound of formula II of the present application, X¹ is selected from C-R^{c1}, and R^{c1} is selected from methoxy.

In some embodiments, in the compound of formula II of the present application, X² is selected from C-R^{c1}, and R^{c1} is selected from F.

In some embodiments, in the compound of formula II of the present application, X³ is selected from C-R^{c1}, and R^{c1} is selected from F.

In some embodiments, in the compound of formula II of the present application, X⁴ is selected from C-R^{c1}, and R^{c1} is selected from H.

In some embodiments, in the compound of formula II of the present application, Ra is selected from 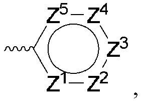 wherein Z¹, Z², Z³, Z⁴, and Z⁵ are each independently selected from N, N⁺-O⁻, and C-R^{a3}; each R^{a3} is independently selected from H, halogen, -N₃, -NO₂, -SCN, -S(F)₅, -CH(O), C₂₋₆ alkenyl, C₂₋₆ alkynyl, C₃₋₆ cycloalkyl, 3-6 membered heterocyclyl, 5-6 membered heteroaryl, -C(O)NR⁴³R⁴⁴, -C(0)NR⁴⁵S(O)₂NR⁴⁶R⁴⁷, -C(S)NR⁴⁸R⁴⁹, -C(=NR⁵⁰)NR⁵¹R⁵², -C(=NR⁵³)R⁵⁴, -NR⁵⁵R⁵⁶, -NR⁵⁷S(O)₂R⁵⁸, -NR⁵⁹S(O)₂NR⁶⁰R⁶¹, -N=S(O)R⁶²R⁶³, -OS(O)₂R⁶⁴, -S(O)₂R⁶⁵, - S(O)₂NR⁶⁶R⁶⁷, -Si(OR⁶⁸)₃, and 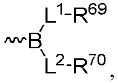 wherein the C₂₋₆ alkenyl, C₂₋₆ alkynyl, C₃₋₆ cycloalkyl, 3-6 membered heterocyclyl, and 5-6 membered heteroaryl are optionally substituted with one or more substituents selected from halogen, hydroxy, -CN, amino, C₁₋₆ alkyl, C₁₋₆ haloalkyl, C₁₋₆ alkoxy, C₁₋₆ haloalkoxy, -C(O)OR⁷¹, and -C(O)NR⁷²R⁷³; or two adjacent R^{a3}, together with the carbon atoms to which they are attached, form 5-6 membered heterocyclyl or a 5-10 membered heteroaromatic ring, wherein the 5-6 membered heterocyclyl or 5-10 membered heteroaromatic ring is optionally substituted with one or more substituents selected from halogen, hydroxy, amino, -CN, =O, C₁₋₆ alkyl, and -C₁₋₆ alkyl NR⁷⁴R⁷⁵;
R⁴³, R⁴⁴, R⁵⁰, and R⁵³ are each independently selected from H, hydroxy, C₁₋₆ alkyl, and C₁₋₆ alkoxy;
R⁴⁵, R⁴⁶, R⁴⁷, R⁴⁸, R⁴⁹, R⁵¹, R⁵², R⁵⁴, R⁵⁷, R⁵⁹, R⁶², R⁶³, R⁶⁸, R⁷¹, R⁷², R⁷³, R⁷⁴, and R⁷⁵ are each independently selected from H, C₁₋₆ alkyl, and C₁₋₆ haloalkyl;
R⁵⁵, R⁵⁶, R⁶⁶, and R⁶⁷ are each independently selected from H, hydroxy, -CN, C₁₋₆ alkyl, -C(O)C₁₋₆ alkyl, -C(O)C₂₋₆ alkenyl, -C(O)C₂₋₆ alkynyl, C₃₋₆ membered cycloalkyl, and 3-6 membered heterocyclyl, wherein the C₁₋₆ alkyl, C₂₋₆ alkenyl, C₂₋₆ alkynyl, C₃₋₆ membered cycloalkyl, and 3-6 membered heterocyclyl are optionally substituted with halogen, hydroxy, amino, -CN, N₃, C₁₋₆ alkyl, C₁₋₆ alkoxy, and C₁₋₆ alkylamino;
R⁵⁸ is independently selected from halogen and C₁₋₆ alkyl, wherein the C₁₋₆ alkyl is optionally substituted with one or more substituents selected from halogen, hydroxy, amino, C₁₋₆ alkoxy, C₁₋₆ alkylamino, and N₃;
R⁶⁰ and R⁶¹ are each independently selected from C₁₋₆ alkyl, wherein the C₁₋₆ alkyl is optionally substituted with one or more substituents selected from halogen, hydroxy, amino, C₁₋₆ alkoxy, C₁₋₆ alkylamino, and N₃;
R⁶⁴ is independently selected from halogen, amino, and C₁₋₆ alkylamino;
R⁶⁵ is independently selected from halogen, N₃, C₁₋₆ alkyl, and C₁₋₆ haloalkyl;
R⁶⁹ and R⁷⁰ are each independently selected from H, halogen, -CN, C₁₋₆ alkyl, C₁₋₆ haloalkyl, -C(O)OC₁₋₆ alkyl, - C(O)NH₂, and -C(O)NHC₁₋₆ alkyl, wherein the C₁₋₆ alkyl and C₁₋₆ haloalkyl are optionally substituted with hydroxy and amino; or R⁶⁹ and R⁷⁰, together with the boron and oxygen atoms to which they are attached, form 5-10 membered heterocyclyl (e.g., 5-6 membered heterocyclyl), wherein the heterocyclyl is optionally substituted with one or more substituents selected from hydrogen, halogen, hydroxy, C₁₋₄ alkyl, and =O;
L¹ and L² are each independently selected from O and N.

In some embodiments, in the compound of formula II of the present application, R^{a} is selected from phenyl, pyridine, and pyridazine substituted with one or more R^{3a}, wherein each R^{3a} is independently selected from H, halogen, -N₃, - NO₂, -SCN, -S(F)₅, -CH(O), C₂₋₆ alkenyl, C₂₋₆ alkynyl, C₃₋₆ cycloalkyl, 3-6 membered heterocyclyl, 5-6 membered heteroaryl, -C(O)NR⁴³R⁴⁴, -C(O)NR⁴⁵S(O)₂NR⁴⁶R⁴⁷, -C(S)NR⁴⁸R⁴⁹, -C(=NR⁵⁰)NR⁵¹R⁵², -C(=NR⁵³)R⁵⁴, -NR⁵⁵R⁵⁶, - NR⁵⁷S(O)₂R⁵⁸, -NR⁵⁹S(P)₂NR⁶⁰R⁶¹, -N=S(O)R⁶²R⁶³, -OS(P)₂R⁶⁴, -S(O)₂R⁶⁵, -S(O)₂NR⁶⁶R⁶⁷, -Si(OR⁶⁸)₃, and 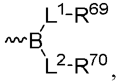 wherein the C₂₋₆ alkenyl, C₂₋₆ alkynyl, C₃₋₆ cycloalkyl, 3-6 membered heterocyclyl, and 5-6 membered heteroaryl are optionally substituted with one or more substituents selected from halogen, hydroxy, -CN, amino, C₁₋₆ alkyl, C₁₋₆ haloalkyl, C₁₋₆ alkoxy, C₁₋₆ haloalkoxy, -C(O)OR⁷¹, and -C(O)NR⁷²R⁷³;
or two adjacent R^{a3}, together with the carbon atoms to which they are attached, form 5-6 membered heterocyclyl or a 5-10 membered heteroaromatic ring, wherein the 5-6 membered heterocyclyl or 5-10 membered heteroaromatic ring is optionally substituted with one or more substituents selected from halogen, hydroxy, amino, -CN, =O, C₁₋₆ alkyl, and -C₁₋₆ alkyl NR⁷⁴R^{75.}

In some embodiments, in the compound of formula II of the present application, R^{a} is selected from phenyl, pyridine, and pyridazine substituted with one or more R^{3a}, wherein each R^{3a} is independently selected from F, N₃, -SCN, N(H)CN, -S(O)₂CHF₂, -S(O)₂CF₃, SF₅, -NO₂, -S(O)₂N(H)CN, -N=S(O)(CH₃)₂, -OS(O)₂NH₂, -NH(OH), - NHS(O)₂NH₂, -NHS(O)₂CH₂CH₃, C(S)NH₂, -S(O)₂N₃, -OS(O)₂F, -NHS(O)₂F, -S(O)₂NH₂, -CH(O), -C(O)NH(OH), 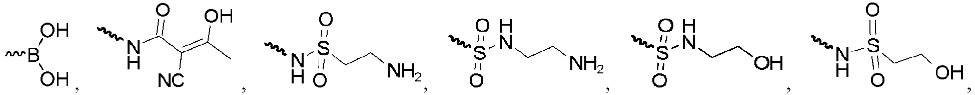 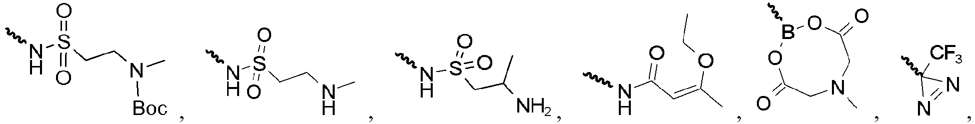 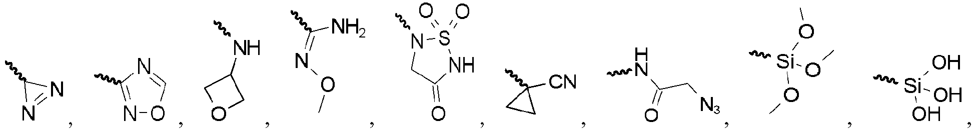 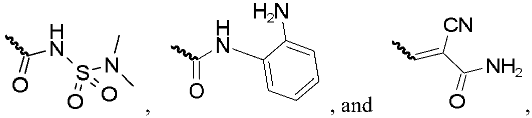 or two adjacent R^{a3}, together with the carbon atoms to which they are attached, form

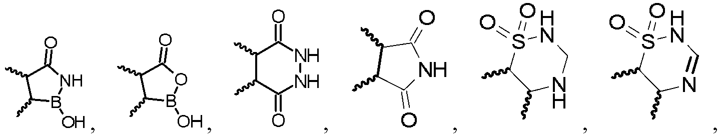

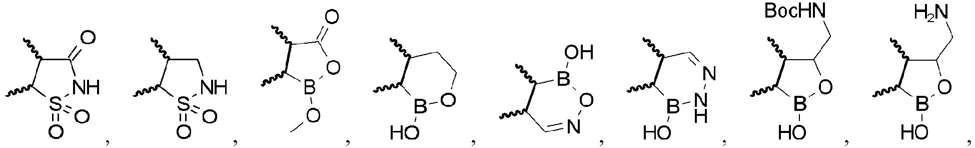

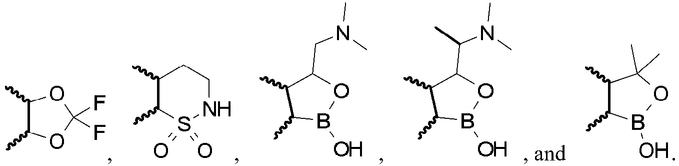

In some embodiments, in the compound of formula II of the present application, R^{a} is selected from 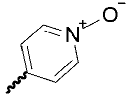 substituted with one or more R^{3a}, wherein each R^{3a} is independently selected from

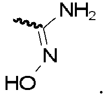

In some embodiments, in the compound of formula II of the present application, R^{a} is selected from wherein each R^{a7} is selected from H.

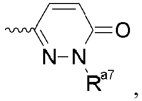

In some embodiments, in the compound of formula II of the present application, R^{a} is selected from 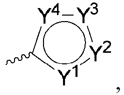 wherein Y¹, Y², Y³, and Y⁴ are each independently selected from O, S, N, N-R^{a1}, and C-R^{a2}; when present, each R^{a1} is independently selected from -S(O)₂-C₃₋₆ cycloalkyl and -S(O)₂-C₁₋₆ haloalkyl; each R^{a2} is independently selected from C₁₋₆ alkyl.

In some embodiments, in the compound of formula II of the present application, R^{a} is selected from 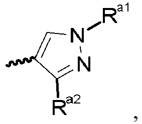 and each R^{a1} is independently selected from -S(O)₂-cyclopropyl; each R^{a2} is independently selected from methyl.

In some embodiments, in the compound of formula II of the present application, R^{a} is selected from:

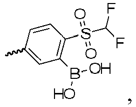

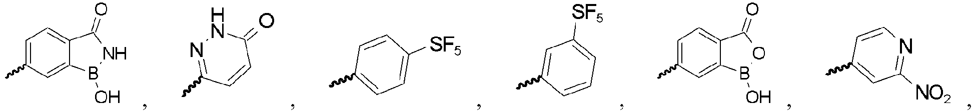

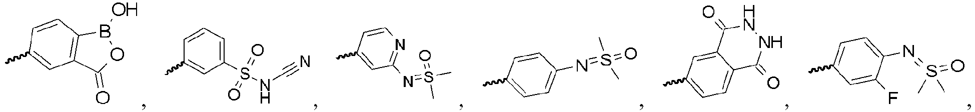

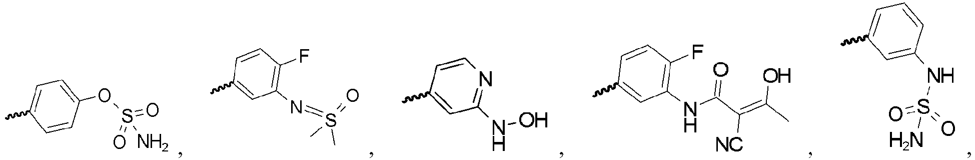

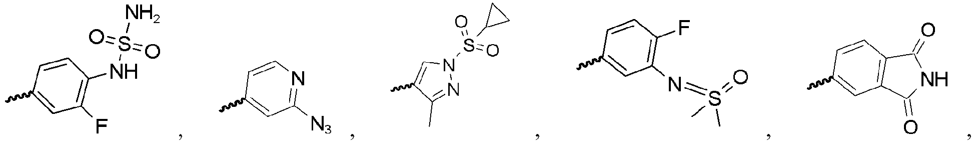

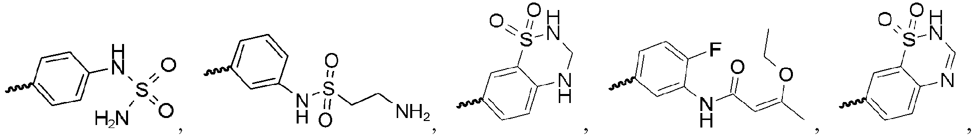

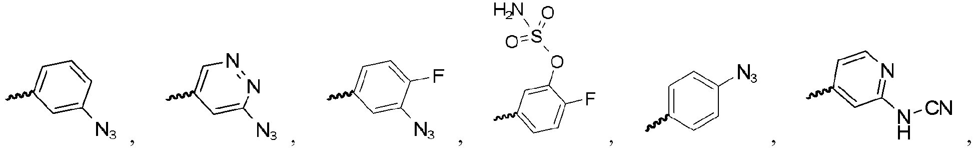

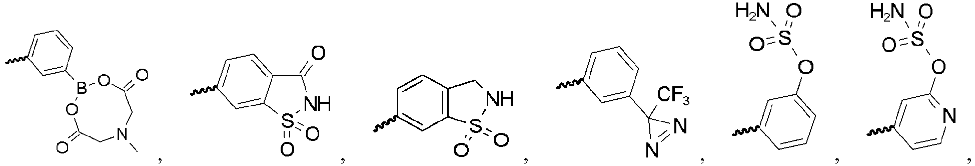

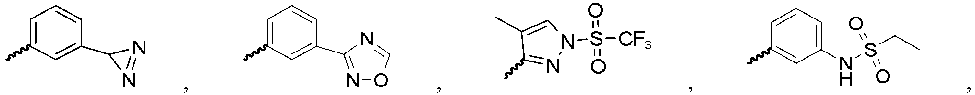

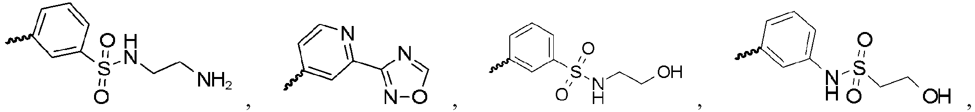

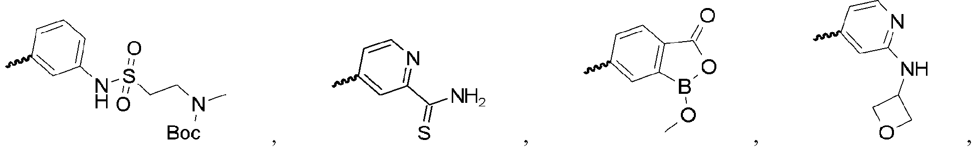

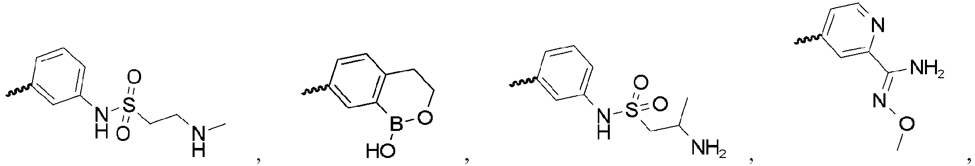

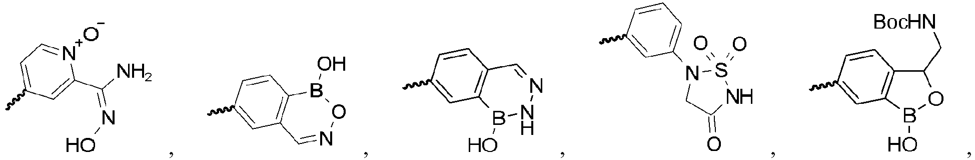

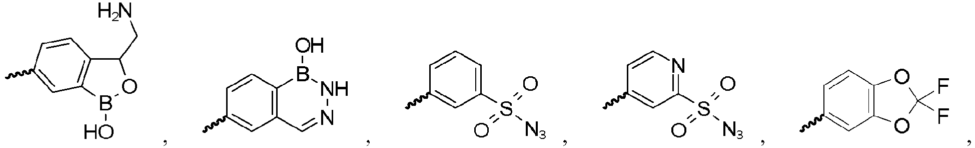

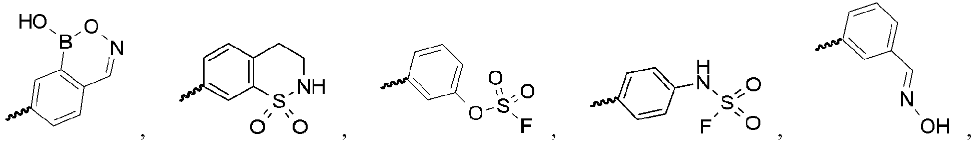

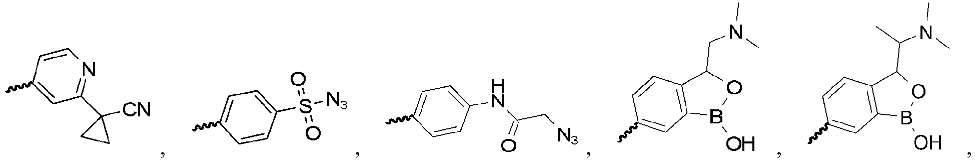

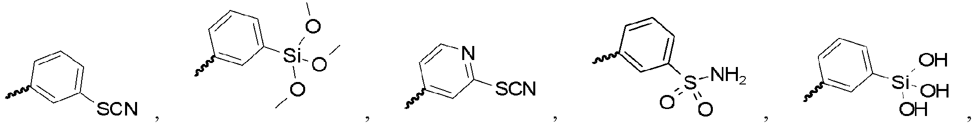

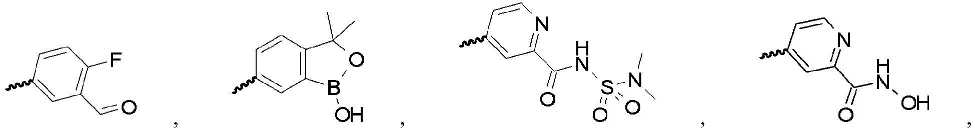

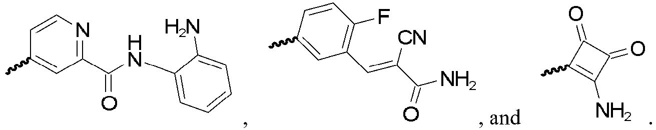

The groups of all embodiments of the present disclosure may be suitably selected in any combination to obtain different general formula ranges or specific schemes. These ranges and schemes all fall within the scope of the present disclosure. The present disclosure encompasses the compounds resulting from any combination of the various embodiments.

In some embodiments, the compound of the present disclosure is selected from:

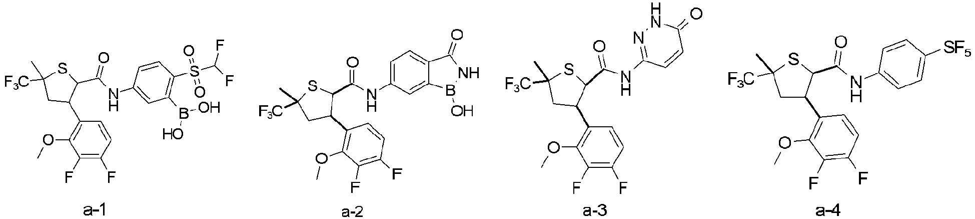

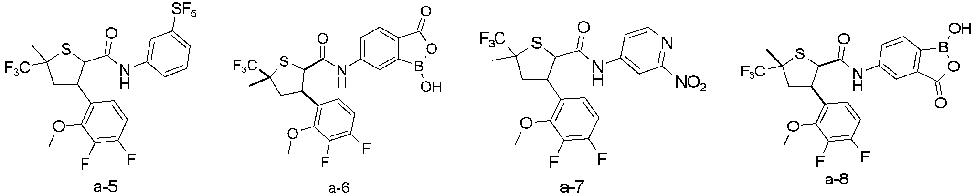

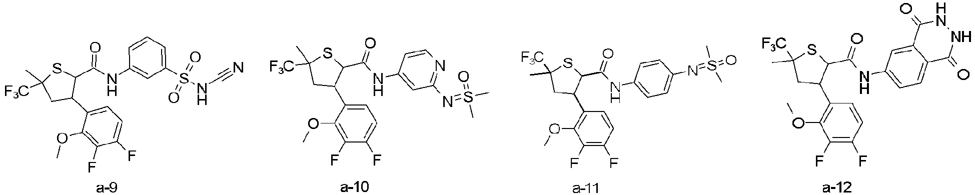

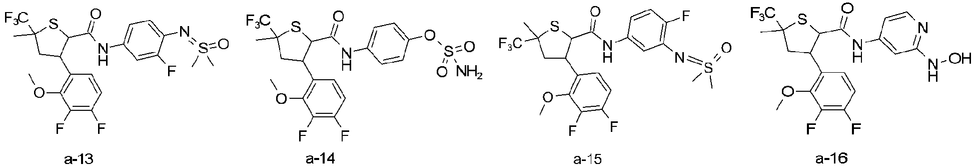

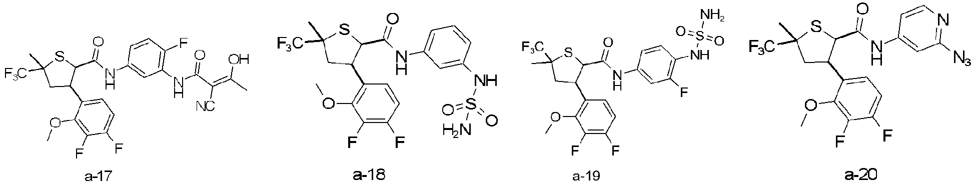

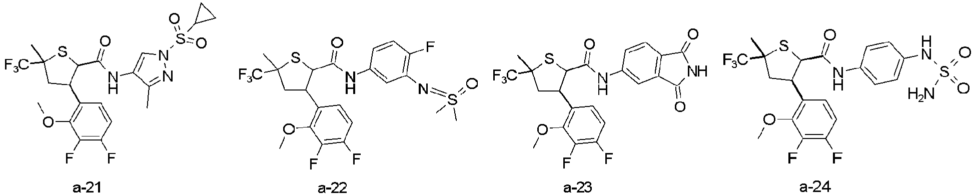

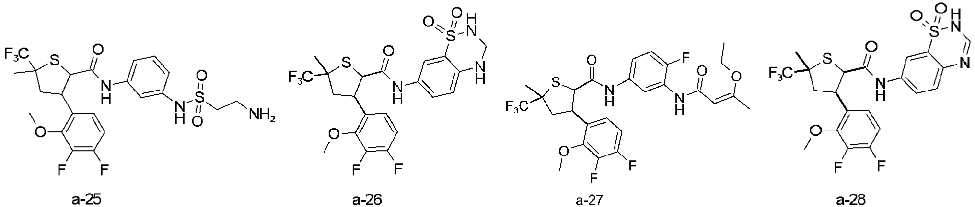

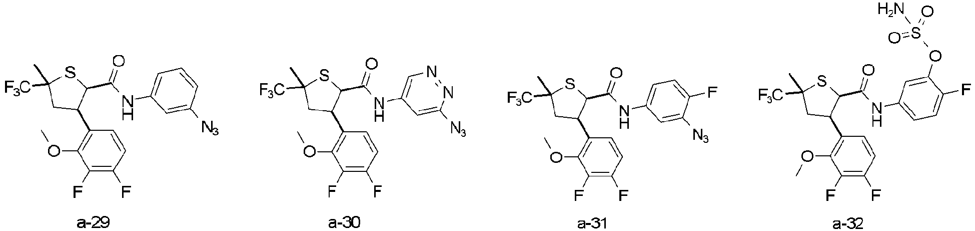

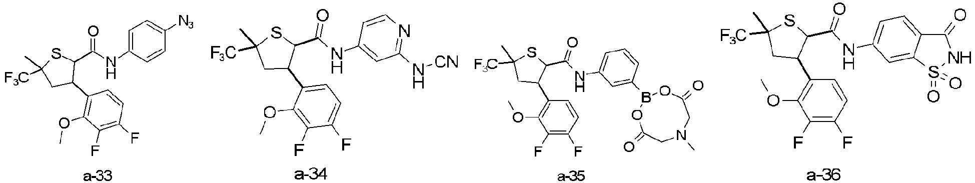

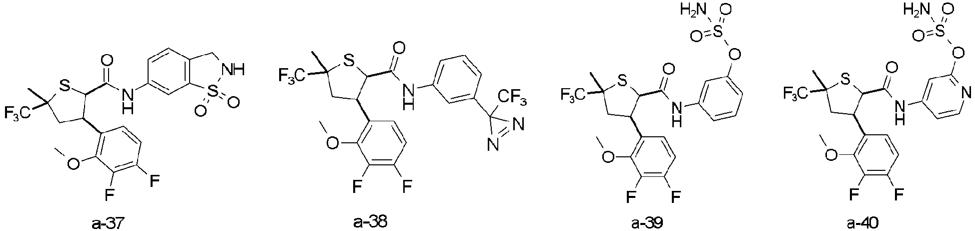

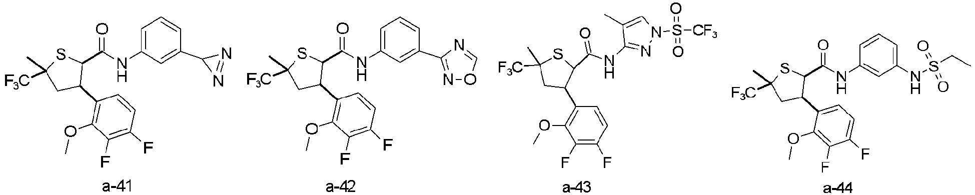

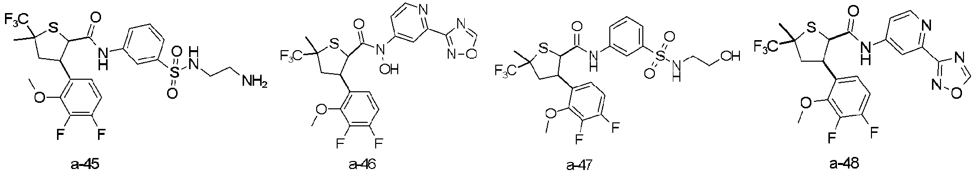

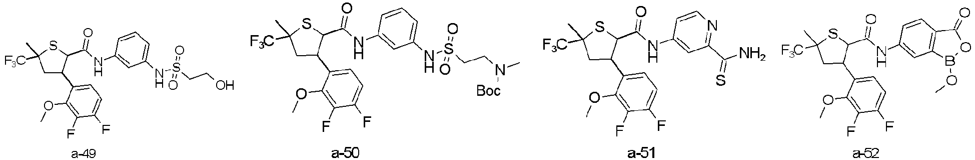

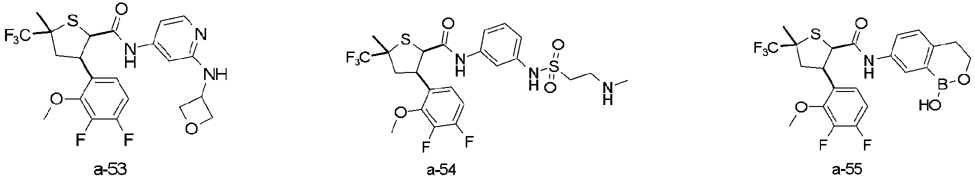

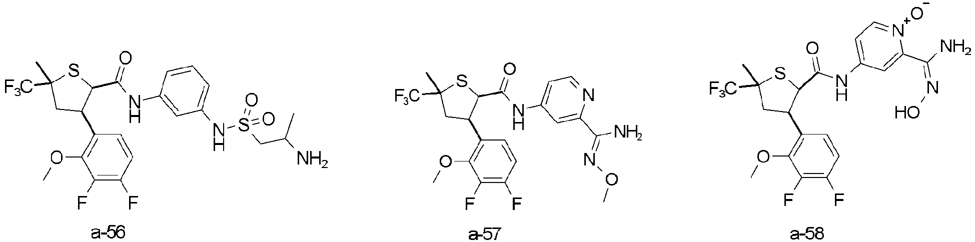

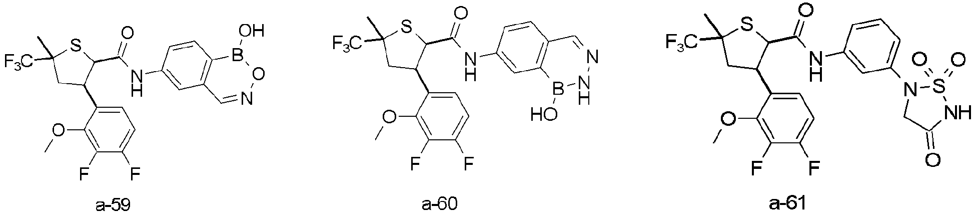

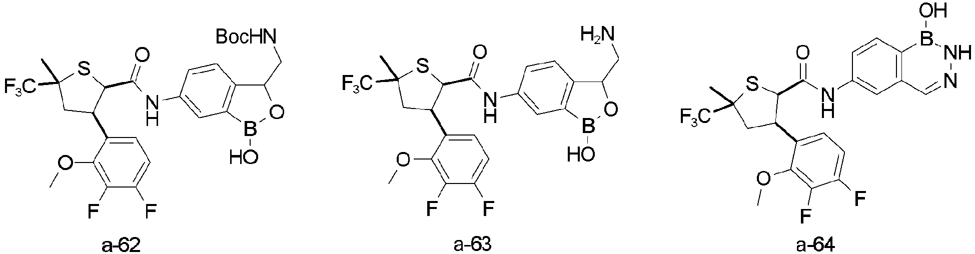

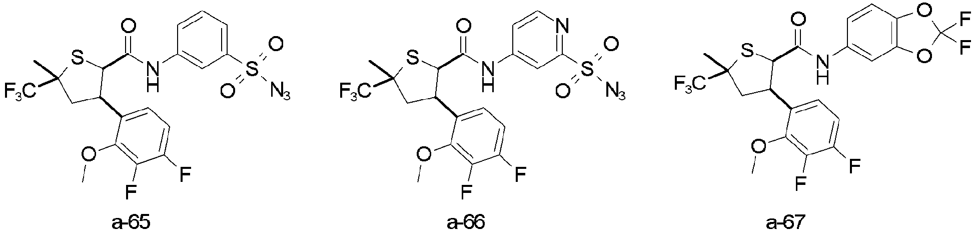

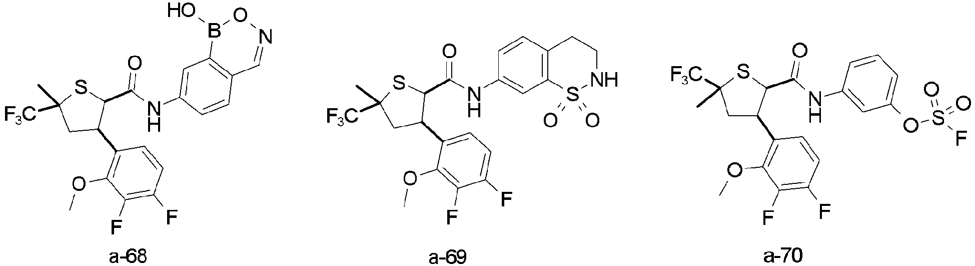

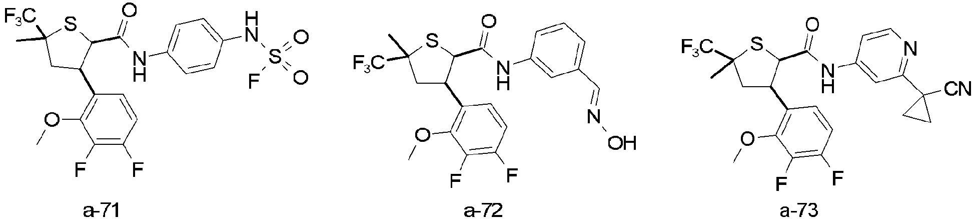

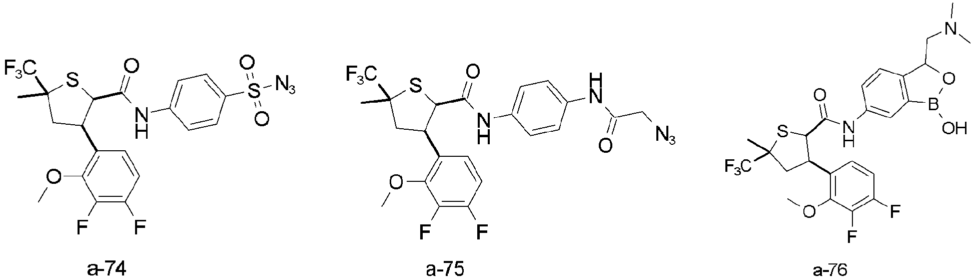

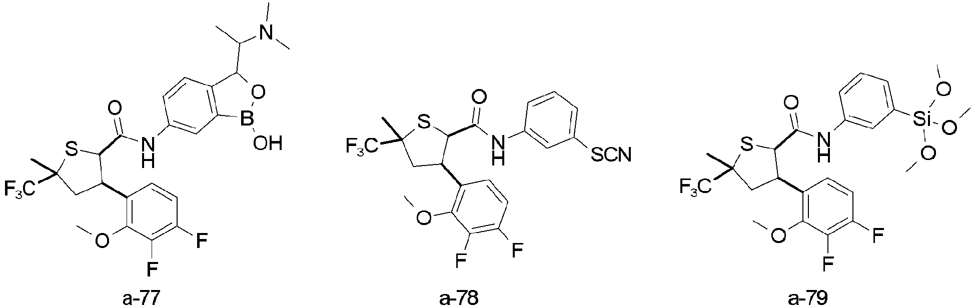

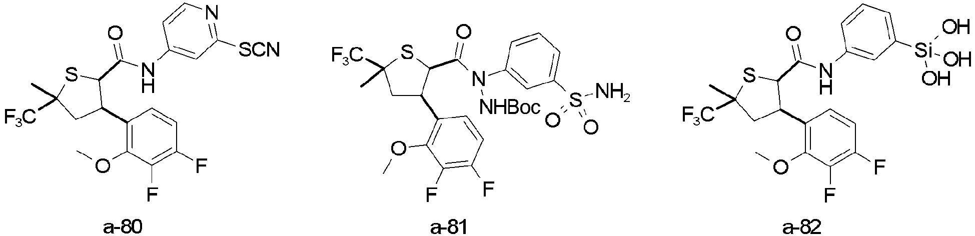

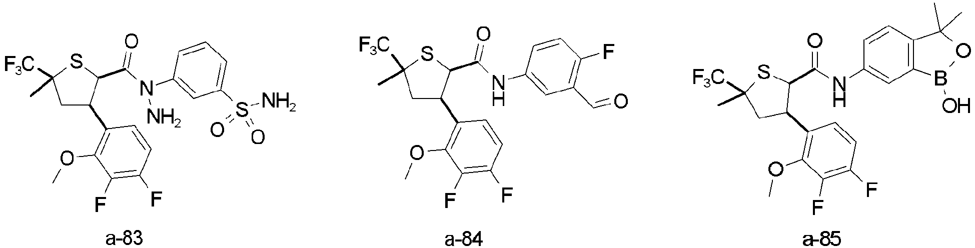

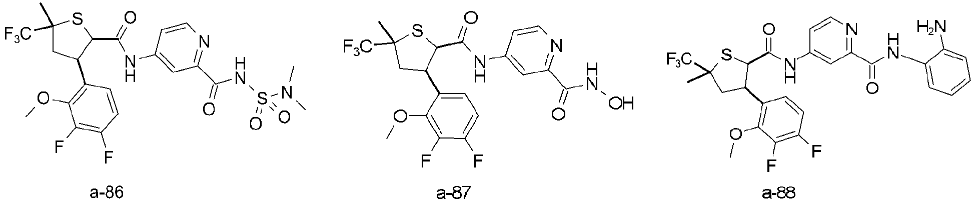

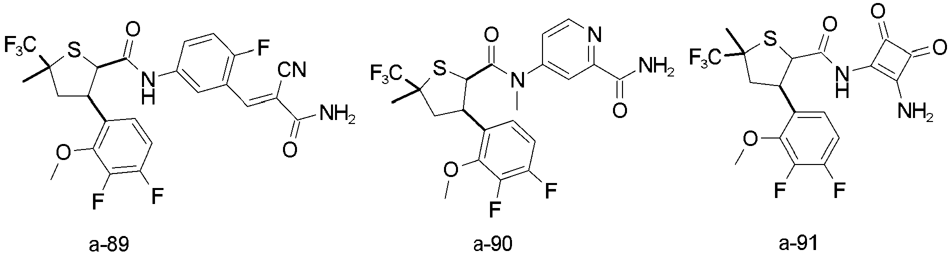

In some embodiments, the compound of the present disclosure is selected from:

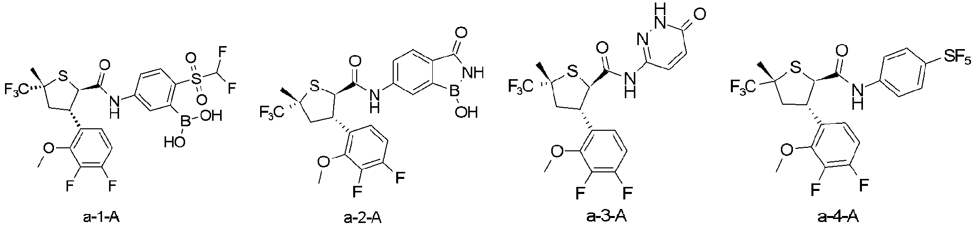

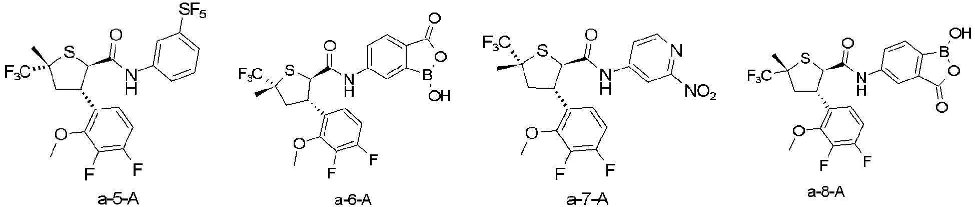

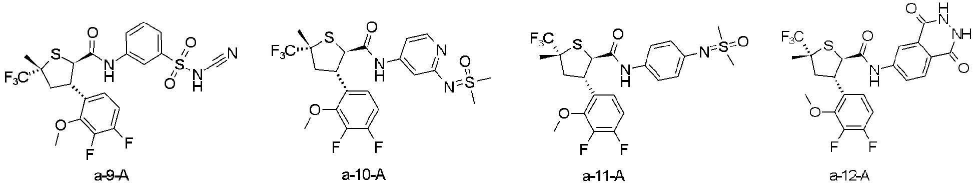

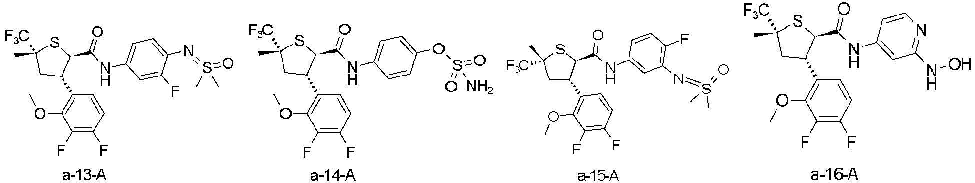

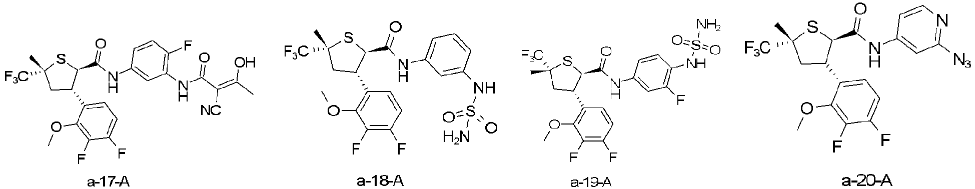

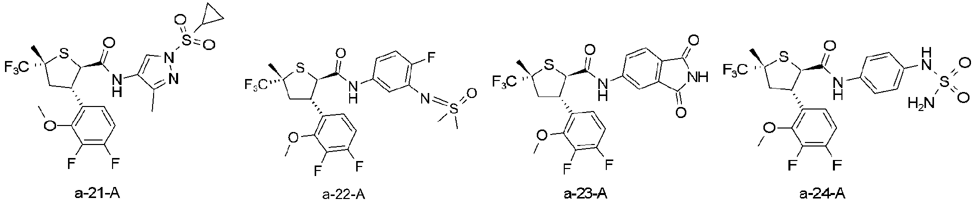

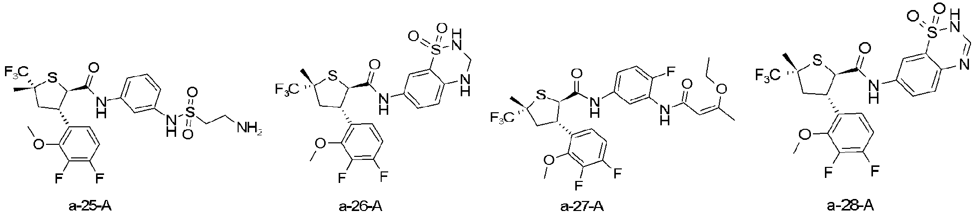

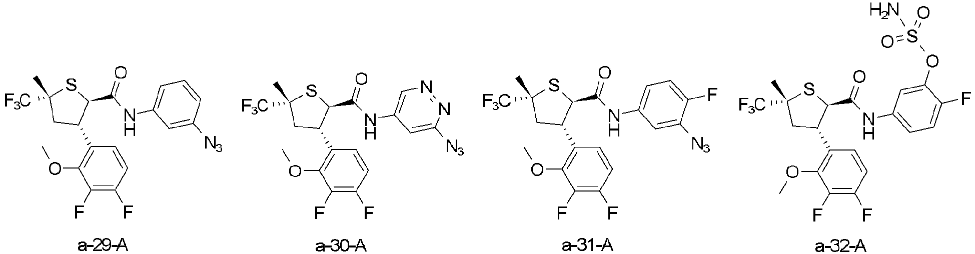

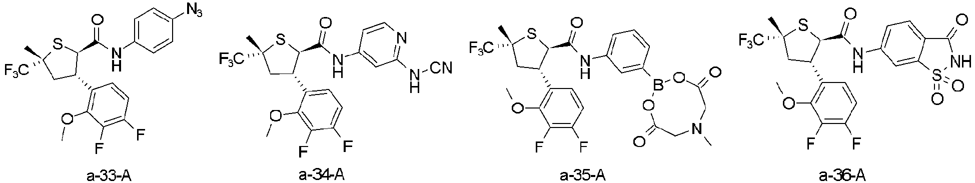

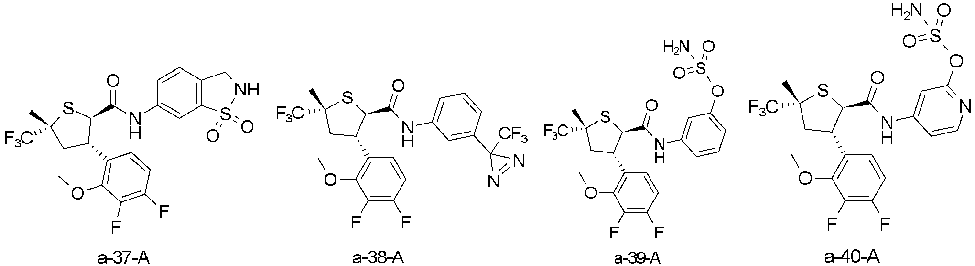

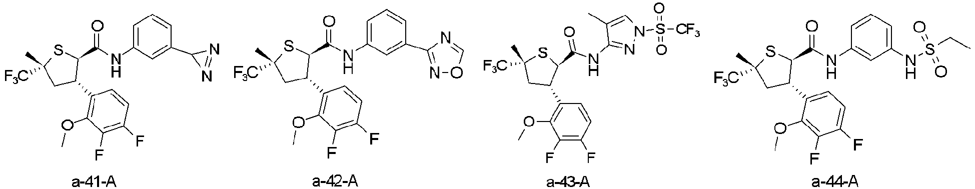

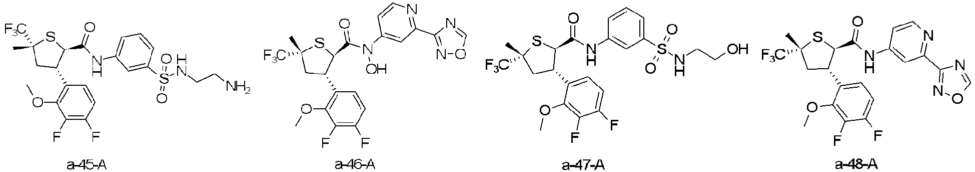

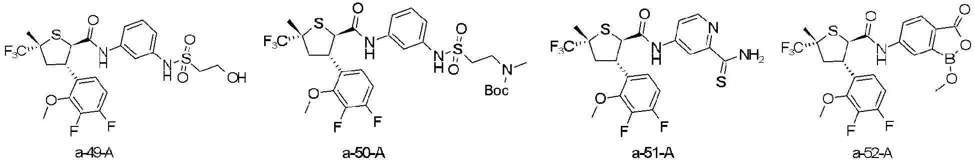

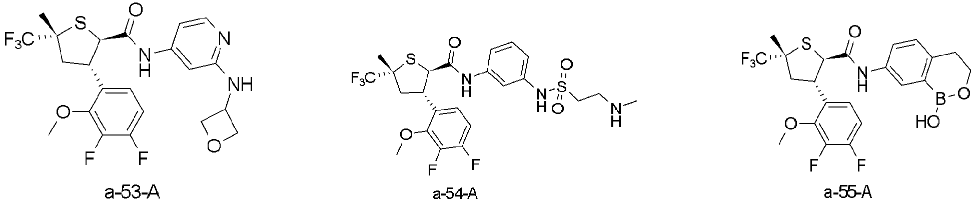

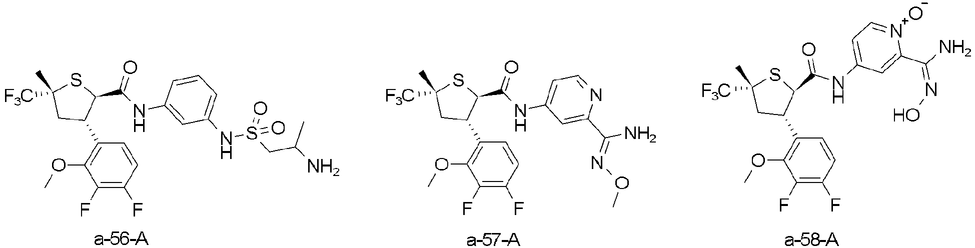

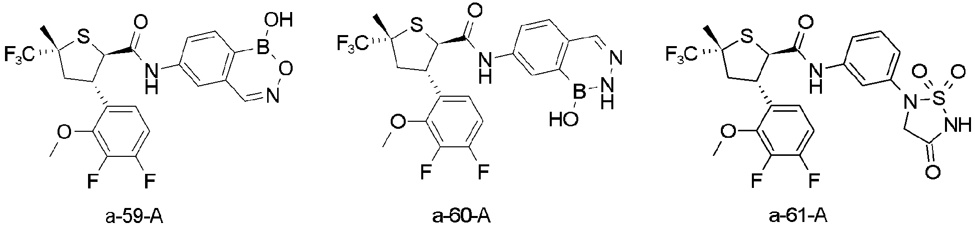

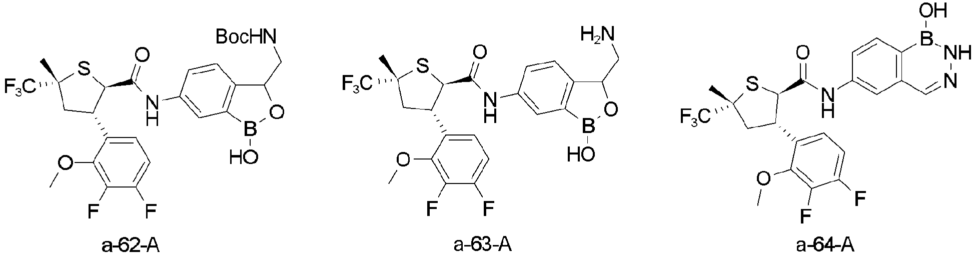

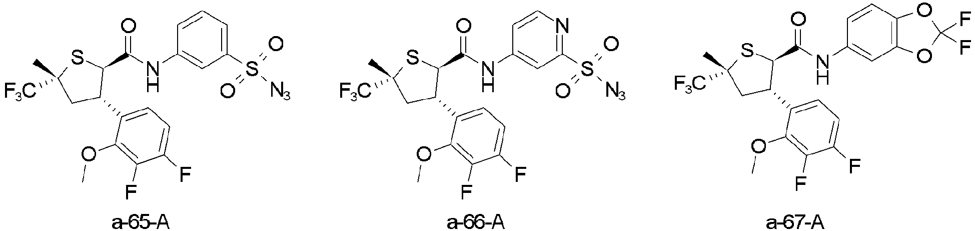

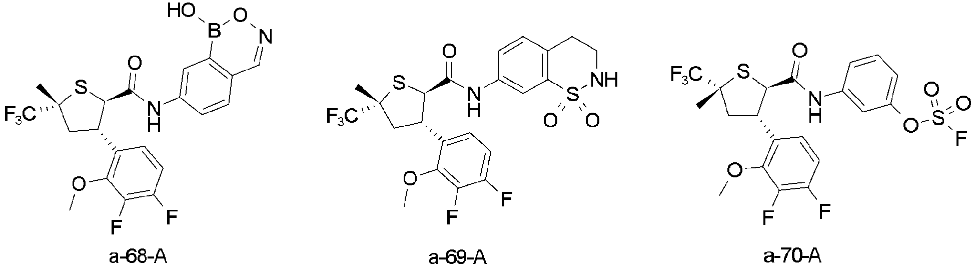

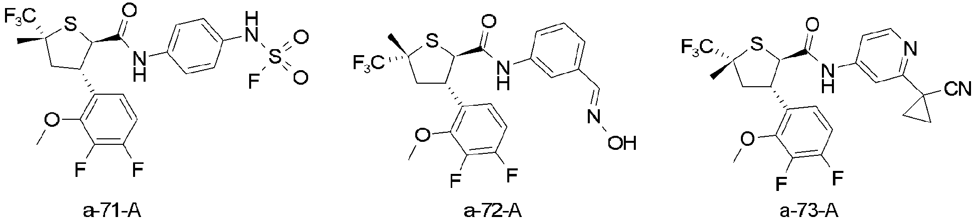

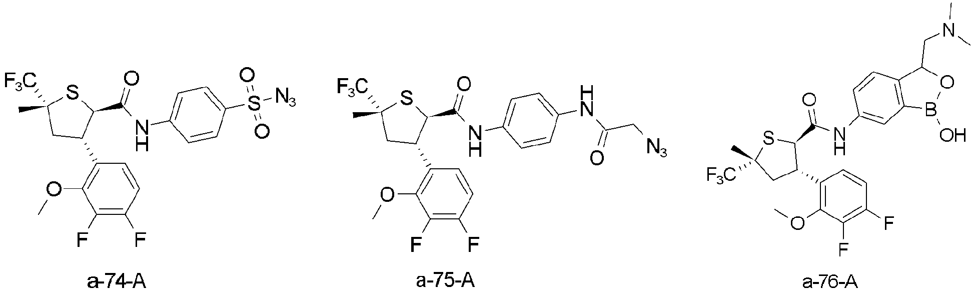

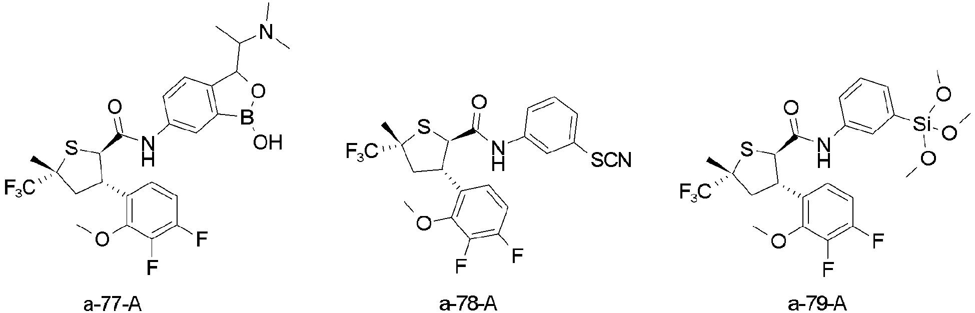

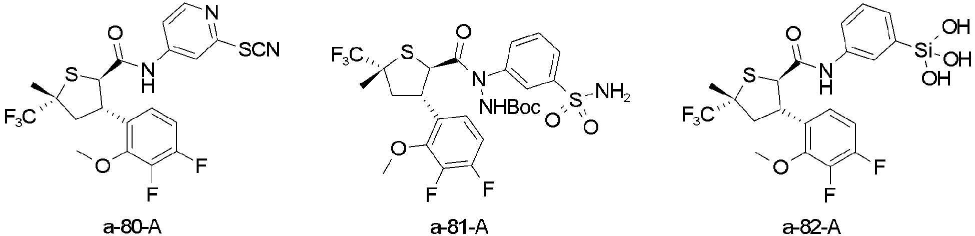

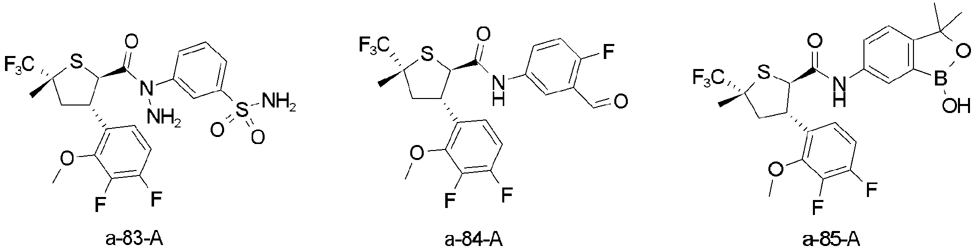

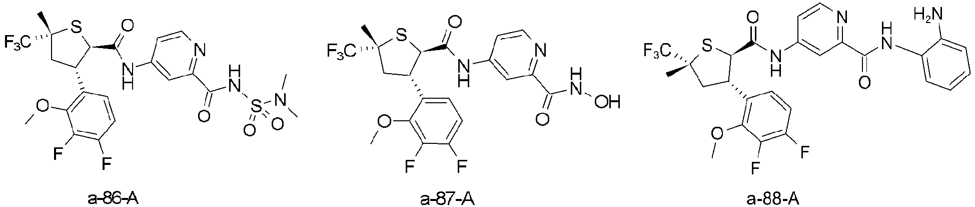

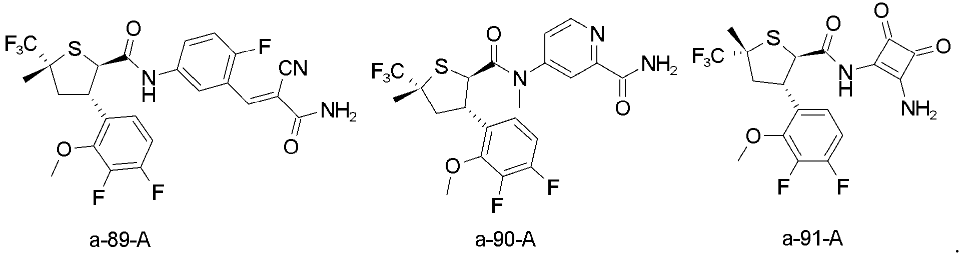

### Pharmaceutical Composition and Kit

Another objective of the present application is to further provide a pharmaceutical composition comprising a prophylactically and/or therapeutically effective amount of the compound represented by formula I or formula II, or the pharmaceutically acceptable salt, the stereoisomer, the solvate, the isotopically labeled compound, or the polymorph thereof of the present application, and one or more pharmaceutically acceptable carriers.

Another objective of the present application is to further provide a kit comprising the compound represented by formula I or formula II, or the pharmaceutically acceptable salt, the stereoisomer, the solvate, the isotopically labeled compound, or the polymorph thereof of the present application, or the pharmaceutical composition of the present application. Optionally, the kit further comprises a package insert.

The term "pharmaceutically acceptable carrier" in the present application refers to a diluent, an adjuvant, an excipient, or a vehicle administered together with a therapeutic agent, which is suitable, within the scope of sound medical judgment, for contact with the tissues of humans and/or other animals without undue toxicity, irritation, allergic response, or other problems or complications relative to a reasonable benefit/risk ratio.

Pharmaceutically acceptable carriers that can be used in the pharmaceutical composition of the present application include, but are not limited to, sterile liquids. The pharmaceutical composition may be, for example, in the form of a solid formulation, a semisolid formulation, a liquid formulation, or a gaseous formulation.

The pharmaceutical composition of the present application may act systemically and/or topically. For this purpose, they can be administered via a suitable route, e.g., by injection or transdermally, or orally or by inhalation.

A content or amount of the compound of the present application in the pharmaceutical composition may be about 0.001 mg to about 5000 mg, suitably 0.01-1000 mg.

In some embodiments, the present application provides a method for preparing the pharmaceutical composition of the present application, which comprises combining the compound, or the pharmaceutically acceptable salt, the stereoisomer, the solvate, the isotopically labeled compound, or the polymorph thereof of the present application with one or more pharmaceutically acceptable carriers.

### Treatment Method and Use

The present application further provides use of the compound represented by formula I or formula II, or the pharmaceutically acceptable salt, the stereoisomer, the solvate, the isotopically labeled compound, or the polymorph thereof of the present application, or the pharmaceutical composition of the present application, or the kit of the present disclosure in the preparation of a medicament for preventing and/or treating a NaV1.8-associated disease, wherein preferably, the NaV1.8-associated disease is pain, multiple sclerosis, Charcot-Marie-Tooth syndrome, incontinence, pathological cough, or arrhythmia.

The present application further provides the compound represented by formula I or formula II, or the pharmaceutically acceptable salt, the stereoisomer, the solvate, the isotopically labeled compound, or the polymorph thereof of the present application, or the pharmaceutical composition of the present application, or the kit of the present disclosure, for use in the prevention and/or treatment of a NaV1.8-associated disease, wherein preferably, the NaV1.8-associated disease is pain, multiple sclerosis, Charcot-Marie-Tooth syndrome, incontinence, pathological cough, or arrhythmia.

The present application further provides a method for preventing and/or treating a NaV1.8-associated disease, which comprises administering to an individual a therapeutically effective amount of the compound represented by formula I or formula II, or the pharmaceutically acceptable salt, the stereoisomer, the solvate, the isotopically labeled compound, or the polymorph thereof of the present application, or the pharmaceutical composition of the present application, or the kit of the present disclosure, wherein preferably, the NaV1.8-associated disease is pain, multiple sclerosis, Charcot-Marie-Tooth syndrome, incontinence, pathological cough, or arrhythmia.

In some embodiments of the present disclosure, the pain is selected from chronic pain, acute pain, inflammatory pain, cancer pain, ICU analgesia, fracture or post-operative pain (e.g., bunionectomy pain, herniorrhaphy pain, and abdominoplasty pain), neuropathic pain (e.g., peripheral neuropathy-related neuralgia, postherpetic neuralgia, peripheral neuralgia, small fiber neuropathy pain, trigeminal neuralgia, idiopathic small fiber neuralgia, or diabetic neuralgia), visceral pain (e.g., intestinal pain), musculoskeletal pain, primary pain, idiopathic pain, osteoarthritis pain, gouty arthritis pain, rheumatic or rheumatoid arthritis pain, odontalgia, arthralgia, labor pain, fibromyalgia, chronic low back pain, bladder pain syndrome, and sciatica.

In some embodiments of the present disclosure, the pain is selected from post-operative pain, neuropathic pain (e.g., postherpetic neuralgia, small fiber neuropathy pain, and diabetic neuralgia), osteoarthritis pain, bladder pain syndrome, and cancer pain.

In some embodiments of the present disclosure, the pain is selected from post-operative pain, e.g., bunionectomy pain, herniorrhaphy pain, and abdominoplasty pain.

In some embodiments of the present disclosure, the pain is selected from neuropathic pain, e.g., postherpetic neuralgia, small fiber neuropathy pain, and diabetic neuralgia.

In some embodiments of the present disclosure, the pain is selected from osteoarthritis pain.

In some embodiments of the present disclosure, the pain is selected from bladder pain syndrome.

In some embodiments of the present disclosure, the pain is selected from cancer pain.

In some embodiments of the present disclosure, the pain is selected from inflammatory pain.

In some embodiments of the present disclosure, the pain is selected from neuropathic pain and inflammatory pain.

In some embodiments of the present disclosure, the compound of the present disclosure or the pharmaceutical composition of the present disclosure is administered at a frequency of once daily, 2 times daily, 3 times daily, 4 times daily, 5 times daily, 6 times daily, 7 times daily, 8 times daily, 9 times daily, 10 times daily, 11 times daily, 12 times daily, 13 times daily, 14 times daily, 15 times daily, 16 times daily, 17 times daily, 18 times daily, 19 times daily, 20 times daily, 21 times daily, 22 times daily, 23 times daily, 24 times daily, once every two days, once every three days, once every four days, once every five days, once every six days, once weekly, 2 times weekly, 3 times weekly, once every 2 weeks, 3 times every 2 weeks, once every 3 weeks, 2 times every 3 weeks, once every 4 weeks, or 3 times every 4 weeks.

In some embodiments, the dose of each administration of the compound of the present disclosure or the pharmaceutical composition of the present disclosure is selected from 0.1-500 mg/kg based on the body weight of a subject to be administered, e.g., 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 15 mg/kg, 20 mg/kg, 25 mg/kg, 30 mg/kg, 35 mg/kg, 40 mg/kg, 45 mg/kg, 50 mg/kg, 55 mg/kg, 60 mg/kg, 65 mg/kg, 70 mg/kg, 75 mg/kg, 80 mg/kg, 85 mg/kg, 90 mg/kg, 95 mg/kg, 100 mg/kg, 105 mg/kg, 110 mg/kg, 115 mg/kg, 120 mg/kg, 125 mg/kg, 130 mg/kg, 135 mg/kg, 140 mg/kg, 145 mg/kg, 150 mg/kg, 160 mg/kg, 165 mg/kg, 170 mg/kg, 180 mg/kg, 190 mg/kg, 200 mg/kg, 210 mg/kg, 22 mg/kg, 230 mg/kg, 240 mg/kg, 250 mg/kg, 260 mg/kg, 270 mg/kg, 280 mg/kg, 290 mg/kg, 300 mg/kg, 310 mg/kg, 320 mg/kg, 330 mg/kg, 340 mg/kg, 350 mg/kg, 360 mg/kg, 370 mg/kg, 380 mg/kg, 390 mg/kg, 400 mg/kg, 410 mg/kg, 420 mg/kg, 430 mg/kg, 440 mg/kg, 450 mg/kg, 460 mg/kg, 470 mg/kg, 480 mg/kg, 490 mg/kg, or 500 mg/kg.

In some embodiments, the dose of the compound of the present disclosure or the pharmaceutical composition of the present disclosure is 0.1-1000 mg, e.g., 0.1 mg, 0.2 mg, 0.3 mg, 0.4 mg, 0.5 mg, 0.6 mg, 0.7 mg, 0.8 mg, 0.9 mg, 1 mg, 2 mg, 3 mg, 4 mg, 5 mg, 6 mg, 7 mg, 8 mg, 9 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 160 mg, 165 mg, 170 mg, 180 mg, 190 mg, 200 mg, 210 mg, 22 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg, 500 mg, 550 mg, 600 mg, 650 mg, 700 mg, 750mg, 800 mg, 850 mg, 900 mg, 950 mg, or 1000 mg.

The term "effective amount" as used herein refers to an amount sufficient to achieve a desired prophylactic or therapeutic effect, e.g., an amount for palliating one or more symptoms associated with the disease to be treated.

The administration regimen may be adjusted to provide the optimum desired response. For example, a single bolus can be administered, several separate doses can be administered over time, or the dose can be proportionally reduced or increased as indicated by the urgent need for treatment. It is noted that dose values may vary with the type and severity of the condition to be palliated, and may include single or multiple doses. It is to be further understood that for any particular individual, the specific administration regimen should be adjusted over time based on the individual's needs and the professional judgment of a person who administers the compound of the present disclosure or supervises the administration of the compound of the present disclosure.

The amount of the compound of the present disclosure administered will depend on the individual being treated, the severity of the disorder or condition, the rate of administration, the disposition of the compound, and the judgment of the prescribing physician. In some cases, dose levels not higher than the lower limit of the aforementioned range may be sufficient, while in other cases, a larger dose may still be employed without causing any adverse side effects, provided that the larger dose is first divided into several smaller doses for administration throughout the day. Unless otherwise stated, the term "treat", "treated", "treating", or "treatment" as used herein means reversing, palliating, or ameliorating the progression of a disorder or condition to which such a term applies or one or more symptoms of such a disorder or condition.

The term "prevent", "prevented", "preventing", or "prevention" refers to inhibiting or delaying the onset of a disease and includes not only prevention prior to the development of the disease but also prevention of a recurrence of the disease after treatment.

The term "individual" as used herein includes humans or non-human animals. Exemplary human individuals include human individuals suffering from a disease (e.g., a disease described herein), which are referred to as patients, or normal individuals. In the present disclosure, "non-human animals" include all vertebrates, such as non-mammals (e.g., birds, amphibians, and reptiles) and mammals, such as non-human primates, livestock, and/or domesticated animals (e.g., sheep, dogs, cats, cows, and pigs). As used herein, " 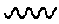 " represents a linking bond.

### DETAILED DESCRIPTION

In order to render the objectives and technical solutions of the present disclosure clearer, the embodiments of the present disclosure will be described in detail below with reference to examples. Those skilled in the art will understand, however, that the following examples are intended only to illustrate the present disclosure, and should not be considered to limit the scope of the present disclosure. Examples without specified conditions were all conducted under conventional conditions or conditions recommended by the manufacturers. Reagents or instruments used without specified manufacturers were all commercially available conventional products.

The structures of the compounds in all examples were determined by nuclear magnetic resonance (¹H-NMR) spectroscopy recorded on a Vian Mercury 400 nuclear magnetic resonance spectrometer, and chemical shifts are expressed in δ (ppm). Unless otherwise specified, the silica gel used for separation was 200-300 mesh silica gel, and the ratios of the eluents are volume ratios.

The following abbreviations are used in the present disclosure: nuclear magnetic resonance (NMR); liquid chromatograph-mass spectrometer (LC-MS); thin-layer chromatography (TLC); preparative liquid chromatography (pre-HPLC); room temperature (RT, rt); equivalent (eq); gram/milligram (g/mg); mole/millimole (mol/mmol); liter/milliliter (L/mL); minute (min(s)); hour (h, hr, hrs); nitrogen (N₂); aqueous solution (aq.); petroleum ether (PE); ethyl acetate (EA); dichloromethane (DCM); methanol (MeOH); methyl tert-butyl ether (MTBE); ethanol (EtOH); trifluoroacetic acid (TFA); N,N,N',N'-tetramethylchloroformamidinium hexafluorophosphate (TCFH); lithium diisopropylamide (LDA); N-methylimidazole (NMI); N,N-dimethylformamide (DMF); and N,N-diisopropylethylamine (DIPEA).

Preparation method for preparative high performance liquid chromatography: instrument model: Agilent 1260; chromatographic column: Waters SunFire Prep C18 OBD (19 mm × 150 mm × 5.0 µm); column temperature: 25 °C; flow rate: 20.0 mL/min; detection wavelength: 214 nm; elution gradient: (0 min: 10% A, 90% B; 16.0 min: 90% A, 10% B); mobile phase A: acetonitrile; mobile phase B: 0.05% formic acid in water.

Aluminum plates (20 × 20 cm) produced by Merck were used for thin-layer chromatography (TLC) silica gel plates. GF 254 plates (1 mm) produced in Yantai were used for thin-layer chromatography separation and purification.

Thin-layer chromatography (TLC) or LC-MS was used for reaction monitoring; the developing solvent systems used included a dichloromethane and methanol system, a n-hexane and ethyl acetate system, and a petroleum ether and ethyl acetate system, and the volume ratio of the solvents was adjusted depending on the polarity of the compound or by adding triethylamine, etc.

A Biotage Initiator+ (400 W, RT to 300 °C) microwave reactor was used for microwave reactions.

200-300 mesh silica gel was generally used as the carrier in column chromatography. Eluent systems included a dichloromethane and methanol system and a petroleum ether and ethyl acetate system, and the volume ratio of the solvents was adjusted depending on the polarity of the compound, or it could be adjusted by adding a small amount of triethylamine.

In the examples, the reaction temperature was room temperature (20 °C-35 °C), unless otherwise specified.

The reagents used in the present disclosure were purchased from Acros Organics, Aldrich Chemical Company, Topbiochem, etc.

### Intermediate 1: Preparation of 3-(3,4-difluoro-2-methoxyphenyl)-5-methyl-5-(trifluoromethyl)tetrahydrothiophene-2-carboxylic acid (1-7)

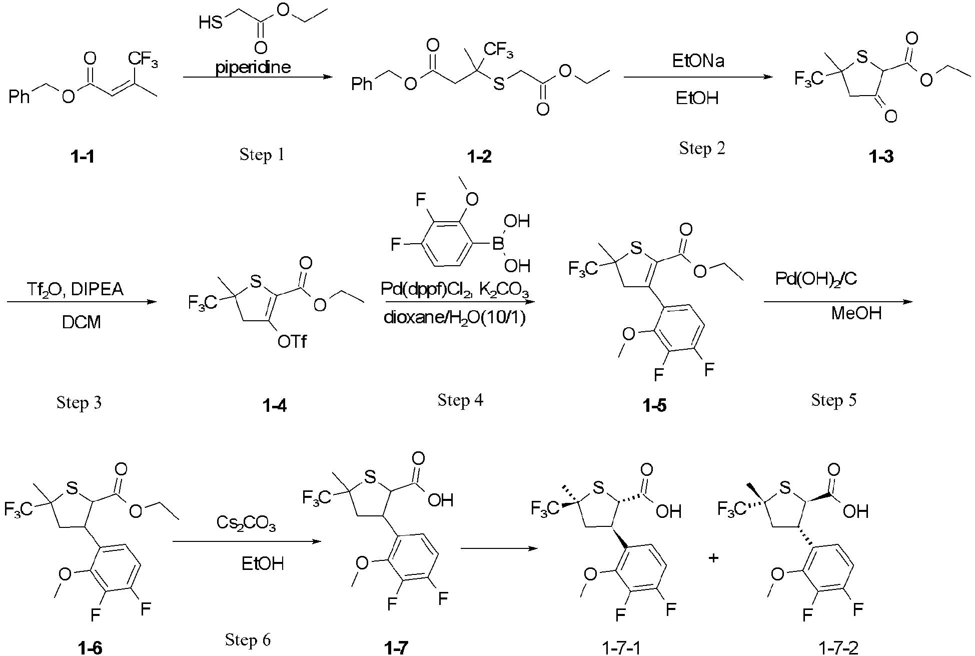

### Step 1: Synthesis of benzyl 3-((2-ethoxy-2-oxoethyl)thio)-4,4,4-trifluoro-3-methylbutanoate (1-2)

Benzyl (Z)-4,4,4-trifluoro-3-methyl-2-butenoate (10 g, 54.9 mmol) and piperidine (0.5 g) were cooled to 0 °C, and ethyl thioglycolate (6.6 g, 54.9 mmol) was added dropwise. The mixture was stirred at this temperature. The reaction was completed as monitored by LC-MS. The reaction solution was directly used in the next step. MS: m/z = 365.2, [M+H]⁺.

### Step 2: Synthesis of ethyl 5-methyl-3-oxo-5-(trifluoromethyl)tetrahydrothiophene-2-carboxylate (1-3)

Sodium ethanolate (11.2 g, 164.73 mmol) was completely dissolved in absolute ethanol (120 mL). The reaction system was then cooled to 0 °C, and the reaction solution obtained in step 1 was slowly added dropwise to the reaction system. The mixture was allowed to naturally warm to room temperature and then stirred. The reaction was completed as monitored by LC-MS. An aqueous citric acid solution (1 M, 100 mL) was added dropwise to the reaction system in an ice bath, and the mixture was extracted with dichloromethane (200 mL). The organic phases were combined, dried over anhydrous sodium sulfate, filtered, concentrated under reduced pressure, and separated by silica gel column chromatography (EA/PE = 20/80) to obtain the target compound (8.7 g, 33.95 mmol, yield: 61.83%). MS: m/z = 257.2, [M+H]⁺.

### Step 3: Synthesis of ethyl 5-methyl-5-(trifluoromethyl)-3-(((trifluoromethyl)sulfonyl)oxy)-4,5-dihydrothiophene-2-carboxylate (1-4)

The product of step 2 (3.1 g, 12.1 mmol), dichloromethane (60 mL), and N,N-diisopropylethylamine (2.35 g, 18.15 mmol) were cooled to -78 °C, and trifluoromethanesulfonic anhydride (4.1 g, 14.52 mmol) was slowly added dropwise to the reaction system. The temperature was maintained, and the reaction was completed. The reaction system was quenched with water (50 mL), and the mixture was extracted with dichloromethane (60 mL). The organic phase was dried over anhydrous sodium sulfate, filtered, concentrated under reduced pressure, and separated by silica gel column chromatography (EA/PE = 10/90) to obtain the target compound (3.5 g, 9.01 mmol, yield: 74.51%). MS: m/z = 389.2, [M+H]⁺.

### Step 4: Synthesis of ethyl 3-(3,4-difluoro-2-methoxyphenyl)-5-methyl-5-(trifluoromethyl)-4,5-dihydrothiophene-2-carboxylate (1-5)

The product of step 3 (3.5 g, 9.01 mmol) was treated with 1,4-dioxane (50 mL) and water (5 mL), and (3,4-difluoro-2-methoxyphenyl)boronic acid (2.53 g, 13.6 mmol), potassium carbonate (3.7 g, 27.2 mmol), and Pd(dppf)Cl₂ (0.63 g, 0.9 mmol) were then sequentially added. The reaction system was purged with nitrogen, warmed to 100 °C, and stirred. The reaction was completed as monitored by LC-MS. The reaction system was cooled to room temperature and concentrated under reduced pressure. Water was added, and the mixture was extracted with dichloromethane. The organic phase was dried over anhydrous sodium sulfate, filtered, concentrated under reduced pressure, and purified by silica gel column chromatography (EA/PE = 9/91) to obtain the target compound (2.8 g, 7.3 mmol, yield: 81.25%). MS: m/z = 383.2, [M+H]⁺.

### Step 5: Synthesis of ethyl 3-(3,4-difluoro-2-methoxyphenyl)-5-methyl-5-(trifluoromethyl)tetrahydrothiophene-2-carboxylate (1-6)

The product of step 4 (2.3 g, 6.02 mmol) was treated with methanol (50 mL) and palladium hydroxide on carbon (3.4 g), and the reaction system was purged with hydrogen, pressurized to 1.5 MPa, and allowed to react at 60 °C for 16 h. The reaction system was cooled to room temperature, and the pressure was released to normal pressure. The reaction solution was filtered through celite and concentrated under reduced pressure. The crude product was separated by Pre-HPLC to obtain the target compound (1.8 g, 4.8 mmol, yield: 77.85%). MS: m/z = 385.2, [M+H]⁺. Step 6: Synthesis of 3-(3,4-difluoro-2-methoxyphenyl)-5-methyl-5-(trifluoromethyl)tetrahydrothiophene-2-carboxylic acid (1-7)

Absolute ethanol (80 mL) and cesium carbonate (1.7 g, 5.2 mmol) were added, and the mixture was warmed to 50 °C. After the reaction system became clear, a solution of the product (1 g, 2.6 mmol) obtained in step 5 in absolute ethanol (20 mL) was added, and then the resulting mixture was stirred for 2 h. The reaction system was cooled to room temperature and concentrated under reduced pressure to remove the solvent. The crude product was dissolved in water (25 mL), and a 1 M aqueous HCl solution was added dropwise to adjust the pH to 3. The mixture was extracted with EA (30 mL × 3). The organic phases were combined, washed with saturated brine, dried over anhydrous sodium sulfate, filtered, and concentrated under reduced pressure. The crude product was separated by Pre-HPLC to obtain the target compound (0.837 g, 2.35 mmol, yield: 90.29%). MS: m/z = 357.1, [M+H]⁺.

Compound 1-7 was resolved by chiral resolution (chromatographic column: AD-H, column temperature: 30 °C, mobile phase (n-hexane-absolute ethanol-isopropanol-diethylamine = 80:16:4:0.05), flow rate: 1 mL/min) to obtain compound 1-7-1 (retention time: 3.149 min) and compound 1-7-2 (retention time: 5.348 min).

### Intermediate 2: Preparation of 3-(3,4-difluoro-2-methoxyphenyl)-5-methyl-5-(trifluoromethyl)tetrahydrothiophene-2-d-2-carboxylic acid (compound 1-8)

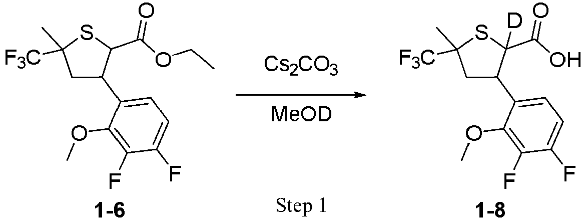

Compound 1-6 (40 mg, 0.10 mmol), cesium carbonate (7 mg, 0.20 mmol), and deuterated methanol (2 mL) were stirred at 80 °C for 3 h. The reaction solution was cooled to room temperature and then concentrated under reduced pressure. The crude product was added to water (2 mL), and a 1 M aqueous hydrochloric acid solution was added dropwise to adjust the pH to 4. The mixture was extracted with ethyl acetate (5 mL × 3). The organic phases were combined, washed with saturated brine, dried over anhydrous sodium sulfate, and concentrated to obtain a crude product 1-8 (35 mg, 0.098 mmol, yield: 99%), which was directly used in the next step. MS: m/z = 358.2, [M+H]⁺. Intermediate 3: Preparation of 3-(3,4-difluoro-2-methoxyphenyl)-5,5-dimethyltetrahydrothiophene-2-carboxylic acid (2-7)

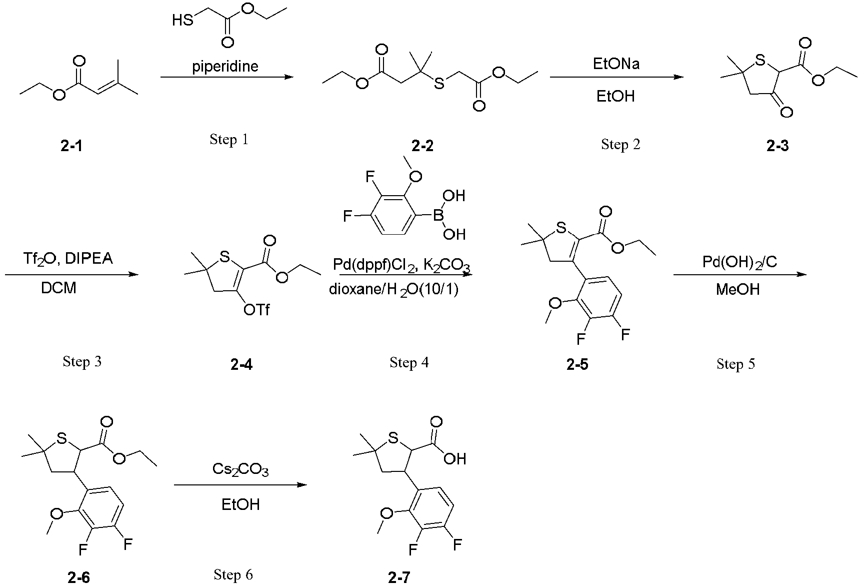

### Step 1: Synthesis of ethyl 3-((2-ethoxy-2-oxoethyl)thio)-3-methylbutanoate (2-2)

Compound 2-1 (15 g, 125 mmol) and piperidine (0.5 g) were cooled to 0 °C, and ethyl thioglycolate (16.3 g, 127.3 mmol) was slowly added dropwise to the reaction system. The mixture was stirred at this temperature. The reaction was completed as monitored by LC-MS. The mixture was concentrated under reduced pressure to obtain the target compound (17.2 g, 69.1 mmol). MS: m/z = 249.2, [M+H]⁺.

### Step 2: Synthesis of ethyl 5,5-dimethyl-3-oxotetrahydrothiophene-2-carboxylate (2-3)

The product of step 1 (17.2 g, 69.1 mmol) and absolute ethanol (200 mL) were cooled to 0 °C, and sodium ethanolate (9.3 g, 138.2 mmol) was slowly added in batches to the reaction system. The reaction system was then warmed to room temperature and stirred. The reaction was completed as monitored by LC-MS. The reaction system was quenched with water (100 mL), and a 1 M aqueous hydrochloric acid solution was added dropwise to adjust the pH to 6. The mixture was extracted with DCM (200 mL × 3). The organic phases were combined, washed with saturated brine, dried over anhydrous sodium sulfate, filtered, and concentrated under reduced pressure to obtain the target compound (13.1 g, 64.8 mmol). MS: m/z = 203.2, [M+H]⁺.

### Step 3: Synthesis of ethyl 5,5-dimethyl-3-(((trifluoromethyl)sulfonyl)oxy)-4,5-dihydrothiophene-2-carboxylate (2-4)

The product of step 2 (13.1 g, 64.8 mmol), dichloromethane (200 mL), and N,N-diisopropylethylamine (25.1 g, 194.4 mmol) were cooled to -78 °C, and trifluoromethanesulfonic anhydride (27.4 g, 97.2 mmol) was slowly added dropwise to the reaction system. The mixture was allowed to naturally warm to room temperature and then stirred for 5 h. The reaction was quenched with water (100 mL), and the mixture was extracted with DCM (200 mL × 3). The organic phases were combined, washed with saturated brine, dried over anhydrous sodium sulfate, filtered, and concentrated under reduced pressure, and the crude product was separated by silica gel column chromatography (EA/PE = 20/80) to obtain the target compound (9.1 g, 27.2 mmol, yield: 41.9%). MS: m/z = 335.2, [M+H]⁺.

### Step 4: Synthesis of ethyl 3-(3,4-difluoro-2-methoxyphenyl)-5,5-dimethyl-4,5-dihydrothiophene-2-carboxylate (2-5)

The product of step 3 (9.1 g, 27.2 mmol) was treated with 1,4-dioxane (100 mL) and water (10 mL), and (3,4-difluoro-2-methoxyphenyl)boronic acid (7.6 g, 40.8 mmol), potassium carbonate (11.2 g, 81.6 mmol), and Pd(dppf)Cl₂ (1.9 g, 0.27 mmol) were then sequentially added. The reaction system was purged with nitrogen three times, warmed to 95 °C, and stirred. The reaction was completed as monitored by LC-MS. The reaction system was cooled to room temperature and concentrated under reduced pressure to remove 1,4-dioxane. Water (100 mL) was added, and the mixture was extracted with DCM (100 mL × 3). The organic phases were combined, washed with saturated brine, dried over anhydrous sodium sulfate, filtered, and concentrated under reduced pressure. The crude product was separated by silica gel column chromatography (EA/PE = 20/80) to obtain the target compound (3.4 g, 10.3 mmol, yield: 38.1%). MS: m/z = 329.2, [M+H]⁺.

### Step 5: Synthesis of ethyl 3-(3,4-difluoro-2-methoxyphenyl)-5,5-dimethyltetrahydrothiophene-2-carboxylate (2-6)

The product of step 4 (3.4 g, 10.3 mmol) was treated with methanol (100 mL) and palladium hydroxide on carbon (3.4 g). The reaction system was purged with hydrogen, pressurized to 1.5 MPa, warmed to 60 °C, and allowed to react for 16 h. The reaction system was cooled to room temperature, and the pressure was released to normal pressure. The reaction solution was filtered through celite and concentrated under reduced pressure. The crude product was separated by silica gel column chromatography (EA/PE = 20/80) to obtain the target compound (2.7 g, 8.1 mmol, yield: 79.1%). MS: m/z = 331.2, [M+H]⁺.

### Step 6: Synthesis of 3-(3,4-difluoro-2-methoxyphenyl)-5,5-dimethyltetrahydrothiophene-2-carboxylic acid (2-7)

The product of step 5 (2.7 g, 8.1 mmol), absolute ethanol (100 mL), and cesium carbonate (5.2 g, 16.2 mmol) were warmed to 50 °C, and the mixture was then stirred for 5 h. The reaction solution was cooled to room temperature and concentrated under reduced pressure. The crude product was dissolved in water (60 mL), and a 1 M aqueous HCl solution was added dropwise to adjust the pH to 3. The mixture was extracted with EA (60 mL × 3). The organic phases were combined, washed with saturated brine, dried over anhydrous sodium sulfate, filtered, and concentrated under reduced pressure. The crude product was separated by Pre-HPLC to obtain the target compound (2.1 g, 7.1 mmol, yield: 88.1%). MS: m/z = 303.1, [M+H]⁺.

### Example 1: Preparation of (3-(3-(3,4-difluoro-2-methoxyphenyl)-5-methyl-5-(trifluoromethyl)tetrahydrothiophene-2-carboxamido)phenyl)boronic acid (compound 9)

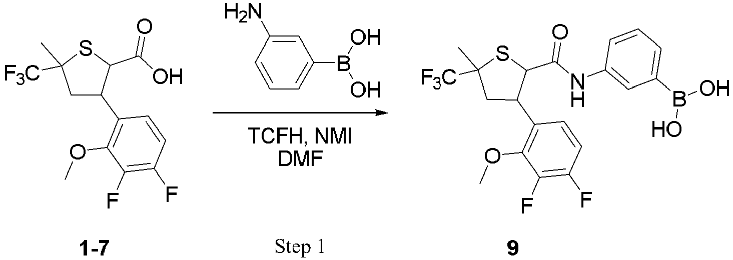

Compound 1-7 (30 mg, 0.08 mmol) was dissolved in anhydrous DMF (5 mL), (3-aminophenyl)boronic acid (33 mg, 0.24 mmol) was added, and TCFH (120 mg, 0.40 mmol) and NMI (56 mg, 0.64 mmol) were added in an ice bath. The mixture was allowed to naturally warm to room temperature and then stirred for 1 h. After the reaction was completed, the mixture was separated by Pre-HPLC to obtain the target compound (79 mg, 0.04 mmol, yield: 50%). MS: m/z = 476.2, [M+H]⁺, ¹H NMR (400 MHz, DMSO) δ 10.17 (s, 1H), 8.00 (s, 2H), 7.76 (s, 1H), 7.59 (d,*J*= 8.1 Hz, 1H), 7.46 (d,*J*= 7.3 Hz, 1H), 7.23 (t,*J*= 7.7 Hz, 1H), 7.20 - 7.11 (m, 2H), 4.62 (d,*J*= 10.7 Hz, 1H), 4.31-4.19 (m, 1H), 3.96 (d,*J* = 1.6 Hz, 3H), 2.39 (t, *J=* 12.9 Hz, 1H), 2.35 - 2.25 (m, 1H), 1.73 (s, 3H).

### Example 2: Preparation of (5-(3-(3,4-difluoro-2-methoxyphenyl)-5-methyl-5-(trifluoromethyl)tetrahydrothiophene-2-carboxamido)-2-methylphenyl)boronic acid (compound 13)

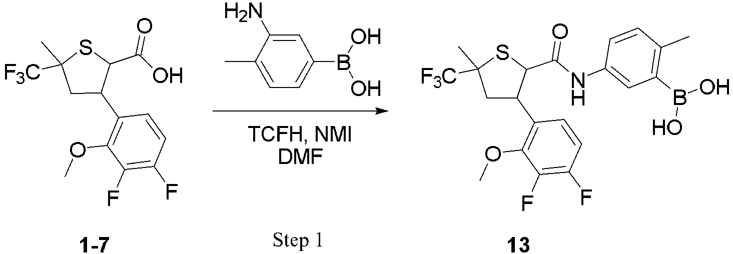

Compound 1-7 (30 mg, 0.08 mmol) was dissolved in anhydrous DMF (5 mL), (3-amino-4-methylphenyl)boronic acid (36 mg, 0.24 mmol) was added, and TCFH (118 mg, 0.41 mmol) and NMI (55 mg, 0.64 mmol) were added in an ice bath. The mixture was allowed to naturally warm to room temperature and then stirred for 1 h. After the reaction was completed, the mixture was separated by Pre-HPLC to obtain the target compound (24.5 mg, 0.05 mmol, yield: 62.50%). MS: m/z = 490.2, [M+H]⁺, ¹H NMR (400 MHz, DMSO) δ 10.07 (s, 1H), 7.96 (s, 2H), 7.45 - 7.40 (m, 2H), 7.19 - 7.12 (m, 2H), 7.00 (d, *J=* 8.0 Hz, 1H), 4.59 (d, *J=* 10.8 Hz, 1H), 4.34-4.22 (m, 1H), 3.96 (d,*J*= 1.6 Hz, 3H), 2.42-2.27 (m, 1H), 1.72 (s, 3H).

### Example 3: Preparation of (3-(3-(3,4-difluoro-2-methoxyphenyl)-5-methyl-5-(trifluoromethyl)tetrahydrothiophene-2-carboxamido)-5-fluorophenyl)boronic acid (compound 17)

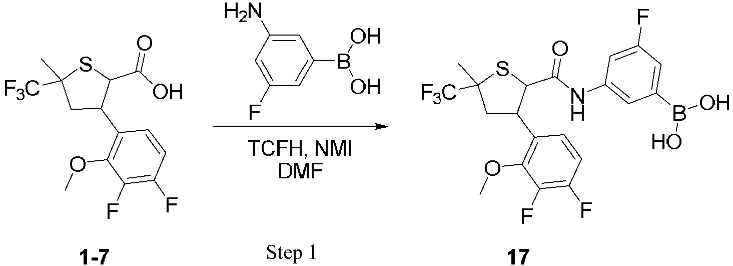

Compound 1-7 (30 mg, 0.08 mmol) was dissolved in anhydrous DMF (5 mL), (3-amino-5-fluorophenyl)boronic acid (70 mg, 0.24 mmol) was added, and TCFH (118 mg, 0.41 mmol) and NMI (55 mg, 0.64 mmol) were added in an ice bath. The mixture was allowed to naturally warm to room temperature and then stirred for 1 h. After the reaction was completed, the mixture was separated by Pre-HPLC to obtain the target compound (20.0 mg, 0.04 mmol, yield: 60.40%). MS: m/z = 494.2, [M+H]⁺, ¹H NMR (400 MHz, DMSO) δ 10.41 (s, 1H), 8.19 (s, 2H), 7.61 - 7.50 (m, 2H), 7.24 - 7.13 (m, 3H), 4.60 (d,*J*= 10.8 Hz, 1H), 4.30-4.23 (m, 1H), 3.96 (d,*J*= 1.6 Hz, 3H), 2.45-2.41 (m, 1H), 2.32-2.28 (m, 1H), 1.73 (s, 3H).

### Example 4: Preparation of (3-(3-(3,4-difluoro-2-methoxyphenyl)-5-methyl-5-(trifluoromethyl)tetrahydrothiophene-2-carboxamido)-4-fluorophenyl)boronic acid (compound 18)

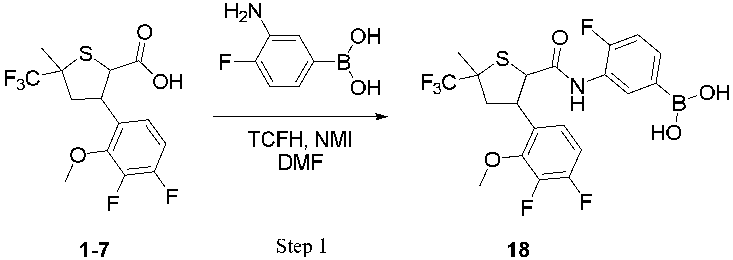

Compound 1-7 (30 mg, 0.08 mmol) was dissolved in anhydrous DMF (5 mL), (3-amino-4-fluorophenyl)boronic acid (70 mg, 0.24 mmol) was added, and TCFH (118 mg, 0.41 mmol) and NMI (55 mg, 0.64 mmol) were added in an ice bath. The mixture was allowed to naturally warm to room temperature and then stirred for 1 h. After the reaction was completed, the mixture was separated by Pre-HPLC to obtain the target compound (16 mg, 0.03 mmol, yield: 40.50%). MS: m/z = 494.2, [M+H]⁺, ¹H NMR (400 MHz, DMSO) δ 9.92 (s, 1H), 8.03 (s, 2H), 7.57-7.56 (m, 2H), 7.30-7.29 (m, 1H), 6.88-6.86 (m, 2H), 4.53(s, 1H), 4.23 (s, 1H), 3.83 (s, 3H), 2.17 - 1.96 (m, 2H), 1.38 (s, 3H).

### Example 5: Preparation of (5-(3-(3,4-difluoro-2-methoxyphenyl)-5-methyl-5-(trifluoromethyl)tetrahydrothiophene-2-carboxamido)-2-fluorophenyl)boronic acid (compound 19)

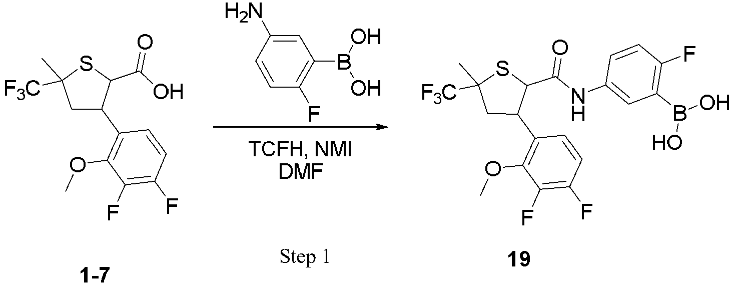

Compound 1-7 (301 mg, 0.8 mmol) was dissolved in anhydrous DMF (5 mL), (5-amino-2-fluorophenyl)boronic acid (703 mg, 2.4 mmol) was added, and TCFH (1.19 g, 4.1 mmol) and NMI (556 mg, 6.4 mmol) were added in an ice bath. The mixture was allowed to naturally warm to room temperature and then stirred for 1 h. After the reaction was completed, the mixture was separated by Pre-HPLC to obtain the target compound (209 mg, 0.43 mmol, yield: 53.7%). MS: m/z = 494.2, [M+H]⁺, ¹H NMR (400 MHz, DMSO) δ 10.22 (s, 1H), 8.17 (s, 2H), 7.59 - 7.51 (m, 2H), 7.22 - 7.14 (m, 2H), 7.09-6.89 (m, 1H), 4.58 (d, *J =* 10.7 Hz, 1H), 4.33 - 4.24 (m, 1H), 3.96 (d, *J =* 1.5 Hz, 3H), 2.45 - 2.35 (m, 1H), 2.32 - 2.25 (m, 1H), 1.73 (s, 3H).

### Example 6: Preparation of (3-cyano-5-(3-(3,4-difluoro-2-methoxyphenyl)-5-methyl-5-(trifluoromethyl)tetrahydrothiophene-2-carboxamido)phenyl)boronic acid (compound 26)

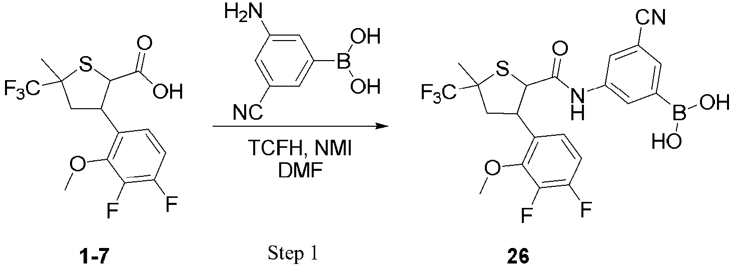

Compound 1-7 (30 mg, 0.08 mmol) was dissolved in anhydrous DMF (5 mL), (3-amino-5-cyanophenyl)boronic acid (62 mg, 0.24 mmol) was added, and TCFH (120 mg, 0.40 mmol) and NMI (56 mg, 0.64 mmol) were added in an ice bath. The mixture was allowed to naturally warm to room temperature and then stirred for 1 h. After the reaction was completed, the mixture was separated by Pre-HPLC to obtain the target compound (18.41 mg, 0.03 mmol, yield: 46%). MS: m/z = 501.2, [M+H]⁺, ¹H NMR (400 MHz, DMSO) δ 10.59 (s, 1H), 8.41 (s, 2H), 8.10 (s, 1H), 8.02 (s, 1H), 7.86 (s, 1H), 7.26 - 7.14 (m, 2H), 4.64 (d,*J*= 10.8 Hz, 1H), 4.36 - 4.25 (m, 1H), 4.00 (d,*J*= 1.6 Hz, 3H), 2.54-2.32 (m, 2H), 1.76 (s, 3H).

### Example 7: Preparation of 3-(3,4-difluoro-2-methoxyphenyl)-N-(1-hydroxy-1,3-dihydrobenzo[c][1,2]oxaborol-6-yl)-5-methyl-5-(trifluoromethyl)tetrahydrothiophene-2-carboxamide (compound 31)

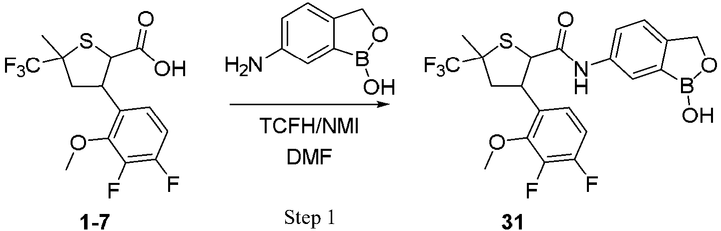

Compound 1-7 (30.0 mg, 0.084 mmol) was dissolved in DMF (1.5 mL), 6-aminobenzo[c][1,2]oxaborol-1(3H)-ol (19.0 mg, 0.126 mmol) and NMI (55.3 mg, 0.673 mmol) were added, and TCHF (94.0 mg, 0.337 mmol) was added in an ice bath. The mixture was allowed to naturally warm to room temperature and then react for 4 h. The reaction was monitored by LC-MS. After the reaction was completed, the mixture was separated by Pre-HPLC to obtain the target compound (23.5 mg, 0.048 mmol, yield: 57.4%). MS: m/z = 488.1, [M+H]⁺, ¹H NMR (400 MHz, DMSO) δ 10.28 (s, 1H), 9.20 (s, 1H), 7.92 (d,*J*= 1.7 Hz, 1H), 7.50 (dd,*J*= 8.3, 2.0 Hz, 1H), 7.30 (d, *J*= 8.3 Hz, 1H), 7.23 - 7.11 (m, 2H), 4.90 (s, 2H), 4.62 (d,*J*= 10.7 Hz, 1H), 4.34 - 4.23 (m, 1H), 3.97 (d,*J*= 1.7 Hz, 3H), 2.47-2.15 (m, 2H), 1.73 (s, 3H).

### Example 8: Preparation of (4-(3-(3,4-difluoro-2-methoxyphenyl)-5-methyl-5-(trifluoromethyl)tetrahydrothiophene-2-carboxamido)thiophen-2-yl)boronic acid (compound 48)

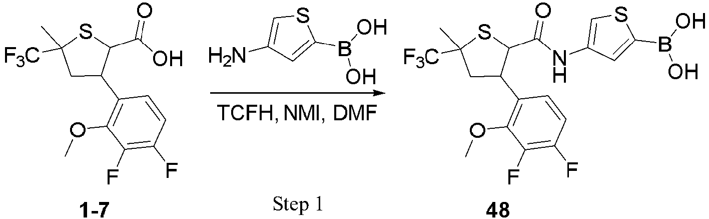

Compound intermediate 1-7 (30 mg, 0.08 mmol) was dissolved in anhydrous DMF (5 mL), (4-aminothiophen-2-yl)boronic acid (36 mg, 0.24 mmol) was added, and TCFH (118 mg, 0.41 mmol) and NMI (55 mg, 0.64 mmol) were added in an ice bath. The mixture was allowed to naturally warm to room temperature and then stirred for 1 h. After the reaction was completed, the mixture was separated by Pre-HPLC to obtain the target compound (18 mg, 0.04 mmol, yield: 46.25%). MS: m/z = 482.2, [M+H]⁺, ¹H NMR (400 MHz, DMSO) δ 10.64 (s, 1H), 8.27 (s, 2H), 7.67 (d, J = 0.6 Hz, 1H), 7.53 (d, J = 0.8 Hz, 1H), 7.20-7.16 (m, 1H), 4.62 (d, J = 10.8 Hz, 1H), 4.34 - 4.26 (m, 1H), 3.99 (d, J = 1.6 Hz, 3H), 2.43-2.33 (m, 1H), 1.75 (s, 3H).

### Example 9: Preparation of (5-(3-(3,4-difluoro-2-methoxyphenyl)-5-methyl-5-(trifluoromethyl)tetrahydrothiophene-2-carboxamido)thiophen-3-yl)boronic acid (compound 49)

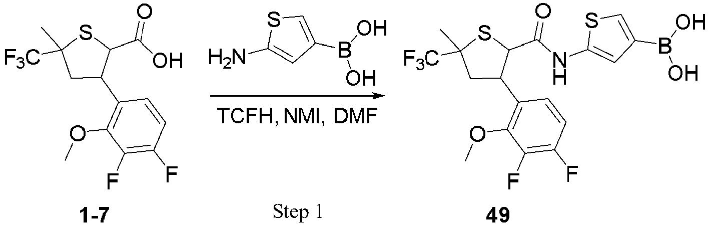

Compound 1-7 (30 mg, 0.08 mmol) was dissolved in anhydrous DMF (5 mL), (5-aminothiophen-3-yl)boronic acid (36 mg, 0.24 mmol) was added, and TCFH (118 mg, 0.41 mmol) and NMI (55 mg, 0.64 mmol) were added in an ice bath. The mixture was allowed to naturally warm to room temperature and then stirred for 1 h. After the reaction was completed, the mixture was separated by Pre-HPLC to obtain the target compound (16 mg, 0.03 mmol, yield: 41.25%). MS: m/z = 482.2, [M+H]⁺, ¹H NMR (400 MHz, DMSO) δ 10.64 (s, 1H), 8.27 (s, 2H), 7.67 (d, J = 0.6 Hz, 1H), 7.53 (d, J = 0.8 Hz, 1H), 7.20-7.16 (m, 1H), 4.62 (d, J = 10.8 Hz, 1H), 4.34 - 4.26 (m, 1H), 3.99 (d, J = 1.6 Hz, 3H), 2.43-2.33 (m, 1H), 1.75 (s, 3H).

### Example 10: Preparation of (6-(3-(3,4-difluoro-2-methoxyphenyl)-5-methyl-5-(trifluoromethyl)tetrahydrothiophene-2-carboxamido)-1H-indol-4-yl)boronic acid (compound 53)

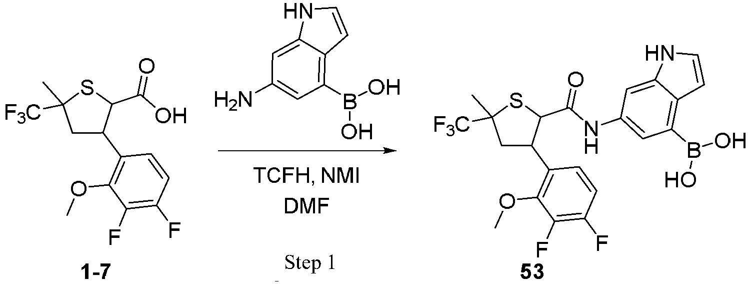

Compound 1-7 (30 mg, 0.08 mmol) was dissolved in anhydrous DMF (5 mL), (6-amino-1H-indol-4-yl)boronic acid (43 mg, 0.24 mmol) was added, and TCFH (118 mg, 0.41 mmol) and NMI (55 mg, 0.64 mmol) were added in an ice bath. The mixture was allowed to naturally warm to room temperature and then stirred for 1 h. After the reaction was completed, the mixture was separated by Pre-HPLC to obtain the target compound (15.4 mg, 0.03 mmol, yield: 40.50%). MS: m/z = 515.2, [M+H]⁺, ¹H NMR (400 MHz, DMSO) δ 10.85 (s, 1H), 10.09 (s, 1H), 7.91 (s, 1H), 7.76 (s, 2H), 7.23-7.11 (m, 4H), 6.66 (s, 1H), 4.67 (d,*J*= 10.8 Hz, 1H), 4.35 - 4.26 (m, 1H), 3.97 (s, 3H), 2.42 - 2.28 (m, 2H), 1.74 (s, 3H).

### Example 11: Preparation of (5-(3-(3,4-difluoro-2-methoxyphenyl)-5-methyl-5-(trifluoromethyl)tetrahydrothiophene-2-carboxamido)-1H-indazol-7-yl)boronic acid (compound 54)

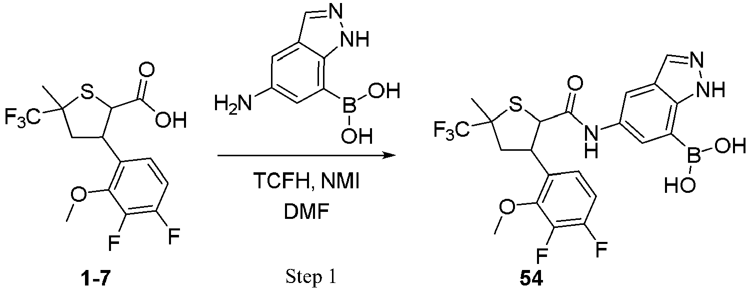

Compound 1-7 (50 mg, 0.14 mmol) was dissolved in anhydrous DMF (5 mL), (5-amino-1H-indazol-7-yl)boronic acid (75 mg, 0.42 mmol) was added, and TCFH (196 mg, 0.70 mmol) and NMI (92 mg, 1.12 mmol) were added in an ice bath. The mixture was allowed to naturally warm to room temperature and then stirred for 1 h. After the reaction was completed, the mixture was separated by Pre-HPLC to obtain the target compound (5.39 mg, 0.03 mmol, yield: 7.47%). MS: m/z = 516.2, [M+H]⁺, ¹H NMR (400 MHz, DMSO) δ 12.32 (s, 1H), 10.22 (s, 1H), 8.36 (s, 2H), 8.09 (s, 1H), 7.99 (s, 1H), 7.65 (d,*J*= 1.6 Hz, 1H), 7.27 - 7.15 (m, 2H), 4.69 (d,*J*= 10.8 Hz, 1H), 4.37 - 4.29 (m, 1H), 4.00 (d,*J=* 1.6 Hz, 3H), 2.46-2.33 (m, 2H), 1.77 (s, 3H).

### Example 12: Preparation of (4-(3-(3,4-difluoro-2-methoxyphenyl)-5-methyl-5-(trifluoromethyl)tetrahydrothiophene-2-carboxamido)phenyl)boronic acid (compound 82)

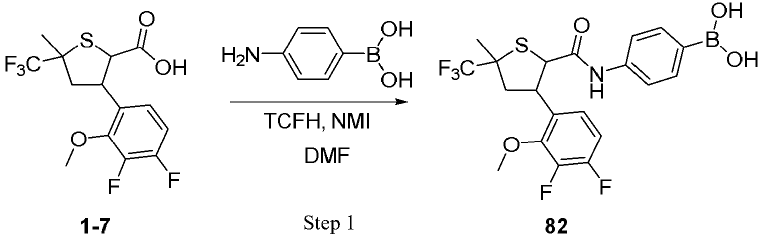

Compound 1-7 (30 mg, 0.08 mmol) was dissolved in anhydrous DMF (5 mL), (4-aminophenyl)boronic acid (33 mg, 0.24 mmol) was added, and TCFH (120 mg, 0.40 mmol) and NMI (56 mg, 0.64 mmol) were added in an ice bath. The mixture was allowed to naturally warm to room temperature and then stirred for 1 h. After the reaction was completed, the mixture was separated by Pre-HPLC to obtain the target compound (21 mg, 0.04 mmol, yield: 55%). MS: m/z = 476.2, [M+H]⁺, ¹H NMR (400 MHz, DMSO) δ 10.24 (s, 1H), 7.89 (s, 2H), 7.68 (d,*J*= 8.3 Hz, 2H), 7.45 (d,*J*= 8.3 Hz, 2H), 7.22 - 7.09 (m, 2H), 4.61 (d,*J*= 10.7 Hz, 1H), 4.35 - 4.23 (m, 1H), 3.97 (d,*J*= 1.4 Hz, 3H), 2.47-2.25 (m, 2H), 1.73 (s, 3H).

### Example 13: Preparation of (4-(3-(3,4-difluoro-2-methoxyphenyl)-5-methyl-5-(trifluoromethyl)tetrahydrothiophene-2-carboxamido)-3-fluorophenyl)boronic acid (compound 87)

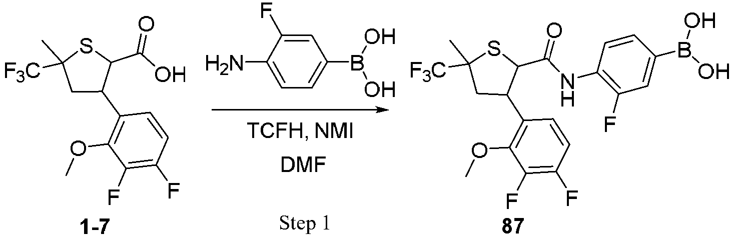

Compound 1-7 (30 mg, 0.08 mmol) was dissolved in anhydrous DMF (5 mL), (4-amino-3-fluorophenyl)boronic acid (70 mg, 0.24 mmol) was added, and TCFH (118 mg, 0.41 mmol) and NMI (55 mg, 0.64 mmol) were added in an ice bath. The mixture was allowed to naturally warm to room temperature and then stirred for 1 h. After the reaction was completed, the mixture was separated by Pre-HPLC to obtain the target compound (19.7 mg, 0.04 mmol, yield: 50.01%). MS: m/z = 494.2, [M+H]⁺, ¹H NMR (400 MHz, DMSO) δ 10.04 (s, 1H), 8.12 (s, 2H), 7.79 (t,*J*= 7.8 Hz, 1H), 7.53 - 7.49 (m, 2H), 7.19-7.15 (m, 2H), 4.84 (d,*J* = 10.8 Hz, 1H), 4.30-4.23 (m, 1H), 3.95 (d, *J =* 1.2 Hz, 3H), 2.37-2.31 (m, 2H), 1.73 (s, 3H).

### Example 14: Preparation of (4-(3-(3,4-difluoro-2-methoxyphenyl)-5-methyl-5-(trifluoromethyl)tetrahydrothiophene-2-carboxamido)-3-methoxyphenyl)boronic acid (compound 94)

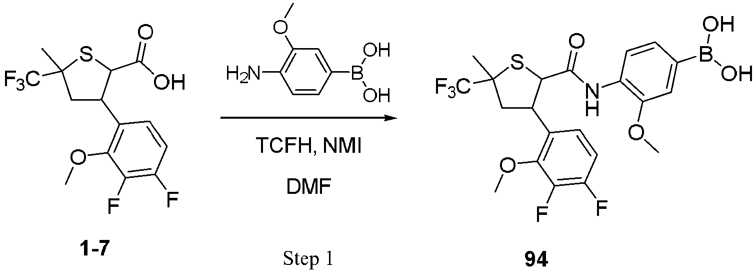

Compound 1-7 (30 mg, 0.08 mmol) was dissolved in anhydrous DMF (5 mL), (4-amino-3-methoxyphenyl)boronic acid (63 mg, 0.24 mmol) was added, and TCFH (118 mg, 0.41 mmol) and NMI (55 mg, 0.64 mmol) were added in an ice bath. The mixture was allowed to naturally warm to room temperature and then stirred for 1 h. After the reaction was completed, the mixture was separated by Pre-HPLC to obtain the target compound (17 mg, 0.03 mmol, yield: 42.10%). MS: m/z = 506.2, [M+H]⁺, ¹H NMR (400 MHz, DMSO) δ 9.42 (s, 1H), 8.01 (s, 2H), 7.88 (d, *J =* 8.0 Hz, 1H), 7.42 (s, 1H), 7.33 (d, *J* = 8.0 Hz, 1H), 7.28 - 7.15 (m, 2H), 5.00 (d, *J =* 10.7 Hz, 1H), 4.29 (d, *J =* 8.0 Hz, 1H), 3.96 (d, *J =* 1.2 Hz, 3H), 3.81 (s, 3H), 2.36 - 2.31 (m, 2H), 1.75 (s, 3H).

### Example 15: Preparation of (2-(3-(3,4-difluoro-2-methoxyphenyl)-5-methyl-5-(trifluoromethyl)tetrahydrothiophene-2-carboxamido)pyrimidin-5-yl)boronic acid (compound 111)

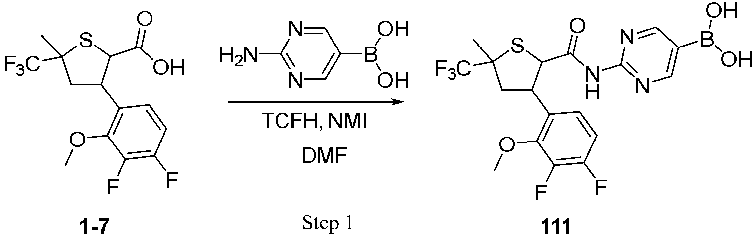

Compound 1-7 (30 mg, 0.08 mmol) was dissolved in anhydrous DMF (5 mL), (2-aminopyrimidin-5-yl)boronic acid (34 mg, 0.24 mmol) was added, and TCFH (118 mg, 0.41 mmol) and NMI (55 mg, 0.64 mmol) were added in an ice bath. The mixture was allowed to naturally warm to room temperature and then stirred for 1 h. After the reaction was completed, the mixture was separated by Pre-HPLC to obtain the target compound (23.9 mg, 0.05 mmol, yield: 62.50%). MS: m/z = 478.2, [M+H]⁺, ¹H NMR (400 MHz, DMSO) δ 10.90 (s, 1H), 8.83 (s, 2H), 8.46 (s, 2H), 7.19 - 7.14 (m, 2H), 4.92 (d, *J =* 10.2 Hz, 1H), 4.36 - 4.28 (m, 1H), 3.95 (d, *J=* 1.6 Hz, 3H), 2.40 - 2.28 (m, 2H), 1.72 (s, 3H).

### Example 16: Preparation of 4-(3-(3,4-difluoro-2-methoxyphenyl)-5,5-dimethyltetrahydrothiophene-2-carboxamido)picolinamide (compound 134)

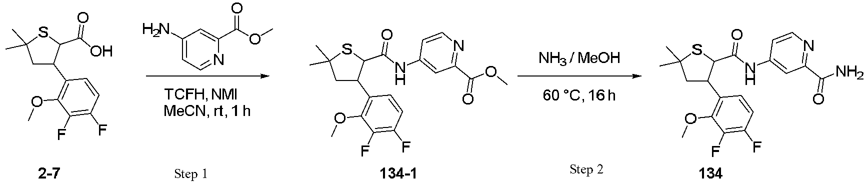

### Step 1: Synthesis of methyl 4-(3-(3,4-difluoro-2-methoxyphenyl)-5,5-dimethyltetrahydrothiophene-2-carboxamido)picolinate

Compound 2-7 (500 mg, 1.65 mmol), acetonitrile (10 mL), NMI (583 mg, 5.7 mmol), and methyl 4-aminopicolinate (275 mg, 1.82 mmol) were mixed well by stirring, TCFH (693 mg, 2.4 mmol) was then added, and the mixture was stirred at room temperature for another 1 h. The reaction mixture was quenched with water and concentrated under reduced pressure. Water was added to the residue, and the resulting mixture was extracted 3 times with ethyl acetate. The organic phase was dried over anhydrous sodium sulfate, filtered, and concentrated under reduced pressure. The crude product was purified by Pre-HPLC to obtain the target compound (523 mg, 1.2 mmol, yield: 78.1%). MS: m/z = 437.1, [M+H]⁺.

### Step 2: Synthesis of 4-(3-(3,4-difluoro-2-methoxyphenyl)-5,5-dimethyltetrahydrothiophene-2-carboxamide)picolinamide

The product of step 1 (523 mg, 1.2 mmol) was treated with a 2 M solution of ammonia in methanol (10 mL), and the reaction mixture was warmed to 60 °C and stirred overnight. The reaction system was cooled to room temperature and concentrated under reduced pressure. The crude product was separated by Pre-HPLC to obtain the target compound (283 mg, 0.67 mmol, yield: 56.1%). MS: m/z = 422.1, [M+H]⁺. ¹H NMR (400 MHz, DMSO) δ 10.60 (s, 1H), 8.45 (d, *J =* 5.2 Hz, 1H), 8.18 (s, 1H), 8.06 (s, 1H), 7.74 - 7.57 (m, 2H), 7.29-7.12 (m, 2H), 4.40 (s, 2H), 3.90 (s, 3H), 2.30-2.12 (m, 2H), 1.60 (s, 3H), 1.48 (s, 3H).

A chiral resolution method was used: chromatographic column: OJ-H; column temperature: 30 °C; mobile phase: n-hexane-absolute ethanol-isopropanol-diethylamine = 80:16:4:0.05; flow rate: 0.6 mL/min. Compound 134-A (retention time: 14.689 min) and compound 134-B (retention time: 17.899 min)

### Example 17: Preparation of 4-(3-(3,4-difluoro-2-methoxyphenyl)-5-methyl-5-(trifluoromethyl)tetrahydrothiophene-2-carboxamido)picolinamide (compound 139)

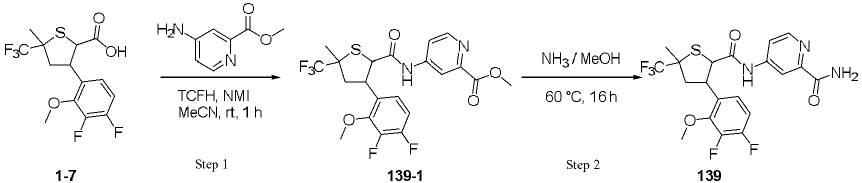

### Step 1: Synthesis of methyl 4-(3-(3,4-difluoro-2-methoxyphenyl)-5-methyl-5-(trifluoromethyl)tetrahydrothiophene-2-carboxamido)picolinate (139-1)

To a mixture of intermediate 1-7 (837 mg, 2.35 mmol), DMF (30 mL), NMI (1.17 g, 11.4 mmol), and methyl 4-aminopicolinate (550 mg, 3.64 mmol), TCFH (2.08 mg, 7.2 mmol) was added in an ice bath. The mixture was allowed to naturally warm to room temperature and then stirred for 1 h. Water (20 mL) was added to quench the reaction, and the mixture was extracted with ethyl acetate (30 mL × 3). The organic phases were combined, washed three times with water, washed once with saturated brine, dried over anhydrous sodium sulfate, filtered, and concentrated under reduced pressure. The crude product was separated by Pre-HPLC to obtain the target compound (910 mg, 1.86 mmol, yield: 78.99%). MS: m/z = 491.1, [M+H]⁺.

### Step 2: Synthesis of 4-(3-(3,4-difluoro-2-methoxyphenyl)-5-methyl-5-(trifluoromethyl)tetrahydrothiophene-2-carboxamido)picolinamide (compound 139)

The product of step 1 (910 mg, 1.86 mmol) was treated with a 2 M solution of ammonia in methanol (15 mL), and the reaction system was warmed to 60 °C and stirred overnight. The reaction system was cooled to room temperature and concentrated under reduced pressure, and the crude product was separated by Pre-HPLC to obtain the target compound (711 mg, 1.5 mmol, yield: 80.6%). MS: m/z = 476.3, [M+H]⁺, ¹H NMR (400 MHz, DMSO) δ 10.81 (s, 1H), 8.46 (d,*J=* 5.5 Hz, 1H), 8.15 (d, *J=* 1.7 Hz, 1H), 8.03 (s, 1H), 7.67 (dd,*J*= 5.5, 2.0 Hz, 1H), 7.60 (s, 1H), 7.28 - 7.09 (m, 2H), 4.61 (d,*J* = 10.6 Hz, 1H), 4.39-7.18 (m, 1H), 3.97 (d,*J=* 1.5 Hz, 3H), 2.47 - 2.13 (m, 2H), 1.74 (s, 3H). A chiral resolution method was used: chromatographic column: AD-H; column temperature: 30 °C; mobile phase: n-hexane-absolute ethanol-isopropanol-diethylamine = 80:16:4:0.05; flow rate: 1 mL/min. Compound 139-A (retention time: 5.387 min) and compound 139-B (retention time: 9.442 min)

### Example 18: Preparation of N-(3-carbamoyl-4-fluorophenyl)-3-(3,4-difluoro-2-methoxyphenyl)-5-methyl-5-(trifluoromethyl)tetrahydrothiophene-2-carboxamide (compound 146).

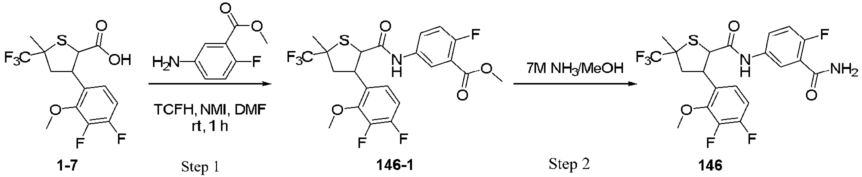

### Step 1: Synthesis of methyl 5-(3,4-difluoro-2-methoxyphenyl)-5-methyl-5-(trifluoromethyl)tetrahydrothiophene-2-carboxamido)-2-fluorobenzoate (compound 146-1).

Compound 1-7 (50.7 mg, 0.1 mmol) was completely dissolved in 3 mL of dry DMF, and methyl 5-amino-2-fluorobenzoate (169.1 mg, 0.1 mmol), NMI (240.2 mg, 3.0 mmol), and TCFH (420 mg, 0.15 mmol) were sequentially added in an ice bath. After the addition, the mixture was allowed to naturally warm to room temperature and react for 4 h. Water (10 mL) was added to quench the reaction, and the mixture was extracted with ethyl acetate (20 mL × 3). The organic phases were combined, washed three times with water, washed once with saturated brine, dried over anhydrous sodium sulfate, filtered, and concentrated. The crude product was separated by Pre-HPLC to obtain the target compound (22.3 mg, 0.044 mmol, yield: 44.0%). MS: m/z = 508.2, [M+H]⁺.

### Step 2: Synthesis of N-(3-carbamoyl-4-fluorophenyl)-3-(3,4-difluoro-2-methoxyphenyl)-5-methyl-5-(trifluoromethyl)tetrahydrothiophene-2-carboxamide (compound 146)

The product of step 1 (22.3 mg, 0.044 mmol) was added to a 7 M solution of NH₃ in MeOH (2 mL), and the mixture was stirred at room temperature overnight. After the reaction was completed, the mixture was concentrated to obtain a crude product, and the crude product was separated by Pre-HPLC to obtain the target compound (17 mg, 0.034 mmol, yield: 77.2%). MS: m/z = 493.2, [M+H]⁺. ¹H NMR (400 MHz, DMSO) δ 10.42 (s, 1H), 7.80 (dd, *J =* 6.4, 2.8 Hz, 1H), 7.71 - 7.56 (m, 3H), 7.18 (dt, *J* = 10.8, 8.5 Hz, 3H), 4.57 (d, *J =* 10.7 Hz, 1H), 4.32 - 4.20 (m, 1H), 3.96 (d, *J* = 1.8 Hz, 3H), 2.43 - 2.29 (m, 2H), 1.73 (s, 3H).

### Example 19: Preparation of 3-(3,4-difluoro-2-methoxyphenyl)-5-methyl-N-(2-oxo-1,2-dihydropyridin-4-yl)-5-(trifluoromethyl)tetrahydrothiophene-2-carboxamide (compound 170)

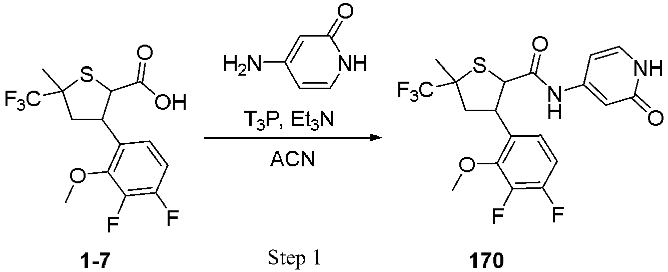

Compound 1-7 (30.0 mg, 0.084 mmol) was dissolved in 1.0 mL of dry MeCN, and T₃P (267.8 mg, 0.842 mmol) and Et₃N (68.16 mg, 0.673 mmol) were sequentially added. The mixture was allowed to react at 60 °C for 0.5 h. 4-Aminopyridin-2(1H)-one (13.9 mg, 0.126 mmol) was added, and the mixture was allowed to react at 60 °C for another 3.0 h. Pre-HPLC separation was performed to obtain the target compound (13.0 mg, 0.289 mmol, yield: 34.43%). MS: m/z = 449.2, [M+H]⁺. ¹H NMR (400 MHz, DMSO) δ 11.23 (s, 1H), 10.31 (s, 1H), 7.24 (d, *J =* 7.2 Hz, 1H), 7.20 - 7.14 (m, 2H), 6.60 (d, *J =* 2.0 Hz, 1H), 6.18 (dd, *J* = 7.2, 1.9 Hz, 1H), 4.55 (d, *J =* 10.6 Hz, 1H), 4.29 - 4.18 (m, 1H), 3.96 (d, *J =* 1.9 Hz, 3H), 2.44 - 2.37 (m, 1H), 2.33 - 2.26 (m, 1H), 1.71 (s, 3H).

### Example 20: Preparation of 3-(3,4-difluoro-2-methoxyphenyl)-5-methyl-N-(1-methyl-2-oxo-1,2-dihydropyridin-4-yl)-5-(trifluoromethyl)tetrahydrothiophene-2-carboxamide (compound 172)

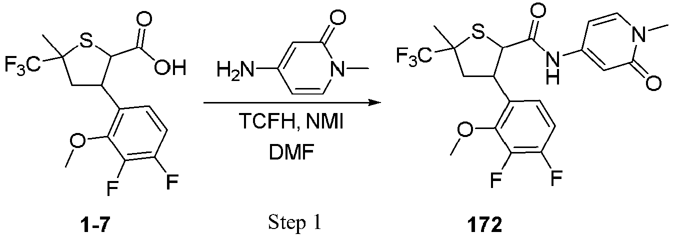

Compound 1-7 (30 mg, 0.084 mmol) was dissolved in anhydrous DMF (2 mL), and 4-amino-1-methylpyridin-2(1H)-one (16 mg, 0.13 mmol) and NMI (33 mg, 0.4 mmol) were added. The mixture was stirred in an ice bath. TCFH (56 mg, 0.2 mmol) was weighed out and added to the reaction flask in one portion, and the mixture was stirred at room temperature for 2 h. 10 mL of water was added to the reaction mixture, and the resulting mixture was extracted with ethyl acetate (20 mL × 3). The organic phases were combined, washed with saturated brine, dried over anhydrous sodium sulfate, filtered, and concentrated. The crude product was separated by Pre-HPLC to obtain the target compound (13 mg, 0.028 mmol, yield: 33%). MS: m/z = 463.1, [M+H]⁺. ¹H NMR (400 MHz, CDCl₃) δ 8.76 (s, 1H), 7.23 (d,*J* = 7.4 Hz, 1H), 7.02 (d, *J=* 6.2 Hz, 1H), 6.95 - 6.78 (m, 2H), 6.54 (s, 1H), 4.49 (d,*J*= 10.7 Hz, 1H), 4.29 - 4.11 (m, 1H), 4.00 (d,*J*= 2.0 Hz, 3H), 3.52 (s, 3H), 2.47 - 2.16 (m, 2H), 1.75 (s, 3H).

### Example 21: Preparation of 3-(3,4-difluoro-2-methoxyphenyl)-5-methyl-N-(6-oxo-1,6-dihydropyridin-3-yl)-5-(trifluoromethyl)tetrahydrothiophene-2-carboxamide (compound 176)

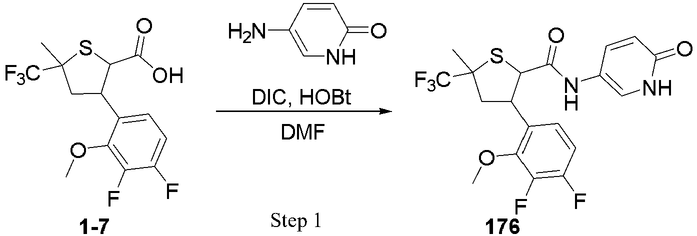

Compound 1-7 (30.0 mg, 0.084 mmol) was dissolved in DMF (1.5 mL), 5-aminopyridin-2(1H)-one (15.0 mg, 0.126 mmol) and HOBt (24.0 mg, 0.168 mmol) were added, and the reaction flask was placed in an ice-water bath. DIC (33.0 mg, 0.252 mmol) was added. The mixture was allowed to naturally warm to room temperature and then react for 4 h. The reaction solution was separated by Pre-HPLC to obtain the target compound (20.8 mg, 0.046 mmol, yield: 55.2%). MS: m/z = 449.2, [M+H]⁺, ¹H NMR (400 MHz, DMSO) δ 11.25 (s, 1H), 10.06 (s, 1H), 7.77 (d,*J*= 2.8 Hz, 1H), 7.30 (dd,*J* = 9.7, 2.9 Hz, 1H), 7.20 - 7.10 (m, 2H), 6.30 (d, *J=* 9.7 Hz, 1H), 4.50 (d,*J* = 10.8 Hz, 1H), 4.29 - 4.16 (m, 1H),, 3.95 (d,*J* = 1.8 Hz, 3H), 2.45 - 2.23 (m, 2H), 1.71 (s, 3H).

### Example 22: Preparation of 3-(3,4-difluoro-2-methoxyphenyl)-5-methyl-N-(6-oxo-1,6-dihydropyridin-3-yl)-5-(trifluoromethyl)tetrahydrothiophene-2-d-2-carboxamide (compound 177)

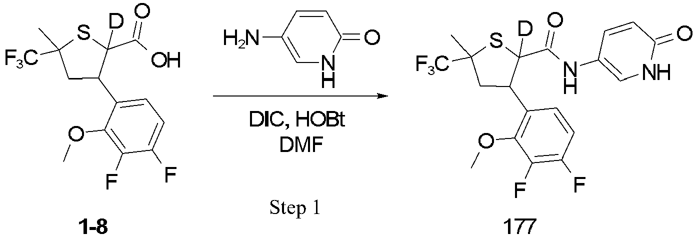

Compound 1-8 (40.0 mg, 0.112 mmol) was dissolved in DMF (2.0 mL), 5-aminopyridin-2(1H)-one (18.0 mg, 0.168 mmol) and HOBt (30.0 mg, 0.224 mmol) were added, and DIC (42.0 mg, 0.336 mmol) was added at 0 °C. The mixture was allowed to naturally warm to room temperature and then react for 4 h. The reaction solution was separated by Pre-HPLC to obtain the target compound (27.6 mg, 0.061 mmol, yield: 54.8%). MS: m/z=450.1,[M+H]⁺. ¹H NMR (400 MHz, DMSO) δ 11.31 (s, 1H), 10.05 (s, 1H), 7.77 (d, *J*= 2.8 Hz, 1H), 7.36-7.25 (m, 1H), 7.22-7.01 (m, 2H), 6.33 - 6.28 (m, 1H), 4.31-4.17 (m, 1H), 3.95 (d, *J=* 1.9 Hz, 3H), 2.46 - 2.23 (m, 2H), 1.71 (s, 3H).

### Example 23: Preparation of 3-(3,4-difluoro-2-methoxyphenyl)-5-methyl-N-(1-methyl-6-oxo-1,6-dihydropyridin-3-yl)-5-(trifluoromethyl)tetrahydrothiophene-2-carboxamide (compound 178)

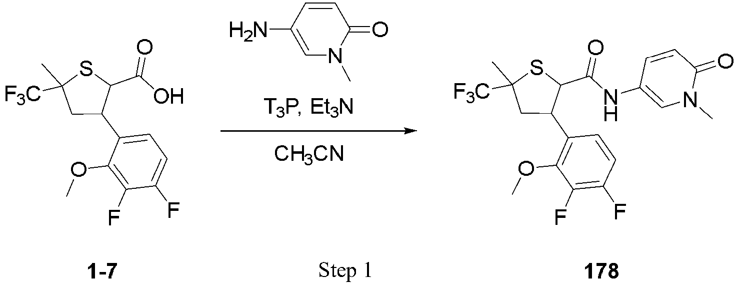

Compound 1-7 (30.0 mg, 0.084 mmol) was dissolved in 1.0 mL of dry MeCN, and T₃P (267.8 mg, 0.842 mmol) and Et₃N (68.16 mg, 0.673 mmol) were sequentially added. The mixture was stirred at 60 °C for 0.5 h. 5-Amino-1-methylpyridin-2(1H)-one (15.6 mg, 0.126 mmol) was added, and the mixture was stirred at 60 °C for another 3.0 h. The reaction solution was separated by Pre-HPLC to obtain the target compound (13.0 mg, 0.028 mmol, yield: 33.45%). MS: m/z = 463.2, [M+H]⁺. ¹H NMR (400 MHz, DMSO) δ 10.42 (s, 1H), 7.80 (dd, *J* = 6.4, 2.8 Hz, 1H), 7.71 - 7.56 (m, 3H), 7.24 - 7.11 (m, 3H), 4.57 (d, *J =* 10.7 Hz, 1H), 4.32 - 4.20 (m, 1H), 3.96 (d, *J =* 1.8 Hz, 3H), 2.43 - 2.31 (m, 2H), 1.73 (s, 3H).

### Example 24: Synthesis of 3-(3,4-difluoro-2-methoxyphenyl)-5-methyl-N-(1-methyl-6-oxo-1,6-dihydropyridin-3-yl)-5-(trifluoromethyl)tetrahydrothiophene-2-d-2-carboxamide (compound 179)

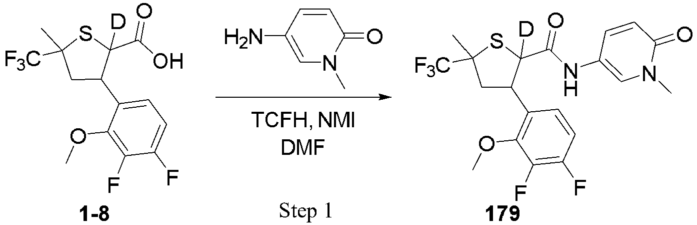

Compound 1-8 (35 mg, 0.098 mmol) was dissolved in anhydrous DMF (2 mL), and 5-amino-1-methylpyridin-2(1H)-one (18 mg, 0.14 mmol) and NMI (33 mg, 0.4 mmol) were added. The mixture was stirred at 0 °C. TCFH (56 mg, 0.2 mmol) was weighed out and added to the reaction flask in one portion, and the mixture was stirred at room temperature for 2 h. The reaction solution was separated by Pre-HPLC to obtain the target compound (15 mg, 0.032 mmol, yield: 32%). MS: m/z = 464.2, [M+H]⁺. ¹H NMR (400 MHz, DMSO) δ 10.05 (s, 1H), 8.07 (d,*J*= 2.8 Hz, 1H), 7.30 - 7.21 (m, 1H), 7.20 - 7.14 (m, 2H), 6.35 (d,*J* = 9.7 Hz, 1H), 4.27 - 4.17 (m, 1H), 3.96 (d, *J=* 1.8 Hz, 3H), 3.36 (s, 3H), 2.44 - 2.25 (m, 2H), 1.71 (s, 3H).

### Example 25: Preparation of 3-((2R,3S,5R)-3-(3,4-difluoro-2-methoxyphenyl)-5-methyl-5-(trifluoromethyl)tetrahydrothiophene-2-carboxamido-2-d)phenyl)boronic acid (compound 10-A)

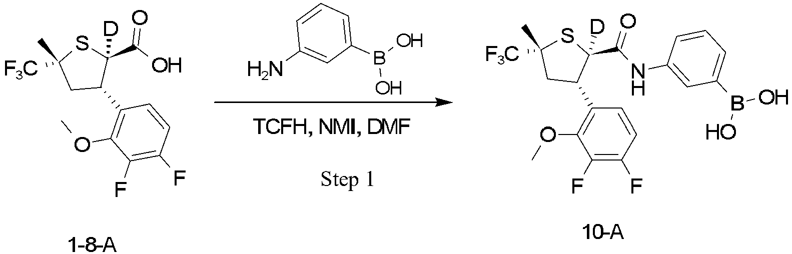

Compound 1-8-A (0.86 g, 2.41 mmol) was dissolved in anhydrous DMF (30 mL), (3-aminophenyl)boronic acid (990.5 mg, 7.23 mmol) was added, and TCFH (3.37 g, 12.05 mmol) and NMI (1.58 g, 19.28 mmol) were added in an ice bath. The mixture was allowed to naturally warm to room temperature and then stirred. After the reaction was completed, the mixture was separated by Pre-HPLC to obtain the target compound (842.6 mg, 1.76 mmol, yield: 73.3%).

MS: m/z = 477.2, [M+H]⁺.

¹H NMR (400 MHz, DMSO) δ 10.02 (s, 1H), 7.59-7.58 (m, 3H), 7.46-7.45(m, 1H), 7.02 (d, *J=*2.0 Hz, 1H), 6.85 (d, *J=2.0* Hz, 1H), 4.20 (s, 2H), 3.83 (s, 3H), 3.51-3.50(m, 1H), 2.17-1.92(m, 2H), 1.38 (s, 3H).

### Example 26: Preparation of 3-(3,4-difluoro-2-methoxyphenyl)-5-methyl-N-(6-oxo-1,6-dihydropyridin-3-yl)-5-(trifluoromethyl)tetrahydrothiophene-2-d-2-carboxamide (compound 177)

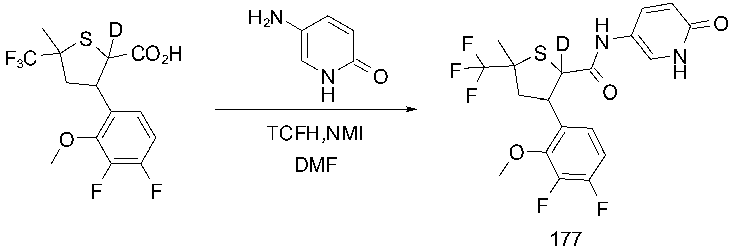

For the experimental procedures, reference may be made to Example 1 (compound 9).

MS: m/z = 450.1, [M+H]+, 1H NMR (400 MHz, DMSO) δ 11.31 (s, 1H), 10.05 (s, 1H), 7.77 (d, J = 2.8 Hz, 1H), 7.30 (dd, J = 9.7, 2.9 Hz, 1H), 7.16 (dd, J = 8.4, 5.5 Hz, 2H), 6.33 - 6.28 (m, 1H), 4.21 (dd, J = 13.1, 6.1 Hz, 1H), 3.95 (d, J = 1.9 Hz, 3H), 2.39 (t, J = 12.9 Hz, 1H), 2.28 (dd, J = 12.6, 6.2 Hz, 1H), 1.71 (s, 3H).

### Example 27: Preparation of (5-(3-(3,4-difluoro-2-methoxyphenyl)-5-methyl-5-(trifluoromethyl)tetrahydrothiophene-2-carboxamido)-2-((difluoromethyl)sulfonyl)phenyl)boronic acid (compound a-1)

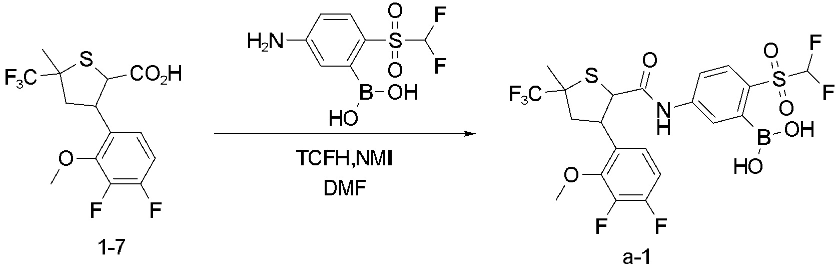

For the experimental procedures, reference may be made to Example 1 (compound 9).

MS: m/z = 590.2 [M+H]⁺.

¹H NMR (400 MHz, CDCl₃) δ 8.26 (s, 1H), 7.99 (d, J = 8.8 Hz, 1H), 7.93 (d, J = 8.7 Hz, 1H), 7.70 (s, 1H), 7.04 - 6.96 (m, 1H), 6.85 (dd, J *=* 16.5, 8.8 Hz, 1H), 6.42 (t, J = 53.8 Hz, 1H), 4.47 (d, J = 10.6 Hz, 1H), 4.23 - 4.11 (m, 1H), 4.00 (d, J = 2.2 Hz, 3H), 2.61 (t, J = 13.0 Hz, 1H), 2.24 (dd, J = 12.9, 5.9 Hz, 1H), 1.81 (s, 3H).

### Example 28: Preparation of 3-(3,4-difluoro-2-methoxyphenyl)-N-(1-hydroxy-1,3-dihydrobenzo[c][1,2]oxaborol-5-yl)-5-methyl-5-(trifluoromethyl)tetrahydrothiophene-2-carboxamide (compound 219)

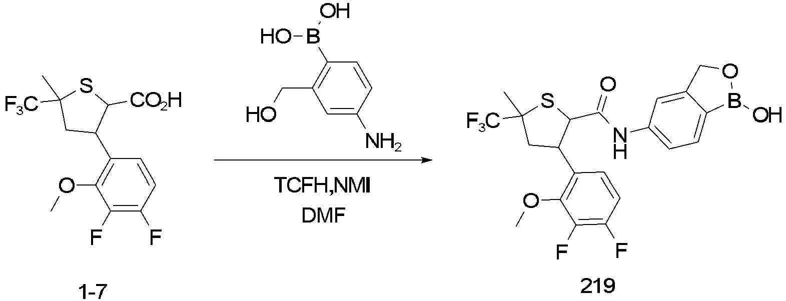

For the experimental procedures, reference may be made to Example 1 (compound 9).

MS: m/z = 488.2 [M+H]⁺.

1H NMR (400 MHz, CDCl₃) δ 7.87 (s, 1H), 7.75 (s, 1H), 7.65 (d, J = 7.9 Hz, 1H), 7.21 - 7.16 (m, 1H), 7.00 (ddd, J = 7.9, 5.6, 2.0 Hz, 1H), 6.86 (dt, J = 16.3, 8.2 Hz, 1H), 5.01 (d, J = 11.4 Hz, 2H), 4.41 (d, J = 10.8 Hz, 1H), 4.22 - 4.11 (m, 1H), 3.98 (d, J = 2.5 Hz, 4H), 2.60 (t, J = 13.0 Hz, 1H), 2.23 (dd, J = 12.9, 6.1 Hz, 1H), 1.81 (s, 4H), 1.62 (s, 2H).

### Example 29: Preparation of (2-bromo-5-(3-(3,4-difluoro-2-methoxyphenyl)-5-methyl-5-(trifluoromethyl)tetrahydrothiophene-2-carboxamido)phenyl)boronic acid (compound 24)

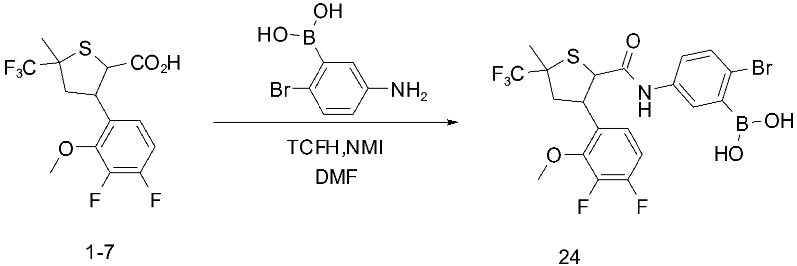

For the experimental procedures, reference may be made to Example 1 (compound 9).

MS: m/z = 555.1 [M+H]⁺.

1H NMR (400 MHz, CDCl₃) δ 7.84 (s, 1H), 7.77 (dd, J = 8.7, 2.8 Hz, 1H), 7.66 (d, J = 2.8 Hz, 1H), 7.45 (d, J = 8.7 Hz, 1H), 7.01 - 6.96 (m, 1H), 6.85 (dt, J = 16.4, 8.2 Hz, 1H), 5.48 (s, 2H), 4.36 (dd, J = 10.9, 5.5 Hz, 1H), 4.19 - 4.08 (m, 1H), 3.97 (d, J = 2.6 Hz, 3H), 2.60 (dd, J = 22.3, 9.3 Hz, 1H), 2.22 (dd, J = 12.9, 6.0 Hz, 1H), 1.80 (s, 3H).

### Example 30: Preparation of (4-((2R,3S,5R)-3-(3,4-difluoro-2-methoxyphenyl)-5-methyl-5-(trifluoromethyl)tetrahydrothiophene-2-carboxamido-2-d)thiophen-2-yl)boronic acid (compound 50-A)

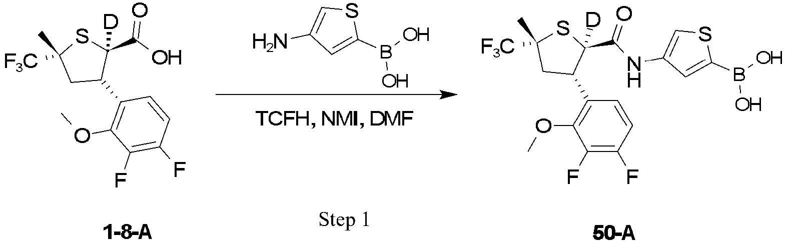

Compound 1-8-A (0.86 g, 2.41 mmol) was dissolved in anhydrous DMF (30 mL), (4-aminothiophen-2-yl)boronic acid (1.03 g, 7.23 mmol) was added, and TCFH (3.37 g, 12.05 mmol) and NMI (1.58 g, 19.28 mmol) were added in an ice bath. The mixture was allowed to naturally warm to room temperature and then stirred. After the reaction was completed, the mixture was separated by Pre-HPLC to obtain the target compound (662.6 mg, 1.37 mmol, yield: 56.9%).

MS: m/z = 483.2, [M+H]⁺.

¹H NMR (400 MHz, DMSO) δ 9.92 (s, 1H), 7.02 (d, *J=*2.0 Hz, 1H), 6.85 (d, *J=*2.0 Hz, 1H), 6.57 (s, 1H), 6.32 (s, 1H), 4.20 (s, 2H), 3.83 (s, 3H), 3.51-3.50(m, 1H), 2.17-1.92(m, 2H), 1.38 (s, 3H).

### Example 31: Preparation of (5-(3-(3,4-difluoro-2-methoxyphenyl)-5-methyl-5-(trifluoromethyl)tetrahydrothiophene-2-carboxamido-2-d)-2-fluorophenyl)boronic acid (compound 20-A)

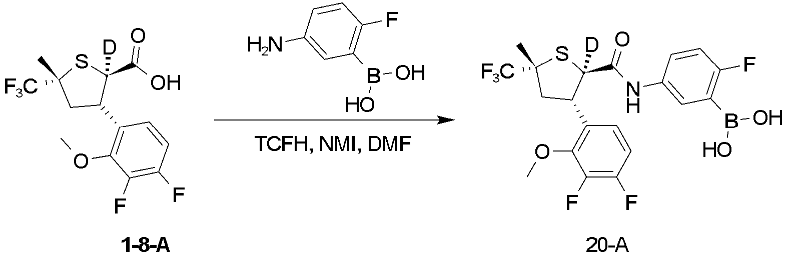

Compound 1-8-A (0.86 g, 2.41 mmol) was dissolved in anhydrous DMF (30 mL), (5-amino-2-fluorophenyl)boronic acid (1.12 g, 7.23 mmol) was added, and TCFH (3.37 g, 12.05 mmol) and NMI (1.58 g, 19.28 mmol) were added in an ice bath. The mixture was allowed to naturally warm to room temperature and then stirred. After the reaction was completed, the mixture was separated by Pre-HPLC to obtain the target compound (886.5 mg, 1.79 mmol, yield: 72.3%).

MS: m/z = 495.2, [M+H]⁺.

¹H NMR (400 MHz, DMSO) δ 10.02 (s, 1H), 7.60-7.58 (m, 2H), 7.30 (d, *J=*2.0 Hz, 1H), 7.02 (d, *J=*2.0 Hz, 1H), 6.85 (d, *J=*2.0 Hz, 1H), 4.20 (s, 2H), 3.83 (s, 3H), 3.51-3.50(m, 1H), 2.17-1.92(m, 2H), 1.38 (s, 3H).

### Example 32: Preparation of (4-((2R,3S,5R)-3-(3,4-difluoro-2-methoxyphenyl)-5-methyl-5-(trifluoromethyl)tetrahydrothiophene-2-carboxamido)-2-(methoxycarbonyl)phenyl)boronic acid (compound 101-A)

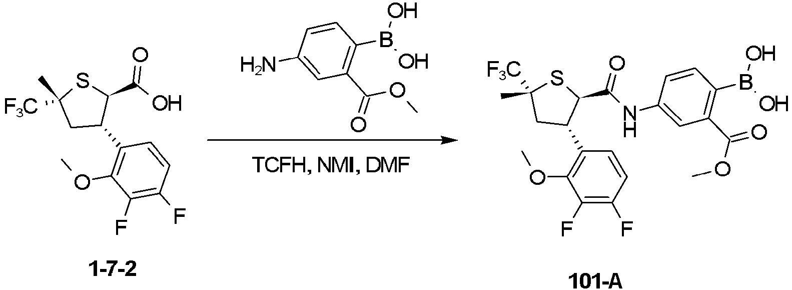

Compound 1-7-2 (50 mg, 0.14 mmol) was dissolved in anhydrous DMF (2 mL), (4-amino-2-(methoxycarbonyl)phenyl)boronic acid (82.3 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (24.6 mg, 0.05 mmol, yield: 32.9%).

Ms: m/z = 534.2, [M+H]⁺.

¹H NMR (400 MHz, DMSO) δ 10.02 (s, 1H), 8.19 (d, *J=*2.0 Hz, 1H), 7.96 (d, *J=*2.0 Hz, 1H), 7.86 (d, *J*=2.0 Hz, 1H), 7.02 (d, *J*=2.0 Hz, 1H), 6.85 (d, *J*=2.0 Hz, 1H), 4.20-4.07 (m, 3H), 3.90 (s, 3H), 3.83 (s, 3H), 3.46-3.43(m, 1H), 2.17-1.92(m, 2H), 1.38 (s, 3H).

### Example 33: Preparation of (4-(3-(3,4-difluoro-2-methoxyphenyl)-5-methyl-5-(trifluoromethyl)tetrahydrothiophene-2-carboxamido)-2-chlorophenyl)boronic acid (compound 92-A)

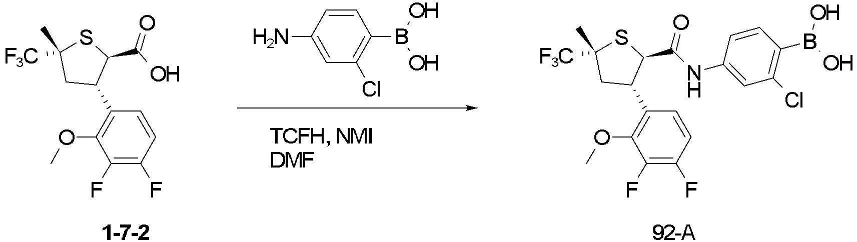

Compound 1-7-2 (50 mg, 0.14 mmol) was dissolved in anhydrous DMF (5 mL), 4-amino-2-chlorophenylboronic acid (72 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (63 mg, 0.12 mmol, yield: 88%). MS: m/z = 510.07, [M+H]⁺.

¹H NMR (400 MHz, DMSO) δ ¹H NMR (400 MHz, DMSO) δ 9.89 (s, 1H), 8.25 (d, *J =* 13.8 Hz, 2H), 7.88 - 7.64 (m, 2H), 7.56 - 7.44 (m, 3H), 4.61 (d, *J =* 10.7 Hz, 1H), 4.38 - 4.24 (m, 1H), 3.98 (d, *J =* 1.6 Hz, 3H), 2.45 (t, *J =* 12.9 Hz, 1H), 2.31 (dd, *J =* 12.6, 6.0 Hz, 1H), 1.76 (s, 3H).

### Example 34: Preparation of (4-(3-(3,4-difluoro-2-methoxyphenyl)-5-methyl-5-(trifluoromethyl)tetrahydrothiophene-2-carboxamido)-2-trifluoromethoxyphenyl)boronic acid (compound 29-A)

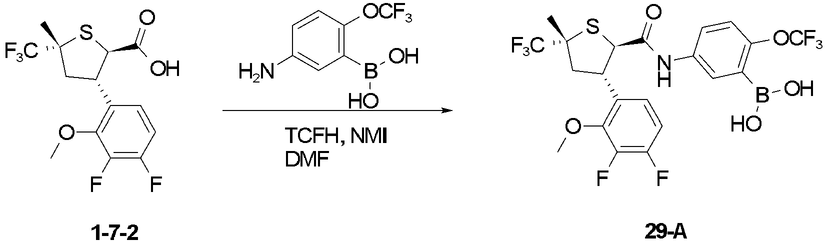

Compound 1-7-2 (50 mg, 0.14 mmol) was dissolved in anhydrous DMF (5 mL), 4-amino-2-trifluoromethoxyphenylboronic acid (93 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (54 mg, 0.096 mmol, yield: 69%).

MS: m/z = 560.06, [M+H]⁺.

¹H NMR (400 MHz, DMSO) δ 9.91 (s, 1H), 8.26 (d, *J =* 13.8 Hz, 2H), 7.87 - 7.63(m, 2H), 7.56 - 7.43 (m, 3H), 4.60 (d, *J* = 10.7 Hz, 1H), 4.38 - 4.24 (m, 1H), 3.99 (d, *J =* 1.6 Hz, 3H), 2.44 (t, *J =* 12.9 Hz, 1H), 2.32 (dd, *J =* 12.6, 6.0 Hz, 1H), 1.77 (s, 3H).

### Example 35: Preparation of (2R,3S,5R)-N-(2-amino-3,4-dioxocyclobut-1-en-1-yl)-3-(3,4-difluoro-2-methoxyphenyl)-5-methyl-5-(trifluoromethyl)tetrahydrothiophene-2-carboxamide (compound a-91-A)

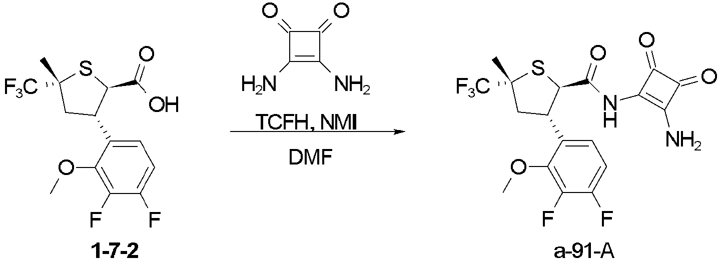

Compound 1-7-2 (50.0 mg, 0.140 mmol) was dissolved in anhydrous DMF (3 mL), and 3,4-diaminocyclobut-3-ene-1,2-dione (15.7 mg, 0.140 mmol) and NMI (22.9 mg, 0.280 mmol) were added. The mixture was stirred at 0 °C. TCFH (78.7 mg, 0.280 mmol) was weighed out and added to the reaction flask in one portion, and the mixture was stirred at room temperature. After the reaction was completed, the reaction solution was separated by Pre-HPLC to obtain the target compound (7.5 mg, 0.017 mmol, yield: 12%).

MS: m/z = 450.1, [M+H]⁺. ¹H NMR (400 MHz, DMSO) δ 11.72 (s, 1H), 8.74 (s, 1H), 7.43 (s, 1H), 7.21 - 7.15 (m, 2H), 4.75 (d, *J =* 10.5 Hz, 1H), 4.30 - 4.16 (m, 1H), 3.95 (d, *J* = 2.0 Hz, 3H), 2.36 (ddd, *J =* 17.5, 12.6, 9.5 Hz, 2H), 1.72 (s, 3H).

### Example 36: Preparation of (5-((2R,3S,5R)-3-(3,4-difluoro-2-methoxyphenyl)-5-methyl-5-(trifluoromethyl)tetrahydrothiophene-2-carboxamido)-2-fluorophenyl)boronic acid (compound 19-A)

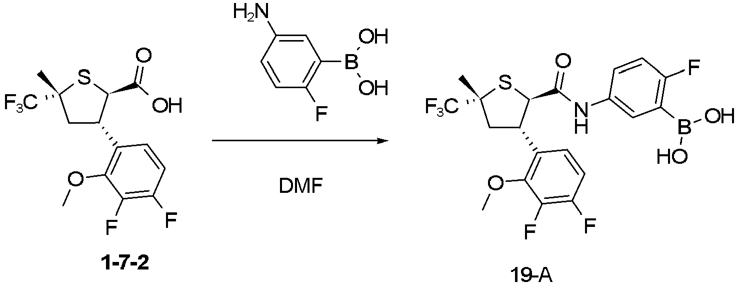

Compound 1-7-2 (50.0 mg, 0.14 mmol) was dissolved in anhydrous DMF (2 mL), (5-amino-2-fluorophenyl)boronic acid (65.1 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (36.8 mg, 0.07 mmol, yield: 53.27%).

MS: m/z = 494.1, [M+H]⁺.

1H NMR (400 MHz, DMSO) δ 10.22 (s, 1H), 8.17 (s, 2H), 7.56 (dd, J = 11.5, 3.9 Hz, 2H), 7.16 (dd, J = 9.9, 6.5 Hz, 2H), 6.98 (d, J = 8.9 Hz, 1H), 4.58 (d, J = 10.7 Hz, 1H), 4.31 - 4.24 (m, 1H), 3.96 (d, J = 1.5 Hz, 3H), 2.39 (d, J = 12.9 Hz, 1H), 2.30 (dd, J *=* 12.5, 6.1 Hz, 1H), 1.73 (s, 3H).

### Example 37: Preparation of 3-(3,4-difluoro-2-methoxyphenyl)-5-methyl-N-(2-sulfamoylpyridin-4-yl)-5-(trifluoromethyl)tetrahydrothiophene-2-carboxamide (compound 193)

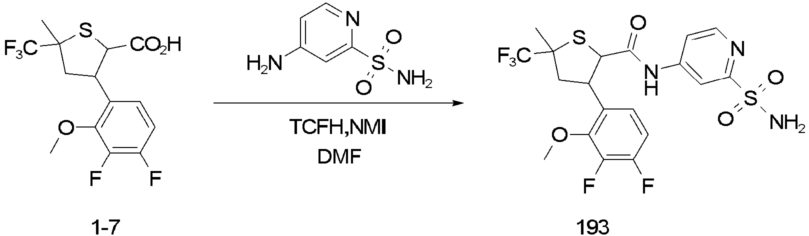

For the experimental procedures, reference may be made to Example 1 (compound 9).

MS: m/z = 512.2 [M+H]⁺.

¹H NMR (400 MHz, MeOD) δ 7.90 - 7.84 (m, 1H), 7.10 (d, J = 2.5 Hz, 1H), 7.00 (ddd, J = 8.1, 5.7, 2.1 Hz, 1H), 6.87 (td, J = 9.3, 7.5 Hz, 1H), 6.75 (dd, J = 6.8, 2.5 Hz, 1H), 4.33 (d, J = 11.0 Hz, 1H), 4.11 - 4.03 (m, 1H), 3.91 (d, J = 2.0 Hz, 3H), 2.37 (t, J = 13.0 Hz, 1H), 2.13 (dd, J = 12.7, 6.0 Hz, 1H), 1.66 (d, J = 9.5 Hz, 3H).

### Example 38: Preparation of (3-((2R,3S,5R)-3-(3,4-difluoro-2-methoxyphenyl)-5-methyl-5-(trifluoromethyl)tetrahydrothiophene-2-carboxamido)phenyl)boronic acid (compound 9-A)

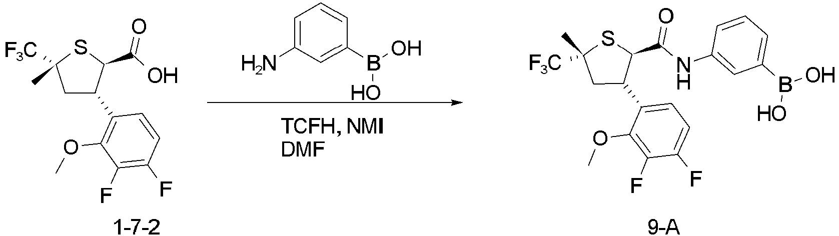

For the experimental procedures, reference may be made to Example 1 (compound 9).

MS: m/z = 476.2, [M+H]⁺.

¹H NMR (400 MHz, CDCl₃) δ 8.13 (d, *J =* 74.5 Hz, 1H), 7.98 - 7.59 (m, 2H), 7.55 - 7.27 (m, 2H), 7.08 - 6.89 (m, 1H), 6.89 - 6.74 (m, 1H), 4.52 - 4.32 (m, 1H), 4.30 - 4.08 (m, 1H), 3.95 (d, *J =* 17.6 Hz, 3H), 2.67 - 2.47 (m, 1H), 2.32 - 2.14 (m, 1H), 1.79 (d, *J =* 13.0 Hz, 3H).

### Example 39: Preparation of (5-(3-(3,4-difluoro-2-methoxyphenyl)-5-methyl-5-(trifluoromethyl)tetrahydrothiophene-2-carboxamido)-2-fluorophenyl)boronic acid (compound 19)

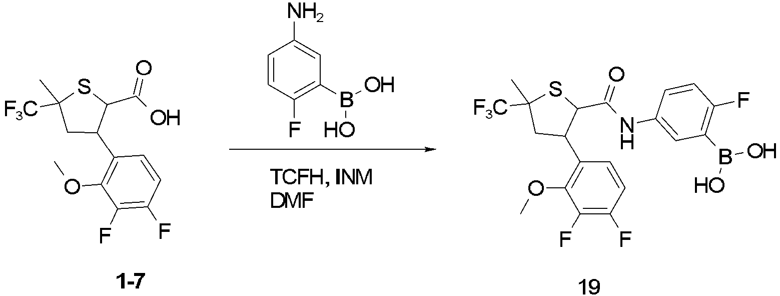

Compound 1-7 (50 mg, 0.14 mmol) was dissolved in anhydrous DMF (5 mL), (5-amino-2-fluorophenyl)boronic acid (65 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (44 mg, 0.089 mmol, yield: 64%). MS: m/z = 494.10, [M+H]⁺.

¹H NMR (400 MHz, DMSO) δ 10.45 (s, 1H), 8.00 (d, *J =* 13.8 Hz, 2H), 7.56 - 7.32 (m, 2H), 7.26 - 7.13 (m, 3H), 4.61 (d, *J =* 10.7 Hz, 1H), 4.38 - 4.24 (m, 1H), 3.99 (d, *J =* 1.6 Hz, 3H), 2.46 (t, *J* = 12.9 Hz, 1H), 2.34 (dd, *J =* 12.6, 6.0 Hz, 1H), 1.76 (s, 3H).

### Example 40: Preparation of (3-((2R,3S,5R)-3-(3,4-difluoro-2-methoxyphenyl)-5-methyl-5-(trifluoromethyl)tetrahydrothiophene-2-carboxamido)phenyl)boronic acid (compound 82-A)

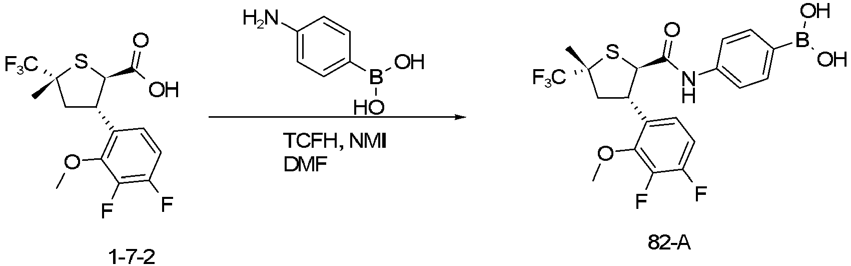

For the experimental procedures, reference may be made to Example 1 (compound 9).

MS: m/z = 476.2, [M+H]⁺.

¹H NMR (400 MHz, CDCl₃) δ 8.08 - 7.95 (m, 2H), 7.94 - 7.66 (m, 1H), 7.48 (dd, *J =* 16.9, 8.3 Hz, 2H), 7.00 (dt, *J* = 10.8, 7.9 Hz, 1H), 6.84 (dt, *J* = 8.8, 7.9 Hz, 1H), 4.41 (dd, *J =* 15.4, 10.8 Hz, 1H), 4.27 - 4.11 (m, 1H), 3.96 (dd, *J* = 10.7, 2.4 Hz, 3H), 2.66 - 2.51 (m, 1H), 2.29 - 2.17 (m, 1H), 1.81 (d, *J =* 8.9 Hz, 3H).

### Example 41: Preparation of (2-bromo-4-(3-(3,4-difluoro-2-methoxyphenyl)-5-methyl-5-(trifluoromethyl)tetrahydrothiophene-2-carboxamido)phenyl)boronic acid (compound 93)

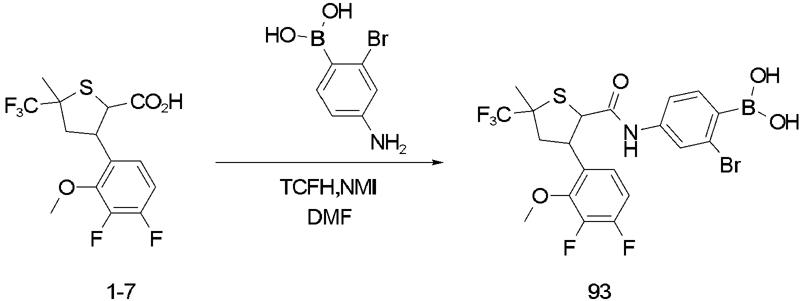

For the experimental procedures, reference may be made to Example 1 (compound 9).

MS: m/z = 555.2 [M+H]⁺.

¹H NMR (400 MHz, CDCl₃) δ 7.88 (dd, J = 8.5, 5.1 Hz, 2H), 7.80 (s, 1H), 7.31 (dd, J = 8.3, 2.0 Hz, 1H), 7.02 - 6.96 (m, 1H), 6.87 (td, J = 9.1, 7.3 Hz, 1H), 4.38 (d, J = 10.8 Hz, 1H), 4.19 - 4.09 (m, 1H), 3.98 (d, J = 2.7 Hz, 3H), 2.60 (t, J = 13.0 Hz, 1H), 2.23 (dd, J = 12.9, 6.0 Hz, 1H), 1.81 (s, 3H).

### Example 42: Preparation of (5-(3-(3,4-difluoro-2-methoxyphenyl)-5-methyl-5-(trifluoromethyl)tetrahydrothiophene-2-carboxamido)-2-(methylthio)phenyl)boronic acid (compound 37)

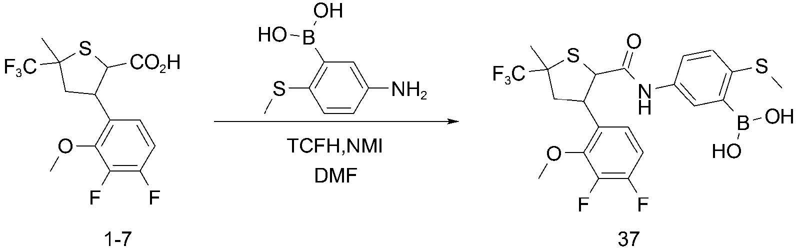

For the experimental procedures, reference may be made to Example 1 (compound 9).

MS: m/z = 522.2 [M+H]⁺.

¹H NMR (400 MHz, CDCl₃) δ 7.93 (dd, J = 8.5, 2.6 Hz, 1H), 7.82 (s, 1H), 7.69 (d, J = 2.6 Hz, 1H), 7.48 (d, J = 8.5 Hz, 1H), 7.02 - 6.96 (m, 1H), 6.85 (dd, J *=* 16.4, 9.1 Hz, 1H), 4.37 (d, J = 10.8 Hz, 1H), 4.18 - 4.09 (m, 1H), 3.97 (d, J = 2.6 Hz, 3H), 2.59 (t, J = 13.0 Hz, 1H), 2.44 (s, 3H), 2.22 (dd, J = 12.9, 6.0 Hz, 1H), 1.81 (s, 3H).

### Example 43: Preparation of 3-(3,4-difluoro-2-methoxyphenyl)-5-methyl-N-(1-methyl-6-oxo-1,6-dihydropyridin-3-yl)-5-(trifluoromethyl)tetrahydrothiophene-2-d-2-carboxamide (compound 179)

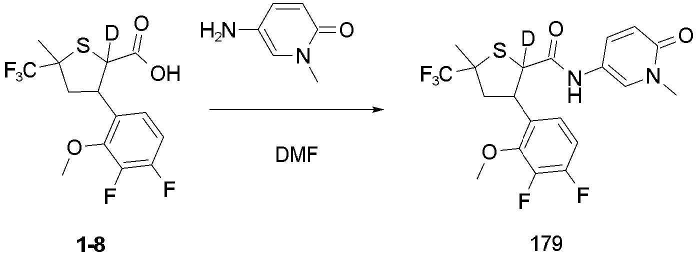

Compound 1-8 (50.0 mg, 0.14 mmol) was dissolved in anhydrous DMF (2 mL), 5-amino-1-methylpyridin-2(1H)-one (52.1 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (35.7 mg, 0.08 mmol, yield: 55.10%).

MS: m/z = 464.1, [M+H]⁺.

¹H NMR (400 MHz, DMSO) δ 10.03 (s, 1H), 8.06 (s, 1H), 7.69 - 7.51 (m, 1H), 7.25 (d, J = 7.3 Hz, 1H), 7.15 (s, 1H), 6.34 (d, J = 9.6 Hz, 1H), 4.22 (d, J = 7.2 Hz, 1H), 3.96 (s, 3H), 3.36 (s, 3H), 2.40 (t, J = 12.8 Hz, 1H), 2.29 (dd, J = 12.2, 5.9 Hz, 1H), 1.71 (s, 3H).

### Example 44: Preparation of (2-((tert-butoxycarbonyl)amino)-5-(3-(3,4-difluoro-2-methoxyphenyl)-5-methyl-5-(trifluoromethyl)tetrahydrothiophene-2-carboxamido)phenyl)boronic acid (compound 220)

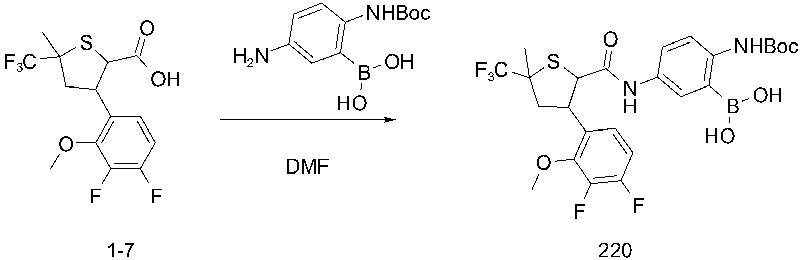

Compound 1-7 (30 mg, 0.08 mmol) was dissolved in anhydrous DMF (5 mL), (5-amino-2-((tert-butoxycarbonyl)amino)phenyl)boronic acid (60.48 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (5.91 mg, 0.01 mmol, yield: 13%).

MS: m/z = 591.2, [M+H]⁺.

¹H NMR (500 MHz, Chloroform ) δ 8.37 (s, 1H), 7.75 (s, 1H), 7.62 (s, 1H), 7.13 (s, 1H), 6.86 (d, *J =* 7.5 Hz, 2H), 4.58 (s, 1H), 3.93 (d, *J =* 12.5 Hz, 4H), 3.31 (s, 1H), 1.89 (s, 1H), 1.70 (s, 2H), 1.49 (s, 9H), 1.38 (s, 3H).

### Example 45: Preparation of (2R,3S,5R)-N-(3-carbamoyl-4-fluorophenyl)-3-(3,4-difluoro-2-methoxyphenyl)-5-methyl-5-(trifluoromethyl)tetrahydrothiophene-2-carboxamide (compound 146-A) and (2S,3R,5S)-N-(3-carbamoyl-4-fluorophenyl)-3-(3,4-difluoro-2-methoxyphenyl)-5-methyl-5-(trifluoromethyl)tetrahydrothiophene-2-carboxamide (compound 146-B)

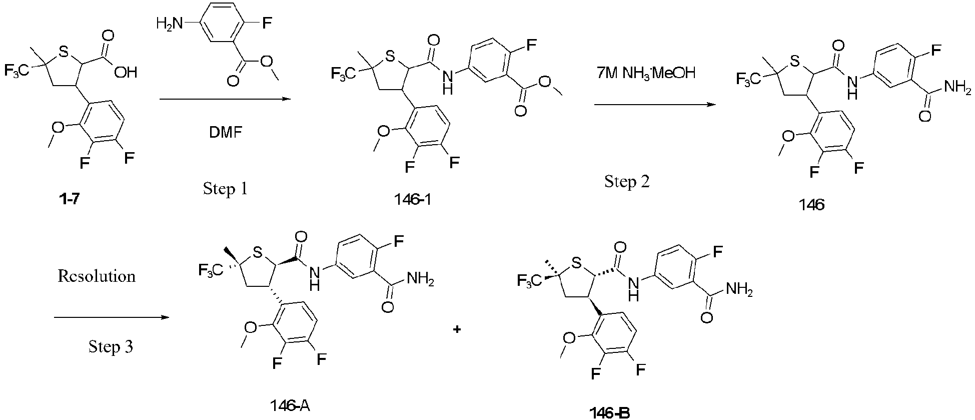

### Step 1: Preparation of (4-(3-(3,4-difluoro-2-methoxyphenyl)-5-methyl-5-(trifluoromethyl)tetrahydrofuran-2-carboxamido)-2-(methoxycarbonyl)phenyl)boronic acid (compound 146-1)

Compound 1-7 (50.0 mg, 0.14 mmol) was dissolved in anhydrous DMF (2 mL), methyl 5-amino-2-fluorobenzoate (71.0 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (44.1 mg, 0.09 mmol, yield: 62.10%). MS: m/z =508.1, [M+H]⁺.

### Step 2: Preparation of N-(3-carbamoyl-4-fluorophenyl)-3-(3,4-difluoro-2-methoxyphenyl)-5-methyl-5-(trifluoromethyl)tetrahydrothiophene-2-carboxamide (compound 146)

The product of step 1 (44.1 mg, 0.09 mmol) was dissolved in 7 M NH₃·MeOH (5 mL), and the mixture was stirred at room temperature. After the reaction was completed, the mixture was separated by Pre-HPLC to obtain the target compound (38.4 mg, 0.08 mmol, yield: 86.71%). MS: m/z =493.1, [M+H]⁺.

### Step 3: Preparation of (2R,3S,5R)-N-(3-carbamoyl-4-fluorophenyl)-3-(3,4-difluoro-2-methoxyphenyl)-5-methyl-5-(trifluoromethyl)tetrahydrothiophene-2-carboxamide (compound 146-A) and (2S,3R,5S)-N-(3-carbamoyl-4-fluorophenyl)-3-(3,4-difluoro-2-methoxyphenyl)-5-methyl-5-(trifluoromethyl)tetrahydrothiophene-2-carboxamide (compound 146-B)

The product of step 2 (38.4 mg, 0.08 mmol) was resolved to obtain the target compounds compound 146-A (16.2 mg, 0.03 mmol, yield: 42.19%) and compound 146-B (16.6 mg, 0.03 mmol, yield: 43.22%).

Compound 146-A:
MS: m/z = 493.1, [M+H]⁺.

¹H NMR (400 MHz, CDCl₃) δ 9.39 (s, 1H), 8.37 (ddd, J = 9.0, 4.4, 2.9 Hz, 1H), 8.00 (dd, J = 6.7, 2.8 Hz, 1H), 7.10 (dd, J = 11.4, 9.1 Hz, 1H), 7.02 - 6.96 (m, 1H), 6.91 (d, J = 13.3 Hz, 1H), 6.82 (td, J = 9.1, 7.4 Hz, 1H), 6.35 (s, 1H), 4.64 (d, J = 10.9 Hz, 1H), 4.33 - 4.23 (m, 1H), 4.03 (d, J = 2.4 Hz, 3H), 2.58 (t, J = 13.1 Hz, 1H), 2.25 (dd, J = 12.9, 6.1 Hz, 1H), 1.80 (s, 3H).

Compound 146-B:
MS: m/z = 493.1, [M+H]⁺.

¹H NMR (400 MHz, CDCl₃) δ 9.42 (s, 1H), 8.38 (ddd, J = 8.9, 4.4, 2.9 Hz, 1H), 8.00 (dd, J = 6.7, 2.8 Hz, 1H), 7.10 (dd, J = 11.4, 9.1 Hz, 1H), 7.02 - 6.96 (m, 1H), 6.91 (d, J = 13.8 Hz, 1H), 6.83 (dt, J = 16.4, 8.2 Hz, 1H), 6.36 (s, 1H), 4.65 (d, J = 10.9 Hz, 1H), 4.33 - 4.24 (m, 1H), 4.03 (d, J = 2.4 Hz, 3H), 2.57 (t, J = 13.1 Hz, 1H), 2.25 (dd, J = 12.9, 6.1 Hz, 1H), 1.80 (s, 3H).

### Example 46: Preparation of (2R,3S,5R)-3-(3,4-difluoro-2-methoxyphenyl)-N-(1-hydroxy-1,3-dihydrobenzo[c][1,2]oxaborol-6-yl)-5-methyl-5-(trifluoromethyl)tetrahydrothiophene-2-carboxamide (compound 31-A) and (2S,3R,5S)-3-(3,4-difluoro-2-methoxyphenyl)-N-(1-hydroxy-1,3-dihydrobenzo[c][1,2]oxaborol-6-yl)-5-methyl-5-(trifluoromethyl)tetrahydrothiophene-2-carboxamide (compound 31-B)

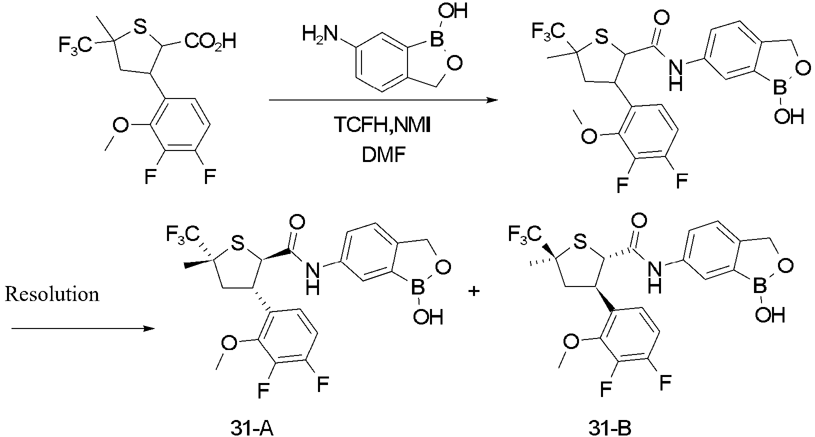

For the experimental procedures, reference may be made to Example 45.

### Compound 31-A:

MS: m/z = 488.1 [M+H]⁺,¹H NMR (400 MHz, CDCl3) δ 7.84 (s, 1H), 7.75 (d, J = 1.8 Hz, 1H), 7.59 (dd, J = 8.3, 2.0 Hz, 1H), 7.28 (d, J = 8.3 Hz, 1H), 7.01 (ddd, J = 5.6, 4.7, 2.2 Hz, 1H), 6.84 (td, J = 9.1, 7.4 Hz, 1H), 5.05 (s, 2H), 4.40 (t, J = 10.1 Hz, 1H), 4.21 - 4.11 (m, 1H), 3.97 (d, J = 2.6 Hz, 3H), 2.60 (t, J = 13.0 Hz, 1H), 2.23 (dd, J = 12.9, 6.0 Hz, 1H), 1.81 (s, 3H).

### Compound 31-B:

MS: m/z = 488.1 [M+H]⁺,¹H NMR (400 MHz, CDCl3) δ 7.84 (s, 1H), 7.75 (d, J = 1.8 Hz, 1H), 7.59 (dd, J = 8.3, 2.0 Hz, 1H), 7.30 - 7.26 (m, 1H), 7.03 - 6.98 (m, 1H), 6.84 (td, J = 9.1, 7.4 Hz, 1H), 5.05 (s, 2H), 4.41 (d, J = 10.8 Hz, 1H), 4.16 (ddd, J = 13.0, 11.0, 6.0 Hz, 1H), 3.97 (d, J = 2.6 Hz, 3H), 2.60 (t, J = 13.0 Hz, 1H), 2.22 (dd, J = 12.9, 6.0 Hz, 1H), 1.81 (s, 3H).

### Example 47: Preparation of (4-(3-(3,4-difluoro-2-methoxyphenyl)-5-methyl-5-(trifluoromethyl)tetrahydrothiophene-2-carboxamido)-2-hydroxy)pyridine (compound 170-A)

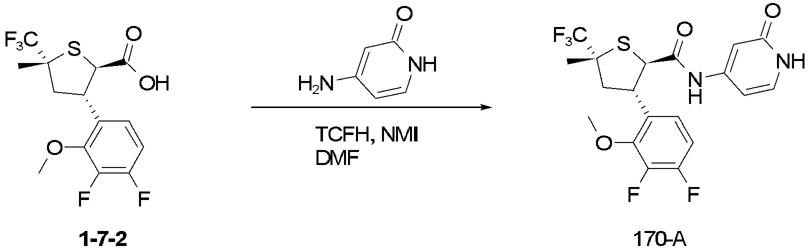

Compound 1-7-2 (50 mg, 0.14 mmol) was dissolved in anhydrous DMF (5 mL), 4-amino-2-hydroxypyridine (46 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (44 mg, 0.098 mmol, yield: 70%).

MS: m/z = 449.09, [M+H]⁺.

¹H NMR (400 MHz, DMSO) δ ¹H NMR (400 MHz, DMSO) δ 9.86 (s, 2H), 7.26 - 7.13 (m, 3H), 5.86 - 5.62 (m, 2H), 4.62 (d, *J =* 10.7 Hz, 1H), 4.35 - 4.21 (m, 1H), 3.98 (d, *J =* 1.6 Hz, 3H), 2.45 (t, *J =* 12.9 Hz, 1H), 2.32 (dd, *J =* 12.6, 6.0 Hz, 1H), 1.75 (s, 3H).

### Example 48: Preparation of (2R,3S,5R)-3-(3,4-difluoro-2-methoxyphenyl)-5-methyl-N-(1-methyl-6-oxo-1,6-dihydropyridin-3-yl)-5-(trifluoromethyl)tetrahydrothiophene-2-carboxamide (compound 178-A)

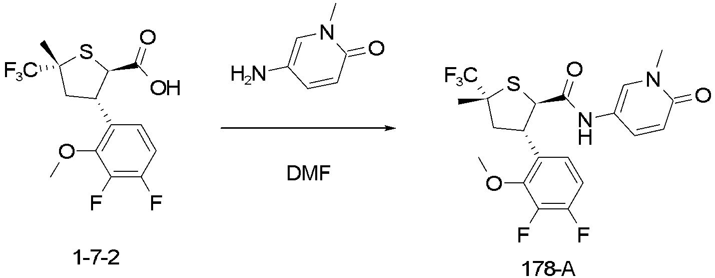

Compound 1-7-2 (30 mg, 0.08 mmol) was dissolved in anhydrous DMF (5 mL), 5-amino-1-methylpyridin-2(1H)-one (29.76 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (18.48 mg, 0.04 mmol, yield: 50%).

MS: m/z = 463.1, [M+H]⁺.

¹H NMR (400 MHz, DMSO) δ 10.71 (s, 1H), 7.84 (d, *J* = 9.3 Hz, 2H), 7.72 (d, *J* = 9.0 Hz, 2H), 7.24 - 7.14 (m, 2H), 4.63 (d, *J* = 10.7 Hz, 1H), 4.38 - 4.21 (m, 1H), 3.99 (d, *J* = 1.8 Hz, 3H), 2.46 (d, *J* = 12.9 Hz, 1H), 2.38 - 2.30 (m, 1H), 1.76 (s, 3H).

### Example 49: Preparation of (2R,3S,5R)-3-(3,4-difluoro-2-methoxyphenyl)-5-methyl-N-(1-methyl-6-oxo-1,6-dihydropyridin-3-yl)-5-(trifluoromethyl)tetrahydrothiophene-2-carboxamide (compound 178-B)

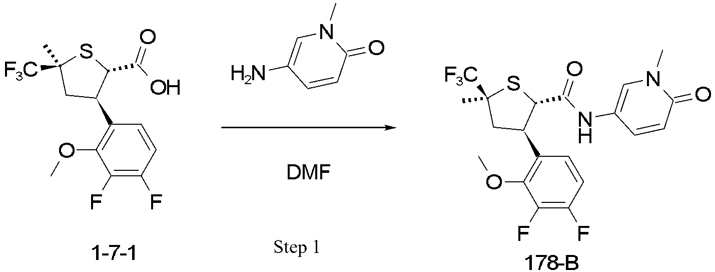

Compound 1-7-1 (36 mg, 0.1 mmol) was dissolved in anhydrous DMF (5 mL), 5-amino-1-methylpyridin-2(1H)-one (37.5 mg, 0.3 mmol) was added, and TCFH (150 mg, 0.50 mmol) and NMI (70 mg, 0.8 mmol) were added in an ice bath. The mixture was allowed to naturally warm to room temperature and then stirred for 1 h. After the reaction was completed, the mixture was separated by Pre-HPLC to obtain the target compound (28.6 mg, 0.062 mmol, yield: 62%).

MS: m/z = 463.1, [M+H]+.

1H NMR (400 MHz, CDCl3) δ 8.41 (d, J = 26.3 Hz, 1H), 8.22 (d, J = 2.2 Hz, 1H), 7.28 (s, 1H), 7.01 (d, J = 2.4 Hz, 1H), 6.90 - 6.75 (m, 1H), 6.59 (d, J = 9.4 Hz, 1H), 4.50 (d, J = 10.8 Hz, 1H), 4.15 (d, J = 2.9 Hz, 2H), 4.01 (d, J = 2.5 Hz, 3H), 3.55 (s, 3H), 2.54 (t, J = 13.0 Hz, 1H), 2.22 (dd, J = 12.9, 6.1 Hz, 1H), 1.78 (d, J = 4.5 Hz, 3H).

### Example 50: Preparation of (4-((2R,3S,5R)-3-(3,4-difluoro-2-methoxyphenyl)-5-methyl-5-(trifluoromethyl)tetrahydrothiophene-2-carboxamido)thiophen-2-yl)boronic acid (compound 48-A)

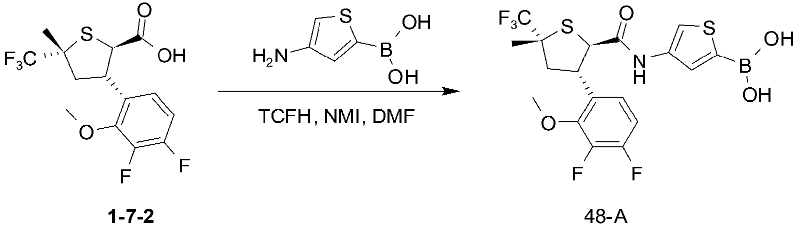

1-7-2 (50 mg, 0.14 mmol) was dissolved in anhydrous DMF (2 mL), (4-aminothiophen-2-yl)boronic acid (60.1 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (18.6 mg, 0.04 mmol, yield: 27.6%).

Ms: m/z = 482.2, [M+H]⁺.

¹H NMR (400 MHz, DMSO) δ 9.92 (s, 1H), 7.02 (d, *J=*2.0 Hz, 1H), 6.85 (d, *J=*2.0 Hz, 1H), 6.57(s, 1H), 6.32 (s, 1H), 4.20-4.07 (m, 3H), 3.83 (s, 3H), 3.46-3.43(m, 1H), 2.17-1.92(m, 2H), 1.38 (s, 3H).

### Example 51: Preparation of 4-((2R,3S,5R)-3-(3,4-difluoro-2-methoxyphenyl)-5-methyl-5-(trifluoromethyl)tetrahydrothiophene-2-carboxamido-2-d)picolinamide (compound 140-A)

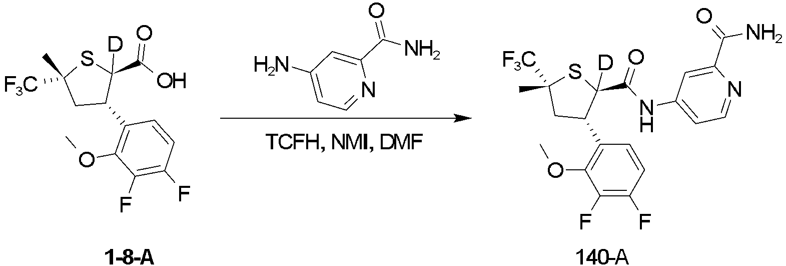

1-8-A (0.86 g, 2.41 mmol) was dissolved in anhydrous DMF (30 mL), methyl 4-aminopyridine-2-carboxylate (990.5 mg, 7.23 mmol) was added, and TCFH (3.37 g, 12.05 mmol) and NMI (1.58 g, 19.28 mmol) were added in an ice bath. The mixture was allowed to naturally warm to room temperature and then stirred. After the reaction was completed, the mixture was separated by Pre-HPLC to obtain the target compound (708.5 mg, 1.48 mmol, yield: 61.6%).

MS: m/z = 477.2, [M+H]⁺.

¹H NMR (400 MHz, DMSO) δ 9.92 (s, 1H), 8.85 (s, 1H), 8.22-8.18 (m, 3H), 7.02 (d, *J=2.0* Hz, 1H), 6.85 (d, *J=2.0* Hz, 1H), 5.82(s, 1H), 3.83 (s, 3H), 3.51-3.50(m, 1H), 2.17-1.92(m, 2H), 1.38 (s, 3H).

### Example 52: Preparation of (2R,3S,5R)-3-(3,4-difluoro-2-methoxyphenyl)-N-(1-hydroxy-3-oxo-2,3-dihydro-1H-benzo[c][1,2]azaborol-6-yl)-5-methyl-5-(trifluoromethyl)tetrahydrothiophene-2-carboxamide (compound 221-A)

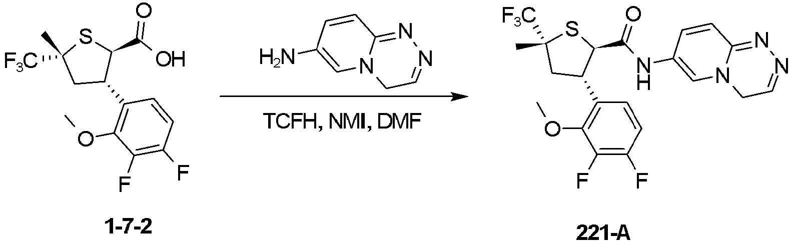

1-7-2 (50 mg, 0.14 mmol) was dissolved in anhydrous DMF (2 mL), 4H-pyrido[2,1-c][1,2,4]triazin-7-amine (62.2 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (22.6 mg, 0.05 mmol, yield: 33.1%).

Ms: m/z = 487.2, [M+H]⁺.

¹H NMR (400 MHz, DMSO) δ 9.30 (s, 1H), 9.15 (d, *J*=2.0 Hz, 1H), 7.52 (d, *J*=2.0 Hz, 1H), 7.02 (d, *J*=2.0 Hz, 1H), 6.85 (d, *J*=2.0 Hz, 1H), 5.82(s, 1H), 4.20-4.07 (m, 2H), 3.83 (s, 3H), 3.46-3.43(m, 1H), 2.17-1.92(m, 2H), 1.38 (s, 3H).

### Example 53: Preparation of 3-(3,4-difluoro-2-methoxyphenyl)-5-methyl-N-(3-methylisoxazol-4-yl)-5-(trifluoromethyl)tetrahydrothiophene-2-carboxamide (compound 197)

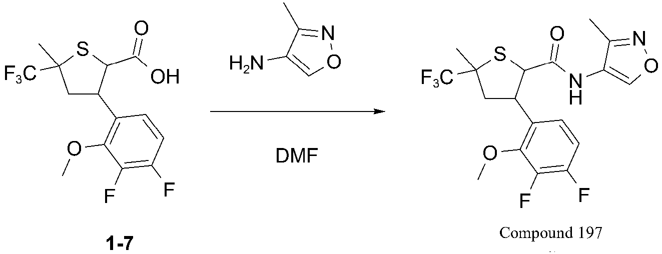

Compound 1-7 (50.0 mg, 0.14 mmol) was dissolved in anhydrous DMF (2 mL), 3-methylisoxazol-4-amine (41.2 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (31.3 mg, 0.07 mmol, yield: 51.30%).

MS: m/z = 437.1, [M+H]⁺.

¹H NMR (400 MHz, DMSO) δ 10.09 (s, 1H), 9.01 (s, 1H), 7.24 - 7.07 (m, 2H), 4.72 (d, J = 10.7 Hz, 1H), 4.27 (ddd, J = 13.0, 10.9, 6.1 Hz, 1H), 3.95 (d, J = 1.9 Hz, 3H), 2.42 (t, J = 12.9 Hz, 1H), 2.30 (dd, J = 12.6, 6.1 Hz, 1H), 2.15 (s, 3H), 1.72 (s, 3H).

### Example 54: Preparation of N-(6-aminopyridin-3-yl)-3-(3,4-difluoro-2-methoxyphenyl)-5-methyl-5-(trifluoromethyl)tetrahydrothiophene-2-carboxamide (compound 181)

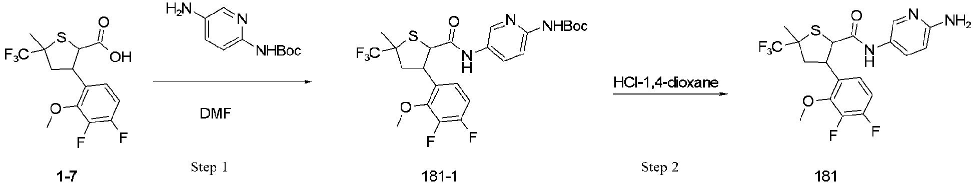

### Step 1: Preparation of tert-butyl (5-(3-(3,4-difluoro-2-methoxyphenyl)-5-methyl-5-(trifluoromethyl)tetrahydrothiophene-2-carboxamido)pyridin-2-yl)carbamate (compound 181-1)

Compound 1-7 (50.0 mg, 0.14 mmol) was dissolved in anhydrous DMF (2 mL), tert-butyl (5-aminopyridin-2-yl)carbamate (121.9 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (47.0 mg, 0.09 mmol, yield: 61.42%). MS: m/z = 548.2, [M+H]⁺.

### Step 2: Preparation of N-(6-aminopyridin-3-yl)-3-(3,4-difluoro-2-methoxyphenyl)-5-methyl-5-(trifluoromethyl)tetrahydrothiophene-2-carboxamide (compound 181)

The product of step 1 (47.0 mg, 0.09 mmol) was dissolved in 1,4-dioxane (5 mL), and the mixture was stirred at room temperature. After the reaction was completed, the mixture was separated by Pre-HPLC to obtain the target compound (32.4 mg, 0.07 mmol, yield: 80.43%).

MS: m/z = 448.1, [M+H]⁺.

¹H NMR (400 MHz, DMSO) δ 9.98 (s, 1H), 7.99 (d, J = 1.8 Hz, 1H), 7.46 (dd, J = 8.8, 2.2 Hz, 1H), 7.15 (dd, J = 16.6, 7.7 Hz, 2H), 6.38 (d, J = 8.8 Hz, 1H), 5.84 (s, 2H), 4.54 (d, J = 10.7 Hz, 1H), 4.29 - 4.18 (m, 1H), 3.95 (s, 3H), 2.39 (t, J = 12.9 Hz, 1H), 2.28 (dd, J = 12.5, 6.0 Hz, 1H), 1.71 (s, 3H).

### Example 55: Preparation of (4-((2R,3S,5R)-3-(3,4-difluoro-2-methoxyphenyl)-5-methyl-5-(trifluoromethyl)tetrahydrothiophene-2-carboxamido-2-d)phenyl)boronic acid (compound 83-A)

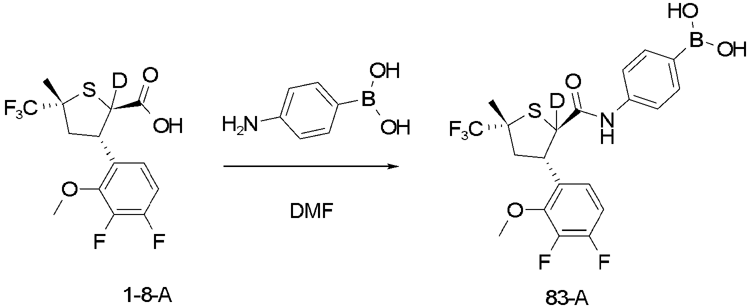

Compound 1-8-A (30 mg, 0.08 mmol) was dissolved in anhydrous DMF (5 mL), (4-aminophenyl)boronic acid (32.64 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (9.52 mg, 0.02 mmol, yield: 25%).

MS: m/z = 477.1, [M+H]⁺.

¹H NMR (400 MHz, DMSO) δ 10.29 (s, 1H), 7.94 (s, 2H), 7.71 (d, *J* = 8.3 Hz, 2H), 7.48 (d, *J =* 8.3 Hz, 2H), 7.24 - 7.16 (m, 2H), 4.30 (dd, *J =* 13.0, 6.0 Hz, 1H), 3.99 (s, 3H), 2.44 (t, *J =* 12.9 Hz, 1H), 2.33 (dd, *J =* 12.5, 6.2 Hz, 1H), 1.76 (s, 3H).

### Example 56: Preparation of (5-(3-(3,4-difluoro-2-methoxyphenyl)-5-methyl-5-(trifluoromethyl)tetrahydrothiophene-2-carboxamido)-2-chlorophenyl)boronic acid (compound 23-A)

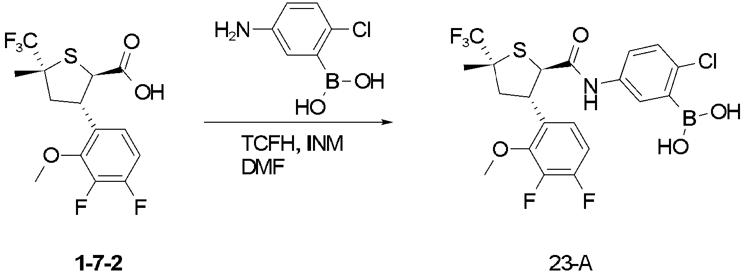

Compound 1-7-2 (50 mg, 0.14 mmol) was dissolved in anhydrous DMF (5 mL), 5-amino-2-chlorophenylboronic acid (72 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (52 mg, 0.10 mmol, yield: 73%). MS: m/z = 510.07, [M+H]⁺.

¹H NMR (400 MHz, DMSO) δ9.89 (s, 1H), 8.27 (d, *J* = 13.8 Hz, 2H), 7.86 - 7.62 (m, 2H), 7.56 - 7.43 (m, 3H), 4.61 (d, *J* = 10.7 Hz, 1H), 4.38 - 4.24 (m, 1H), 3.99 (d, *J =* 1.6 Hz, 3H), 2.45 (t, *J* = 12.9 Hz, 1H), 2.33 (dd, *J* = 12.6, 6.0 Hz, 1H), 1.76 (s, 3H).

### Example 57: Preparation of N-(6-aminopyridin-3-yl)-3-(3,4-difluoro-2-methoxyphenyl)-5-methyl-5-(trifluoromethyl)tetrahydrothiophene-2-carboxamide (compound 222)

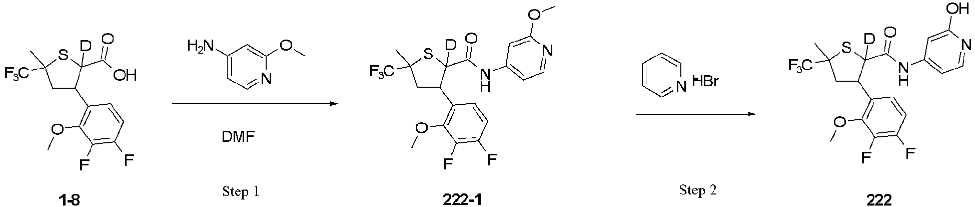

### Step 1: Preparation of 3-(3,4-difluoro-2-methoxyphenyl)-N-(2-methoxypyridin-4-yl)-5-methyl-5-(trifluoromethyl)tetrahydrothiophene-2-d-2-carboxamide (compound 222-1)

Compound 1-8 (50.0 mg, 0.14 mmol) was dissolved in anhydrous DMF (2 mL), 2-methoxypyridin-4-amine (52.1 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (35.8 mg, 0.08 mmol, yield: 55.25%). MS: m/z = 464.1, [M+H]⁺.

### Step 2: Preparation of 3-(3,4-difluoro-2-methoxyphenyl)-N-(2-hydroxypyridin-4-yl)-5-methyl-5-(trifluoromethyl)tetrahydrothiophene-2-d-2-carboxamide (compound 222)

The product of step 1 (35.8 mg, 0.08 mmol) was dissolved in DMF (5 mL), and pyridine hydrobromide (64.0 mg, 0.40 mmol) was added. The mixture was stirred at room temperature. After the reaction was completed, the mixture was separated by Pre-HPLC to obtain the target compound (27.9 mg, 0.06 mmol, yield: 77.62%).

MS: m/z = 450.1, [M+H]⁺.

¹H NMR (400 MHz, DMSO) δ 11.22 (s, 1H), 10.31 (s, 1H), 7.24 (d, J = 7.2 Hz, 1H), 7.21 - 7.11 (m, 2H), 6.60 (d, J = 1.6 Hz, 1H), 6.18 (dd, J = 7.2, 1.7 Hz, 1H), 4.23 (dd, J = 13.1, 6.1 Hz, 1H), 3.96 (s, 3H), 2.42 (t, J = 12.9 Hz, 1H), 2.30 (dd, J = 12.6, 6.1 Hz, 1H), 1.72 (s, 3H).

### Example 58: Preparation of 3-(3,4-difluoro-2-methoxyphenyl)-5-methyl-N-(tetrazolo[1,5-a]pyridin-7-yl)-5-(trifluoromethyl)tetrahydrothiophene-2-carboxamide (compound 203)

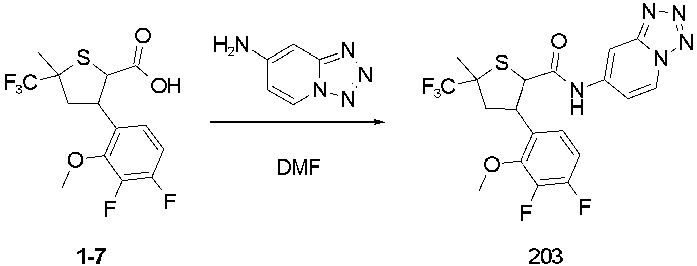

Compound 1-7 (50.0 mg, 0.14 mmol) was dissolved in anhydrous DMF (2 mL), tetrazolo[1,5-a]pyridin-7-amine (56.8 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (36.7 mg, 0.08 mmol, yield: 55.38%).

MS: m/z = 474.1, [M+H]⁺.

¹H NMR (400 MHz, DMSO) δ 11.05 (s, 1H), 9.17 (d, J = 7.4 Hz, 1H), 8.38 (d, J = 1.1 Hz, 1H), 7.26 - 7.15 (m, 3H), 4.64 (d, J = 10.6 Hz, 1H), 4.35 - 4.27 (m, 1H), 3.99 (s, 3H), 2.46 (s, 1H), 2.37 - 2.32 (m, 1H), 1.75 (s, 3H).

### Example 59: Preparation of (2R,3S,5R)-3-(3,4-difluoro-2-methoxyphenyl)-N-(1-hydroxy-3-oxo-2,3-dihydro-1H-benzo[c][1,2]azaborol-6-yl)-5-methyl-5-(trifluoromethyl)tetrahydrothiophene-2-carboxamide (compound a-2)

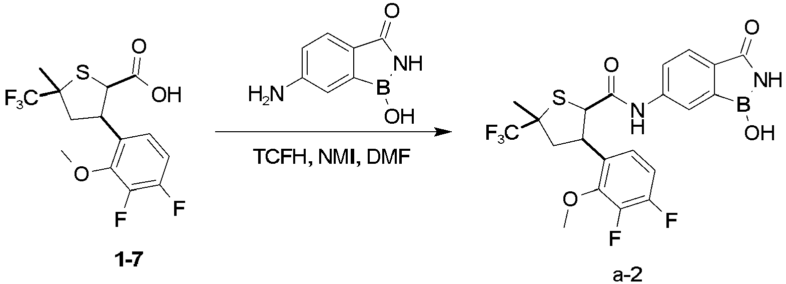

1-7 (50 mg, 0.14 mmol) was dissolved in anhydrous DMF (2 mL), 6-amino-1-hydroxy-1,2-dihydro-3H-benzo[c][1,2]azaborol-3-one (68.0 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (22.5 mg, 0.04 mmol, yield: 32.0%).

Ms: m/z = 501.2, [M+H]⁺.

¹H NMR (400 MHz, DMSO) δ 10.02 (s, 1H), 8.09 (d, *J=*2.0 Hz, 1H), 7.82-7.77 (m, 3H), 7.02 (d, *J=*2.0 Hz, 1H), 6.85 (d, *J=2.0* Hz, 1H), 4.22-4.02 (m, 2H), 3.82(s, 3H), 3.48-3.45(m, 1H), 2.17-1.92(m, 2H), 1.38 (s, 3H).

### Example 60: Preparation of (2R,3S,5R)-3-(3,4-difluoro-2-methoxyphenyl)-5-methyl-N-(6-oxo-1,6-dihydropyridazin-3-yl)-5-(trifluoromethyl)tetrahydrothiophene-2-carboxamide (compound a-3-A)

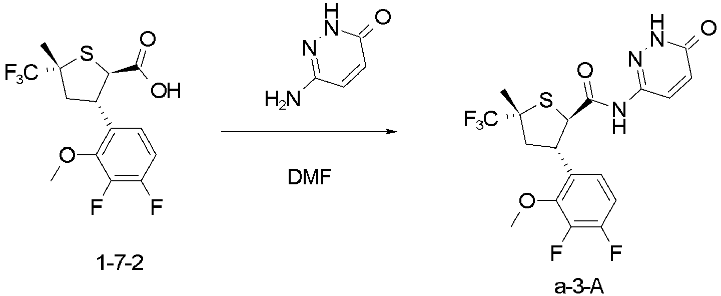

Compound 1-7-2 (30 mg, 0.08 mmol) was dissolved in anhydrous DMF (5 mL), 6-aminopyridazin-3(2H)-one (26.64 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (6.74 mg, 0.015 mmol, yield: 19%).

MS: m/z = 450.1, [M+H]⁺.

¹H NMR (400 MHz, DMSO) δ 12.71 (s, 1H), 10.82 (s, 1H), 7.84 (d, *J=* 10.1 Hz, 1H), 7.20 (t, *J* = 6.0 Hz, 2H), 6.90 (d, *J* = 10.1 Hz, 1H), 4.66 (d, *J =* 10.6 Hz, 1H), 4.28 (td, *J =* 12.8, 6.2 Hz, 1H), 3.97 (d, *J =* 1.7 Hz, 3H), 2.43 (t, *J =* 12.9 Hz, 1H), 2.33 (dd, *J =* 12.6, 6.2 Hz, 1H), 1.75 (s, 3H).

### Example 61: Preparation of (2R,3S,5R)-3-(3,4-difluoro-2-methoxyphenyl)-5-methyl-N-(4-(pentafluoro-16-thio)phenyl)-5-(trifluoromethyl)tetrahydrothiophene-2-carboxamide (a-4-A)

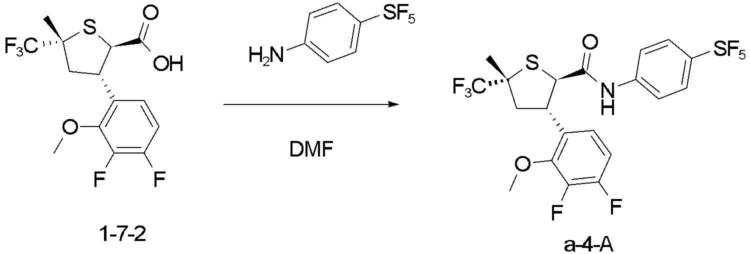

Compound 1-7-2 (30 mg, 0.08 mmol) was dissolved in anhydrous DMF (5 mL), 4-(pentafluoro-6-sulfonyl)aniline (52.56 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (11.14 mg, 0.02 mmol, yield: 25%).

MS: m/z = 558.2, [M+H]⁺.

¹H NMR (400 MHz, DMSO) δ 12.71 (s, 1H), 10.82 (s, 1H), 7.84 (d, J = 10.1 Hz, 1H), 7.20 (t, J = 6.0 Hz, 2H), 6.90 (d, J = 10.1 Hz, 1H), 4.66 (d, J = 10.6 Hz, 1H), 4.28 (td, J = 12.8, 6.2 Hz, 1H), 3.97 (d, J = 1.7 Hz, 3H), 2.43 (t, J = 12.9 Hz, 1H), 2.33 (dd, J = 12.6, 6.2 Hz, 1H), 1.75 (s, 3H).

### Example 62: Preparation of (4-(3-(3,4-difluoro-2-methoxyphenyl)-5-methyl-5-(trifluoromethyl)tetrahydrothiophene-2-carboxamido)-2-fluorophenyl)boronic acid (compound 88)

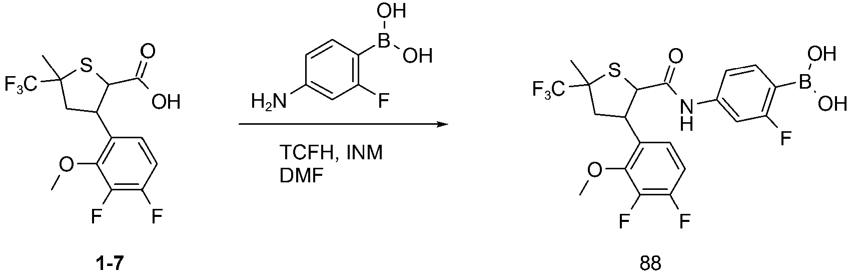

Compound 1-7 (50 mg, 0.14 mmol) was dissolved in anhydrous DMF (5 mL), (4-amino-2-fluorophenyl)boronic acid (65 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (48 mg, 0.097 mmol, yield: 69%). MS: m/z = 494.10, [M+H]⁺.

¹H NMR (400 MHz, DMSO) δ10.43 (s, 1H), 8.00 (d, J = 13.8 Hz, 2H), 7.56 - 7.32 (m, 2H), 7.26 - 7.13 (m, 3H), 4.62 (d, J = 10.7 Hz, 1H), 4.36 - 4.22 (m, 1H), 3.99 (d, J = 1.6 Hz, 3H), 2.46 (t, J = 12.9 Hz, 1H), 2.34 (dd, J = 12.6, 6.0 Hz, 1H), 1.75 (s, 3H).

### Example 63: Preparation of (4-(3-(3,4-difluoro-2-methoxyphenyl)-5-methyl-5-(trifluoromethyl)tetrahydrothiophene-2-carboxamido)-1-difluoromethyl-3-methyl)pyrazole (compound 199)

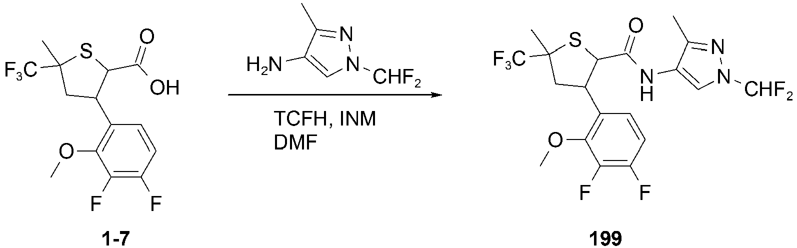

Compound 1-7 (50 mg, 0.14 mmol) was dissolved in anhydrous DMF (5 mL), 1-difluoromethyl-3-methyl-4-amino-1H-pyrazole (62 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (40 mg, 0.082 mmol, yield: 59%).

MS: m/z = 486.10, [M+H]⁺.

¹H NMR (400 MHz, DMSO) δ 9.84 (s, 1H), 8.27 (t, *J* = 114.7 Hz, 1H), 7.56 - 7.43 (m, 3H), 4.61 (d, *J* = 10.7 Hz, 1H), 4.38 - 4.24 (m, 1H), 3.99 (d, *J* = 1.6 Hz, 3H), 2.63 (s, 1H), 2.45 (t, *J =* 12.9 Hz, 1H), 2.33 (dd, *J* = 12.6, 6.0 Hz, 1H), 1.76 (s, 3H).

### Example 64: Preparation of (2R,3S,5R)-3-(3,4-difluoro-2-methoxyphenyl)-N-(1-hydroxy-1,3-dihydrobenzo[c][1,2]oxaborol-5-yl)-5-methyl-5-(trifluoromethyl)tetrahydrothiophene-2-carboxamide (compound 119-A)

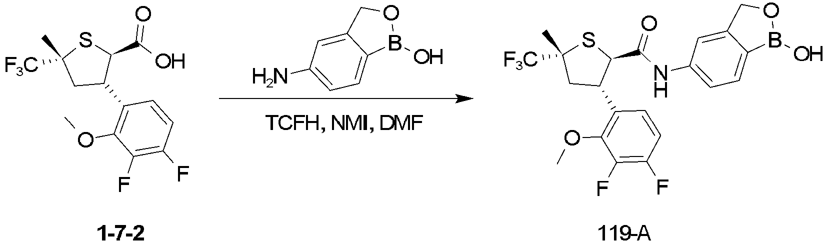

1-7-2 (50 mg, 0.14 mmol) was dissolved in anhydrous DMF (2 mL), 5-aminobenzo[c][1,2]oxaborol-1(3H)-ol (62.6 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (44.5 mg, 0.09 mmol, yield: 65.1 %).

Ms: m/z = 488.2, [M+H]⁺.

¹H NMR (400 MHz, DMSO) δ 10.02 (s, 1H), 7.88 (s, 1H), 7.84 (d, *J*=2.0 Hz, 1H), 7.62 (d, *J*=2.0 Hz, 1H), 7.02 (d, *J*=2.0 Hz, 1H), 6.85 (d, *J*=2.0 Hz, 1H), 5.12 (s, 2H), 4.22-4.02 (m, 2H), 3.82(s, 3H), 3.48-3.45(m, 1H), 2.17-1.92(m, 2H), 1.38 (s, 3H).

### Example 65: Preparation of (2R,3S,5R)-3-(3,4-difluoro-2-methoxyphenyl)-5-methyl-N-(3-sulfamoylphenyl)-5-(trifluoromethyl)tetrahydrothiophene-2-carboxamide (compound 223-A) and (2S,3R,5S)-3-(3,4-difluoro-2-methoxyphenyl)-5-methyl-N-(3-sulfamoylphenyl)-5-(trifluoromethyl)tetrahydrothiophene-2-carboxamide (compound 223-B)

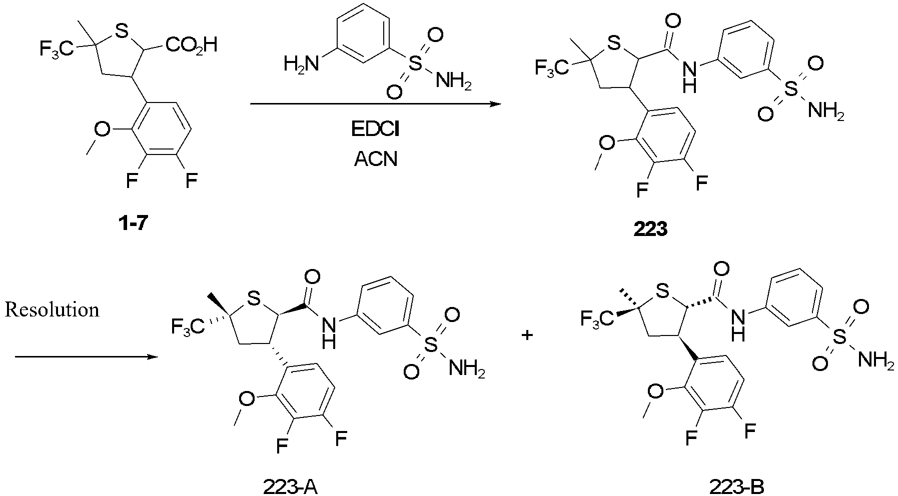

For the experimental procedures, reference may be made to Example 45.

Compound 223-A: MS: m/z = 511.1 [M+H]+, 1H NMR (400 MHz, DMSO) δ 10.60 (s, 1H), 8.07 (s, 1H), 7.64 (dd, J = 12.1, 10.5 Hz, 2H), 7.53 - 7.44 (m, 2H), 7.23 - 7.11 (m, 2H), 4.68 (t, J = 5.5 Hz, 1H), 4.61 (d, J = 10.7 Hz, 1H), 4.33 - 4.23 (m, 1H), 3.97 (d, J = 1.6 Hz, 3H), 2.76 (t, J = 6.4 Hz, 2H), 2.44 (t, J = 12.9 Hz, 1H), 2.31 (dd, J = 12.6, 6.1 Hz, 1H), 2.07 (s, 1H), 1.73 (s, 3H).

Compound 223-B: MS: m/z = 511.1[M+H]+, 1H NMR (400 MHz, CDCl3) δ 8.07 (s, 1H), 8.00 (t, J = 1.8 Hz, 1H), 7.74 (dd, J = 8.2, 1.2 Hz, 1H), 7.66 (d, J = 7.9 Hz, 1H), 7.45 (t, J = 8.0 Hz, 1H), 7.02 - 6.96 (m, 1H), 6.84 (dt, J = 16.4, 8.2 Hz, 1H), 4.92 (s, 2H), 4.42 (d, J = 10.8 Hz, 1H), 4.15 (ddd, J = 13.0, 10.9, 6.0 Hz, 1H), 2.60 (t, J = 13.0 Hz, 1H), 2.23 (dd, J = 12.9, 6.0 Hz, 1H), 1.81 (s, 3H).

### Example 66: Preparation of (2R,3S,5R)-3-(3,4-difluoro-2-methoxyphenyl)-N-(1-(ethylsulfonyl)-3-methyl-1H-pyrazol-4-yl)-5-methyl-5-(trifluoromethyl)tetrahydrothiophene-2-carboxamide (compound 224-A)

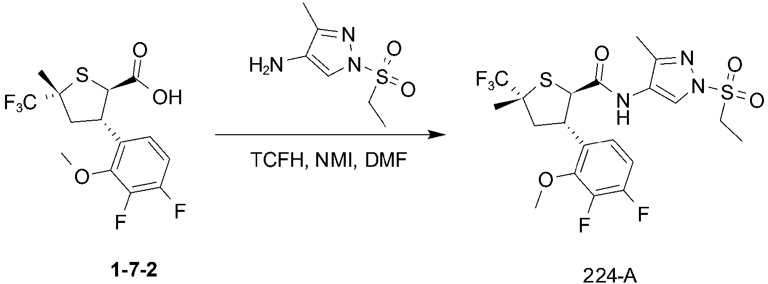

1-7-2 (50 mg, 0.14 mmol) was dissolved in anhydrous DMF (2 mL), 1-(ethylsulfonyl)-3-methyl-1H-pyrazol-4-amine (79.5 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (39.4 mg, 0.07 mmol, yield: 53.2%).

Ms: m/z = 528.2, [M+H]⁺.

¹H NMR (400 MHz, DMSO) δ 9.92 (s, 1H), 8.08 (s, 1H), 7.02 (d, *J*=2.0 Hz, 1H), 6.85 (d, *J=*2.0 Hz, 1H), 4.22-4.02 (m, 1H), 3.82(s, 3H), 3.48-3.45(m, 3H), 2.75 (s, 3H), 2.17-1.92(m, 2H), 1.38 (s, 3H), 1.22(t, *J=*4.0 Hz, 1H).

### Example 67: Preparation of (2-cyclopropyl-5-(3-(3,4-difluoro-2-methoxyphenyl)-5-methyl-5-(trifluoromethyl)tetrahydrothiophene-2-carboxamido)phenyl)boronic acid (compound 16)

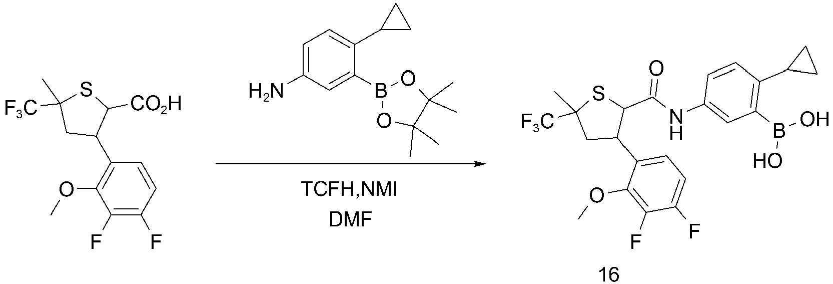

For the experimental procedures, reference may be made to Example 1.

MS: m/z = 516.3[M+H]+.

¹H NMR (400 MHz, CDCl3) δ 8.09 - 7.52 (m, 2H), 7.19 - 6.63 (m, 3H), 4.73 - 4.34 (m, 1H), 4.32 - 4.11 (m, 1H), 4.00 - 3.87 (m, 3H), 3.05 - 2.80 (m, 1H), 2.68 - 2.43 (m, 2H), 2.21 (tt, J = 16.4, 8.2 Hz, 1H), 1.85 - 1.73 (m, 3H), 1.31 (ddd, J = 27.6, 10.6, 4.7 Hz, 1H), 1.00 - 0.79 (m, 2H), 0.77 - 0.49 (m, 2H).

### Example 68: Preparation of (4-(3-(3,4-difluoro-2-methoxyphenyl)-5-methyl-5-(trifluoromethyl)tetrahydrothiophene-2-carboxamido)-2-(dimethylamino)phenyl)boronic acid (compound 98)

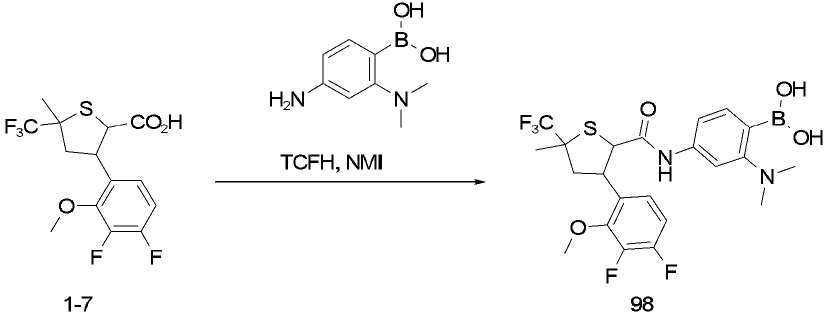

For the experimental procedures, reference may be made to Example 1.

MS: m/z = 519.3[M+H]⁺.

¹H NMR (400 MHz, CDCl3) δ 7.82 (d, J = 26.1 Hz, 3H), 7.02 (dd, J = 15.8, 7.6 Hz, 2H), 6.86 (dd, J = 16.5, 8.5 Hz, 1H), 4.41 (d, J = 10.8 Hz, 1H), 4.22 - 4.12 (m, 1H), 3.98 (d, J = 1.9 Hz, 3H), 2.68 (s, 6H), 2.59 (t, J = 13.1 Hz, 1H), 2.23 (dd, J = 12.9, 6.0 Hz, 1H), 1.81 (s, 3H).

### Example 69: Preparation of (5-(3-(3,4-difluoro-2-methoxyphenyl)-5-methyl-5-(trifluoromethyl)tetrahydrothiophene-2-carboxamido)-2-vinylphenyl)boronic acid (compound 15)

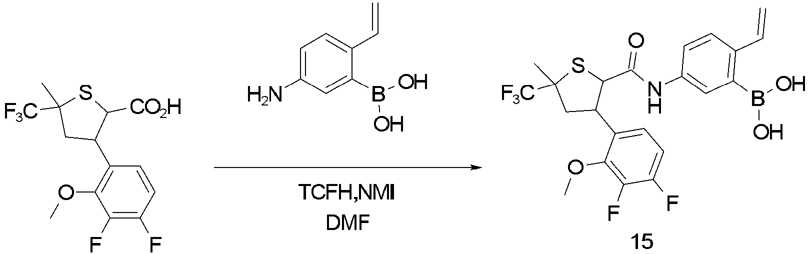

For the experimental procedures, reference may be made to Example 1.

MS: m/z = 502.3[M+H]⁺.

¹H NMR (400 MHz, CD3OD_SPE) δ 7.48 - 7.41 (m, 2H), 7.36 (d, J = 1.2 Hz, 1H), 7.10 (ddd, J = 8.2, 5.7, 2.2 Hz, 1H), 6.94 (td, J = 9.3, 7.6 Hz, 1H), 6.66 (dd, J = 17.5, 11.0 Hz, 1H), 5.61 - 5.53 (m, 1H), 5.16 (d, J = 11.3 Hz, 1H), 4.53 (t, J = 9.3 Hz, 1H), 4.32 (ddd, J = 13.2, 10.9, 6.0 Hz, 1H), 3.98 (d, J = 2.2 Hz, 3H), 2.52 (t, J = 13.0 Hz, 1H), 2.25 (dd, J = 12.7, 6.0 Hz, 1H), 1.76 (s, 3H).

### Example 70: Preparation of 3-((2R,3S,5R)-3-(3,4-difluoro-2-methoxyphenyl)-5-methyl-5-(trifluoromethyl)tetrahydrothiophene-2-carboxamido)benzenesulfonyl fluoride (compound a-70-A)

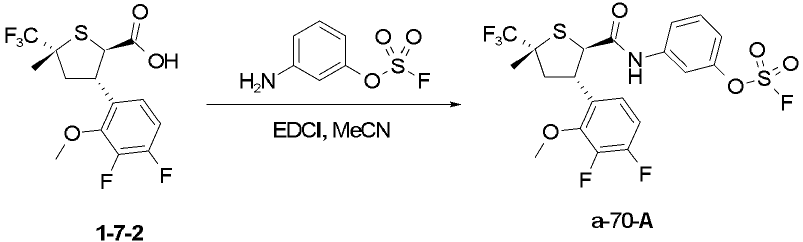

Compound 1-7-2 (100 mg, 0.28 mmol) was dissolved in anhydrous MeCN (5 mL), 3-aminophenyl fluorosulfate (54 mg, 0.28 mmol) was added, and EDCI (44 mg, 0.28 mmol) was added in an ice bath. The mixture was allowed to naturally warm to room temperature and then stirred. After the reaction was completed, the mixture was separated by Pre-HPLC to obtain the target compound (83 mg, 0.16 mmol, yield: 55.85%).

MS: m/z = 529.1, [M+H]+.

¹H NMR (400 MHz, DMSO) δ 10.67 (s, 1H), 7.87 (s, 1H), 7.49 (s, 2H), 7.19 (ddd, *J =* 22.7, 19.2, 5.8 Hz, 3H), 4.60 (d, *J* = 10.6 Hz, 1H), 4.33 - 4.23 (m, 1H), 3.96 (s, 3H), 2.43 (d, *J* = 12.9 Hz, 1H), 2.31 (dd, *J* = 12.4, 5.9 Hz, 1H), 1.73 (s, 3H).

### Example 71: Preparation of 3-(3,4-difluoro-2-methoxyphenyl)-5-methyl-N-(3-(S-methylsulfonimidoyl)phenyl)-5-(trifluoromethyl)tetrahydrothiophene-2-carboxamide (compound 225)

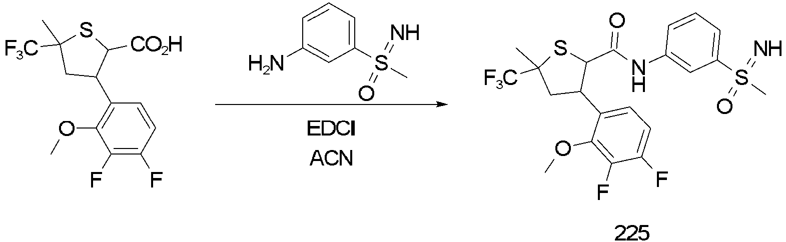

For the experimental procedures, reference may be made to Example 70.

MS: m/z = 509.1[M+H]⁺, ¹H NMR (400 MHz, DMSO) δ 7.30 - 7.08 (m, 3H), 7.00 (dt, J = 5.8, 2.0 Hz, 1H), 6.84 - 6.58 (m, 2H), 5.73 (d, J = 6.8 Hz, 2H), 4.56 (dd, J = 11.1, 4.4 Hz, 1H), 4.08 - 3.97 (m, 1H), 3.88 (dd, J = 32.2, 1.4 Hz, 3H), 3.28 (d, J = 13.2 Hz, 3H), 2.35 - 2.15 (m, 2H), 1.65 (d, J = 10.7 Hz, 3H).

### Example 72: Preparation of 3-(3,4-difluoro-2-methoxyphenyl)-5-methyl-N-(3-(pentafluorosulfanyl)phenyl)-5-(trifluoromethyl)tetrahydrothiophene-2-carboxamide (compound a-5)

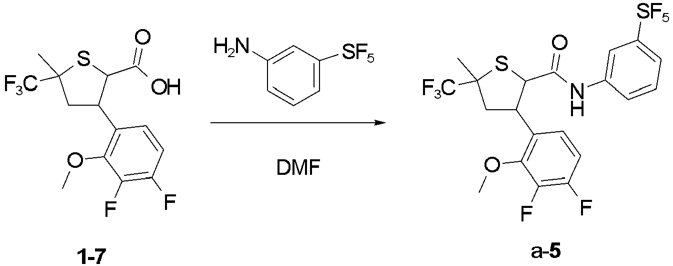

Compound 1-7 (50.0 mg, 0.14 mmol) was dissolved in anhydrous DMF (2 mL), (3-aminophenyl)sulfur pentafluoride (92.1 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (44.8 mg, 0.08 mmol, yield: 57.48%).

MS: m/z = 558.0, [M+H]⁺.

¹H NMR (400 MHz, DMSO) δ 10.80 (s, 1H), 8.92 (d, J = 7.4 Hz, 1H), 8.13 (d, J = 1.1 Hz, 1H), 7.04 - 6.96 (m, 2H), 6.98 - 6.80 (m, 2H), 4.39 (d, J = 10.6 Hz, 1H), 4.07 (td, J = 13.0, 6.1 Hz, 1H), 3.74 (d, J = 1.7 Hz, 3H), 2.21 (s, 1H), 2.09 (dt, J = 11.5, 5.8 Hz, 1H), 1.50 (s, 3H).

### Example 73: Preparation of 3-(3,4-difluoro-2-methoxyphenyl)-N-(2-(2,3-dihydroxypropoxy)pyridin-4-yl)-5-methyl-5-(trifluoromethyl)tetrahydrothiophene-2-carboxamide (compound 191)

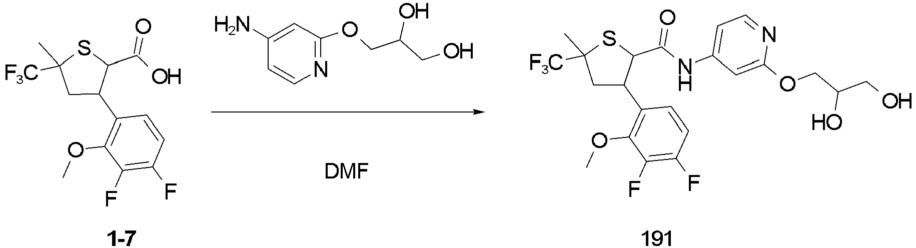

Compound 1-7 (50.0 mg, 0.14 mmol) was dissolved in anhydrous DMF (2 mL), 3-((4-aminopyridin-2-yl)oxy)propane-1,2-diol (77.3 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (44.8 mg, 0.08 mmol, yield: 57.48%).

MS: m/z = 523.1, [M+H]⁺, ¹H NMR (400 MHz, DMSO) δ 7.57 (d, J = 5.7 Hz, 1H), 7.35 - 7.23 (m, 1H), 7.14 - 7.00 (m, 1H), 6.16 (dd, J = 7.9, 3.4 Hz, 1H), 5.97 (d, J = 14.6 Hz, 2H), 5.82 - 5.74 (m, 1H), 4.98 - 4.85 (m, 1H), 4.65 (td, J = 11.5, 3.7 Hz, 1H), 4.11 = 3.97 (m, 3H), 3.94 (d, J = 1.8 Hz, 3H), 3.90 - 3.78 (m, 1H), 2.41 - 2.20 (m, 2H), 1.67 (s, 3H).

### Example 74: Preparation of 3-(3,4-difluoro-2-methoxyphenyl)-5-methyl-N-(tetrazolo[1,5-a]pyridin-6-yl)-5-(trifluoromethyl)tetrahydrothiophene-2-carboxamide (compound 202)

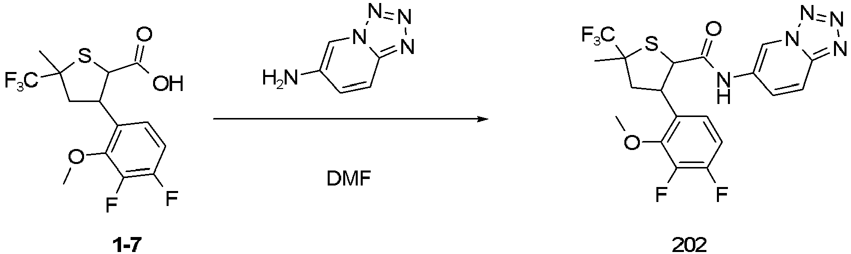

Compound 1-7 (50.0 mg, 0.14 mmol) was dissolved in anhydrous DMF (2 mL), tetrazolo[1,5-a]pyridin-6-amine (56.7 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (31.0 mg, 0.07 mmol, yield: 46.69%).

MS: m/z = 474.1, [M+H]⁺, ¹H NMR (400 MHz, DMSO) δ 10.95 (s, 1H), 9.64 (s, 1H), 8.17 (d, J = 9.6 Hz, 1H), 7.64 (dd, J = 9.6, 1.6 Hz, 1H), 7.28 - 7.05 (m, 2H), 4.65 (d, J = 10.6 Hz, 1H), 4.32 (td, J = 13.0, 6.1 Hz, 1H), 3.99 (d, J = 1.7 Hz, 3H), 2.45 (s, 1H), 2.34 (dd, J = 12.6, 6.0 Hz, 1H), 1.74 (s, 3H).

### Example 75: Preparation of (2R,3S,5R)-3-(3,4-difluoro-2-methoxyphenyl)-N-(1-hydroxy-3-oxo-1,3-dihydrobenzo[c][1,2]oxaborol-6-yl)-5-methyl-5-(trifluoromethyl)tetrahydrothiophene-2-carboxamide (compound a-6-A)

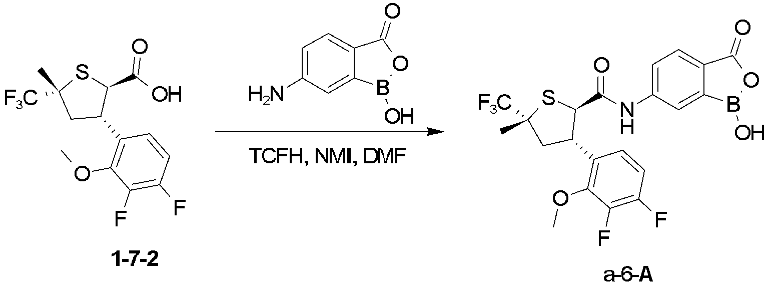

1-7-2 (50 mg, 0.14 mmol) was dissolved in anhydrous DMF (2 mL), 6-amino-1-hydroxybenzo[c][1,2]oxaborol-3(1H)-one (68.5 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (33.2 mg, 0.07 mmol, yield: 42.2%).

Ms: m/z = 502.2, [M+H]⁺,¹H NMR (400 MHz, DMSO) δ 10.02 (s, 1H), 8.27 (d, J=1.6 Hz, 1H), 7.85 (d, *J*=1.6 Hz, 1H), 7.79 (s, 1H), 7.02 (d, *J*=2.0 Hz, 1H), 6.85 (d, *J*=2.0 Hz, 1H), 4.22-4.02 (m, 2H), 3.82(s, 3H), 3.47-3.46(m, 1H), 2.50 (s, 3H), 2.16-1.92(m, 2H), 1.38 (s, 3H).

### Example 76: Preparation of (4-(3-(3,4-difluoro-2-methoxyphenyl)-5-methyl-5-(trifluoromethyl)tetrahydrothiophene-2-carboxamido)-2-methylsulfonyl)pyridine (compound 192)

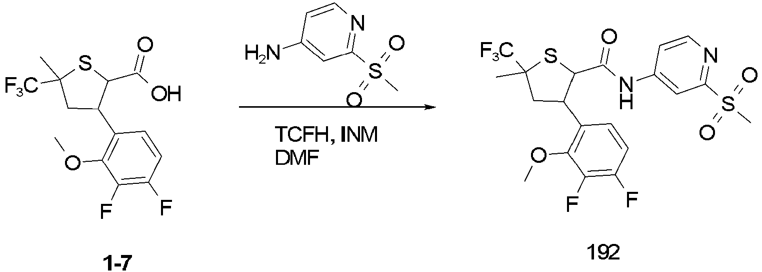

Compound 1-7 (50 mg, 0.14 mmol) was dissolved in anhydrous DMF (5 mL), 2-methylsulfonyl-4-aminopyridine (72 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (53 mg, 0.10 mmol, yield: 74%). MS: m/z = 511.07, [M+H]⁺, ¹H NMR (400 MHz, DMSO) δ 9.56 (m, 2H), 7.86 - 7.62 (m, 2H), 7.56 - 7.43 (m, 2H), 4.61 (d, *J =* 10.7 Hz, 1H), 4.38 - 4.24 (m, 1H), 3.99 (d, *J =* 1.6 Hz, 3H), 3.41 (s, 3H), 2.45 (t, *J =* 12.9 Hz, 1H), 2.33 (dd, *J =* 12.6, 6.0 Hz, 1H), 1.75 (s, 3H).

### Example 77: Preparation of (4-(3-(3,4-difluoro-2-methoxyphenyl)-5-methyl-5-(trifluoromethyl)tetrahydrothiophene-2-carboxamido)-2-nitro)pyridine (compound a-7)

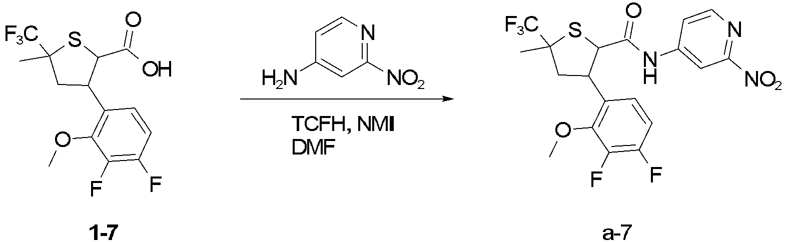

Compound 1-7 (50 mg, 0.14 mmol) was dissolved in anhydrous DMF (5 mL), 2-nitro-4-aminopyridine (58 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (35 mg, 0.073 mmol, yield: 52%).

MS: m/z = 478.08, [M+H]⁺, ¹H NMR (400 MHz, DMSO) δ 9.87 (s, 1H), 8.58 - 8.48 (m, 1H), 7.96 - 7.72 (m, 2H), 7.54 - 7.41 (m, 2H), 4.61 (d, *J =* 10.7 Hz, 1H), 4.38 - 4.24 (m, 1H), 3.99 (d, *J =* 1.6 Hz, 3H), 2.43 (t, *J =* 12.9 Hz, 1H), 2.31 (dd, *J =* 12.6, 6.0 Hz, 1H), 1.75 (s, 3H).

### Example 78: Preparation of (2R,3S,5R)-3-(3,4-difluoro-2-methoxyphenyl)-N-(1-hydroxy-3-oxo-1,3-dihydrobenzo[c][1,2]oxaborol-5-yl)-5-methyl-5-(trifluoromethyl)tetrahydrothiophene-2-carboxamide (compound a-8-A)

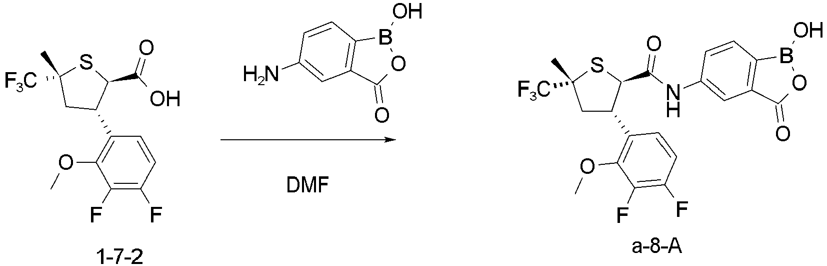

Compound 1-7-2 (30 mg, 0.08 mmol) was dissolved in anhydrous DMF (5 mL), 5-amino-1-hydroxybenzo[c][1,2]oxaborol-3(1H)-one (39.12 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (10.04 mg, 0.02 mmol, yield: 25%).

MS: m/z = 502.2, [M+H]⁺, ¹H NMR (400 MHz, DMSO) δ 12.71 (s, 1H), 10.82 (s, 1H), 7.84 (d, J = 10.1 Hz, 1H), 7.20 (t, J = 6.0 Hz, 2H), 6.90 (d, J = 10.1 Hz, 1H), 4.66 (d, J = 10.6 Hz, 1H), 4.28 (td, J = 12.8, 6.2 Hz, 1H), 3.97 (d, J = 1.7 Hz, 3H), 2.43 (t, J = 12.9 Hz, 1H), 2.33 (dd, J = 12.6, 6.2 Hz, 1H), 1.75 (s, 3H).

### Example 80: Preparation of N-(3-(N-cyanosulfamoyl)phenyl)-3-(3,4-difluoro-2-methoxyphenyl)-5-methyl-5-(trifluoromethyl)tetrahydrothiophene-2-carboxamide (compound a-9)

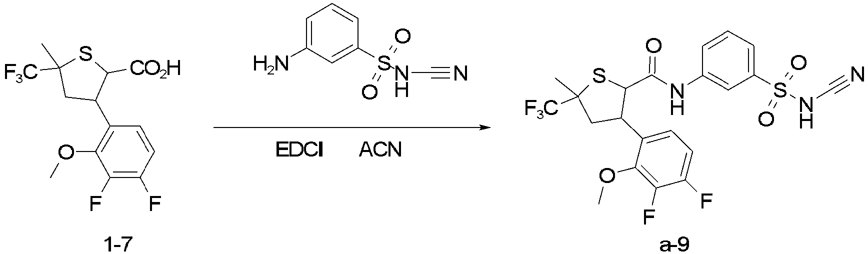

For the experimental procedures, reference may be made to Example 70.

MS: m/z = 553.1[M+H]⁺, ¹H NMR (400 MHz, CDCl3) δ 9.10 (s, 1H), 7.68 - 7.38 (m, 3H), 7.08 - 6.59 (m, 4H), 4.49 (d, J = 10.3 Hz, 1H), 4.28 - 4.16 (m, 1H), 3.88 (d, J = 1.4 Hz, 3H), 2.44 (d, J = 6.3 Hz, 1H), 2.21 - 2.11 (m, 1H), 1.69 (s, 4H).

### Example 81: Preparation of 3-(3,4-difluoro-2-methoxyphenyl)-N-(2-((dimethyl(oxo)-16-sulfanylidene)amino)pyridin-4-yl)-5-methyl-5-(trifluoromethyl)tetrahydrothiophene-2-carboxamide (compound a-10)

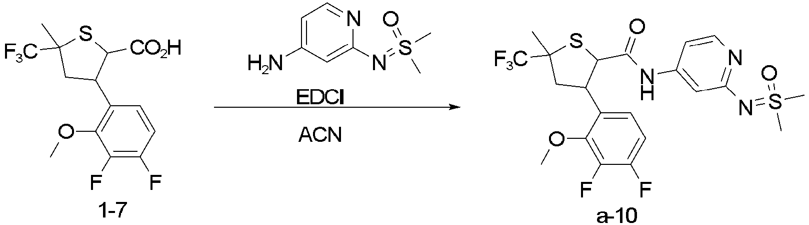

For the experimental procedures, reference may be made to Example 70.

MS: m/z = 524.1[M+H]⁺, ¹H NMR (400 MHz, CDCl3) δ 8.20 (s, 1H), 8.02 (d, J = 5.9 Hz, 1H), 7.08 (dd, J = 5.8, 1.7 Hz, 1H), 7.00 (ddd, J = 7.9, 5.5, 2.0 Hz, 1H), 6.90 - 6.81 (m, 2H), 4.43 (d, J = 10.8 Hz, 1H), 4.17 (ddd, J = 13.0, 10.9, 6.1 Hz, 1H), 3.97 (d, J = 2.5 Hz, 3H), 3.33 (s, 6H), 2.55 (t, J = 13.0 Hz, 1H), 2.21 (dd, J = 12.9, 6.1 Hz, 1H), 1.77 (d, J = 10.7 Hz, 3H).

### Example 82: Preparation of (2R,3S,5R)-3-(3,4-difluoro-2-methoxyphenyl)-N-(4-((dimethyl(oxo)-16-sulfonamido)amino)phenyl)-5-methyl-5-(trifluoromethyl)tetrahydrothiophene-2-carboxamide (compound a-11-A)

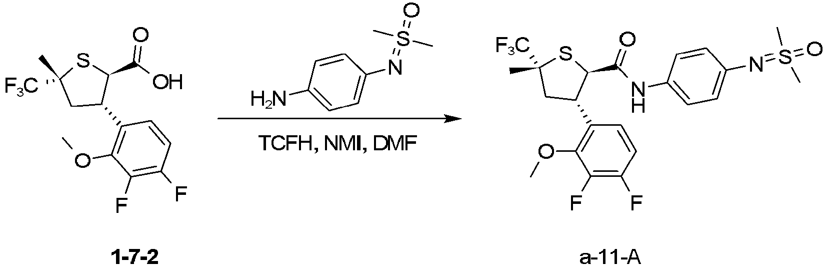

1-7-2 (50 mg, 0.14 mmol) was dissolved in anhydrous DMF (2 mL), ((4-aminophenyl)imino)dimethyl-6-thione (77.3 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (25.1 mg, 0.05 mmol, yield: 34.3%).

Ms: m/z = 523.2, [M+H]⁺,¹H NMR (400 MHz, DMSO) δ 10.02 (s, 1H), 7.56 (d, *J*=1.6 Hz, 2H), 7.34 (d, *J=*2.0 Hz, 2H), 7.02 (d, *J=*2.0 Hz, 1H), 6.85 (d, *J=*2.0 Hz, 1H), 4.22-4.02 (m, 1H), 3.82(s, 3H), 3.47-3.46(m, 1H), 2.50 (s, 3H), 2.16-1.92(m, 2H), 1.38 (s, 3H).

### Example 83: Preparation of 3-(3,4-difluoro-2-methoxyphenyl)-N-(1,4-dioxo-1,2,3,4-tetrahydrophthalazin-6-yl)-5-methyl-5-(trifluoromethyl)tetrahydrothiophene-2-carboxamide (compound a-12)

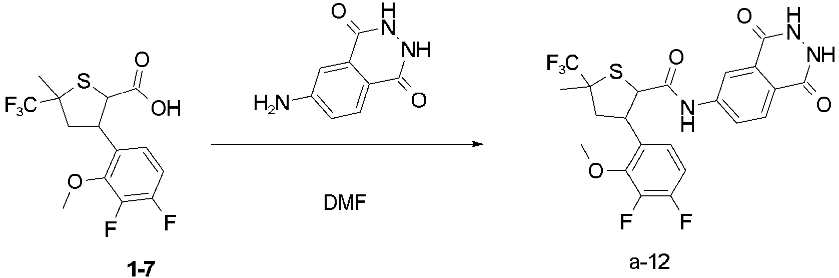

Compound 1-7 (50.0 mg, 0.14 mmol) was dissolved in anhydrous DMF (2 mL), 6-amino-2,3-dihydrophthalazine-1,4-dione (41.2 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (31.0 mg, 0.08 mmol, yield: 55.03%).

MS: m/z = 516.1, [M+H]⁺, ¹H NMR (400 MHz, DMSO) δ 11.97 (s, 1H), 7.87 (d, J = 8.7 Hz, 1H), 7.47 (t, J = 6.7 Hz, 1H), 7.24 - 7.17 (m, 1H), 7.04 (dd, J = 8.7, 2.0 Hz, 1H), 6.46 - 6.37 (m, 3H), 5.12 (d, J = 10.9 Hz, 1H), 4.28 (td, J = 12.2, 6.2 Hz, 1H), 3.97 (d, J = 1.4 Hz, 3H), 2.47 - 2.32 (m, 2H), 1.78 (s, 3H).

### Example 84: Preparation of 3-(3,4-difluoro-2-methoxyphenyl)-N-(4-((dimethyl(oxo)-16-sulfonamido)amino)-3-fluorophenyl)-5-methyl-5-(trifluoromethyl)tetrahydrothiophene-2-carboxamide (compound a-13)

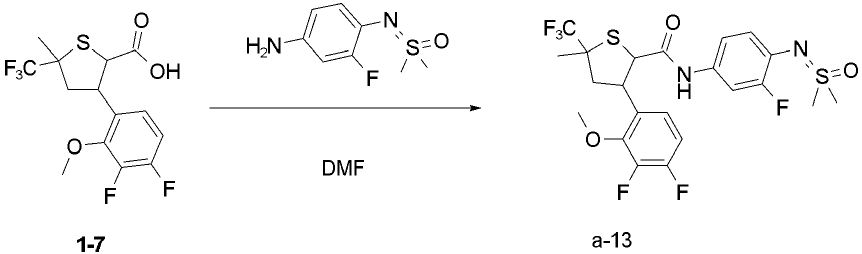

Compound 1-7 (50.0 mg, 0.14 mmol) was dissolved in anhydrous DMF (2 mL), (4-amino-2-fluorophenyl)dimethyl-6-sulfonic acid (84.9 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (34.0 mg, 0.06 mmol, yield: 45.01%).

MS: m/z = 541.1, [M+H]⁺, ¹H NMR (400 MHz, DMSO) δ 10.27 (s, 1H), 7.47 - 7.38 (m, 1H), 7.20 - 7.12 (m, 2H), 7.05 - 6.99 (m, 2H), 4.55 (d, J = 10.8 Hz, 1H), 4.25 (td, J = 12.8, 6.1 Hz, 1H), 3.96 (d, J = 1.3 Hz, 3H), 3.18 (s, 6H), 2.45 - 2.24 (m, 2H), 1.72 (s, 3H).

### Example 85: Preparation of 4-(3-(3,4-difluoro-2-methoxyphenyl)-5-methyl-5-(trifluoromethyl)tetrahydrothiophene-2-carboxamido)phenylsulfamate (compound a-14)

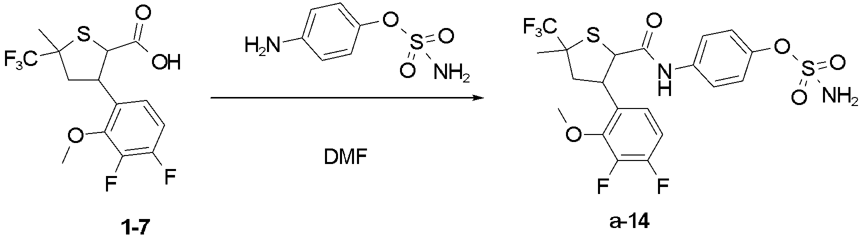

Compound 1-7 (50.0 mg, 0.14 mmol) was dissolved in anhydrous DMF (2 mL), 4-aminophenyl sulfamate (79.0 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (31.0 mg, 0.06 mmol, yield: 42.13%).

MS: m/z = 527.1, [M+H]⁺, ¹H NMR (400 MHz, DMSO) δ 10.38 (s, 1H), 7.89 (s, 2H), 7.54 (d, J = 9.0 Hz, 2H), 7.16 (t, J = 9.0 Hz, 4H), 4.59 (d, J = 10.7 Hz, 1H), 4.27 (td, J = 12.8, 6.1 Hz, 1H), 3.96 (d, J = 1.6 Hz, 3H), 2.42 (t, J = 12.9 Hz, 1H), 2.31 (dd, J = 12.6, 6.1 Hz, 1H), 1.73 (s, 3H).

### Example 86: Preparation of 4-((2R,3S,5R)-3-(3,4-difluoro-2-methoxyphenyl)-5-methyl-5-(trifluoromethyl)tetrahydrothiophene-2-carboxamido)-2-hydroxybenzoic acid (compound 226-A)

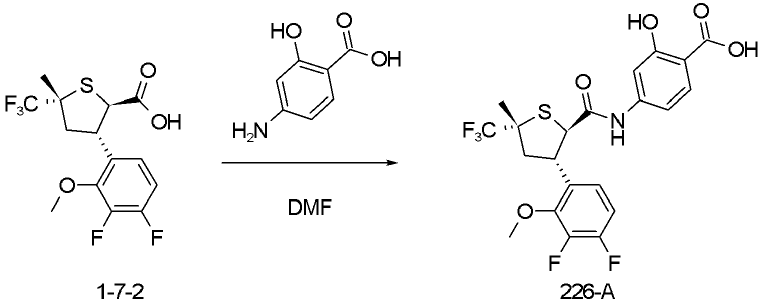

Compound 1-7-2 (30 mg, 0.08 mmol) was dissolved in anhydrous DMF (5 mL), 4-amino-2-hydroxybenzoic acid (36.72 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (9.82 mg, 0.02 mmol, yield: 25%). MS: m/z = 492.2, [M+H]⁺, ¹H NMR (400 MHz, DMSO) δ 10.17 (s, 1H), 7.56 (d, *J* = 8.4 Hz, 1H), 7.23 - 7.16 (m, 2H), 6.97 (s, 1H), 6.76 (dd, *J* = 8.4, 1.4 Hz, 1H), 4.62 (d, *J =* 10.7 Hz, 1H), 4.29 (td, *J =* 12.6, 6.2 Hz, 1H), 3.99 (s, 3H), 2.42 (t, *J =* 12.9 Hz, 1H), 2.32 (dd, *J =* 12.6, 6.3 Hz, 1H), 1.75 (s, 3H).

### Example 87: Preparation of 3-(3,4-difluoro-2-methoxyphenyl)-N-(3-((dimethyl(oxo)-16-sulfonamido))amino)-4-fluorophenyl)-5-methyl-5-(trifluoromethyl)tetrahydrothiophene-2-carboxamide (compound a-15)

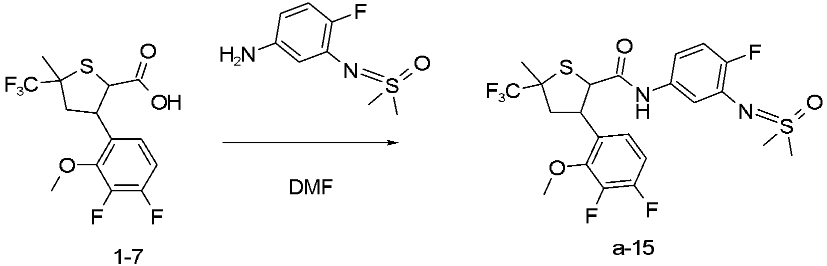

Compound 1-7 (30 mg, 0.08 mmol) was dissolved in anhydrous DMF (5 mL), (5-amino-2-fluorophenyl)imino)dimethyl-6-thione (48.48 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (10.81 mg, 0.02 mmol, yield: 25%).

MS: m/z = 541.3, [M+H]⁺, ¹H NMR (400 MHz, DMSO) δ 10.20 (s, 1H), 7.33 (d, *J =* 5.9 Hz, 1H), 7.22 - 7.16 (m, 2H), 7.12 (d, *J =* 8.2 Hz, 1H), 7.01 (t, *J =* 9.7 Hz, 1H), 4.58 (d, *J* = 10.7 Hz, 1H), 4.29 (td, *J =* 12.5, 6.4 Hz, 1H), 3.99 (s, 3H), 3.26 (s, 6H), 2.42 (t, *J =* 12.8 Hz, 1H), 2.32 (dd, *J =* 12.5, 6.2 Hz, 1H), 1.75 (s, 3H).

### Example 88: Preparation of (4-(3-(3,4-difluoro-2-methoxyphenyl)-5-methyl-5-(trifluoromethyl)tetrahydrothiophene-2-carboxamido)-2-amino)pyridine (compound 175)

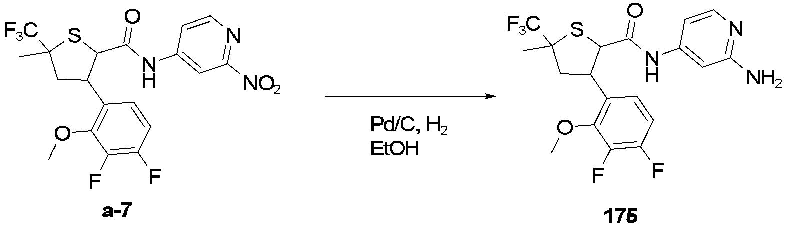

Compound a-7 (20 mg, 0.14 mmol) was dissolved in absolute EtOH (5 mL), and Pd/C (20 mg) was added. The mixture was stirred at room temperature under hydrogen atmosphere. After the reaction was completed, the mixture was separated by Pre-HPLC to obtain the target compound (10 mg, 0.022 mmol, yield: 53%).

MS: m/z = 448.10, [M+H]⁺, ¹H NMR (400 MHz, DMSO) δ 9.87 (s, 1H), 7.88 - 7.64 (m, 2H), 7.56 - 7.43 (m, 3H), 4.61 (d, *J* = 10.7 Hz, 1H), 4.38 - 4.24 (m, 1H), 3.98 (d, *J* = 1.6 Hz, 3H), 2.45 (t, *J* = 12.9 Hz, 1H), 2.33 (dd, *J =* 12.6, 6.0 Hz, 1H), 1.98 (s, 2H), 1.75 (s, 3H).

### Example 89: Preparation of 4-(3-(3,4-difluoro-2-methoxyphenyl)-4,5-dimethyl-5-(trifluoromethyl)tetrahydrothiophene-2-carboxamido)picolinamide (compound 136)

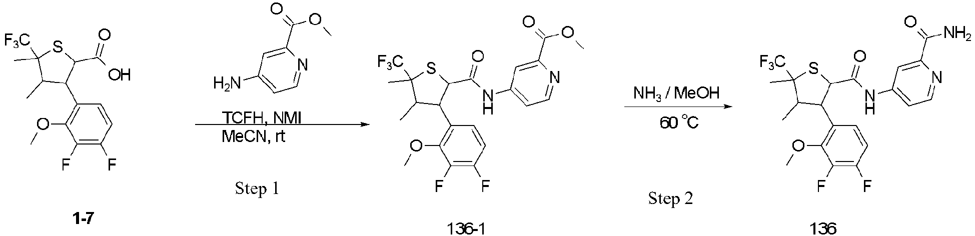

### Step 1: Synthesis of methyl 4-(3-(3,4-difluoro-2-methoxyphenyl)-4,5-methyl-5-(trifluoromethyl)tetrahydrothiophene-2-carboxamido)picolinate (136-1)

To a mixture of compound 1-7 (37 mg, 0.1 mmol), anhydrous acetonitrile (3 mL), NMI (65 mg, 0.8 mmol), and methyl 4-aminopicolinate (41 mg, 0.3 mmol), TCFH (140 mg, 0.5 mmol) was added in an ice bath. The mixture was allowed to naturally warm to room temperature and then stirred. The reaction was quenched with water (20 mL), and the mixture was extracted with ethyl acetate (30 mL × 3). The organic phases were combined and washed with water and saturated brine, and the crude product was separated by Pre-HPLC to obtain the target compound (38 mg, 0.075 mmol, yield: 75.24%). MS: m/z = 505.1, [M+H]⁺.

### Step 2: Synthesis of 4-(3-(3,4-difluoro-2-methoxyphenyl)-4,5-dimethyl-5-(trifluoromethyl)tetrahydrothiophene-2-carboxamido)picolinamide (compound 136)

The product of step 2 (38 mg, 0.075 mmol) and a 7 M solution of ammonia in methanol (2 mL) were stirred at 25 °C and concentrated under reduced pressure. The crude product was separated by Pre-HPLC to obtain the target compound (21 mg, 0.043 mmol, yield: 57.33%).

MS: m/z = 490.2, [M+H]⁺, ¹H NMR (400 MHz, DMSO) δ 10.79 (s, 1H), 8.48 (d, J = 5.5 Hz, 1H), 8.17 (d, J = 1.8 Hz, 1H), 8.12 - 8.04 (m, 1H), 7.70 - 7.59 (m, 2H), 7.24 - 7.16 (m, 2H), 4.49 (d, J = 10.5 Hz, 1H), 3.98 (d, J = 1.2 Hz, 3H), 3.96 - 3.86 (m, 2H), 2.90 - 2.72 (m, 1H), 1.63 (s, 3H), 0.85 (d, J = 6.7 Hz, 3H).

### Example 90: Preparation of 3-(3,4-difluoro-2-methoxyphenyl)-N-(4-fluoro-3-(S-methylsulfonimidoyl)phenyl)-5-methyl-5-(trifluoromethyl)tetrahydrothiophene-2-carboxamide (compound 227)

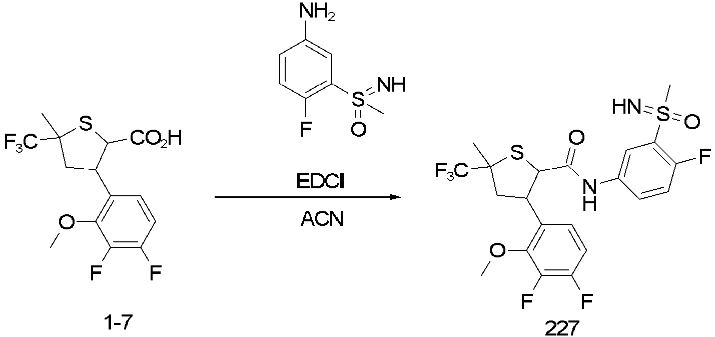

For the experimental procedures, reference may be made to Example 70.

MS: m/z = 527.2[M+H]+, ¹H NMR (400 MHz, DMSO) δ 10.60 (d, J = 22.1 Hz, 1H), 8.08 (ddd, J = 12.9, 6.4, 2.7 Hz, 1H), 7.81 - 7.72 (m, 1H), 7.35 (t, J = 9.3 Hz, 1H), 7.23 - 7.10 (m, 1H), 4.65 (t, J = 8.1 Hz, 1H), 4.59 (d, J = 10.7 Hz, 1H), 4.31 - 4.22 (m, 1H), 3.97 (s, 1H), 3.13 (d, J = 6.6 Hz, 1H), 2.41 (d, J = 13.0 Hz, 1H), 2.29 (s, 1H), 1.72 (d, J = 8.1 Hz, 1H).

### Example 91: Preparation of 3-(3,4-difluoro-2-methoxyphenyl)-5-methyl-N-(4-(S-methylsulfonimidoyl)phenyl)-5-(trifluoromethyl)tetrahydrothiophene-2-carboxamide (compound 228)

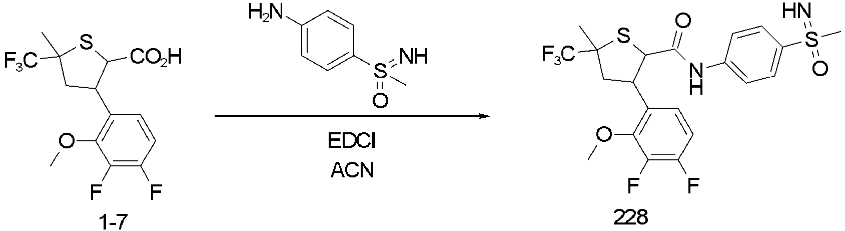

For the experimental procedures, reference may be made to Example 70.

MS: m/z = 509.1[M+H]+, 1H NMR (400 MHz, DMSO) δ 10.64 (s, 1H), 7.85 - 7.80 (m, 1H), 7.68 (dd, J = 7.7, 6.0 Hz, 1H), 7.17 (dt, J = 16.2, 7.4 Hz, 1H), 4.62 (d, J = 10.7 Hz, 1H), 4.33 - 4.24 (m, 1H), 4.11 (s, 1H), 3.97 (d, J = 1.7 Hz, 1H), 2.98 (d, J = 6.2 Hz, 1H), 2.36 - 2.28 (m, 1H), 1.74 (s, 1H), 1.51 (d, J = 2.6 Hz, 1H).

### Example 92: Preparation of (4-(3-(3,4-difluoro-2-methoxyphenyl)-5-methyl-5-(trifluoromethyl)tetrahydrothiophene-2-carboxamido)-2-hydroxylamine)pyridine (compound a-16)

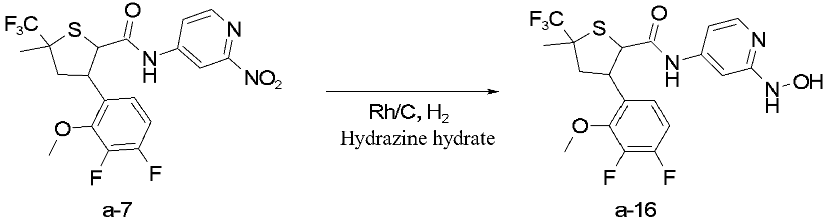

Compound a-7 (20 mg, 0.14 mmol) was dissolved in hydrazine hydrate (5 mL), and Rh/C (20 mg) was added. The mixture was stirred at room temperature under hydrogen atmosphere. After the reaction was completed, the mixture was separated by Pre-HPLC to obtain the target compound (13 mg, 0.028 mmol, yield: 67%).

MS: m/z = 464.10, [M+H]⁺, ¹H NMR (400 MHz, DMSO) δ 9.89 (s, 1H), 7.89 - 7.63 (m, 2H), 7.56 - 7.41 (m, 3H), 4.62 (d, *J =* 10.7 Hz, 1H), 4.38 - 4.24 (m, 1H), 3.99 (d, *J =* 1.6 Hz, 3H), 2.45 (t, *J =* 12.9 Hz, 1H), 2.33 (dd, *J =* 12.6, 6.0 Hz, 1H), 2.21 - 1.99 (m, 2H), 1.75 (s, 3H).

### Example 93: Preparation of (2R,3S,5R)-3-(3,4-difluoro-2-methoxyphenyl)-N-(2-methanesulfonylpyridin-4-yl)-5-methyl-5-(trifluoromethyl)tetrahydrothiophene-2-carboxamide (compound 192-A)

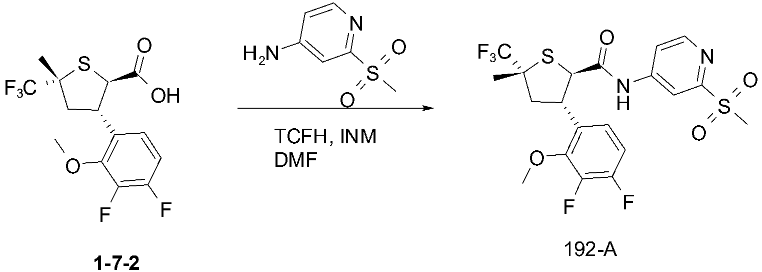

Compound 1-7-2 (50 mg, 0.14 mmol) was dissolved in anhydrous DMF (5 mL), 2-methylsulfonyl-4-aminopyridine (72 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (53 mg, 0.10 mmol, yield: 74%). MS: m/z = 511.07, [M+H]⁺, ¹H NMR (400 MHz, DMSO) δ 9.57 (m, 2H), 7.86 - 7.62 (m, 2H), 7.56 - 7.43 (m, 2H), 4.61 (d, *J =* 10.7 Hz, 1H), 4.37 - 4.23 (m, 1H), 3.99 (d, *J =* 1.6 Hz, 3H), 3.42 (s, 3H), 2.45 (t, *J =* 12.9 Hz, 1H), 2.33 (dd, *J =* 12.6, 6.0 Hz, 1H), 1.76 (s, 3H).

### Example 94: Preparation of (2S,3S,5R)-3-(3,4-difluoro-2-methoxyphenyl)-5-methyl-N-(2-nitropyridin-4-yl)-5-(trifluoromethyl)tetrahydrothiophene-2-carboxamide (compound a-7-A)

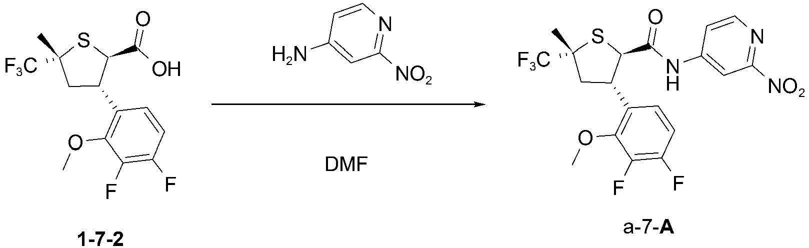

Compound 1-7-2 (50.0 mg, 0.14 mmol) was dissolved in anhydrous DMF (2 mL), 2-nitropyridin-4-amine (58.4 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (41.6 mg, 0.09 mmol, yield: 62.23%).

MS: m/z = 478.1, [M+H]⁺, 1H NMR (400 MHz, DMSO) δ 9.52 (s, 1H), 8.82 (d, J = 8.0 Hz, 1H), 8.47 (s, 1H), 7.62 (dt, J = 8.1, 4.0 Hz, 1H), 7.23-7.01 (m, 1H), 6.93-6.72 (m, 1H), 4.59 (d, J = 10.7 Hz, 1H), 4.31-4.25 (m, 1H), 4.00 (d, J = 2.5 Hz, 3H), 2.49 (t, J = 13.1 Hz, 1H), 2.24 (dd, J = 12.9, 6.2 Hz, 1H), 1.78 (s, 3H).

### Example 95: Preparation of (2R,3S,5R)-3-(3,4-difluoro-2-methoxyphenyl)-N-(3-fluoro-4-(4,4,5,5-tetramethyl-1,3,2-dioxaborolan-2-yl)phenyl)-5-methyl-5-(trifluoromethyl)tetrahydrothiophene-2-carboxamide (compound 229-A)

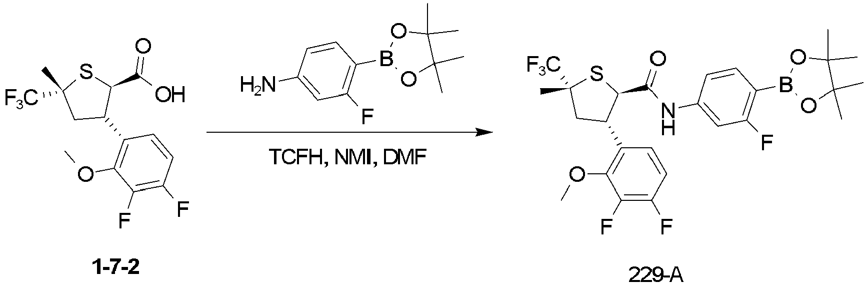

1-7-2 (50 mg, 0.14 mmol) was dissolved in anhydrous DMF (2 mL), 3-fluoro-4-(4,4,5,5-tetramethyl-1,3,2-dioxaborolan-2-yl)aniline (99.6 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (38.8 mg, 0.07 mmol, yield: 48.2%).

Ms: m/z = 576.2, [M+H]⁺,¹H NMR (400 MHz, DMSO) δ 10.02 (s, 1H), 7.82-7.75 (m, 2H), 7.41 (d, *J*=1.6 Hz, 1H), 7.01 (d, *J*=2.0 Hz, 1H), 6.85 (d, *J*=2.0 Hz, 1H), 4.22-4.02 (m, 1H), 3.82(s, 3H), 3.47-3.46(m, 1H), 2.63 (s, 3H), 2.16-1.92(m, 2H), 1.37 (s, 3H) , 1.20 (s, 3H).

### Example 96: Preparation of (4-((2R,3S,5R)-3-(3,4-difluoro-2-methoxyphenyl)-5-methyl-5-(trifluoromethyl)tetrahydrothiophene-2-carboxamido)-2-fluorophenyl)boronic acid (compound 88-A)

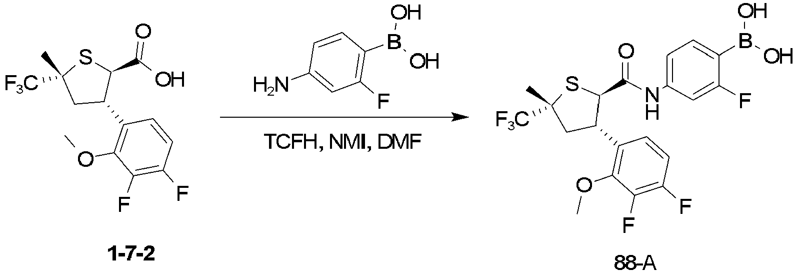

1-7-2 (50 mg, 0.14 mmol) was dissolved in anhydrous DMF (2 mL), (4-amino-2-fluorophenyl)boronic acid (71.4 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (45.8 mg, 0.09 mmol, yield: 64.2%).

Ms: m/z = 494.2, [M+H]⁺,¹H NMR (400 MHz, DMSO) δ 10.02 (s, 1H), 7.83-7.75 (m, 2H), 7.42 (d, *J*=1.6 Hz, 1H), 7.02 (d, *J*=2.0 Hz, 1H), 6.86 (d, *J*=2.0 Hz, 1H), 4.23-4.02 (m, 3H), 3.83(s, 3H), 3.48-3.46(m, 1H), 2.64 (s, 3H), 2.17-1.92(m, 2H), 1.38 (s, 3H).

### Example 97: Preparation of (2R,3S,5R)-3-(3,4-difluoro-2-methoxyphenyl)-N-(4-(dimethylphosphoryl)phenyl)-5-methyl-5-(trifluoromethyl)tetrahydrothiophene-2-carboxamide (230-A)

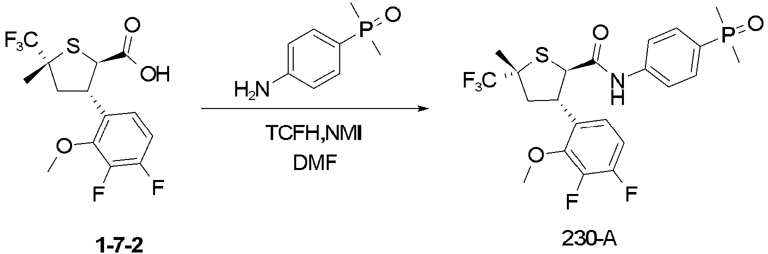

For the experimental procedures, reference may be made to Example 1.

MS: m/z = 508.2[M+H]⁺, ¹H NMR (400 MHz, CDCl3) δ 10.03 (s, 1H), 7.73 (d, J = 7.0 Hz, 2H), 7.57 (dd, J = 11.2, 8.6 Hz, 2H), 7.03 - 6.97 (m, 1H), 6.78 (dt, J = 16.5, 8.3 Hz, 1H), 4.65 (d, J = 10.8 Hz, 1H), 4.29 (ddd, J = 13.2, 10.9, 6.1 Hz, 1H), 4.00 (d, J = 2.3 Hz, 3H), 2.49 (t, J = 13.0 Hz, 1H), 2.24 (dd, J = 12.8, 6.1 Hz, 2H), 1.81 - 1.69 (m, 9H).

### Example 98: Preparation of (2R,3S,5R)-N-(3-((Z)-2-cyano-3-hydroxybut-2-enyl)-4-fluorophenyl)-3-(3,4-difluoro-2-methoxyphenyl)-5-methyl-5-(trifluoromethyl)tetrahydrothiophene-2-carboxamide (a-17-A)

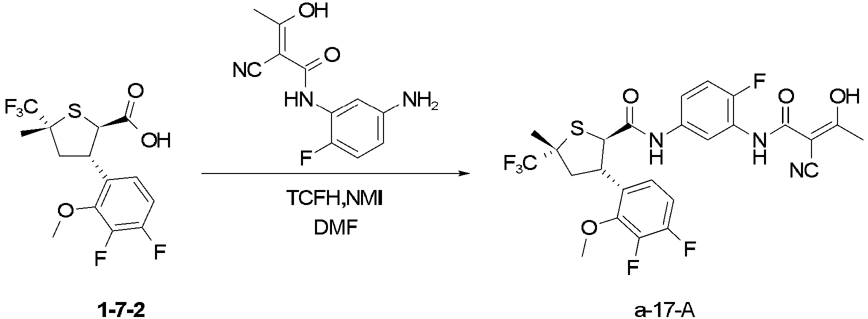

For the experimental procedures, reference may be made to Example 1.

MS: m/z = 573.1[M+H]⁺, ¹H NMR (400 MHz, DMSO) δ 12.16 (s, 1H), 10.32 (s, 1H), 8.51 - 8.46 (m, 1H), 7.36 - 7.27 (m, 2H), 7.25 - 7.14 (m, 3H), 7.12 - 7.01 (m, 2H), 4.67 (d, J = 10.8 Hz, 1H), 4.37 - 4.27 (m, 1H), 4.02 (d, J = 1.7 Hz, 3H), 2.43 (t, J = 12.8 Hz, 1H), 2.34 (dd, J = 12.6, 6.3 Hz, 1H), 2.08 (s, 3H), 1.78 (s, 3H).

### Example 99: Preparation of (2R,3S,5R)-3-(3,4-difluoro-2-methoxyphenyl)-5-methyl-N-(3-(sulfamoylamino)phenyl)-5-(trifluoromethyl)tetrahydrothiophene-2-carboxamide (a-18-A)

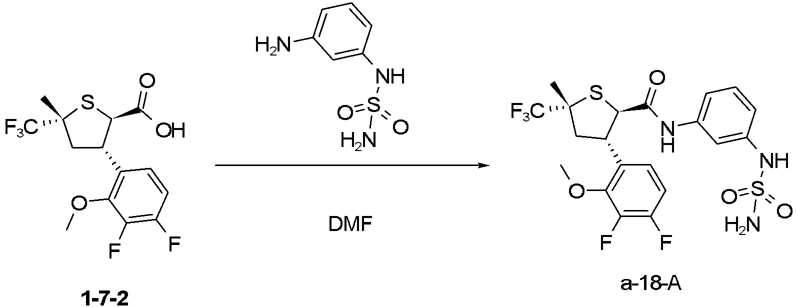

Compound 1-7-2 (50.0 mg, 0.14 mmol) was dissolved in anhydrous DMF (2 mL), 4-aminophenylsulfamide (78.6 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (29.4 mg, 0.06 mmol, yield: 40.00%).

MS: m/z = 526.1, [M+H]⁺, ¹H NMR (400 MHz, DMSO) δ 10.22 (s, 1H), 7.29 (s, 1H), 7.24 - 6.91 (m, 6H), 6.88 (d, J = 8.3 Hz, 1H), 4.62 (d, J = 10.7 Hz, 1H), 4.27 (td, J = 12.9, 6.2 Hz, 1H), 3.96 (d, J = 1.5 Hz, 3H), 2.43 - 2.27 (m, 2H), 1.72 (s, 3H).

### Example 100: Preparation of (2R,3S,5R)-3-(3,4-difluoro-2-methoxyphenyl)-5-methyl-N-(3-(S-methylsulfonimidoyl)phenyl)-5-(trifluoromethyl)tetrahydrothiophene-2-carboxamide (compound 225-A)

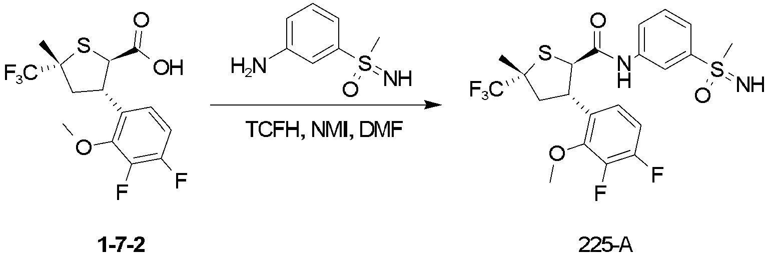

Compound 1-7-2 (50 mg, 0.14 mmol) was dissolved in anhydrous DMF (2 mL), (3-aminophenyl)(imino)(methyl)-λ⁶-thione (71.4 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (45.8 mg, 0.09 mmol, yield: 64.2%).

Ms: m/z = 509.2, [M+H]⁺,¹H NMR (400 MHz, DMSO) δ 10.02 (s, 1H), 7.75-7.66 (m, 3H), 7.33 (d, *J*=1.6 Hz, 1H), 7.02 (d, *J*=2.0 Hz, 1H), 6.86 (d, *J*=2.0 Hz, 1H), 4.23-4.02 (m, 2H), 3.83(s, 3H), 3.48-3.46(m, 1H), 2.64 (s, 3H), 2.17-1.92(m, 2H), 1.38 (s, 3H).

### Example 101: Preparation of (2R,3S,5R)-3-(3,4-difluoro-2-methoxyphenyl)-5-methyl-N-(6-(methylthio)pyridin-3-yl)-5-(trifluoromethyl)tetrahydrothiophene-2-carboxamide (compound 231-A)

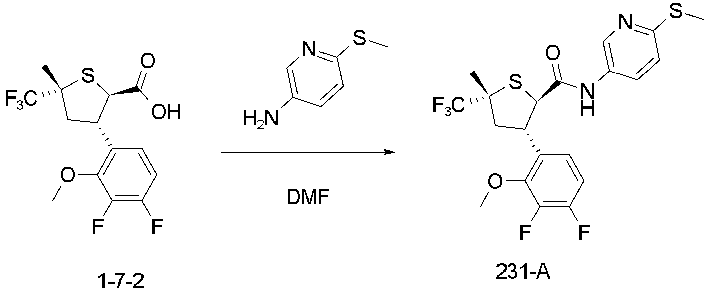

Compound 1-7-2 (30 mg, 0.08 mmol) was dissolved in anhydrous DMF (5 mL), 6-(methylthio)pyridin-3-amine (33.62 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (9.56 mg, 0.02 mmol, yield: 25%). MS: m/z = 479.3, [M+H]⁺, ¹H NMR (400 MHz, DMSO) δ ¹H NMR: δ8.07 (dd, J = 1.8, 0.5 Hz, 1H), 7.34 (dd, J = 8.6, 1.8 Hz)), 7.15-7.40 (2H, 7.22 (dd, J = 8.6, 0.5 Hz), 7.09 (d, J = 8.1 Hz, 1H), 6.87 (d, J = 8.1 Hz, 1H), 3.98 (d, J = 5.5 Hz, 1H), 3.77 (s, 3H), 3.61 (1H, ddd, J = 8.1, 6.1, 5.5 Hz), 2.44 (3H, s), 1.93 (2H, dd, J = 16.0, 7.1 Hz), 1.75 (s, 3H).

### Example 102: Preparation of (2R,3S,5R)-3-(3,4-difluoro-2-methoxyphenyl)-N-[3-fluoro-4-(sulfamoylamino)phenyl]-5-methyl-5-(trifluoromethyl)thiolane-2-carboxamide (compound a-19-A)

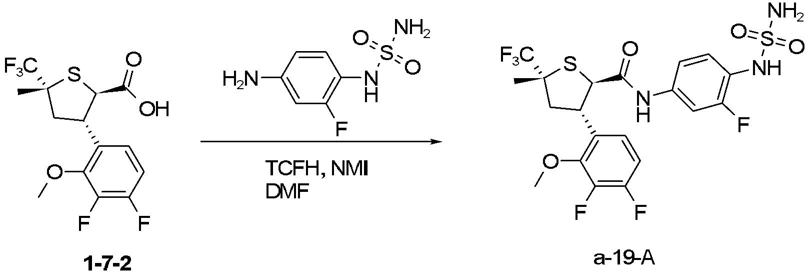

Compound 1-7-2 (50 mg, 0.14 mmol) was dissolved in anhydrous DMF (5 mL), 2-fluoro-4-aminosulfamide (86 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (45 mg, 0.083 mmol, yield: 59%).

MS: m/z = 544.07, [M+H]⁺, ¹H NMR (400 MHz, DMSO) δ 9.87 (s, 1H), 7.88 - 7.54 (m, 3H), 7.36 - 7.23 (m, 2H), 5.62 (s, 1H), 4.61 (d, *J* = 10.7 Hz, 1H), 4.47 (s, 2H), 4.38 - 4.24 (m, 1H), 3.98 (d, *J =* 1.6 Hz, 3H), 2.43 (t, *J =* 12.9 Hz, 1H), 2.32 (dd, *J =* 12.6, 6.0 Hz, 1H), 1.75 (s, 3H).

### Example 103: Preparation of (2R,3S,5R)-3-(3,4-difluoro-2-methoxyphenyl)-N-(1-hydroxy-3-oxo-2,3-dihydro-1H-benzo[c][1,2]azaborol-6-yl)-5-methyl-5-(trifluoromethyl)tetrahydrothiophene-2-carboxamide (compound a-2-A)

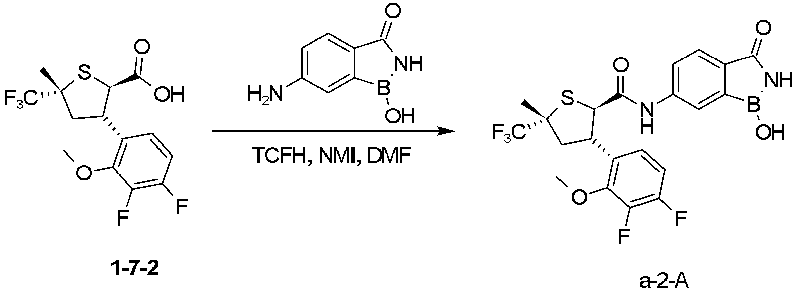

Compound 1-7-2 (50 mg, 0.14 mmol) was dissolved in anhydrous DMF (2 mL), 6-amino-1-hydroxy-1,2-dihydro-3H-benzo[c][1,2]azaborol-3-one (68.0 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (40.1 mg, 0.08 mmol, yield: 51.1%).

Ms: m/z = 501.2, [M+H]⁺,¹H NMR (400 MHz, DMSO) δ 10.02 (s, 1H), 8.09 (d, *J=2.0* Hz, 1H), 7.82-7.70 (m, 3H), 7.07 (d, *J*=1.6 Hz, 1H), 6.86 (*d, J*=2.0 Hz, 1H), 4.23-4.02 (m, 2H), 3.83(s, 3H), 3.48-3.46(m, 1H), 2.17-1.92(m, 2H), 1.38 (s, 3H).

### Example 104: Preparation of (2R,3S,5R)-3-(3,4-difluoro-2-methoxyphenyl)-5-methyl-N-(tetrazolo[1,5-a]pyridin-6-yl)-5-(trifluoromethyl)tetrahydrothiophene-2-carboxamide (compound 202-A)

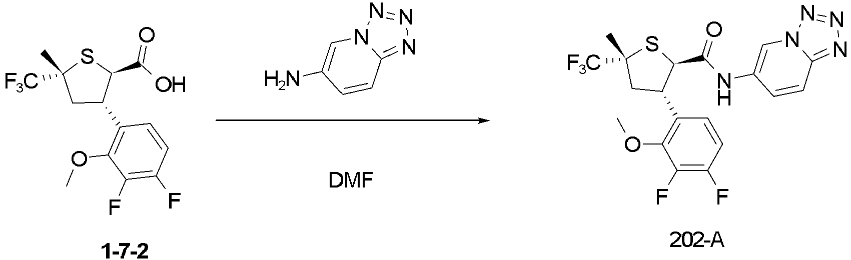

Compound 1-7-2 (50.0 mg, 0.14 mmol) was dissolved in anhydrous DMF (2 mL), tetrazolo[1,5-a]pyridin-6-amine (56.7 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (31.3 mg, 0.07 mmol, yield: 47.23%).

MS: m/z = 474.1, [M+H]⁺, 1H NMR (400 MHz, DMSO) δ 10.85 (s, 1H), 9.54 (s, 1H), 8.07 (d, J = 9.6 Hz, 1H), 7.54 (dd, J = 9.6, 1.6 Hz, 1H), 7.18 - 7.03 (m, 2H), 4.57 (d, J = 10.6 Hz, 1H), 4.22 (td, J = 13.0, 6.1 Hz, 1H), 3.89 (d, J = 1.7 Hz, 3H), 2.35 (s, 1H), 2.24 (dd, J = 12.6, 6.0 Hz, 1H), 1.75 (s, 3H).

### Example 105: Preparation of (2R,3S,5R)-N-(2-aminopyridin-4-yl)-3-(3,4-difluoro-2-methoxyphenyl)-5-methyl-5-(trifluoromethyl)tetrahydrothiophene-2-carboxamide (compound 175-A)

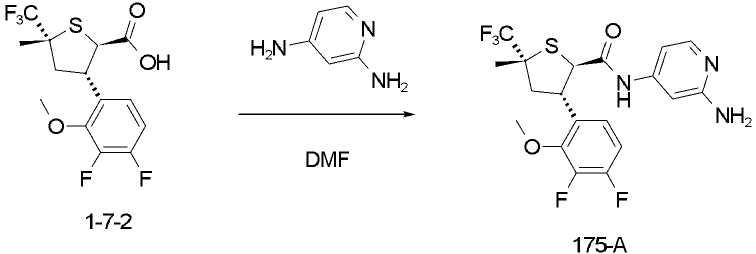

Compound 1-7 (30 mg, 0.08 mmol) was dissolved in anhydrous DMF (5 mL), pyridine-2,4-diamine (26.16 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (4.93 mg, 0.02 mmol, yield: 14%).

MS: m/z = 448.1, [M+H]⁺, ¹H NMR (400 MHz, DMSO) δ 10.17 (s, 1H), 7.50 (dd, J = 5.2, 0.4 Hz, 1H), 7.28(s,2H), 7.23 - 7.16 (m, 2H), 6.14 (dd, J = 5.2, 1.8 Hz, 1H), 5.99 (dd, J = 1.8, 0.4 Hz, 1H), 4.66 (d, J = 10.6 Hz, 1H), 4.28 (td, J = 12.8, 6.2 Hz, 1H), 3.97 (d, J = 1.7 Hz, 3H), 2.43 (t, J = 12.9 Hz, 1H), 2.33 (dd, J = 12.6, 6.2 Hz, 1H), 1.75 (s, 3H).

### Example 106: Preparation of (2R,3S,5R)-N-(2-azidopyridin-4-yl)-3-(3,4-difluoro-2-methoxyphenyl)-5-methyl-5-(trifluoromethyl)tetrahydrothiophene-2-carboxamide (compound a-20-A)

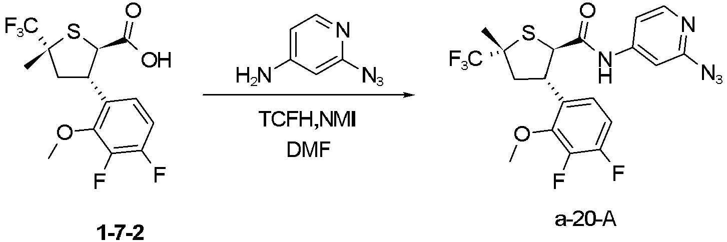

For the experimental procedures, reference may be made to Example 1.

MS: m/z = 472.4[M+H]+, ¹H NMR (400 MHz, CDCl₃) δ 9.32 (s, 1H), 8.72 (d, *J =* 7.4 Hz, 1H), 8.43 (s, 1H), 7.54 (dt, *J =* 8.1, 4.0 Hz, 1H), 7.01 (ddd, *J =* 7.8, 5.5, 1.9 Hz, 1H), 6.83 (dt, *J =* 16.4, 8.2 Hz, 1H), 4.59 (d, *J =* 10.7 Hz, 1H), 4.27 (ddd, *J* = 13.1, 10.8, 6.1 Hz, 1H), 4.00 (d, *J =* 2.5 Hz, 3H), 2.56 (t, *J =* 13.1 Hz, 1H), 2.24 (dd, *J =* 12.9, 6.2 Hz, 1H), 1.78 (s, 3H).

### Example 107: Preparation of (2R,3S,5R)-3-(3,4-difluoro-2-methoxyphenyl)-N-(2-(2,3-dihydroxypropoxy)pyridin-4-yl)-5-methyl-5-(trifluoromethyl)tetrahydrothiophene-2-carboxamide (compound 191-A)

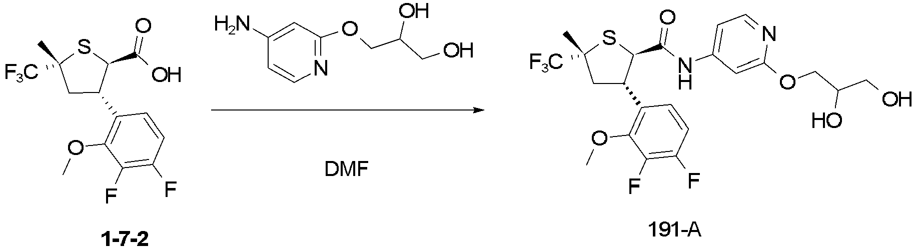

Compound 1-7-2 (50.0 mg, 0.14 mmol) was dissolved in anhydrous DMF (2 mL), 3-((4-aminopyridin-2-yl)oxy)propane-1,2-diol (77.3 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (40.9 mg, 0.08 mmol, yield: 55.98%).

MS: m/z = 523.1, [M+H]⁺, 1H NMR (400 MHz, DMSO) δ 10.57 (s, 1H), 7.97 (d, J = 5.7 Hz, 1H), 7.23 - 7.11 (m, 2H), 7.04 (s, 1H), 6.95 (dd, J = 5.7, 1.4 Hz, 1H), 4.88 (d, J = 5.1 Hz, 1H), 4.65 - 4.53 (m, 2H), 4.31 - 4.18 (m, 2H), 4.09 - 4.02 (m, 1H), 3.96 (s, 3H), 3.78 - 3.68 (m, 1H), 3.38 (t, J = 5.6 Hz, 2H), 2.44 (t, J = 12.9 Hz, 1H), 2.31 (dd, J = 12.6, 6.1 Hz, 1H), 1.72 (s, 3H).

### Example 108: Preparation of (2R,3S,5R)-N-(1-(cyclopropylsulfonyl)-3-methyl-1H-pyrazol-4-yl)-3-(3,4-difluoro-2-methoxyphenyl)-5-methyl-5-(trifluoromethyl)tetrahydrothiophene-2-carboxamide (compound a-21-A)

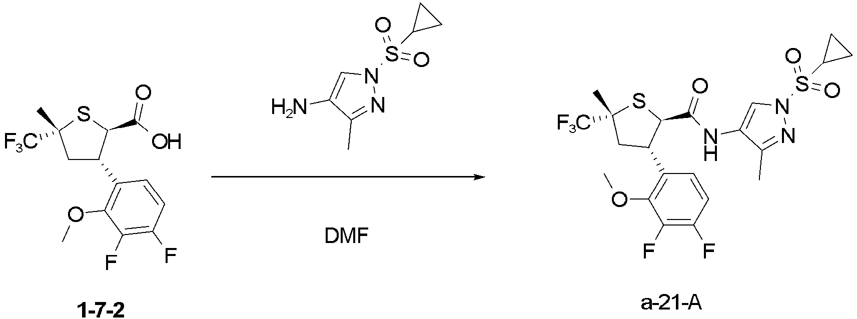

Compound 1-7-2 (50.0 mg, 0.14 mmol) was dissolved in anhydrous DMF (2 mL), 1-(cyclopropylsulfonyl)-3-methyl-1H-pyrazol-4-amine (84.4 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (37.9 mg, 0.07 mmol, yield: 50.18%).

MS: m/z = 540.1, [M+H]⁺, ¹H NMR (400 MHz, DMSO ) δ 9.21 (s, 1H), 8.14 (s, 1H), 7.13 (dd, J = 15.0, 10.0 Hz, 1H), 6.90 - 6.79 (m, 1H), 4.45 (d, J = 3.3 Hz, 1H), 4.23 (td, J = 7.9, 3.2 Hz, 1H), 3.92 (s, 3H), 3.76 (dd, J = 24.7, 7.9 Hz, 1H), 2.43 (s, 3H), 2.11 (dd, J = 24.7, 7.9 Hz, 1H), 1.82 - 1.61 (m, 1H), 1.38 (s, 3H), 1.12 - 0.85 (m, 4H).

### Example 109: Preparation of 3-(3,4-difluoro-2-methoxyphenyl)-5-methyl-N-(6-(methylsulfinyl)pyridin-3-yl)-5-(trifluoromethyl)tetrahydrothiophene-2-carboxamide (compound 232)

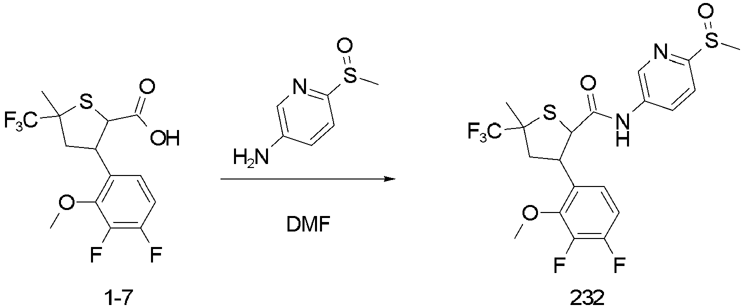

Compound 1-7 (30 mg, 0.08 mmol) was dissolved in anhydrous DMF (5 mL), 6-(methylsulfinyl)pyridin-3-amine (37.44 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (14.86 mg, 0.03 mmol, yield: 37%).

MS: m/z = 495.2, [M+H]⁺, ¹H NMR (400 MHz, DMSO) δ 10.33 (s, 1H), 7.27 (d, *J* = 7.1 Hz, 1H), 7.21 - 7.06 (m, 2H), 6.63 (s, 1H), 6.21 (d, *J =* 7.0 Hz, 1H), 4.58 (d, *J =* 10.6 Hz, 1H), 4.39 - 4.19 (m, 1H), 4.02 (dd, *J* = 20.4, 1.7 Hz, 3H), 3.63 (dd, *J =* 61.8, 11.1 Hz, 1H), 2.46 (t, *J* = 12.9 Hz, 1H), 2.39 - 2.24 (m, 1H), 1.73 (d, *J* = 8.8 Hz, 3H), 1.48 (s, 1H), 1.43 - 1.31 (m, 1H).

### Example 110: Preparation of 3-(3,4-difluoro-2-methoxyphenyl)-N-(3-((dimethyl(oxo)-16-sulfonamido)amino)-4-fluorophenyl)-5-methyl-5-(trifluoromethyl)tetrahydrothiophene-2-carboxamide (compound a-22)

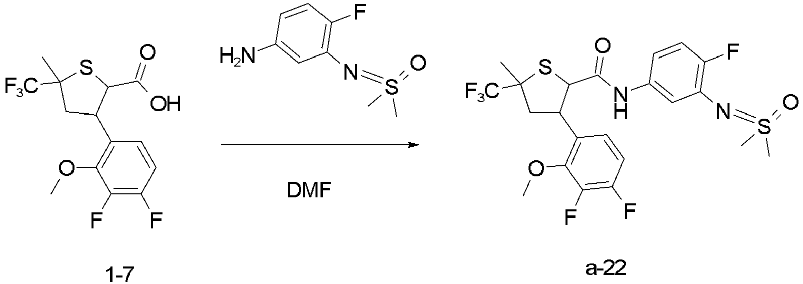

Compound 1-7 (30 mg, 0.08 mmol) was dissolved in anhydrous DMF (5 mL), (5-amino-2-fluorophenyl)imino)dimethyl-6-thione (48.48 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (21.60 mg, 0.04 mmol, yield: 50%).

MS: m/z = 541.1, [M+H]⁺, ¹H NMR (400 MHz, DMSO) δ 10.20 (s, 1H), 7.33 (d, *J* = 5.9 Hz, 1H), 7.22 - 7.16 (m, 2H), 7.12 (d, *J =* 8.2 Hz, 1H), 7.01 (t, *J* = 9.7 Hz, 1H), 4.58 (d, *J =* 10.7 Hz, 1H), 4.29 (td, *J* = 12.5, 6.4 Hz, 1H), 3.99 (s, 3H), 3.26 (s, 6H), 2.42 (t, *J* = 12.8 Hz, 1H), 2.32 (dd, *J* = 12.5, 6.2 Hz, 1H), 1.75 (s, 3H).

### Example 111: Preparation of (2R,3S,5R)-3-(3,4-difluoro-2-methoxyphenyl)-N-(1,3-dioxoisoindol-5-yl)-5-methyl-5-(trifluoromethyl)tetrahydrothiophene-2-carboxamide (compound a-23-A)

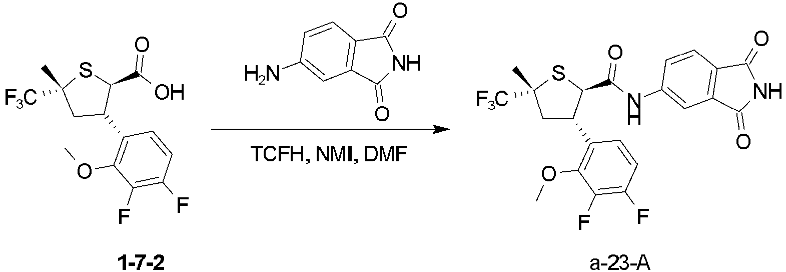

1-7-2 (50 mg, 0.14 mmol) was dissolved in anhydrous DMF (2 mL), 5-aminoisoindoline-1,3-dione (68.0 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (43.5 mg, 0.09 mmol, yield: 62.0%).

Ms: m/z = 501.2, [M+H]⁺,¹H NMR (400 MHz, DMSO) δ 13.51 (s, 1H), 10.02 (s, 1H), 8.35(s, 1H), 8.27 (d, *J=*2.0 Hz, 1H), 8.07 (d, *J*=2.0 Hz, 1H), 7.07 (d, *J*=1.6 Hz, 1H), 6.86 (d, *J*=2.0 Hz, 1H), 4.23-4.02 (m, 1H), 3.83(s, 3H), 3.48-3.46(m, 1H), 2.17-1.92(m, 2H), 1.38 (s, 3H).

### Example 112: Preparation of (2R,3S,5R)-3-(3,4-difluoro-2-methoxyphenyl)-5-methyl-N-(2-sulfamoylpyridin-4-yl)-5-(trifluoromethyl)tetrahydrothiophene-2-carboxamide (compound 234-A)

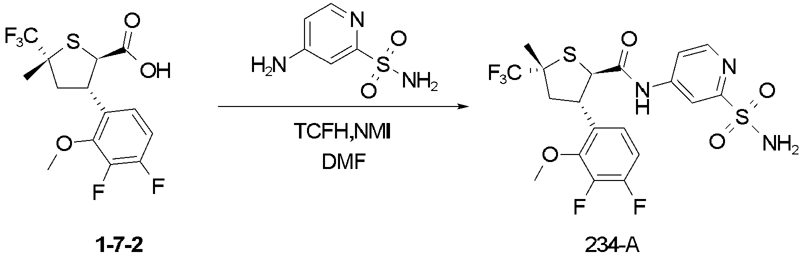

For the experimental procedures, reference may be made to Example 1.

MS: m/z = 512.3[M+H]⁺, ¹H NMR (400 MHz, DMSO) δ 7.86 (d, J = 6.0 Hz, 1H), 7.29 (s, 1H), 7.14 - 6.96 (m, 3H), 6.59 (d, J = 4.7 Hz, 1H), 4.29 (d, J = 10.8 Hz, 1H), 4.01 - 3.90 (m, 1H), 3.84 (s, 3H), 2.24 - 2.09 (m, 2H), 1.60 (s, 3H).

### Example 113: Preparation of 3-(3,4-difluoro-2-methoxyphenyl)-N-(3-fluoro-4-(S-methylsulfonimidoyl)phenyl)-5-methyl-5-(trifluoromethyl)tetrahydrothiophene-2-carboxamide (compound 235)

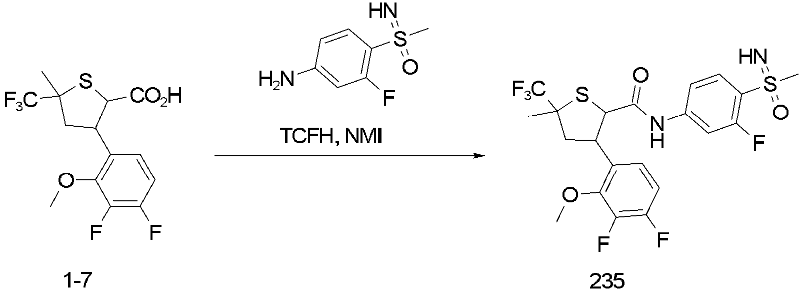

For the experimental procedures, reference may be made to Example 1.

MS: m/z = 527.1[M+H]⁺, ¹H NMR (400 MHz, DMSO) δ 10.79 (s, 1H), 7.76 (t, J = 8.5 Hz, 1H), 7.64 (d, J = 12.2 Hz, 1H), 7.36 (dd, J = 8.7, 1.7 Hz, 1H), 7.23 - 7.11 (m, 2H), 4.62 - 4.57 (m, 2H), 4.32 - 4.22 (m, 1H), 3.96 (d, J = 1.7 Hz, 3H), 3.10 (d, J = 0.8 Hz, 3H), 2.44 (d, J = 12.9 Hz, 1H), 2.35 - 2.29 (m, 1H), 1.73 (s, 3H).

### Example 114: Preparation of (2R,3S,5R)-3-(3,4-difluoro-2-methoxyphenyl)-5-methyl-N-(4-(sulfamoylamino)phenyl)-5-(trifluoromethyl)tetrahydrothiophene-2-carboxamide (compound a-24-A)

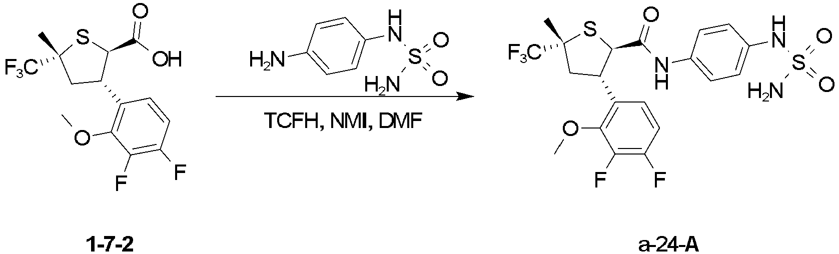

1-7-2 (50 mg, 0.14 mmol) was dissolved in anhydrous DMF (2 mL), N-(4-aminophenyl)sulfamide (78.5 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (44.6 mg, 0.08 mmol, yield: 55.2%).

Ms: m/z = 526.2, [M+H]⁺,¹H NMR (400 MHz, DMSO) δ 10.58 (s, 1H), 10.02 (s, 1H), 7.37 (d, *J=*2.0 Hz, 1H), 7.07 (d, *J*=1.6 Hz, 1H), 6.90-6.86 (m, 2H), 5.51 (s, 1H), 4.23-4.02 (m, 1H), 3.83(s, 3H), 3.48-3.46(m, 1H), 2.17-1.92(m, 2H), 1.38 (s, 3H) .

### Example 115: Preparation of N-(2-aminopyrimidin-4-yl)-3-(3,4-difluoro-2-methoxyphenyl)-5-methyl-5-(trifluoromethyl)tetrahydrothiophene-2-carboxamide (compound 236)

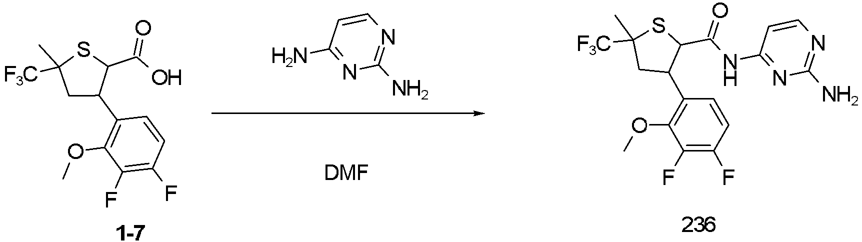

Compound 1-7 (50.0 mg, 0.14 mmol) was dissolved in anhydrous DMF (2 mL), pyrimidine-2,4-diamine (46.2 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (27.0 mg, 0.06 mmol, yield: 43.06%).

MS: m/z = 449.1, [M+H]⁺, ¹H NMR (400 MHz, DMSO) δ 10.18 (s, 1H), 7.87 (d, J = 5.8 Hz, 1H), 7.17 (d, J = 7.8 Hz, 2H), 6.92 - 6.81 (m, 2H), 6.12 (d, J = 5.8 Hz, 1H), 4.28 (dd, J = 17.8, 11.5 Hz, 1H), 3.95 (d, J = 1.4 Hz, 3H), 3.74 (s, 1H), 2.38 - 2.18 (m, 2H), 1.70 (s, 3H).

### Example 116: Preparation of (2R,3S,5R)-3-(3,4-difluoro-2-methoxyphenyl)-5-methyl-N-(6-(methylsulfinyl)pyridin-3-yl)-5-(trifluoromethyl)tetrahydrothiophene-2-carboxamide (compound 238-A)

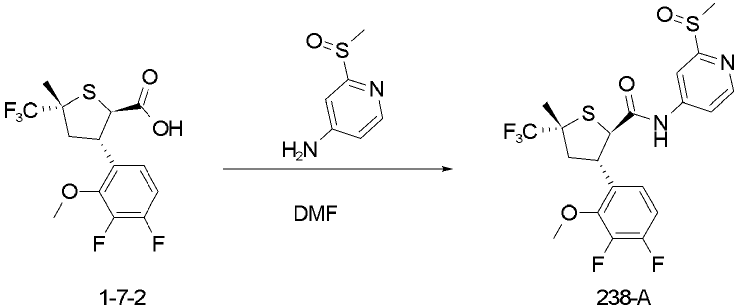

Compound 1-7-2 (30 mg, 0.08 mmol) was dissolved in anhydrous DMF (5 mL), 6-(methylsulfinyl)pyridin-3-amine (37.44 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (14.86 mg, 0.03 mmol, yield: 37%).

MS: m/z = 495.2, [M+H]⁺, ¹H NMR (400 MHz, DMSO) δ 10.33 (s, 1H), 7.27 (d, *J =* 7.1 Hz, 1H), 7.21 - 7.06 (m, 2H), 6.63 (s, 1H), 6.21 (d, *J =* 7.0 Hz, 1H), 4.58 (d, *J =* 10.6 Hz, 1H), 4.39 - 4.19 (m, 1H), 4.02 (dd, *J* = 20.4, 1.7 Hz, 3H), 3.63 (dd, *J =* 61.8, 11.1 Hz, 1H), 2.46 (t, *J* = 12.9 Hz, 1H), 2.39 - 2.24 (m, 1H), 1.73 (d, *J* = 8.8 Hz, 3H), 1.48 (s, 1H), 1.43 - 1.31 (m, 1H).

### Example 117: Preparation of (2R,3S,5R)-3-(3,4-difluoro-2-methoxyphenyl)-N-(3-(N-isopropylsulfamoyl)phenyl)-5-methyl-5-(trifluoromethyl)tetrahydrothiophene-2-carboxamide (compound 239-A)

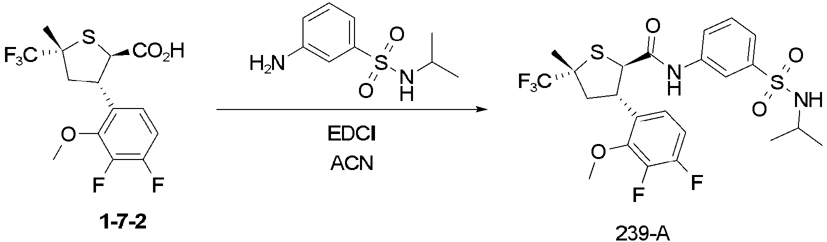

For the experimental procedures, reference may be made to Example 70.

MS: m/z = 553.3[M+H]+, ¹H NMR (400 MHz, DMSO) δ 10.59 (s, 1H), 8.11 - 8.07 (m, 1H), 7.63 (ddd, *J* = 7.3, 4.6, 2.5 Hz, 2H), 7.52 - 7.45 (m, 2H), 7.24 - 7.10 (m, 2H), 4.60 (d, *J =* 10.7 Hz, 1H), 4.28 (ddd, *J =* 13.1, 10.9, 6.1 Hz, 1H), 3.96 (d, *J =* 1.9 Hz, 3H), 3.19 (dq, *J* = 13.3, 6.6 Hz, 1H), 2.44 (t, *J =* 12.9 Hz, 1H), 2.31 (dd, *J =* 12.6, 6.1 Hz, 1H), 1.73 (s, 3H), 0.92 (dd, *J* = 6.5, 0.6 Hz, 6H).

### Example 118: Preparation of (2R,3S,5R)-3-(3,4-difluoro-2-methoxyphenyl)-N-(3-(N,N-dimethylsulfamoyl)phenyl)-5-methyl-5-(trifluoromethyl)tetrahydrothiophene-2-carboxamide (compound 240-A)

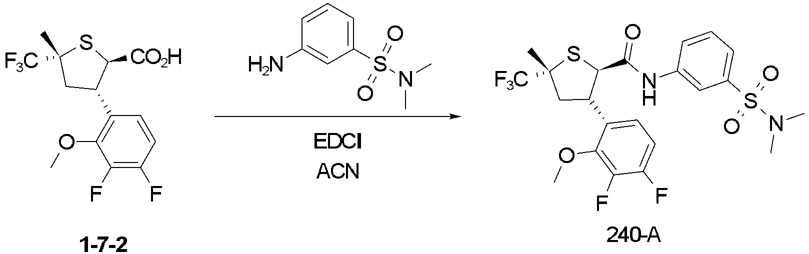

For the experimental procedures, reference may be made to Example 70.

MS: m/z = 539.3[M+H]⁺, ¹H NMR (400 MHz, DMSO) δ 10.65 (s, 1H), 7.99 (t, J = 1.8 Hz, 1H), 7.77 - 7.73 (m, 1H), 7.55 (t, J = 8.0 Hz, 1H), 7.43 - 7.39 (m, 1H), 7.24 - 7.11 (m, 2H), 4.59 (d, J = 10.7 Hz, 1H), 4.27 (ddd, J = 13.1, 10.9, 6.1 Hz, 1H), 3.96 (d, J = 1.9 Hz, 3H), 2.58 (s, 6H), 2.48 - 2.40 (m, 1H), 2.31 (dd, J = 12.6, 6.1 Hz, 1H), 1.73 (s, 3H).

### Example 119: Preparation of (2R,3S,5R)-3-(3,4-difluoro-2-methoxyphenyl)-5-methyl-N-(3-(N-methylsulfamoyl)phenyl)-5-(trifluoromethyl)tetrahydrothiophene-2-carboxamide (compound 241-A)

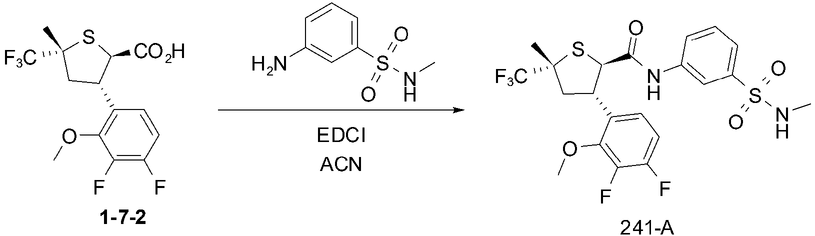

For the experimental procedures, reference may be made to Example 70.

MS: m/z = 542.3[M+H]⁺, ¹H NMR (400 MHz, DMSO) δ 10.61 (s, 1H), 8.07 (t, J = 1.8 Hz, 1H), 7.68 - 7.64 (m, 1H), 7.54 - 7.42 (m, 3H), 7.23 - 7.11 (m, 2H), 4.61 (d, J = 10.7 Hz, 1H), 4.28 (ddd, J = 13.1, 10.9, 6.1 Hz, 1H), 3.97 (d, J = 1.9 Hz, 3H), 2.44 (t, J = 12.9 Hz, 1H), 2.38 (d, J = 4.9 Hz, 3H), 2.31 (dd, J = 12.6, 6.1 Hz, 1H), 1.73 (s, 3H).

### Example 120: Preparation of (2R,3S,5R)-N-(2-cyanopyridin-4-yl)-3-(3,4-difluoro-2-methoxyphenyl)-5-methyl-5-(trifluoromethyl)tetrahydrothiophene-2-carboxamide (compound 242-A)

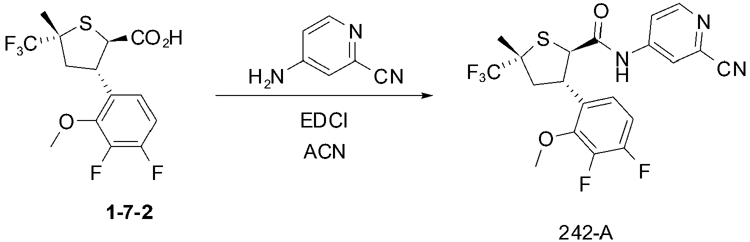

For the experimental procedures, reference may be made to Example 70.

MS: m/z = 458.2[M+H]⁺, ¹H NMR (400 MHz, DMSO) δ 11.03 (s, 1H), 8.56 (d, *J =* 5.6 Hz, 1H), 8.03 (d, *J =* 1.8 Hz, 1H), 7.70 (dd, *J =* 5.6, 2.1 Hz, 1H), 7.24 - 7.10 (m, 2H), 4.61 (d, *J =* 10.6 Hz, 1H), 4.32 - 4.22 (m, 1H), 3.97 (d,*J =* 1.8 Hz, 3H), 2.45 (d, *J =* 13.0 Hz, 1H), 2.32 (dd, *J =* 12.6, 6.1 Hz, 1H), 1.73 (s, 3H).

### Example 121: Preparation of N-(3-((2-aminoethyl)sulfonamido)phenyl)-3-(3,4-difluoro-2-methoxyphenyl)-5-methyl-5-(trifluoromethyl)tetrahydrothiophene-2-carboxamide (compound a-25)

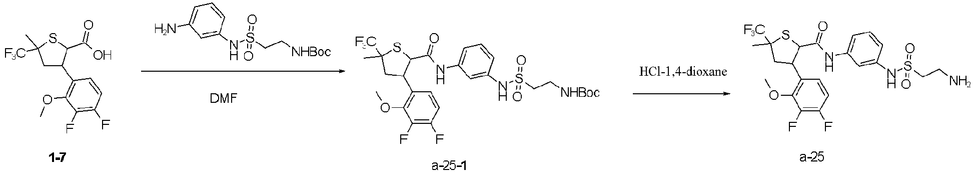

### Step 1: Preparation of tert-butyl (5-(3-(3,4-difluoro-2-methoxyphenyl)-5-methyl-5-(trifluoromethyl)tetrahydrothiophene-2-carboxamido)pyridin-2-yl)carbamate (compound a-25-1)

Compound 1-7 (50.0 mg, 0.14 mmol) was dissolved in anhydrous DMF (2 mL), tert-butyl (2-(N-(3-aminophenyl)sulfamoyl)ethyl)carbamate (132.4 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (60.7 mg, 0.09 mmol, yield: 66.42%). MS: m/z = 654.2, [M+H]⁺.

### Step 2: Preparation of N-(3-((2-aminoethyl)sulfonamido)phenyl)-3-(3,4-difluoro-2-methoxyphenyl)-5-methyl-5-(trifluoromethyl)tetrahydrothiophene-2-carboxamide (compound a-25)

The product of step 1 (60.7 mg, 0.09 mmol) was dissolved in 1,4-dioxane (5 mL), and the mixture was stirred at room temperature. After the reaction was completed, the mixture was separated by Pre-HPLC to obtain the target compound (40.5 mg, 0.07 mmol, yield: 81.29%).

MS: m/z = 554.1, [M+H]⁺, ¹H NMR (400 MHz, DMSO) δ 10.37 (s, 1H), 8.25 (s, 1H), 7.44 (s, 1H), 7.27 - 7.14 (m, 4H), 6.87 (d, J = 7.8 Hz, 1H), 4.61 (d, J = 10.7 Hz, 1H), 4.31 - 4.24 (m, 1H), 3.96 (d, J = 1.6 Hz, 3H), 3.20 (d, J = 7.3 Hz, 2H), 2.93 (t, J = 7.0 Hz, 2H), 2.40 (t, J = 12.9 Hz, 1H), 2.30 (dd, J = 12.6, 6.2 Hz, 1H), 1.98 (s, 2H), 1.72 (s, 3H).

### Example 122: Preparation of (2R,3S,5R)-3-(3,4-difluoro-2-methoxyphenyl)-N-(1,1-dioxide-3,4-dihydro-2H-benzo[e][1,2,4]thiadiazin-7-yl)-5-methyl-5-(trifluoromethyl)tetrahydrothiophene-2-carboxamide (compound a-26-A)

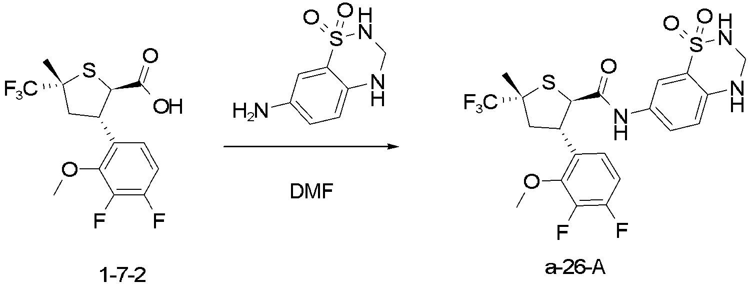

Compound 1-7-2 (30 mg, 0.08 mmol) was dissolved in anhydrous DMF (5 mL), 7-amino-3,4-dihydro-2H-benzo[e][1,2,4]thiadiazine-1,1-dioxide (47.76 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (10.76 mg, 0.02 mmol, yield: 25%).

MS: m/z = 538.2, [M+H]⁺, ¹H NMR (400 MHz, DMSO) δ 10.19 (s, 1H), 7.83 (d, *J* = 2.4 Hz, 1H), 7.64 (t, *J =* 7.7 Hz, 1H), 7.30 (dd, *J =* 9.0, 2.5 Hz, 1H), 7.24 - 7.09 (m, 2H), 7.03 (s, 1H), 6.72 (d, *J* = 9.0 Hz, 1H), 4.57 (t, *J =* 9.3 Hz, 3H), 4.36 - 4.19 (m, 1H), 3.99 (d, *J =* 1.7 Hz, 3H), 2.44 (t, *J =* 12.9 Hz, 1H), 2.32 (dd, *J* = 12.6, 6.1 Hz, 1H), 1.75 (s, 3H).

### Example 123: Preparation of (2R,3S,5R)-3-(3,4-difluoro-2-methoxyphenyl)-N-(3-((Z)-3-ethoxybut-2-enyl)-4-fluorophenyl)-5-methyl-5-(trifluoromethyl)tetrahydrothiophene-2-carboxamide (compound a-27-A)

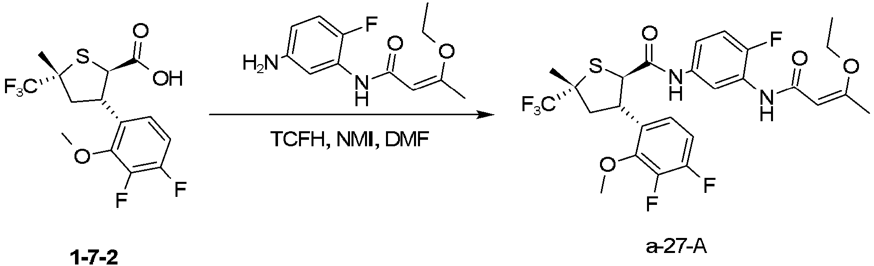

Compound 1-7-2 (50 mg, 0.14 mmol) was dissolved in anhydrous DMF (2 mL), (Z)-N-(5-amino-2-fluorophenyl)-3-ethoxybut-2-enamide (100.1 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (44.6 mg, 0.08 mmol, yield: 55.2%).

MS: m/z = 577.2, [M+H]⁺,¹H NMR (400 MHz, DMSO) δ 10.08-10.02 (m, 2H), 7.75 (s, 1H), 7.44-7.40 (m, 2H), 7.07 (d, *J*=1.6 Hz, 1H), 6.86 (d, *J*=1.6 Hz, 1H), 5.05 (s, 1H), 4.23-4.02 (m, 1H), 3.83(s, 3H), 3.48-3.46(m, 1H), 2.29(s, 3H), 2.17-1.92(m, 2H), 1.38 (s, 3H), 1.23 (s, 3H).

### Example 124: Preparation of 7-(3-(3,4-difluoro-2-methoxyphenyl)-5-methyl-5-(trifluoromethyl)tetrahydrothiophene-2-carboxamido)-4H-benzo[e][1,2,4]thiadiazine-1,1-dioxide (compound a-28-A)

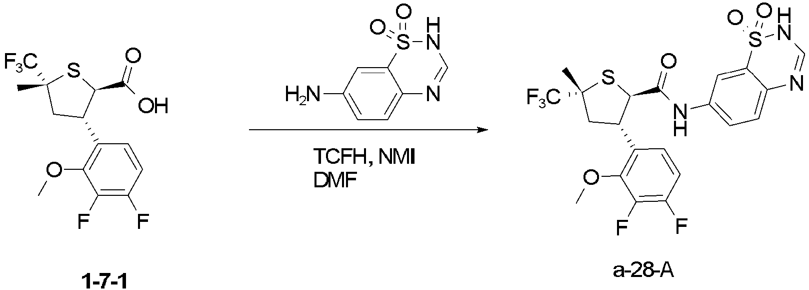

Compound 1-7-1 (50 mg, 0.14 mmol) was dissolved in anhydrous DMF (5 mL), 7-amino-4H-benzo[e][1,2,4]thiadiazine-1,1-dioxide (83 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (48 mg, 0.090 mmol, yield: 64%).

MS: m/z = 536.07, [M+H]⁺, ¹H NMR (400 MHz, DMSO) δ 9.86 (s, 1H), 9.29 (s, 1H), 7.86 - 7.62 (m, 2H), 7.56 - 7.43 (m, 3H), 4.62 (d, *J =* 10.7 Hz, 1H), 4.38 - 4.24 (m, 1H), 3.98 (d, *J =* 1.6 Hz, 3H), 2.43 (t, *J =* 12.9 Hz, 1H), 2.33 (dd, *J =* 12.6, 6.0 Hz, 1H), 2.11 (s, 1H), 1.76 (s, 3H).

### Example 125: Preparation of (2R,3S,5R)-3-(3,4-difluoro-2-methoxyphenyl)-N-(2-(difluoromethyl)pyridin-4-yl)-5-methyl-5-(trifluoromethyl)tetrahydrothiophene-2-carboxamide (compound 244-A)

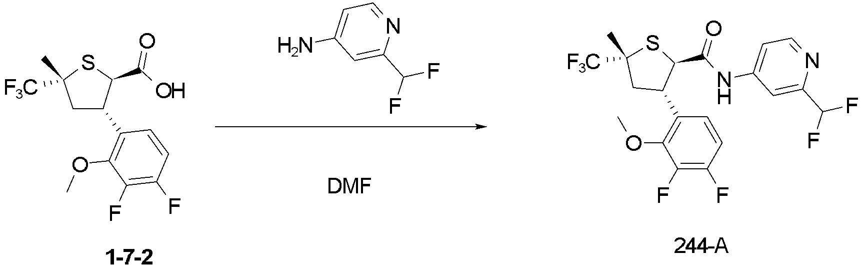

Compound 1-7-2 (50.0 mg, 0.14 mmol) was dissolved in anhydrous DMF (2 mL), 2-(difluoromethyl)pyridin-4-amine (60.5 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (38.6 mg, 0.08 mmol, yield: 57.18%).

MS: m/z = 483.1, [M+H]⁺, ¹H NMR (400 MHz, DMSO) δ 10.89 (s, 1H), 8.51 (d, J = 5.5 Hz, 1H), 7.86 (d, J = 1.8 Hz, 1H), 7.56 (dd, J = 5.3, 1.5 Hz, 1H), 7.17 (ddd, J = 17.0, 7.9, 4.5 Hz, 2H), 6.88 (t, J = 55.0 Hz, 1H), 4.61 (d, J = 10.6 Hz, 1H), 4.28 (ddd, J = 13.1, 10.8, 6.0 Hz, 1H), 3.97 (t, J = 3.8 Hz, 3H), 2.45 (d, J = 12.9 Hz, 1H), 2.32 (dd, J = 12.6, 6.1 Hz, 1H), 1.73 (s, 3H).

### Example 126: Preparation of (2R,3S,5R)-N-(3-azidophenyl)-3-(3,4-difluoro-2-methoxyphenyl)-5-methyl-5-(trifluoromethyl)tetrahydrothiophene-2-carboxamide (compound a-29-A)

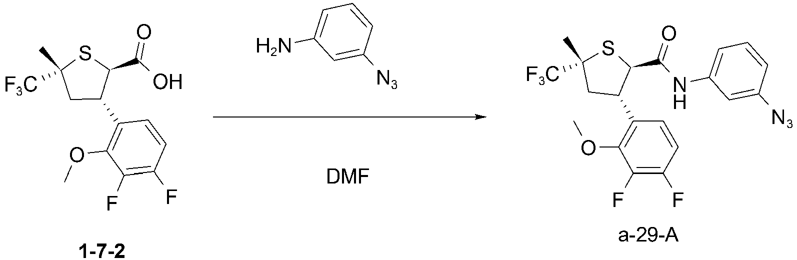

Compound 1-7-2 (50.0 mg, 0.14 mmol) was dissolved in anhydrous DMF (2 mL), 3-azidoaniline (56.3 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (34.4 mg, 0.07 mmol, yield: 51.98%).

MS: m/z = 483.1, [M+H]⁺, ¹H NMR (400 MHz, DMSO) δ 10.42 (s, 1H), 7.42 (t, J = 1.9 Hz, 1H), 7.32 - 7.09 (m, 4H), 6.77 (dd, J = 7.7, 1.0 Hz, 1H), 4.59 (d, J = 10.7 Hz, 1H), 4.34 - 4.18 (m, 1H), 3.96 (d, J = 1.7 Hz, 3H), 2.42 (t, J = 12.9 Hz, 1H), 2.29 (dd, J = 12.6, 6.1 Hz, 1H), 1.72 (s, 3H).

### Example 127: Preparation of (2R,3S,5R)-(4-(3-(3,4-difluoro-2-methoxyphenyl)-5-methyl-5-(trifluoromethyl)tetrahydrothiophene-2-carboxamido)-6-azido)pyridazine (compound a-30-A)

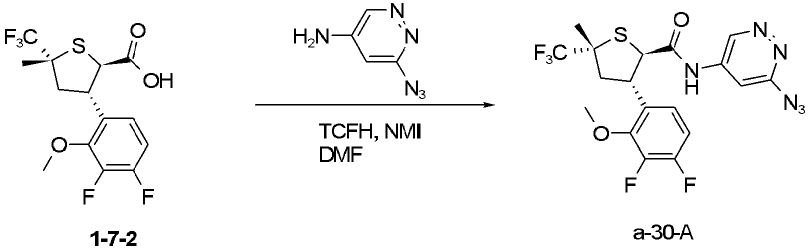

Compound 1-7-2 (50 mg, 0.14 mmol) was dissolved in anhydrous DMF (5 mL), 6-azidopyridazin-4-amine (57 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (61 mg, 0.128 mmol, yield: 91%).

MS: m/z = 475.09, [M+H]⁺, ¹H NMR (400 MHz, DMSO) δ 9.68 (s, 1H), 8.93 (s, 1H), 7.76 (s, 1H), 7.56 - 7.43 (m, 2H), 4.62 (d, *J =* 10.7 Hz, 1H), 4.38 - 4.25 (m, 1H), 3.97 (d, *J* = 1.6 Hz, 3H), 2.43 (t, *J =* 12.9 Hz, 1H), 2.35 (dd, *J* = 12.6, 6.0 Hz, 1H), 1.75 (s, 3H).

### Example 128: Preparation of (2R,3S,5R)-N-(3-azido-4-fluorophenyl)-3-(3,4-difluoro-2-methoxyphenyl)-5-methyl-5-(trifluoromethyl)tetrahydrothiophene-2-carboxamide (compound a-31-A)

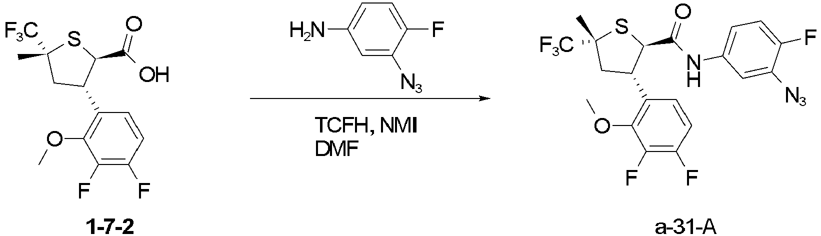

Compound 1-7-2 (50 mg, 0.14 mmol) was dissolved in anhydrous DMF (5 mL), 3-azido-4-fluoroaniline (64 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (40 mg, 0.082 mmol, yield: 58%).

MS: m/z = 491.09, [M+H]⁺, ¹H NMR (400 MHz, DMSO) δ 10.41 (s, 1H), 7.57 - 7.33 (m, 2H), 7.26 - 7.13 (m, 3H), 4.63 (d, *J =* 10.7 Hz, 1H), 4.35 - 4.21 (m, 1H), 3.99 (d, *J* = 1.6 Hz, 3H), 2.47 (t, *J =* 12.9 Hz, 1H), 2.33 (dd, *J* = 12.6, 6.0 Hz, 1H), 1.75 (s, 3H).

### Example 129: Preparation of (2R,3S,5R)-N-(4-azidophenyl)-3-(3,4-difluoro-2-methoxyphenyl)-5-methyl-5-(trifluoromethyl)tetrahydrothiophene-2-carboxamide (compound a-32-A)

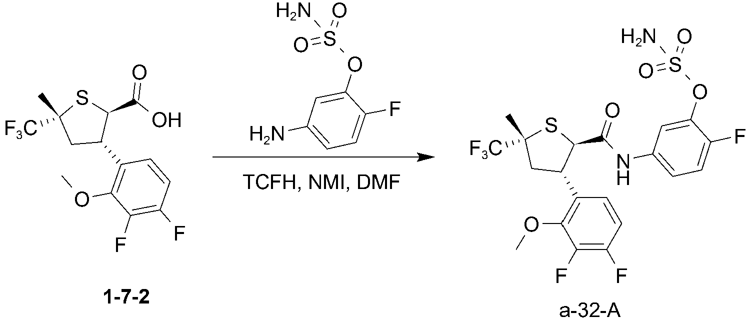

1-7-2 (50 mg, 0.14 mmol) was dissolved in anhydrous DMF (2 mL), 5-amino-2-fluorophenylsulfamate (86.6 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (26.4 mg, 0.05 mmol, yield: 34.6%).

MS: m/z = 545.2, [M+H]⁺,¹H NMR (400 MHz, DMSO) δ 10.02 (s, 1H), 7.16-7.07 (m, 6H), 6.86 (d, *J*=1.6 Hz, 1H), 4.23-4.02 (m, 1H), 3.83(s, 3H), 3.48-3.46(m, 1H), 2.17-1.92(m, 2H), 1.38 (s, 3H).

### Example 130: Preparation of (2R,3S,5R)-N-(4-azidophenyl)-3-(3,4-difluoro-2-methoxyphenyl)-5-methyl-5-(trifluoromethyl)tetrahydrothiophene-2-carboxamide (compound a-33-A)

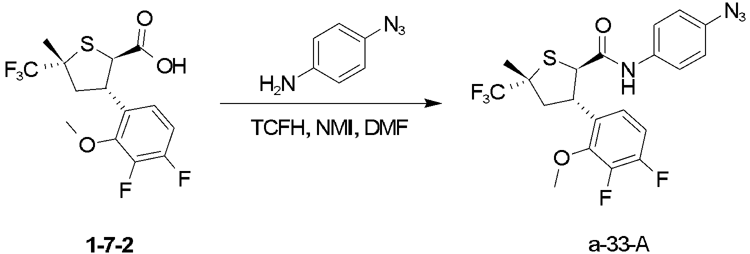

1-7-2 (50 mg, 0.14 mmol) was dissolved in anhydrous DMF (2 mL), 4-azidoaniline (56.3 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (42.2 mg, 0.09 mmol, yield: 63.7%).

MS: m/z = 473.2, [M+H]⁺,¹H NMR (400 MHz, DMSO) δ 10.02 (s, 1H), 7.56 (d, *J*=2.4 Hz, 2H), 7.34 (d, *J*=2.4 Hz, 2H), 7.02-6.86 (m, 2H), 4.23-4.02 (m, 1H), 3.83(s, 3H), 3.48-3.46(m, 1H), 2.17-1.92(m, 2H), 1.38 (s, 3H).

### Example 131: Preparation of (2R,3S,5R)-N-(6-aminopyridazin-4-yl)-3-(3,4-difluoro-2-methoxyphenyl)-5-methyl-5-(trifluoromethyl)tetrahydrothiophene-2-carboxamide (compound 245-A)

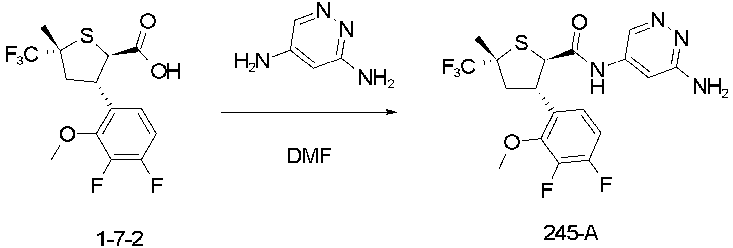

Compound 1-7-2 (30 mg, 0.08 mmol) was dissolved in anhydrous DMF (5 mL), pyridazine-3,5-diamine (26.41 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (14.37 mg, 0.03 mmol, yield: 40%).

MS: m/z = 449.2, [M+H]⁺, ¹H NMR (400 MHz, DMSO) δ 10.83 (s, 1H), 8.27 (d, *J =* 2.4 Hz, 1H), 7.36 (d, *J* = 1.9 Hz, 1H), 7.21 (dd, *J =* 8.5, 4.4 Hz, 2H), 6.52 (s, 2H), 4.83 (d, *J* = 10.6 Hz, 1H), 4.49 - 4.18 (m, 1H), 3.97 (d, *J =* 1.4 Hz, 3H), 1.75 (s, 3H).

### Example 132: Preparation of (2R,3S,5R)-N-(2-cyanoaminopyridin-4-yl)-3-(3,4-difluoro-2-methoxyphenyl)-5-methyl-5-(trifluoromethyl)tetrahydrothiophene-2-carboxamide (compound a-34-A)

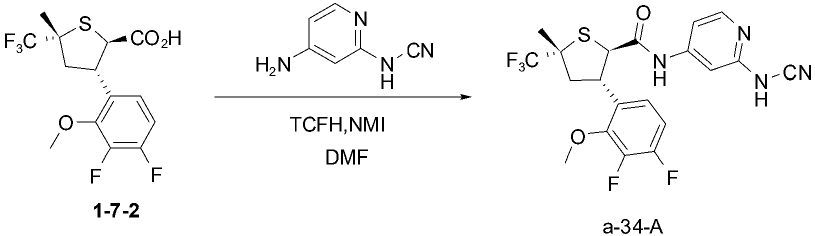

For the experimental procedures, reference may be made to Example 1.

MS: m/z = 473.2[M+H]+, 1H NMR (400 MHz, DMSO) δ 10.85 (s, 1H), 7.62 (d, J = 6.9 Hz, 1H), 7.40 (d, J = 1.8 Hz, 1H), 7.24 - 7.09 (m, 2H), 6.70 - 6.62 (m, 1H), 4.59 (d, J = 10.5 Hz, 1H), 4.25 (ddd, J = 13.1, 10.7, 6.1 Hz, 1H), 3.97 (d, J = 2.0 Hz, 3H), 3.16 (d, J = 4.9 Hz, 1H), 2.46 (d, J = 12.9 Hz, 1H), 2.32 (dd, J = 12.5, 6.1 Hz, 1H), 1.74 (d, J = 9.9 Hz, 3H).

### Example 133: Preparation of (2R,3S,5R)-3-(3,4-difluoro-2-methoxyphenyl)-5-methyl-N-(3-(6-methyl-4,8-dioxo-1,3,6,2-diazaborinin-2-yl)phenyl)-5-(trifluoromethyl)tetrahydrothiophene-2-carboxamide (compound a-35-A)

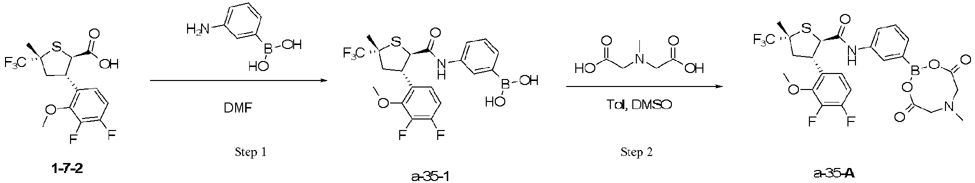

### Step 1: Preparation of (3-((2R,3S,5R)-3-(3,4-difluoro-2-methoxyphenyl)-5-methyl-5-(trifluoromethyl)tetrahydrothiophene-2-carboxamido)phenyl)boronic acid (compound a-35-1)

Compound 1-7-2 (50.0 mg, 0.14 mmol) was dissolved in anhydrous DMF (2 mL), (3-aminophenyl)boronic acid (57.6 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (46.1 mg, 0.10 mmol, yield: 69.33%). MS: m/z = 476.1, [M+H]⁺.

### Step 2: Preparation of (2R,3S,5R)-3-(3,4-difluoro-2-methoxyphenyl)-5-methyl-N-(3-(6-methyl-4,8-dioxo-1,3,6,2-diazaborinin-2-yl)phenyl)-5-(trifluoromethyl)tetrahydrothiophene-2-carboxamide (compound a-35-A)

The product of step 1 (46.1 mg, 0.10 mmol) and 2,2'-(methylazanediyl)diacetic acid (22.1 mg, 0.15 mmol) were dissolved in toluene (30 mL) and dimethyl sulfoxide (3 mL). The mixture was heated to 160 °C and stirred. After the reaction was completed, the mixture was separated by Pre-HPLC to obtain the target compound (26.9 mg, 0.05 mmol, yield: 45.87%).

MS: m/z = 587.1, [M+H]⁺, ¹H NMR (400 MHz, DMSO) δ 10.25 (d, J = 23.2 Hz, 1H), 7.88 - 6.98 (m, 6H), 4.65 (d, J = 7.8 Hz, 1H), 4.36 (d, J = 17.1 Hz, 2H), 4.11 (d, J = 16.7 Hz, 1H), 4.00 (s, 3H), 3.39 (s, 3H), 2.53 - 2.31 (m, 4H), 1.76 (s, 3H).

### Example 134: Preparation of (2R,3S,5R)-3-(3,4-difluoro-2-methoxyphenyl)-N-(1,1-dioxide-3-oxo-2,3-dihydrobenzo[d]isothiazol-6-yl)-5-methyl-5-(trifluoromethyl)tetrahydrothiophene-2-carboxamide (compound a-36-A)

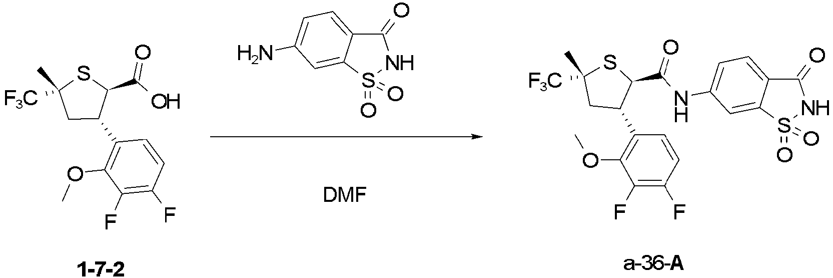

Compound 1-7-2 (50.0 mg, 0.14 mmol) was dissolved in anhydrous DMF (2 mL), 6-aminobenzo[d]isothiazol-3(2H)-one-1,1-dioxide (83.2 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (45.6 mg, 0.09 mmol, yield: 60.77%).

MS: m/z = 537.1, [M+H]⁺, ¹H NMR (400 MHz, DMSO) δ 10.65 (s, 1H), 8.21 (s, 1H), 7.90 (d, J = 1.2 Hz, 1H), 7.52 (dt, J = 20.0, 4.9 Hz, 2H), 7.22 - 7.06 (m, 2H), 4.61 (d, J = 10.7 Hz, 1H), 4.36 - 4.20 (m, 1H), 3.98 (d, J = 1.6 Hz, 3H), 2.44 (t, J = 12.9 Hz, 1H), 2.32 (dd, J = 12.6, 6.1 Hz, 1H), 1.74 (s, 3H).

### Example 135: Preparation of (2R,3S,5R)-3-(3,4-difluoro-2-methoxyphenyl)-5-methyl-N-((1,2,3)triazolo(1,5-a)pyridin-5-yl)-5-(trifluoromethyl)tetrahydrothiophene-2-carboxamide (compound 246-A)

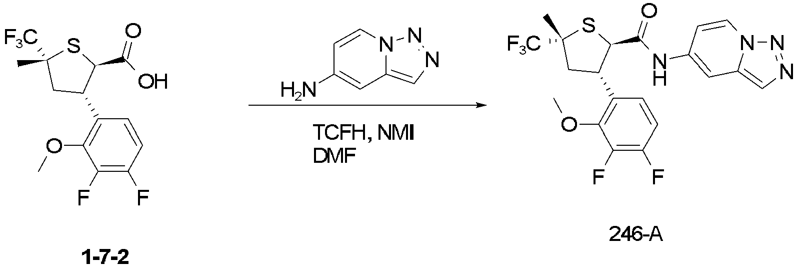

Compound 1-7-2 (50 mg, 0.14 mmol) was dissolved in anhydrous DMF (5 mL), [1,2,3]triazolo[1,5-a]pyridin-5-amine (56 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (47 mg, 0.10 mmol, yield: 71%). MS: m/z = 473.10, [M+H]⁺, ¹H NMR (400 MHz, DMSO) δ 9.53 (m, 2H), 7.87 - 7.63 (m, 2H), 7.36 - 7.23 (m, 3H), 4.63 (d, *J=* 10.7 Hz, 1H), 4.35 - 4.21 (m, 1H), 3.98 (d, *J* = 1.6 Hz, 3H), 2.46 (t, *J =* 12.9 Hz, 1H), 2.33 (dd, *J =* 12.6, 6.0 Hz, 1H), 1.75 (s, 3H).

### Example 136: Preparation of (2R,3S,5R)-3-(3,4-difluoro-2-methoxyphenyl)-N-(1, 1 -dioxide-2,3-dihydrobenzo[d]isothiazol-6-yl)-5-methyl-5-(trifluoromethyl)tetrahydrothiophene-2-carboxamide (compound a-37-A)

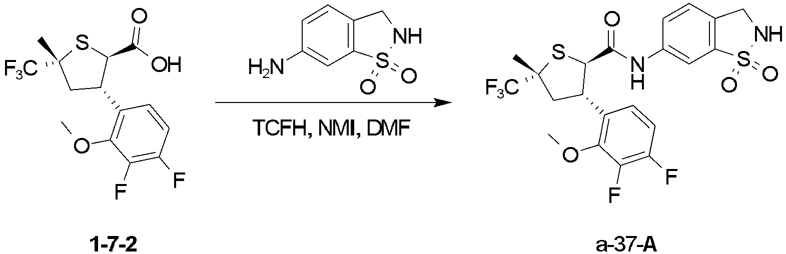

1-7-2 (50 mg, 0.14 mmol) was dissolved in anhydrous DMF (2 mL), ethyl 2-(4-aminopyridin-2-yl)acetate (75.7 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (28.8 mg, 0.05 mmol, yield: 39.6%).

MS: m/z = 523.2, [M+H]⁺,¹H NMR (400 MHz, DMSO) δ 9.92 (s, 1H), 8.85 (d, *J*=2.4 Hz, 1H), 7.49 (s, 1H), 7.41 (d, *J*=2.4 Hz, 1H), 7.02-6.86 (m, 2H), 4.23-4.02 (m, 5H), 3.83(s, 3H), 3.48-3.46(m, 1H), 2.17-1.92(m, 2H), 1.38 (s, 3H), 1.21 (s, 3H).

### Example 137: Preparation of (2R,3S,5R)-3-(3,4-difluoro-2-methoxyphenyl)-N-(6-mercaptopyridin-3-yl)-5-methyl-5-(trifluoromethyl)tetrahydrothiophene-2-carboxamide (compound 247-A)

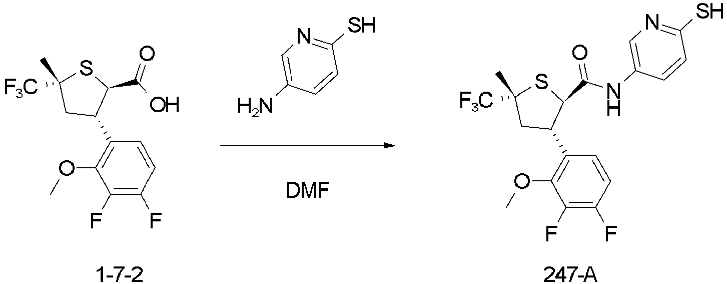

Compound 1-7-2 (30 mg, 0.08 mmol) was dissolved in anhydrous DMF (5 mL), 5-aminopyridine-2-thiol (30.24 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (9.28 mg, 0.02 mmol, yield: 25%).

MS: m/z = 465.1, [M+H]⁺, ¹H NMR (400 MHz, DMSO) δ 13.38 (s, 1H), 10.43 (s, 1H), 8.15 (d, *J =* 3.2 Hz, 1H), 7.23 (dtd, *J* = 13.1, 9.5, 4.6 Hz, 4H), 4.55 (d, *J =* 10.7 Hz, 1H), 4.32 - 4.19 (m, 1H), 3.99 (d, *J =* 1.9 Hz, 3H), 2.46 (t, *J =* 12.9 Hz, 1H), 2.33 (dd, *J* = 12.6, 6.1 Hz, 1H), 1.74 (s, 3H).

### Example 138: Preparation of (2R,3S,5R)-3-(3,4-difluoro-2-methoxyphenyl)-5-methyl-5-(trifluoromethyl)-N-(3-(3-(trifluoromethane)-3H-diazirin-3-yl)phenyl)tetrahydrothiophene-2-carboxamide (compound a-38-A)

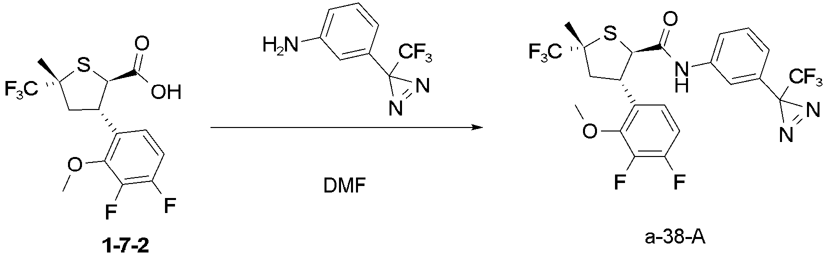

Compound 1-7-2 (50.0 mg, 0.14 mmol) was dissolved in anhydrous DMF (2 mL), 3-(3-(trifluoromethyl)-3H-diazirin-3-yl)aniline (84.6 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (41.8 mg, 0.08 mmol, yield: 57.37%).

MS: m/z = 537.1, [M+H]⁺, 1H NMR (400 MHz, DMSO) δ 10.52 (s, 1H), 7.64 - 7.54 (m, 2H), 7.43 - 7.36 (m, 1H), 7.24 - 7.08 (m, 2H), 6.89 (d, J = 7.8 Hz, 1H), 4.58 (d, J = 10.7 Hz, 1H), 4.35 - 4.20 (m, 1H), 3.98 (dd, J = 12.1, 5.0 Hz, 3H), 2.44 (t, J = 12.9 Hz, 1H), 2.30 (dd, J = 12.6, 6.1 Hz, 1H), 1.73 (s, 3H).

### Example 139: Preparation of (3-((2R,3S,5R)-3-(3,4-difluoro-2-methoxyphenyl)-5-methyl-5-(trifluoromethyl)tetrahydrothiophene-2-carboxamido)phenyl sulfamate (compound a-39-A)

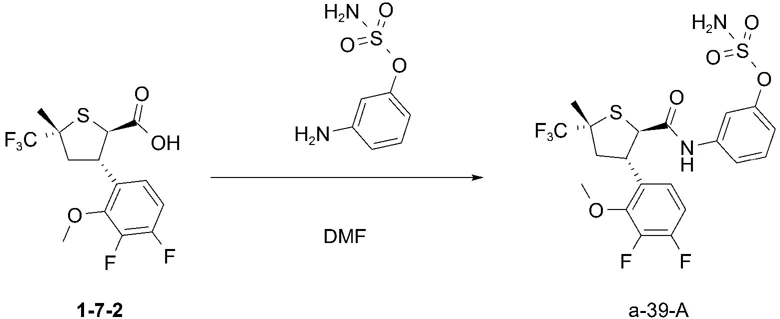

Compound 1-7-2 (50.0 mg, 0.14 mmol) was dissolved in anhydrous DMF (2 mL), 3-aminophenyl sulfamate (79.0 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (35.1 mg, 0.07 mmol, yield: 47.62%).

MS: m/z = 527.1, [M+H]⁺, 1H NMR (400 MHz, DMSO) δ 10.46 (s, 1H), 7.98 (s, 2H), 7.58 (s, 1H), 7.46 - 7.29 (m, 2H), 7.25 - 7.08 (m, 2H), 6.97 (t, J = 11.9 Hz, 1H), 4.60 (d, J = 10.7 Hz, 1H), 4.28 (td, J = 12.7, 6.1 Hz, 1H), 3.97 (s, 3H), 2.43 (t, J = 12.9 Hz, 1H), 2.30 (dd, J = 12.5, 6.1 Hz, 1H), 1.73 (s, 3H).

### Example 140: Preparation of 4-((2R,3S,5R)-3-(3,4-difluoro-2-methoxyphenyl)-5-methyl-5-(trifluoromethyl)tetrahydrothiophene-2-carboxamido)pyridin-2-yl sulfamate (compound a-40-A)

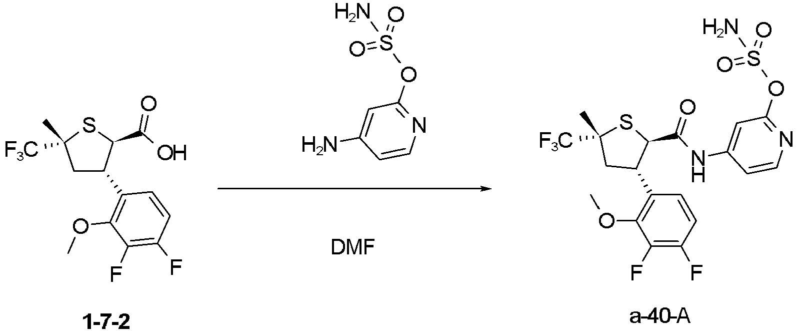

Compound 1-7-2 (50.0 mg, 0.14 mmol) was dissolved in anhydrous DMF (2 mL), 4-aminopyridin-2-sulfamate (79.4 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (33.8 mg, 0.06 mmol, yield: 45.79%).

MS: m/z = 527.1, [M+H]⁺, ¹H NMR (400 MHz, DMSO) δ 10.00 (s, 1H), 7.46 (s, 1H), 7.35 - 7.04 (m, 2H), 5.95 (d, J = 5.2 Hz, 1H), 4.52 (d, J = 11.1 Hz, 1H), 4.08 (td, J = 12.3, 6.1 Hz, 1H), 3.95 (d, J = 1.2 Hz, 3H), 2.33 - 2.18 (m, 2H), 1.67 (s, 3H).

### Example 141: Preparation of (2R,3S,5R)-N-(3-(3H-diazirin-3-yl)phenyl)-3-(3,4-difluoro-2-methoxyphenyl)-5-methyl-5-(trifluoromethyl)tetrahydrothiophene-2-carboxamide (compound a-41-A)

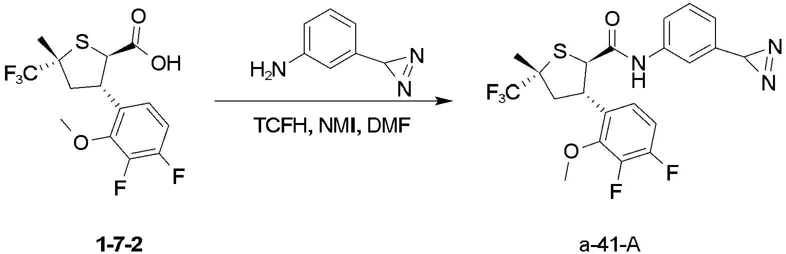

Compound 1-7-2 (50 mg, 0.14 mmol) was dissolved in anhydrous DMF (2 mL), 3-(3H-diazirin-3-yl)aniline (55.9 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (18.9 mg, 0.04 mmol, yield: 28.6%).

MS: m/z = 472.2, [M+H]⁺,¹H NMR (400 MHz, DMSO) δ 10.02 (s, 1H), 7.72 (s, 1H), 7.63 (d, *J*=2.4 Hz, 1H), 7.29 (d, *J*=2.0 Hz, 1H), 7.02-6.86 (m, 3H), 4.23-4.21 (m, 1H), 3.83(s, 3H), 3.48-3.46(m, 1H), 2.79 (s, 1H), 2.17-1.92(m, 2H), 1.38 (s, 3H).

### Example 142: Preparation of (2R,3S,5R)-N-(3-(1,2,4-oxadiazol-3-yl)phenyl)-3-(3,4-difluoro-2-methoxyphenyl)-5-methyl-5-(trifluoromethyl)tetrahydrothiophene-2-carboxamide (compound a-42-A)

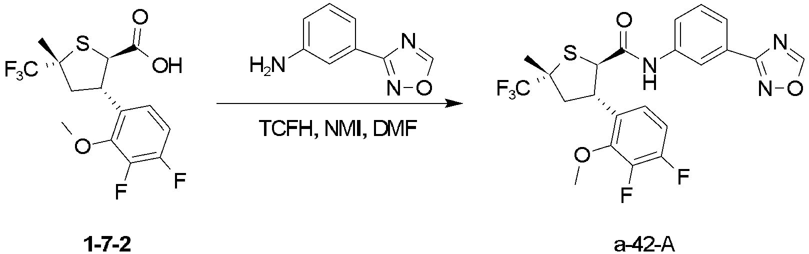

Compound 1-7-2 (50 mg, 0.14 mmol) was dissolved in anhydrous DMF (2 mL), 3-(1,2,4-oxadiazol-3-yl)aniline (67.6 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (22.9 mg, 0.05 mmol, yield: 32.7%).

MS: m/z = 500.2, [M+H]⁺,¹H NMR (400 MHz, DMSO) δ 10.02 (s, 1H), 8.12 (d, *J*=2.0 Hz, 1H),7.74-7.68 (m, 3H), 7.07 (d, *J*=2.4 Hz, 1H), 6.86 (d, *J*=2.4 Hz, 1H), 4.23-4.21 (m, 1H), 3.83(s, 3H), 3.48-3.46(m, 1H), 2.17-1.92(m, 2H), 1.38 (s, 3H).

### Example 143: Preparation of (2R,3S,5R)-N-(benzo[c][1,2,5]oxadiazol-5-yl)-3-(3,4-difluoro-2-methoxyphenyl)-5-methyl-5-(trifluoromethyl)tetrahydrothiophene-2-carboxamide (compound 248-A)

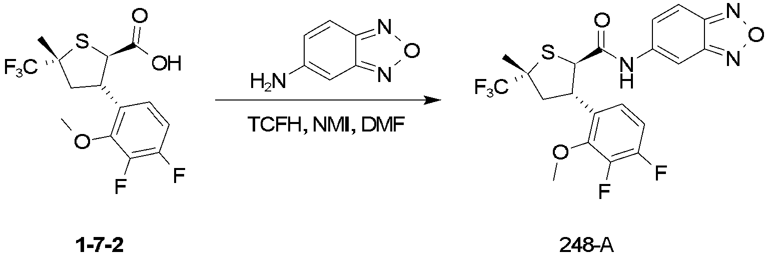

Compound 1-7-2 (50 mg, 0.14 mmol) was dissolved in anhydrous DMF (2 mL), benzo[c][1,2,5]oxadiazol-5-amine (56.7 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (28.1 mg, 0.06 mmol, yield: 42.3%).

MS: m/z = 474.2, [M+H]⁺,¹H NMR (400 MHz, DMSO) δ 10.02 (s, 1H), 7.89-7.88 (m, 2H), 7.37 (d, *J*=2.0 Hz, 1H), 7.07 (d, *J*=2.0 Hz, 1H), 6.86 (d, *J*=2.0 Hz, 1H), 4.23-4.21 (m, 1H), 3.83(s, 3H), 3.48-3.46(m, 1H), 2.17-1.92(m, 2H), 1.38 (s, 3H).

### Example 144: Preparation of (2R,3S,5R)-3-(3,4-difluoro-2-methoxyphenyl)-5-methyl-N-(4-methyl-1-((trifluoromethyl)sulfonyl)-1H-pyrazol-3-yl)-5-(trifluoromethane)tetrahydrothiophene-2-carboxamide (compound a-43-A)

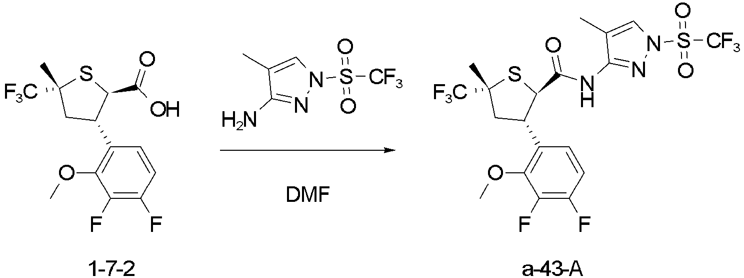

Compound 1-7-2 (30 mg, 0.08 mmol) was dissolved in anhydrous DMF (5 mL), 4-methyl-1-((trifluoromethyl)sulfonyl)-1H-pyrazol-3-amine (57.96 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (11.35 mg, 0.02 mmol, yield: 25%).

MS: m/z = 567.8, [M+H]⁺, ¹H NMR (400 MHz, DMSO) δ 10.46 (s, 1H), 8.53 (s, 1H), 7.20 (dd, *J =* 10.2, 6.7 Hz, 2H), 4.80 (d, *J =* 10.7 Hz, 1H), 4.36 - 4.26 (m, 1H), 3.99 (d, *J =* 1.8 Hz, 3H), 2.48 (t, *J =* 12.9 Hz, 1H), 2.38 - 2.33 (m, 1H), 2.30 (s, 3H), 1.75 (s, 3H).

### Example 145: Preparation of (2R,3S,5R)-3-(3,4-difluoro-2-methoxyphenyl)-N-(3-(ethylsulfonamido)phenyl)-5-methyl-5-(trifluoromethyl)tetrahydrothiophene-2-carboxamide (compound a-44-A)

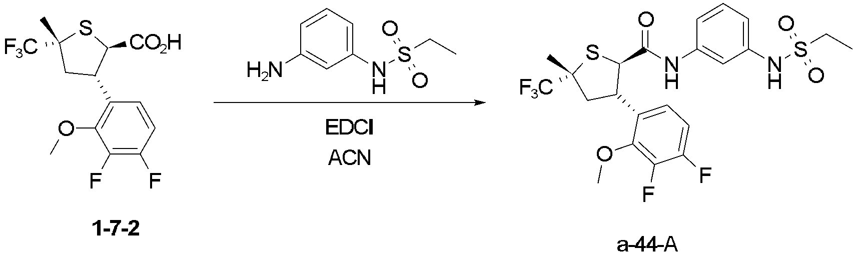

For the experimental procedures, reference may be made to Example 70.

MS: m/z = 539.1[M+H]+, ¹H NMR (400 MHz, DMSO) δ 10.85 (s, 1H), 7.62 (d, J = 6.9 Hz, 1H), 7.40 (d, J = 1.8 Hz, 1H), 7.24 - 7.09 (m, 2H), 6.70 - 6.62 (m, 1H), 4.59 (d, J = 10.5 Hz, 1H), 4.25 (ddd, J = 13.1, 10.7, 6.1 Hz, 1H), 3.97 (d, J = 2.0 Hz, 3H), 3.16 (d, J = 4.9 Hz, 1H), 2.46 (d, J = 12.9 Hz, 1H), 2.32 (dd, J = 12.5, 6.1 Hz, 1H), 1.74 (d, J = 9.9 Hz, 3H).

### Example 146: Preparation of (2R,3S,5R)-N-(3-(N-(2-aminoethyl)sulfamoyl)phenyl)-3-(3,4-difluoro-2-methoxyphenyl)-5-methyl-5-(trifluoromethyl)tetrahydrothiophene-2-carboxamide (compound a-45-A)

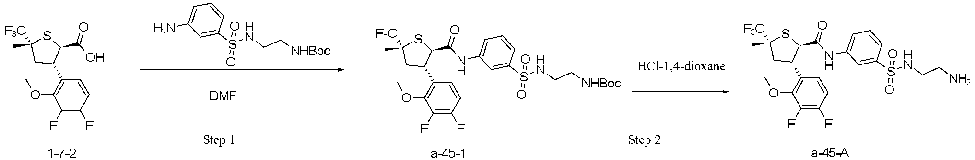

### Step 1: Preparation of tert-butyl (2-(3-((2R,3S,5R)-3-(3,4-difluoro-2-methoxyphenyl)-5-methyl-5-(trifluoromethyl)tetrahydrothiophene-2-carboxamido)phenylsulfonamido)ethyl)carbamate (compound a-45-1)

Compound 1-7-2 (50.0 mg, 0.14 mmol) was dissolved in anhydrous DMF (2 mL), tert-butyl (2-((3-aminophenyl)sulfonamido)ethyl)carbamate (132.4 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (50.6 mg, 0.08 mmol, yield: 55.31%). MS: m/z = 654.2, [M+H]⁺.

### Step 2: Preparation of (2R,3S,5R)-N-(3-(N-(2-aminoethyl)sulfamoyl)phenyl)-3-(3,4-difluoro-2-methoxyphenyl)-5-methyl-5-(trifluoromethyl)tetrahydrothiophene-2-carboxamide (compound a-45-A)

The product of step 1 (50.6 mg, 0.08 mmol) was dissolved in 1,4-dioxane (5 mL), and the mixture was stirred at room temperature. After the reaction was completed, the mixture was separated by Pre-HPLC to obtain the target compound (36.8 mg, 0.07 mmol, yield: 83.19%).

MS: m/z = 554.1, [M+H]⁺, ¹H NMR (400 MHz, DMSO) δ 10.71 (s, 1H), 8.18 (s, 1H), 7.90 (dd, J = 16.8, 11.1 Hz, 4H), 7.65 (d, J = 8.1 Hz, 1H), 7.52 (dd, J = 17.0, 9.1 Hz, 2H), 7.25 - 7.12 (m, 2H), 4.64 (d, J = 10.7 Hz, 1H), 4.28 (td, J = 12.9, 6.1 Hz, 1H), 3.97 (d, J = 1.5 Hz, 3H), 2.89 (dd, J = 18.2, 12.6 Hz, 4H), 2.44 (t, J = 12.9 Hz, 1H), 2.32 (dt, J = 12.5, 6.3 Hz, 1H), 1.73 (s, 3H).

### Example 147: Preparation of (2R,3S,5R)-N-(2-(1,2,4-oxadiazol-3-yl)pyridin-4-yl)-3-(3,4-difluoro-2-methoxyphenyl)-N-hydroxy-5-methyl-5-(trifluoromethyl)tetrahydrothiophene-2-carboxamide (compound a-46-A)

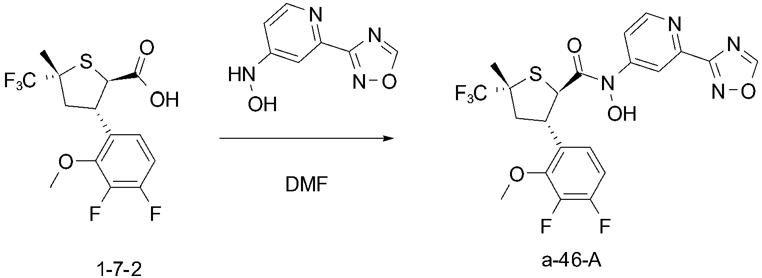

Compound 1-7-2 (30 mg, 0.08 mmol) was dissolved in anhydrous DMF (5 mL), N-(2-(1,2,4-oxadiazol-3-yl)pyridin-4-yl)hydroxyamine (42.72 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (15.48 mg, 0.03 mmol, yield: 37%).

MS: m/z = 516.9, [M+H]⁺, ¹H NMR (400 MHz, DMSO) δ 10.94 (s, 1H), 9.79 (d, *J =* 7.0 Hz, 1H), 8.62 (d, *J =* 5.5 Hz, 1H), 8.36 (d, *J =* 1.3 Hz, 1H), 7.66 (dd, *J* = 5.5, 1.7 Hz, 1H), 7.36 - 7.08 (m, 2H), 4.65 (d, *J =* 10.6 Hz, 1H), 4.42 - 4.22 (m, 1H), 3.99 (d, *J* = 11.6 Hz, 3H), 2.49 (d, *J =* 13.0 Hz, 1H), 2.38 - 2.28 (m, 1H), 1.77 (s, 3H).

### Example 148: Preparation of (2R,3S,5R)-N-(4-amino-3-sulfamoylphenyl)-3-(3,4-difluoro-2-methoxyphenyl)-5-methyl-5-(trifluoromethyl)tetrahydrothiophene-2-carboxamide (compound 249-A)

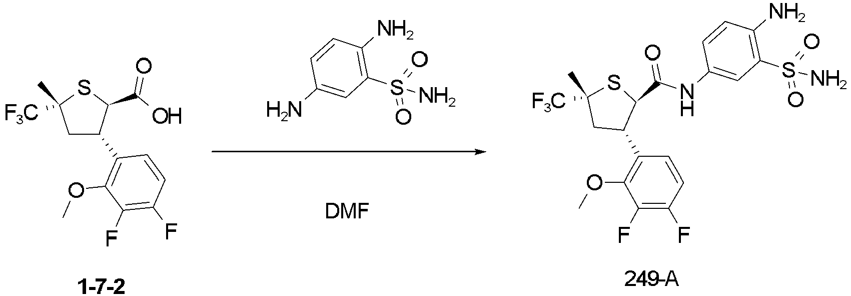

Compound 1-7-2 (50.0 mg, 0.14 mmol) was dissolved in anhydrous DMF (2 mL), 2,5-diaminobenzenesulfonamide (78.6 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (26.3 mg, 0.05 mmol, yield: 35.73%).

MS: m/z = 526.1, [M+H]⁺, 1H NMR (400 MHz, DMSO) δ 10.08 (s, 1H), 7.79 (s, 1H), 7.36 - 7.10 (m, 5H), 6.70 (d, J = 8.7 Hz, 1H), 5.69 (s, 1H), 4.54 (d, J = 10.7 Hz, 1H), 4.26 (d, J = 12.1 Hz, 1H), 3.96 (s, 3H), 2.39 (t, J = 12.8 Hz, 1H), 2.28 (dd, J = 12.3, 5.8 Hz, 1H), 1.72 (s, 3H).

### Example 149: Preparation of (2R,3S,5R)-3-(3,4-difluoro-2-methoxy-phenyl)-5-methyl-N-[2-(1,2,4-oxadiazol-3-yl)-4-pyridyl]-5-(trifluoromethyl)tetrahydrothiophene-2-carboxamide (compound a-48-A)

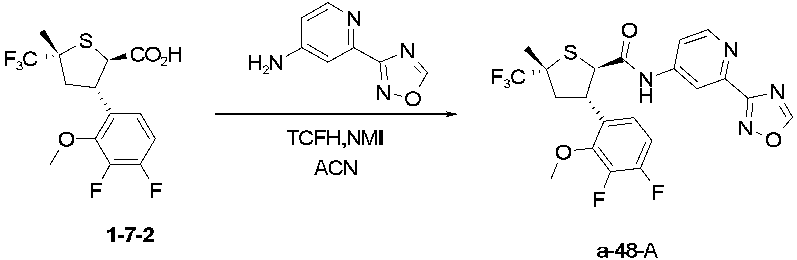

For the experimental procedures, reference may be made to Example 1.

MS: m/z = 501.3[M+H]+, 1H NMR (400 MHz, DMSO) δ 10.93 (s, 1H), 9.75 (s, 1H), 8.59 (d, J = 5.5 Hz, 1H), 8.33 (d, J = 1.9 Hz, 1H), 7.64 (dd, J = 5.5, 2.1 Hz, 1H), 7.26 - 7.09 (m, 2H), 4.63 (d, J = 10.6 Hz, 1H), 4.29 (ddd, J = 13.1, 10.8, 6.1 Hz, 1H), 3.98 (d, J = 1.9 Hz, 3H), 2.46 (d, J = 12.9 Hz, 1H), 2.33 (dd, J = 12.6, 6.1 Hz, 1H), 1.74 (s, 3H).

### Example 150: Preparation of (2R,3S,5R)-3-(3,4-difluoro-2-methoxy-phenyl)-N-[3-(2-hydroxyethylsulfonylamino)phenyl]-5-methyl-5-(trifluoromethyl)tetrahydrothiophene-2-carboxamide (compound a-49-A)

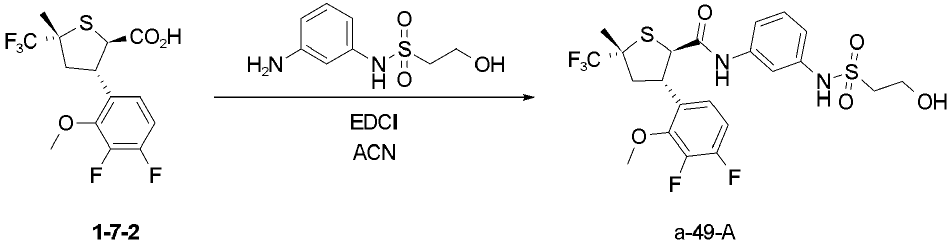

For the experimental procedures, reference may be made to Example 70.

MS: m/z = 555.3[M+H]+, ¹H NMR (400 MHz, DMSO) δ 10.32 (s, 1H), 7.42 (t, *J =* 1.9 Hz, 1H), 7.31 - 7.26 (m, 1H), 7.23 - 7.14 (m, 3H), 6.87 (dd, *J =* 8.0, 1.1 Hz, 1H), 4.60 (d, *J =* 10.7 Hz, 1H), 4.32 - 4.22 (m, 1H), 3.97 (d, *J =* 1.8 Hz, 3H), 3.70 (t, *J =* 6.7 Hz, 2H), 3.20 (t, *J =* 6.8 Hz, 2H), 2.40 (t, *J =* 12.9 Hz, 1H), 2.30 (dd, *J =* 12.6, 6.2 Hz, 1H), 1.72 (s, 3H).

### Example 151: Preparation of tert-butyl N-(2-((3-(((2R,3S,5R)-3-(3,4-difluoro-2-methoxy-phenyl)-5-methyl-5-(trifluoromethyl)tetrahydrothiophene-2-carbonyl)amino)phenyl)sulfamoyl)ethyl)-N-methylcarbamate (compound a-50-A)

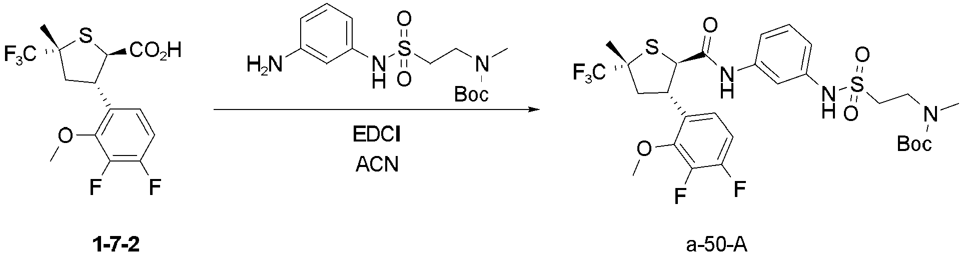

For the experimental procedures, reference may be made to Example 70.

MS: m/z =668.4[M+H]+, ¹H NMR (400 MHz, DMSO) δ 10.31 (s, 1H), 9.92 (s, 1H), 7.46 (s, 1H), 7.30 - 7.11 (m, 4H), 6.88 (d, *J =* 7.6 Hz, 1H), 4.60 (d, *J =* 10.7 Hz, 1H), 4.33 - 4.22 (m, 1H), 3.96 (d, *J =* 1.8 Hz, 3H), 3.44 (s, 2H), 3.28 (s, 2H), 2.73 (s, 3H), 2.40 (t, *J =* 12.9 Hz, 1H), 2.30 (dd, *J =* 12.6, 6.2 Hz, 1H), 1.72 (s, 3H), 1.23 (d, *J =* 77.2 Hz, 9H).

### Example 152: Preparation of (2R,3S,5R)-N-(2-aminomethylthio-4-pyridyl)-3-(3,4-difluoro-2-methoxy-phenyl)-5-methyl-5-(trifluoromethyl)tetrahydrothiophene-2-carboxamide (compound a-51-A)

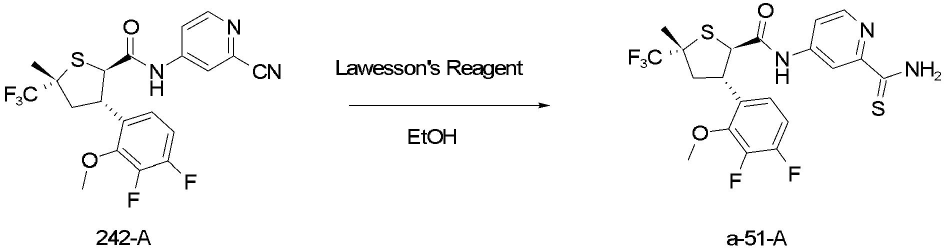

Compound 242-A (85 mg, 185.83 µmol) was added to a pressure-resistant tube and dissolved in EtOH (2 mL), and Lawesson's reagent (375.80 mg, 929.13 µmol) was added. The mixture was refluxed at 90 °C. After the reaction was completed, the crude product was subjected to preparative chromatography to obtain the target compound (36 mg, 73.25 µmol, yield: 39.42%).

MS: m/z =492.2[M+H]+, ¹H NMR (400 MHz, DMSO) δ 10.92 (s, 1H), 10.17 (d, *J =* 2.3 Hz, 1H), 9.89 (d, *J =* 2.6 Hz, 1H), 8.57 (d, *J* = 2.0 Hz, 1H), 8.43 (d, *J =* 5.5 Hz, 1H), 7.78 (dd, *J* = 5.5*,* 2.1 Hz, 1H), 7.24 - 7.10 (m, 2H), 4.63 (d, *J* = 10.6 Hz, 1H), 4.34 - 4.23 (m, 1H), 3.97 (d, *J =* 1.9 Hz, 3H), 2.44 (d, *J =* 12.9 Hz, 1H), 2.32 (dd, *J =* 12.6, 6.1 Hz, 1H), 1.74 (d, *J =* 8.3 Hz, 3H).

### Example 153: Preparation of 6-(3-(3,4-difluoro-2-methoxyphenyl)-5-methyl-5-(trifluoromethyl)tetrahydrothiophene-2-carboxamido)-1-methoxybenzo[c][1,2]oxaborol-3(1H)-one (compound a-52)

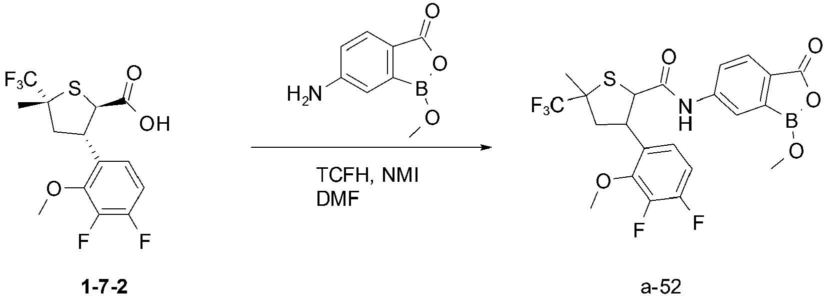

Compound 1-7-2 (50 mg, 0.14 mmol) was dissolved in anhydrous DMF (5 mL), 6-amino-1-methoxybenzo[c][1,2]oxaborol-3(1H)-one (74 mg, 0.42 mmol) was added, and TCFH (197 mg, 0.70 mmol) and NMI (92 mg, 1.12 mmol) were added in an ice bath. The mixture was allowed to naturally warm to room temperature and then stirred for 1 h. After the reaction was completed, the mixture was separated by Pre-HPLC to obtain the target compound (35 mg, 0.068 mmol, yield: 49%). MS: m/z = 516.10, [M+H]⁺, ¹H NMR (400 MHz, DMSO) δ 9.98 (s, 1H), 7.57 = 7.33 (m, 3H), 7.26 - 7.13 (m, 2H), 4.63 (d, J = 10.7 Hz, 1H), 4.35 - 4.21 (m, 1H), 3.99 (d, J = 1.6 Hz, 3H), 3.42 (s, 3H), 2.47 (t, J = 12.9 Hz, 1H), 2.33 (dd, J = 12.6, 6.0 Hz, 1H), 1.75 (s, 3H).

### Example 154: Preparation of 3-(1-((2R,3S,SR)-3-(3,4-difluoro-2-methoxyphenyl)-5-methyl-5-(trifluoromethyl)tetrahydrothiophene-2-formyl)hydrazineyl)benzenesulfonamide (compound a-83-A)

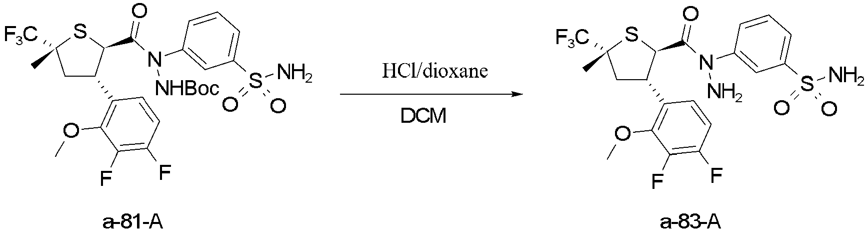

Compound a-81-A (30 mg, 0.05 mmol) was dissolved in anhydrous DCM (2 mL), and a solution of hydrochloric acid in dioxane (2 mL) was added in an ice bath. The mixture was allowed to naturally warm to room temperature and then stirred. After the reaction was completed, the mixture was separated by Pre-HPLC to obtain the target compound (15 mg, 0.03 mmol, yield: 57.62%).

MS: m/z = 525.1, ¹H NMR (400 MHz, DMSO) δ 7.85 (s, 1H), 7.69 (d, *J =* 7.7 Hz, 1H), 7.59 (d, *J =* 6.5 Hz, 1H), 7.47 (s, 3H), 7.15 (t, *J =* 8.1 Hz, 1H), 4.27 (s, 1H), 4.00 (d, *J =* 13.2 Hz, 4H), 2.41 - 2.26 (m, 2H), 2.08 (s, 2H), 1.73 (s, 3H).

### Example 155: Preparation of (2R,3S,5R)-3-(3,4-difluoro-2-methoxyphenyl)-5-methyl-N-(3-((2-(methylamino)ethyl)sulfonamido)phenyl)-5-(trifluoromethyl)tetrahydrothiophene-2-carboxamide (compound a-54-A)

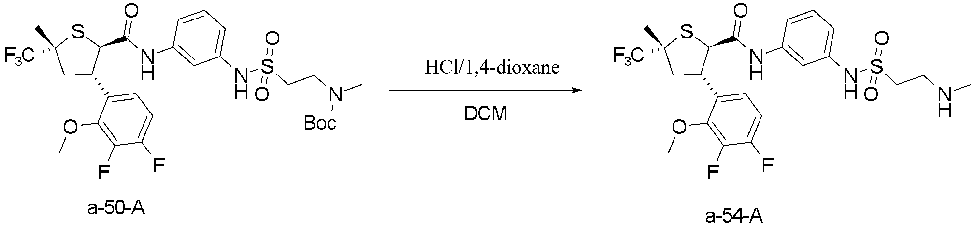

For the experimental procedures, reference may be made to Example 154.

MS: m/z =568.2[M+H]+, ¹H NMR (400 MHz, DMSO) δ 10.36 (s, 1H), 8.24 (s, 1H), 7.44 (s, 1H), 7.28 (d, *J =* 8.4 Hz, 1H), 7.18 (dt, *J =* 9.5, 7.2 Hz, 3H), 6.87 (d, *J =* 7.9 Hz, 1H), 4.60 (d, *J =* 10.7 Hz, 1H), 4.30 - 4.24 (m, 1H), 3.96 (d, *J =* 1.7 Hz, 4H), 3.23 (t, *J =* 7.2 Hz, 2H), 2.87 (t, *J =* 7.2 Hz, 2H), 2.40 (dd, *J =* 16.9, 8.9 Hz, 1H), 2.30 (dd, *J =* 12.6, 6.2 Hz, 1H), 2.25 (s, 3H), 1.72 (s, 3H).

### Example 156: Preparation of (4-(3-(3,4-difluoro-2-methoxyphenyl)-5-methyl-5-(trifluoromethyl)tetrahydrothiophene-2-carboxamido)-2-(3-oxetane)amino)pyridine (compound a-53-A)

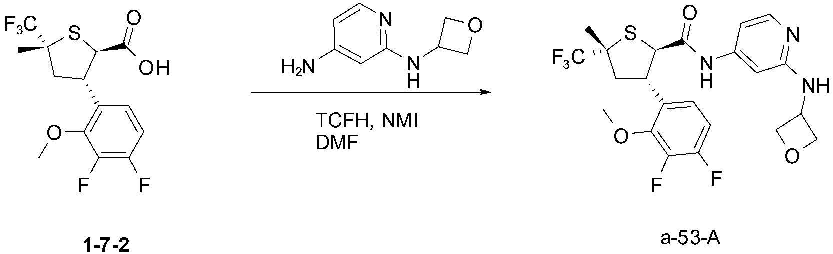

Compound 1-7-2 (50 mg, 0.14 mmol) was dissolved in anhydrous DMF (5 mL), 4-amino-2-(3-oxetane)aminopyridine (69 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (25 mg, 0.050 mmol, yield: 35%).

MS: m/z = 504.13, [M+H]⁺, ¹H NMR (400 MHz, DMSO) δ 9.85 (s, 1H), 7.88 - 7.64 (m, 2H), 7.56 - 7.43 (m, 3H), 5.03 - 4.81 (m, 4H), 4.61 (d, *J =* 10.7 Hz, 1H), 4.38 - 4.24 (m, 1H), 4.11 (s, 1H), 3.97 (d, *J =* 1.6 Hz, 3H), 3.87-3.74 (m, 1H), 2.43 (t, *J =* 12.9 Hz, 1H), 2.31 (dd, *J =* 12.6, 6.0 Hz, 1H), 1.76 (s, 3H).

### Example 157: Preparation of (2R,3S,5R)-3-(3,4-difluoro-2-methoxyphenyl)-N-(1-hydroxy-3,4-dihydro-1H-benzo[c][1,2]oxaborinden-7-yl)-5-methyl-5-(trifluoromethyl)tetrahydrothiophene-2-carboxamide (compound a-55-A)

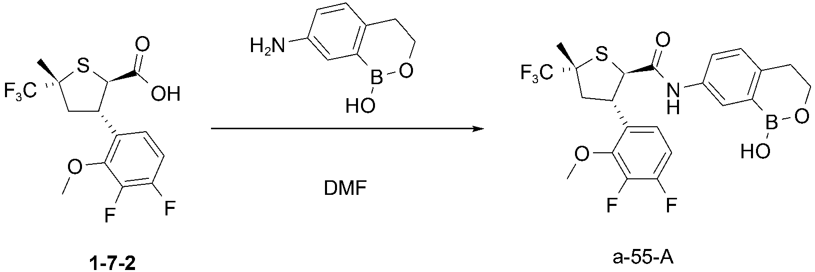

Compound 1-7-2 (50.0 mg, 0.14 mmol) was dissolved in anhydrous DMF (2 mL), 7-amino-3,4-dihydro-1H-benzo[c][1,2]oxaborolan-1-ol (68.5 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred for 1 h. After the reaction was completed, the mixture was separated by Pre-HPLC to obtain the target compound (46.2 mg, 0.09 mmol, yield: 65.88%).

MS: m/z = 526.1, [M+H]⁺, 1H NMR (400 MHz, DMSO) δ 10.16 (s, 1H), 8.40 (s, 1H), 7.73 (d, J = 2.1 Hz, 1H), 7.51 (dd, J = 8.2, 2.2 Hz, 1H), 7.22 - 7.09 (m, 3H), 4.60 (d, J = 10.7 Hz, 1H), 4.27 (td, J = 12.8, 6.1 Hz, 1H), 4.01 (t, J = 5.8 Hz, 2H), 3.96 (d, J = 1.5 Hz, 3H), 2.78 (t, J = 5.8 Hz, 2H), 2.40 (t, J = 12.9 Hz, 1H), 2.30 (dd, J = 12.6, 6.2 Hz, 1H), 1.73 (s, 3H).

### Example 158: Preparation of (2R,3S,5R)-N-(3-((2-aminopropyl)sulfonamido)phenyl)-3-(3,4-difluoro-2-methoxyphenyl)-5-methyl-5-(trifluoromethyl)tetrahydrothiophene-2-carboxamide (a-56-A)

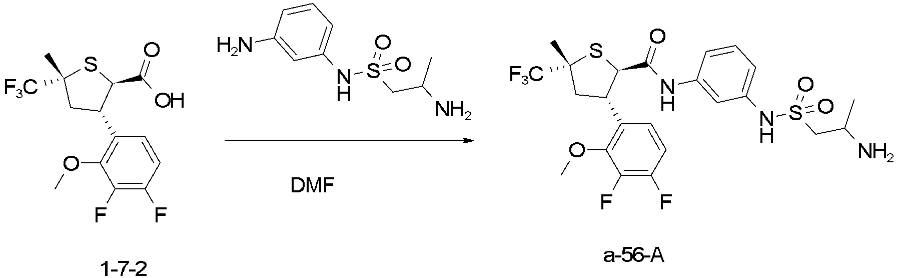

Compound 1-7-2 (30 mg, 0.08 mmol) was dissolved in anhydrous DMF (5 mL), 2-amino-N-(3-aminophenyl)propane-1-sulfonamide (54.96 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (17.01 mg, 0.03 mmol, yield: 37%).

MS: m/z = 568.1, [M+H]⁺, ¹H NMR (400 MHz, DMSO) δ 10.33 (s, 1H), 8.26 (s, 1H), 7.40 (dt, *J =* 3.9, 2.0 Hz, 1H), 7.31 - 7.21 (m, 1H), 7.22 - 7.05 (m, 3H), 6.86 (dd, *J =* 8.0, 1.0 Hz, 1H), 4.61 (d, *J =* 10.7 Hz, 1H), 4.36 - 4.21 (m, 1H), 4.03 (d, *J* = 7.1 Hz, 1H), 3.97 (d, *J =* 1.8 Hz, 3H), 3.31 (td, *J =* 12.6, 6.3 Hz, 2H), 3.13 - 2.99 (m, 2H), 2.39 (d, *J* = 12.9 Hz, 1H), 2.30 (dd, *J =* 12.6, 6.2 Hz, 1H), 1.73 (s, 3H), 1.09 (dd, *J* = 6.5, 1.3 Hz, 3H).

### Example 159: Preparation of (5-((2R,3S,5R)-3-(3,4-difluoro-2-methoxyphenyl)-5-methyl-5-(trifluoromethyl)tetrahydrothiophene-2-carboxamido)-2-((dimethylamino)methyl)phenyl)boronic acid (compound 250-A)

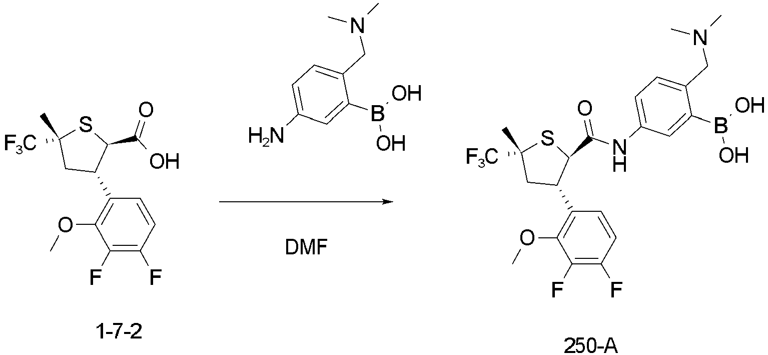

Compound 1-7-2 (30 mg, 0.08 mmol) was dissolved in anhydrous DMF (5 mL), (5-amino-2-((dimethylamino)methyl)phenyl)boronic acid (46.56 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (21.33 mg, 0.04 mmol, yield: 50%).

MS: m/z = 533.2, [M+H]⁺, ¹H NMR (400 MHz, DMSO) δ 10.17 (s, 1H), 7.23 - 7.16 (m, 2H), 4.66 (d, J = 10.6 Hz, 1H), 4.28 (td, J = 12.8, 6.2 Hz, 1H), 3.97 (d, J = 1.7 Hz, 3H), 2.43 (t, J = 12.9 Hz, 1H), 2.33 (dd, J = 12.6, 6.2 Hz, 1H), 1.75 (s, 3H).

### Example 160: Preparation of 2-cyano-4-((2R,3S,5R)-3-(3,4-difluoro-2-methoxyphenyl)-5-methyl-5-(trifluoromethyl)tetrahydrothiophene-2-carboxamido)pyridine 1-oxide (compound 251-A)

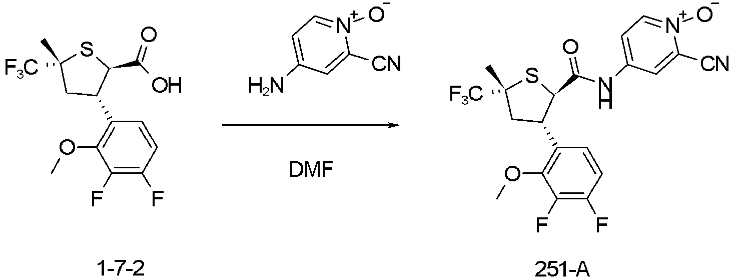

Compound 1-7-2 (30 mg, 0.08 mmol) was dissolved in anhydrous DMF (5 mL), 4-amino-2-cyanopyridine 1-oxide (32.40 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (9.48 mg, 0.02 mmol, yield: 25%). MS: m/z = 474.3, ¹H NMR (400 MHz, DMSO) δ 10.19 (s, 1H), 8.17 (d, *J =* 7.2 Hz, 1H), 8.10 (d, *J =* 3.1 Hz, 1H), 7.53 (dd, *J* = 7.2, 3.1 Hz, 1H), 7.20 (dt, *J =* 9.3, 8.5 Hz, 2H), 4.58 (d, *J =* 10.6 Hz, 1H), 4.28 (td, *J =* 13.0, 6.1 Hz, 1H), 3.99 (d, *J =* 1.7 Hz, 3H), 2.48 (d, *J =* 13.0 Hz, 1H), 2.34 (dd, *J* = 12.6, 6.0 Hz, 1H), 1.75 (s, 3H).

### Example 161: Preparation of (2R,3S,5R)-3-(3,4-difluoro-2-methoxyphenyl)-N-(2-((Z)-N'-methoxycarbamoyl)pyridin-4-yl)-5-methyl-5-(trifluoromethyl)tetrahydrothiophene-2-carboxamide (a-57-A)

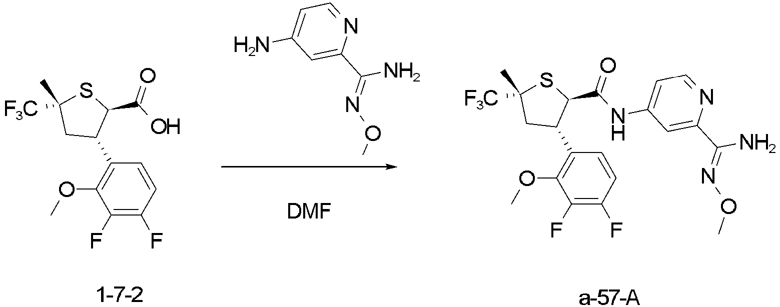

Compound 1-7-2 (30 mg, 0.08 mmol) was dissolved in anhydrous DMF (5 mL), (Z)-4-amino-N'-methoxypicolinamide (39.84 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (10.11 mg, 0.02 mmol, yield: 25%).

MS: m/z = 505.3, [M+H]⁺, ¹H NMR (400 MHz, DMSO) δ 10.19 (s, 1H), 8.17 (d, *J =* 7.2 Hz, 1H), 8.10 (d, *J =* 3.1 Hz, 1H), 7.53 (dd, *J =* 7.2, 3.1 Hz, 1H), 7.20 (dt, *J =* 9.3, 8.5 Hz, 2H), 6.79 (s, 2H), 4.58 (d, *J =* 10.6 Hz, 1H), 4.32(s, 3H), 4.28 (td, *J =* 13.0, 6.1 Hz, 1H), 3.99 (d, *J* = 1.7 Hz, 3H), 2.48 (d, *J =* 13.0 Hz, 1H), 2.34 (dd, *J =* 12.6, 6.0 Hz, 1H), 1.75 (s, 3H).

### Example 162: Preparation of 4-((2R,3S,5R)-3-(3,4-difluoro-2-methoxyphenyl)-5-methyl-5-(trifluoromethyl)tetrahydrothiophene-2-carboxamido)-2-((E)-N'-hydroxycarbamoyl)pyridine 1-oxide (compound a-58-A)

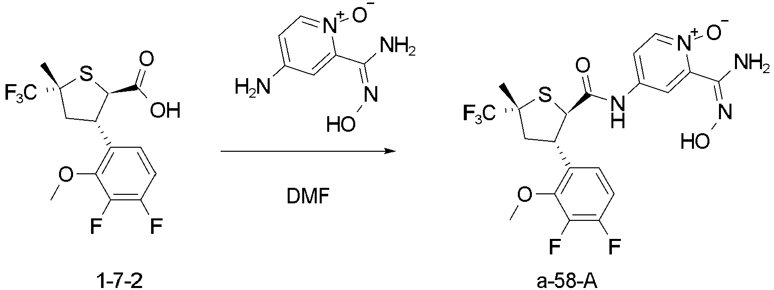

Compound 1-7-2 (30 mg, 0.08 mmol) was dissolved in anhydrous DMF (5 mL), (E)-4-amino-2-(N'-hydroxycarbamimidoyl)pyridine 1-oxide (40.32 mg, 0.24 mmol) was added, and TCFH (120 mg, 0.40 mmol) and NMI (56 mg, 0.64 mmol) were added in an ice bath. The mixture was allowed to naturally warm to room temperature and then stirred. After the reaction was completed, the mixture was separated by Pre-HPLC to obtain the target compound (10.15 mg, 0.02 mmol, yield: 25%).

MS: m/z = 507.3, [M+H]⁺, ¹H NMR (400 MHz, DMSO) δ 10.83 (s, 1H), 10.19 (s, 1H), 8.17 (d, *J* = 7.2 Hz, 1H), 8.10 (d, *J =* 3.1 Hz, 1H), 7.53 (dd, *J =* 7.2, 3.1 Hz, 1H), 7.20 (dt, *J =* 9.3, 8.5 Hz, 2H), 6.79 (s, 2H), 4.58 (d, *J =* 10.6 Hz, 1H), 4.28 (td, *J =* 13.0, 6.1 Hz, 1H), 3.99 (d, *J =* 1.7 Hz, 3H), 2.48 (d, *J =* 13.0 Hz, 1H), 2.34 (dd, *J =* 12.6, 6.0 Hz, 1H), 1.75 (s, 3H).

### Example 163: Preparation of (2R,3S,5R)-3-(3,4-difluoro-2-methoxyphenyl)-N-(1-hydroxy-1H-benzo[d][1,2,6]oxazolin-6-yl)-5-methyl-5-(trifluoromethyl)tetrahydrothiophene-2-carboxamide (compound a-59-A)

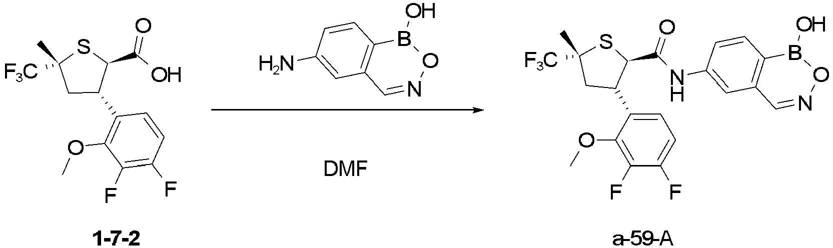

Compound 1-7-2 (50.0 mg, 0.14 mmol) was dissolved in anhydrous DMF (2 mL), 6-amino-1H-benzo[d][1,2,6]oxazolin-1-ol (68.1 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (47.1 mg, 0.09 mmol, yield: 67.32%).

MS: m/z = 501.1, [M+H]⁺, 1H NMR (400 MHz, DMSO) δ 10.66 (s, 1H), 9.25 (s, 1H), 8.59 (s, 1H), 8.05 - 7.94 (m, 2H), 7.69 (dd, J = 8.2, 1.4 Hz, 1H), 7.21 - 7.12 (m, 2H), 4.66 (d, J = 10.6 Hz, 1H), 4.30 (td, J = 12.9, 6.1 Hz, 1H), 3.97 (d, J = 1.5 Hz, 3H), 2.45 (t, J = 13.0 Hz, 1H), 2.32 (dd, J = 12.6, 6.1 Hz, 1H), 1.74 (s, 3H).

### Example 164: Preparation of (2R,3S,5R)-3-(3,4-difluoro-2-methoxyphenyl)-N-(1-hydroxy-1,2-dihydrobenzo[d][1,2,3]diazaborinin-7-yl)-5-methyl-5-(trifluoromethyl)tetrahydrothiophene-2-carboxamide (compound a-60-A)

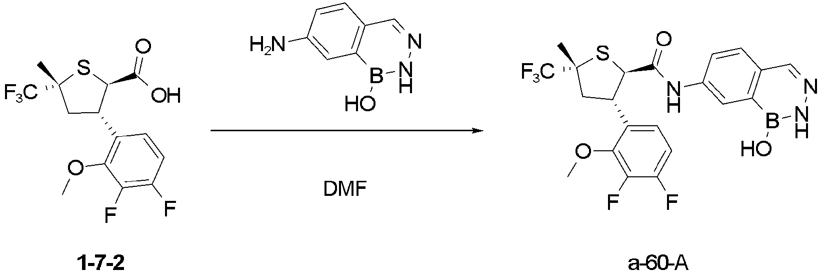

Compound 1-7-2 (50.0 mg, 0.14 mmol) was dissolved in anhydrous DMF (2 mL), 7-aminobenzo[d][1,2,3]diazaborinin-1(2H)-ol (67.7 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (41.8 mg, 0.08 mmol, yield: 59.77%).

MS: m/z = 500.1, [M+H]⁺, ¹H NMR (400 MHz, DMSO) δ 10.46 (s, 1H), 9.71 (s, 1H), 8.26 (d, J = 2.0 Hz, 1H), 8.08 (s, 1H), 7.88 (s, 1H), 7.73 (dd, J = 8.5, 2.2 Hz, 1H), 7.63 (d, J = 8.6 Hz, 1H), 7.24 - 7.10 (m, 2H), 4.67 (d, J = 10.7 Hz, 1H), 4.35 - 4.25 (m, 1H), 3.98 (d, J = 1.8 Hz, 3H), 2.48 - 2.27 (m, 2H), 1.74 (s, 3H).

### Example 165: Preparation of (2R,3S,5R)-3-(3,4-difluoro-2-methoxyphenyl)-N-(3-(1,1-dioxide-4-oxo-1,2,5-thiadiazolidin-2-yl)phenyl)-5-methyl-5-(trifluoromethyl)tetrahydrothiophene-2-carboxamide (compound a-61-A)

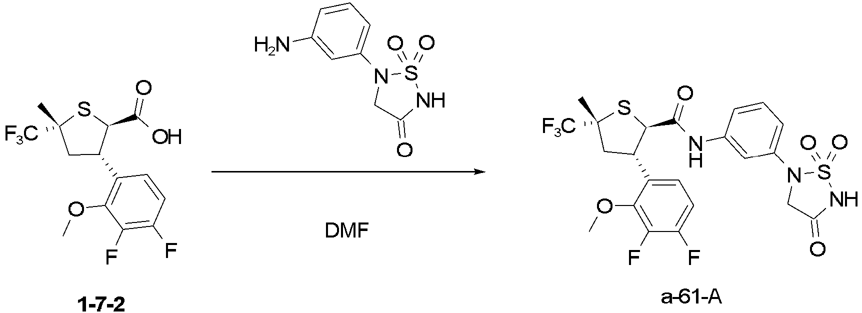

Compound 1-7-2 (50.0 mg, 0.14 mmol) was dissolved in anhydrous DMF (2 mL), 5-(3-aminophenyl)-1,2,5-thiadiazolidin-3-one-1,1-dioxide (95.4 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (45.5 mg, 0.08 mmol, yield: 44.73%).

MS: m/z = 566.1, [M+H]⁺, 1H NMR (400 MHz, DMSO) δ 9.95 (s, 1H), 8.85 (d, J=3.2 Hz, 2H), 7.49 (s, 1H), 7.41 (d, J=2.4 Hz, 1H), 7.02-6.86 (m, 2H), 4.65 (d, J = 10.0 Hz, 1H), 4.23-4.02 (m, 3H), 3.83(s, 3H), 3.48-3.46(m, 1H), 2.17-1.92(m, 2H), 1.71 (s, 3H).

### Example 166: Preparation of tert-butyl (6-((2R,3S,5R)-3-(3,4-difluoro-2-methoxyphenyl)-5-methyl-5-(trifluoromethyl)tetrahydrothiophene-2-carboxamido)-1-hydroxy-1,3-dihydrobenzo[c][1,2]oxaborol-3-yl)methyl)carbamate (compound a-62-A)

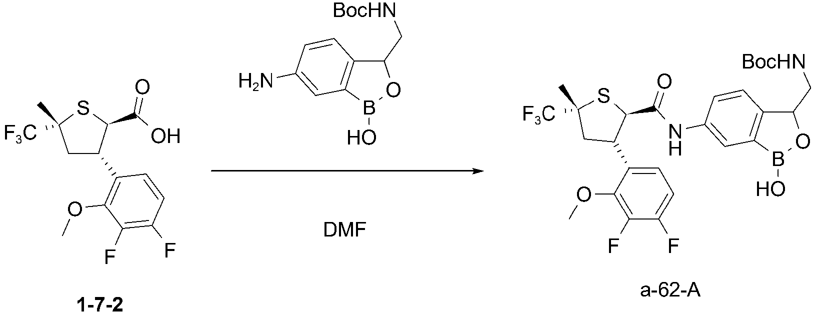

Compound 1-7-2 (50.0 mg, 0.14 mmol) was dissolved in anhydrous DMF (2 mL), tert-butyl (6-amino-1-hydroxy-1,3-dihydrobenzo[c][1,2]oxaborol-3-yl)methyl)carbamate (116.8 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (43.4 mg, 0.07 mmol, yield: 50.27%).

MS: m/z = 617.2, [M+H]⁺, ¹H NMR (400 MHz, DMSO) δ 10.27 (s, 1H), 9.22 (s, 1H), 7.89 (dd, J = 4.5, 1.7 Hz, 1H), 7.48 (dd, J = 10.2, 3.9 Hz, 1H), 7.28 (d, J = 8.3 Hz, 1H), 7.20 - 7.13 (m, 2H), 6.91 (t, J = 5.3 Hz, 1H), 5.05 (dd, J = 6.8, 4.6 Hz, 1H), 4.62 (d, J = 10.7 Hz, 1H), 4.27 (tt, J = 18.3, 9.1 Hz, 1H), 3.96 (d, J = 1.6 Hz, 3H), 3.31 - 3.26 (m, 1H), 3.06 - 2.94 (m, 1H), 2.44 - 2.27 (m, 2H), 1.73 (s, 3H), 1.34 (s, 9H).

### Example 167: Preparation of (2R,3S,5R)-N-(3-(aminomethyl)-1-hydroxy-1,3-dihydrobenzo[c][1,2]oxaborol-6-yl)-3-(3,4-difluoro-2-methoxyphenyl)-5-methyl-5-(trifluoromethyl)tetrahydrothiophene-2-carboxamide hydrochloride (compound a-63-A)

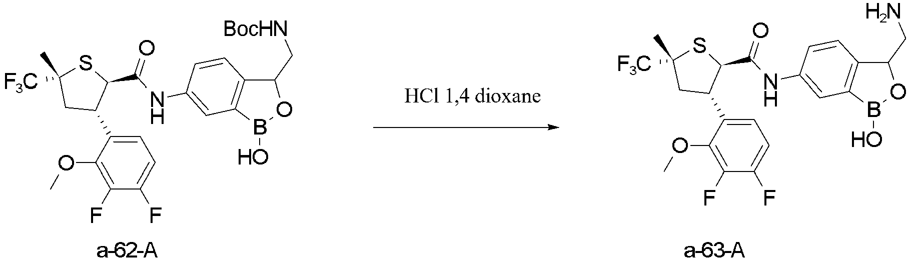

Compound a-62-A (30.0 mg, 0.05 mmol) was dissolved in 1,4-dioxane (5 mL). The mixture was then stirred at room temperature for 1 h. After the reaction was completed, the mixture was concentrated to dryness by rotary evaporation to obtain the target compound (22.2 mg, 0.04 mmol, yield: 80.27%). MS: m/z = 517.1, [M+H]⁺, 1H NMR (400 MHz, DMSO) δ 10.25 (s, 1H), 9.45 (s, 1H), 7.90 (dd, J = 4.4, 1.6 Hz, 1H), 7.48 (dd, J = 10.2, 3.9 Hz, 1H), 7.42 (d, J = 8.4 Hz, 1H), 7.25 - 7.11 (m, 2H), 5.11(s, 2H), 5.35 (dd, J = 6.8, 4.4 Hz, 1H), 4.64 (d, J = 10.7 Hz, 1H), 4.15 (tt, J = 18.4, 9.6 Hz, 1H), 3.66 (d, J = 1.6 Hz, 3H), 3.30 - 3.15 (m, 1H), 3.07 - 2.94 (m, 1H), 2.50 - 2.27 (m, 2H), 1.75 (s, 3H).

### Example 168: Preparation of (2R,3S,5R)-3-(3,4-difluoro-2-methoxyphenyl)-N-(1-hydroxy-1,2-dihydrobenzo[d][1,2,3]diazaborinin-6-yl)-5-methyl-5-(trifluoromethyl)tetrahydrothiophene-2-carboxamide (compound a-64-A)

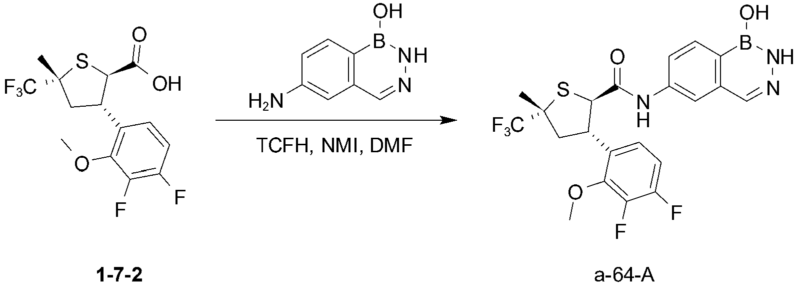

1-7-2 (50 mg, 0.14 mmol) was dissolved in anhydrous DMF (2 mL), 6-aminobenzo[d][1,2,3]diazaborinin-1(2H)-ol (67.6 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (28.2 mg, 0.06 mmol, yield: 40.2%).

MS: m/z = 500.2, [M+H]⁺,¹H NMR (400 MHz, DMSO) δ 10.02 (s, 1H), 8.59 (s, 1H), 8.05 (s, 1H), 7.73-7.53 (m, 2H), 7.02 (d, *J*=2.0 Hz, 1H), 6.88(d, *J*=2.0 Hz, 1H), 4.23-4.21 (m, 1H), 3.83(s, 3H), 3.48-3.46(m, 1H), 2.17-1.92(m, 2H), 1.38 (s, 3H).

### Example 169: Preparation of 3-((2R,3S,5R)-3-(3,4-difluoro-2-methoxyphenyl)-5-methyl-5-(trifluoromethyl)tetrahydrothiophene-2-carboxamido)benzenesulfonyl azide (compound a-65-A)

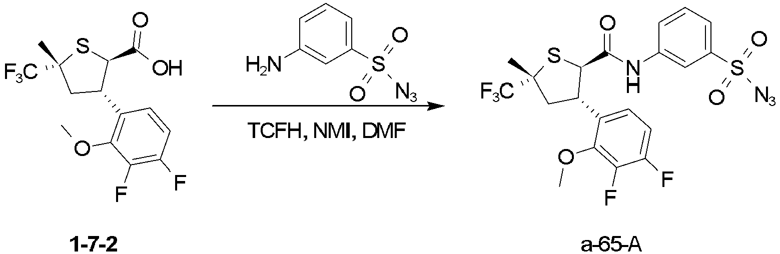

Compound 1-7-2 (50 mg, 0.14 mmol) was dissolved in anhydrous DMF (2 mL), 3-aminobenzenesulfonyl azide (83.6 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (34.2 mg, 0.06 mmol, yield: 45.5%).

MS: m/z = 537.2, [M+H]⁺,¹H NMR (400 MHz, DMSO) δ 10.02 (s, 1H), 8.03-7.97 (m, 2H), 7.80-7.64 (m, 2H), 7.07(d, *J=2.0* Hz, 1H), 6.88(d, *J*=2.0 Hz, 1H), 4.23-4.21 (m, 1H), 3.83(s, 3H), 3.48-3.46(m, 1H), 2.17-1.92(m, 2H), 1.38 (s, 3H).

### Example 170: Preparation of 4-((2R,3S,5R)-3-(3,4-difluoro-2-methoxyphenyl)-5-methyl-5-(trifluoromethyl)tetrahydrothiophene-2-carboxamido)pyridine-2-sulfonyl azide (compound a-66-A)

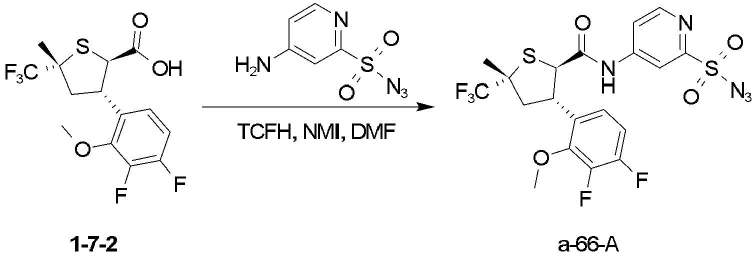

Compound 1-7-2 (50 mg, 0.14 mmol) was dissolved in anhydrous DMF (2 mL), 4-aminopyridine-2-sulfonyl azide (83.6 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (6.8 mg, 0.01 mmol, yield: 9.0%). MS: m/z = 538.2, [M+H]⁺,¹H NMR (400 MHz, DMSO) δ 9.92 (s, 1H), 8.70 (d, *J*=2.0 Hz, 1H), 8.37 (s, 1H), 7.96 (d, *J*=2.0 Hz, 1H), 7.07(d, *J*=2.0 Hz, 1H), 6.88(d, *J=*2.0 Hz, 1H), 4.07-4.06 (m, 1H), 3.83(s, 3H), 3.48-3.46(m, 1H), 2.17-1.92(m, 2H), 1.38 (s, 3H).

### Example 171: Preparation of (2R,3S,5R)-3-(3,4-difluoro-2-methoxyphenyl)-N-(2,2-difluorobenzo[d][1,3]dioxol-5-yl)-5-methyl-5-(trifluoromethyl)tetrahydrothiophene-2-carboxamide (compound a-67-A)

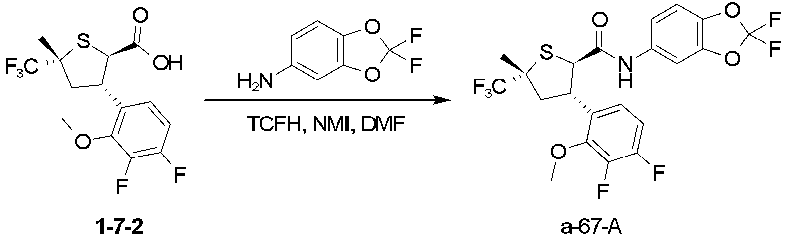

Compound 1-7-2 (50 mg, 0.14 mmol) was dissolved in anhydrous DMF (2 mL), 2,2-difluorobenzo[d][1,3]dioxolan-5-amine (72.7 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (36.2 mg, 0.07 mmol, yield: 50.5%).

MS: m/z = 512.2, [M+H]⁺,¹H NMR (400 MHz, DMSO) δ 10.02 (s, 1H), 7.23 (s, 1H), 7.03-7.02 (m, 2H), 6.87-6.86 (m, 2H), 4.23-4.21(m, 1H), 3.83(s, 3H), 3.48-3.46(m, 1H), 2.17-1.92(m, 2H), 1.38 (s, 3H).

### Example 172: Preparation of (2R,3S,5R)-3-(3,4-difluoro-2-methoxyphenyl)-N-(1-hydroxy-1H-2,3,1-benzoxazaborol-7-yl)-5-methyl-5-(trifluoromethyl)tetrahydrothiophene-2-carboxamide (compound a-68-A)

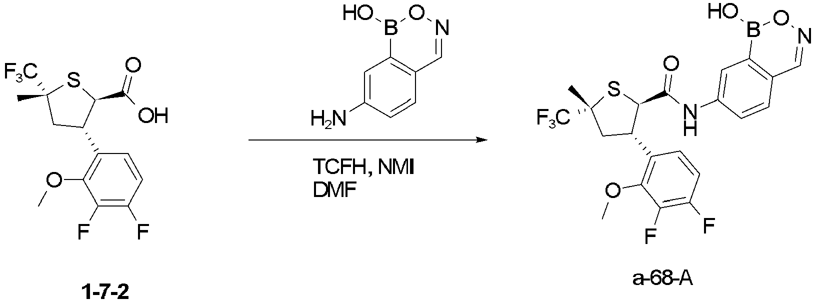

Compound 1-7-2 (50 mg, 0.14 mmol) was dissolved in anhydrous DMF (5 mL), 7-amino-1H-benzo[d][1,2,6]oxazolin-1-ol (68 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (51 mg, 0.102 mmol, yield: 73%).

MS: m/z = 501.10, [M+H]⁺, ¹H NMR (400 MHz, DMSO) δ 9.93 (s, 1H), 8.00 (s, 1H), 7.56 - 7.32 (m, 3H), 7.25 - 7.12 (m, 3H), 4.62 (d, *J =* 10.7 Hz, 1H), 4.36 - 4.22 (m, 1H), 3.99 (d, *J =* 1.6 Hz, 3H), 2.44 (t, *J =* 12.9 Hz, 1H), 2.33 (dd, *J =* 12.6, 6.0 Hz, 1H), 1.76 (s, 3H).

### Example 173: Preparation of (2R,3S,5R)-3-(3,4-difluoro-2-methoxyphenyl)-N-(1,1-dioxide-3,4-dihydro-2H-benzo[e][1,2]thiazin-7-yl)-5-methyl-5-(trifluoromethyl)tetrahydrothiophene-2-carboxamide (a-69-A)

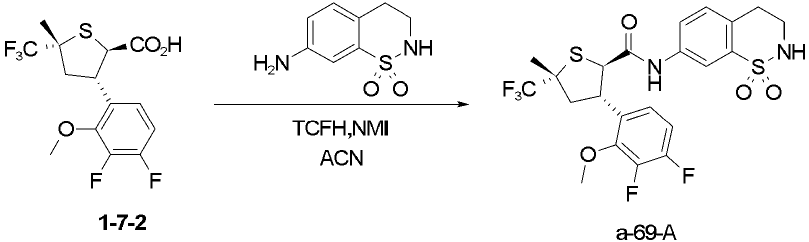

For the experimental procedures, reference may be made to Example 1.

MS: m/z =554.1[M+H]+, ¹H NMR (400 MHz, DMSO) δ 10.53 (s, 1H), 8.03 (d, *J =* 2.2 Hz, 1H), 7.50 (dd, *J =* 8.5, 2.2 Hz, 1H), 7.26 (d, *J =* 8.6 Hz, 1H), 7.23 - 7.10 (m, 2H), 4.58 (d, *J =* 10.7 Hz, 1H), 4.27 (ddd, *J =* 13.1, 10.9, 6.1 Hz, 1H), 3.97 (d, *J =* 1.9 Hz, 3H), 3.52 (t, *J =* 6.0 Hz, 2H), 2.82 (t, *J =* 5.9 Hz, 2H), 2.45 (t, *J =* 12.9 Hz, 1H), 2.31 (dd, *J* = 12.6, 6.1 Hz, 1H), 1.73 (s, 3H).

### Example 174: Preparation of (2R,3S,5R)-N-(2-acrylamidopyridin-4-yl)-3-(3,4-difluoro-2-methoxyphenyl)-5-methyl-5-(trifluoromethyl)tetrahydrothiophene-2-carboxamide (compound 252-A)

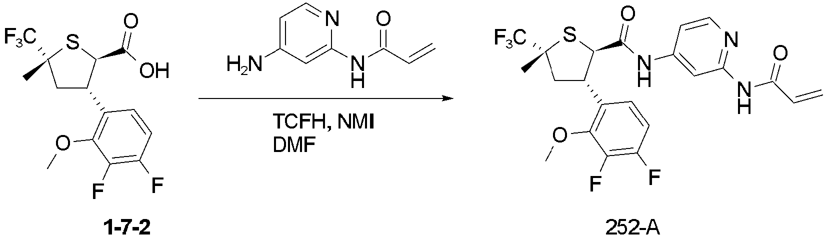

Compound 1-7-2 (100 mg, 0.28 mmol) was dissolved in anhydrous DMF (5 mL), N-(4-aminopyridin-2-yl)acrylamide (64 mg, 0.28 mmol) was added, and TCFH (395 mg, 1.40 mmol) and NMI (184 mg, 2.25 mmol) were added in an ice bath. The mixture was allowed to naturally warm to room temperature and then stirred. After the reaction was completed, the mixture was separated by Pre-HPLC to obtain the target compound (76 mg, 0.05 mmol, yield: 54.00%). MS: m/z = 501.1, [M+H]+, ¹H NMR (400 MHz, DMSO) δ 10.70 (d, *J =* 34.7 Hz, 2H), 8.33 (d, *J =* 1.5 Hz, 1H), 8.19 (d, *J =* 5.6 Hz, 1H), 7.42 (dd, *J =* 5.6, 1.8 Hz, 1H), 7.17 (ddd, *J =* 26.6, 11.9, 5.9 Hz, 3H), 6.60 (dt, *J =* 21.2, 10.5 Hz, 1H), 6.32 (dd, *J =* 17.0, 1.8 Hz, 1H), 5.84 - 5.72 (m, 1H), 4.67 (d, *J =* 10.6 Hz, 1H), 4.31 (td, *J =* 12.5, 6.1 Hz, 1H), 4.00 (d, *J =* 1.7 Hz, 4H), 2.35 (dd, *J =* 12.7, 6.1 Hz, 2H), 1.76 (s, 4H), 1.69 (s, 1H).

### Example 175: Preparation of (4-(3-(3,4-difluoro-2-methoxyphenyl)-5-methyl-5-(trifluoromethyl)tetrahydrothiophene-2-carboxamido)phenyl)sulfonyl fluoride (compound a-71-A)

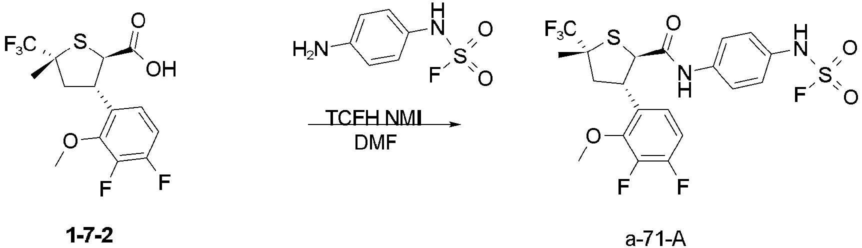

Compound 1-7-2 (39 mg, 0.11 mmol) was dissolved in anhydrous DMF (2 mL), (4-aminophenyl)sulfonyl fluoride (65.51 mg, 0.35 mmol) was added, and TCFH (154 mg, 0.55 mmol) and NMI (65.6 mg, 0.88 mmol) were added in an ice bath. The mixture was allowed to naturally warm to room temperature and then stirred. After the reaction was completed, the mixture was separated by Pre-HPLC to obtain the target compound (38 mg, 0.07 mmol, yield: 65.60%). MS:m/z=529.06, [M+H]⁺,¹HNMR (400 MHZ, DMSO) δ 10.11 (s, 1H), 7.87 (s, 1H), 7.16 (dd, *J*= 8.7, 3.8 Hz, 2H), 7.11(d,*J*=8.1 Hz,1H),7.05(t *J*= 1.9 Hz,1H),6.93(t *J=* 8.1 Hz,1H),6.71-6.59 (m,1H),4.66(d *J* = 10.8Hz, 1H), 4.31-4.20 (m,1H),3.96(d, J= 1.6 Hz,3H),2.31(dd, J= 11.8,9.9 Hz,2H),1.72(s,3H).

### Example 176: Preparation of (2R,3S,5R)-3-(3,4-difluoro-2-methoxyphenyl)-N-(3-((E)-(hydroxy(methyl)-14-azaneylidene)methyl)phenyl)-5-methyl-5-(trifluoromethyl)tetrahydrothiophene-2-carboxamide (a-72-A)

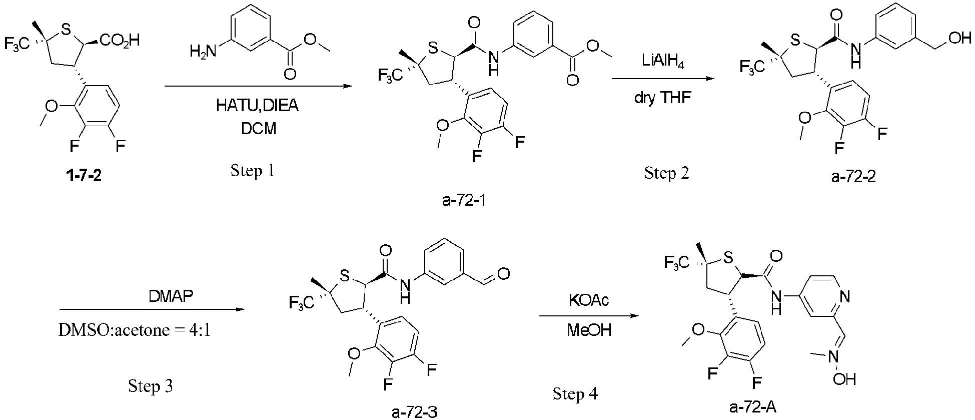

### Step 1: Synthesis of methyl 3-((2R,3S,5R)-3-(3,4-difluoro-2-methoxyphenyl)-5-methyl-5-(trifluoromethyl)tetrahydrothiophene-2-carboxamido)benzoate (a-72-1)

Compound 1-7-2 (450 mg, 1.26 mmol) and HATU (720.32 mg, 1.89 mmol) were dissolved in DCM (5 mL), DIEA (326.45 mg, 2.53 mmol) was added, and finally, methyl 3-aminobenzoate (229.09 mg, 1.52 mmol) was added. The mixture was allowed to react at room temperature. After the reaction was completed, the crude product was purified to obtain the target compound (600 mg, 1.23 mmol, yield: 97.06%). MS: m/z =507.1[M+H]⁺.

### Step 2: Synthesis of (2R,3S,5R)-3-(3,4-difluoro-2-methoxyphenyl)-N-(3-(hydroxymethyl)phenyl)-5-methyl-5-(trifluoromethyl)tetrahydrothiophene-2-carboxamide (a-72-2)

Methyl 3-((2R,3R,5R)-3-(3,4-difluoro-2-methoxyphenyl)-5-methyl-5-(trifluoromethyl)tetrahydrothiophene-2-carboxamido)benzoate (600 mg, 1.23 mmol) was dissolved in THF (5 mL). The mixture was purged with nitrogen and cooled to -40 °C. Lithium aluminum hydride (69.79 mg, 1.84 mmol) was added. After the addition, the mixture was allowed to react at -40 °C. LiAlH4:15% NaOH:H₂O = 1:3:1 was added to quench the reaction at a low temperature. The mixture was filtered, concentrated, and purified to obtain the title compound (300 mg, 650.13 µmol, yield: 53.04%). MS: m/z =462.1[M+H]⁺.

Step 3: Synthesis of (2R,3S,5R)-3-(3,4-difluoro-2-methoxyphenyl)-N-(3-formylphenyl)-5-methyl-5-(trifluoromethyl)tetrahydrothiophene-2-carboxamide (a-72-3) (2R,3S,5R)-3-(3,4-Difluoro-2-methoxyphenyl)-N-(3-(hydroxymethyl)phenyl)-5-methyl-5-(trifluoromethyl)tetrahydrothiophene-2-carboxamide (105 mg, 227.55 µmol) was dissolved in DMSO (2.0 mL) and acetone (0.5 mL). The mixture was cooled to 0 °C, and Dess-Martin periodinane (193.02 mg, 455.09 µmol) was added. After the addition, the mixture was stirred at 0 °C, and the reaction was quenched with saturated sodium thiosulfate at a low temperature. The mixture was extracted with DCM and concentrated at a low temperature to obtain a crude product of the target compound (100 mg, 217.66 µmol, yield: 95.66%). The crude product was directly used in the next step.

Step 4: Synthesis of (2R,3S,5R)-3-(3,4-difluoro-2-methoxyphenyl)-N-(3-((E)-(hydroxy(methyl)-14-azaneylidene)methyl)phenyl)-5-methyl-5-(trifluoromethyl)tetrahydrothiophene-2-carboxamide (a-72-A) (2R,3S,5R)-3-(3,4-Difluoro-2-methoxyphenyl)-N-(3-formylphenyl)-5-methyl-5-(trifluoromethyl)tetrahydrothiophene-2-carboxamide (100 mg, 217.66 µmol) was dissolved in methanol (5 mL). Potassium acetate (42.72 mg, 435.32 µmol) and N-methylhydroxyamine hydrochloride (23.63 mg, 282.96 µmol) were added. After the addition, the mixture was allowed to react at 65 °C. The crude product was purified to obtain the target compound (56 mg, 114.41 µmol, yield: 52.56%).

MS: m/z =490.1[M+H]+, ¹H NMR (400 MHz, DMSO) δ 10.40 (s, 1H), 8.48 (s, 1H), 7.80 (s, 1H), 7.74 - 7.64 (m, 2H), 7.32 (t, *J* = 8.0 Hz, 1H), 7.22 - 7.11 (m, 2H), 4.63 (d, *J =* 10.7 Hz, 1H), 4.28 (ddd, *J* = 13.0, 10.9, 6.2 Hz, 1H), 3.96 (d, *J =* 1.8 Hz, 3H), 3.76 (s, 3H), 2.40 (t, *J =* 12.9 Hz, 1H), 2.30 (dd, *J* = 12.6, 6.2 Hz, 1H), 2.07 (s, 1H), 1.73 (s, 3H).

### Example 177: Preparation of (2R,3S,5R)-N-(2-(1-cyanocyclopropyl)pyridin-4-yl)-3-(3,4-difluoro-2-methoxyphenyl)-5-methyl-5-(trifluoromethyl)tetrahydrothiophene-2-carboxamide (compound a-73-A)

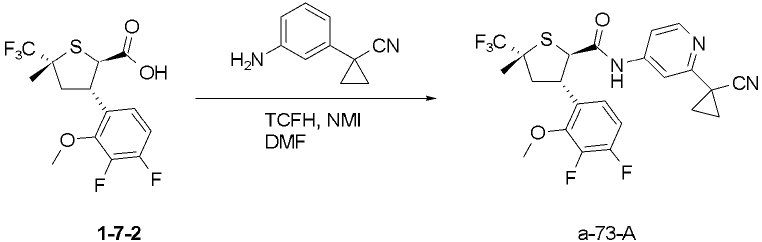

Compound 1-7-2 (100 mg, 0.28 mmol) was dissolved in anhydrous DMF (5 mL), 1-(3-aminophenyl)cyclopropanecarbonitrile (44 mg, 0.28 mmol) was added, and TCFH (395 mg, 1.40 mmol) and NMI (184 mg, 2.25 mmol) were added in an ice bath. The mixture was allowed to naturally warm to room temperature and then stirred. After the reaction was completed, the mixture was separated by Pre-HPLC to obtain the target compound (68 mg, 0.05 mmol, yield: 48.32%).

MS: m/z = 497.1, [M+H]+, ¹H NMR (400 MHz, DMSO) δ 10.81 (s, 1H), 8.33 (d, *J =* 5.5 Hz, 1H), 7.83 (d, *J =* 1.7 Hz, 1H), 7.46 (dd, *J* = 5.6, 1.9 Hz, 1H), 7.27 - 7.11 (m, 2H), 4.64 (d, *J =* 10.6 Hz, 1H), 4.30 (tt, *J =* 20.5, 7.9 Hz, 1H), 4.03 - 3.98 (m, 3H), 2.47 (d, *J* = 12.9 Hz, 1H), 2.35 (dd, *J* = 12.6, 6.1 Hz, 1H), 1.79 - 1.73 (m, 6H), 1.65 (dd, *J* = 7.8, 4.3 Hz, 2H).

### Example 178: Preparation of 4-((2R,3S,5R)-3-(3,4-difluoro-2-methoxyphenyl)-5-methyl-5-(trifluoromethyl)tetrahydrothiophene-2-carboxamido)benzenesulfonyl azide (compound a-74-A)

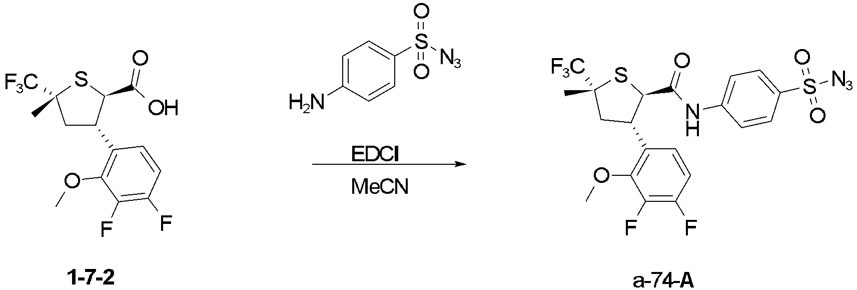

Compound 4-aminobenzenesulfonyl azide (300 mg, 0.84 mmol) was dissolved in anhydrous MeCN (5 mL), 1-7-2 (189 mg, 0.92 mmol) was added, and EDCI (177 mg, 0.92 mmol) was added in an ice bath. The mixture was allowed to naturally warm to room temperature and then stirred. After the reaction was completed, the mixture was separated by Pre-HPLC to obtain the target compound (114 mg, 0.21 mmol, yield: 22.82%).

MS: m/z = 537.06, [M+H]⁺. ¹H NMR (400 MHz, CDCl₃) δ 9.05 (s, 1H), 8.70 (d, *J =* 5.5 Hz, 1H), 8.17 (s, 1H), 8.09 (dd, *J* = 5.5, 1.9 Hz, 1H), 7.13 (t, *J =* 7.1 Hz, 1H), 6.96 (dd, *J* = 16.6, 9.0 Hz, 1H), 4.67 (d, *J =* 10.7 Hz, 1H), 4.17 (d, *J =* 2.9 Hz, 1H), 4.14 (d, *J* = 2.2 Hz, 1H), 4.11 (d, *J =* 2.5 Hz, 3H), 2.68 (t, *J =* 12.9 Hz, 1H), 1.91 (s, 3H).

### Example 179: Preparation of N-(4-(2-azidoacetamido)phenyl)-3-(3,4-difluoro-2-methoxyphenyl)-5-methyl-5-(trifluoromethyl)tetrahydrothiophene-2-carboxamide (compound a-75-A)

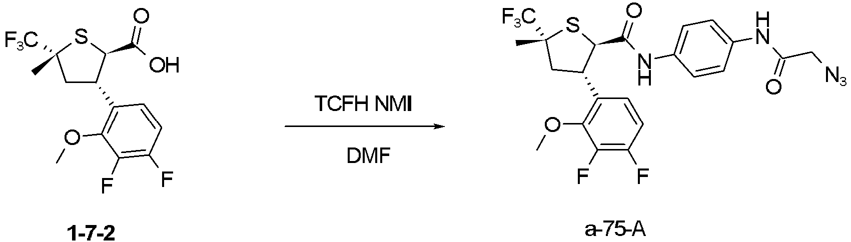

For the experimental procedures, reference may be made to Example 1.

MS:m/z=530.12,[M+H]⁺,¹HNMR (400 MHZ, DMSO) δ 9.33 (s, 1H), 7.87 (s, 1H), 7.07(dd, *J*= 5.6, 3.8 Hz, 2H), 7.01(d,*J*=7.7 Hz,1H).6.93 (t *J*= 3.7 Hz,1H),6.81 (t *J*= 6.3 Hz,1H),6.65-6.48 (m,1H),4.57(d *J*= 9.7Hz, 1H), 4.44-4.31 (m,1H),3.83 (d, J= 1.6 Hz,3H),2.57 (s,1H),2.24 (dd, J= 11.8,9.9 Hz,2H),1.63 (s,3H).

### Example 180: Preparation of (2R,3S,5R)-3-(3,4-difluoro-2-methoxyphenyl)-N-(3-((dimethylamino)methyl)-1-hydroxy-1,3-dihydrobenzo[c][1,2]oxaborol-6-yl)-5-methyl-5-(trifluoromethyl)tetrahydrothiophene-2-carboxamide (compound a-76-A)

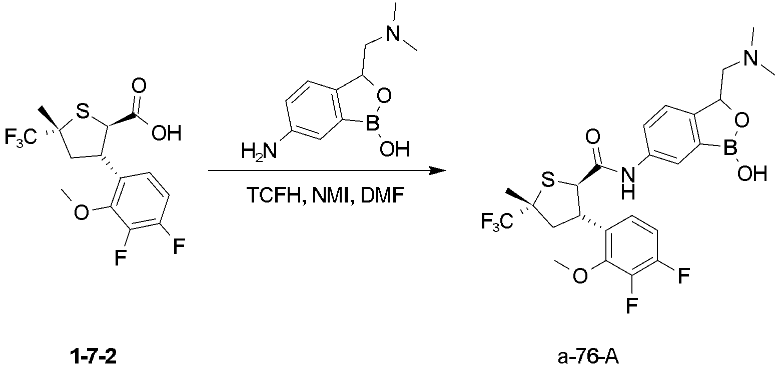

Compound 1-7-2 (50 mg, 0.14 mmol) was dissolved in anhydrous DMF (2 mL), 6-amino-3-((dimethylamino)methyl)benzo[c][1,2]oxaborol-1(3H)-ol (86.5 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (43.5 mg, 0.08 mmol, yield: 57.1%).

MS: m/z = 545.1, [M+H]⁺,¹H NMR (400 MHz, DMSO) δ 9.98 (s, 1H), 7.61-7.38 (m, 3H), 7.05-7.02 (m, 1H), 6.91-6.86 (m, 1H),4.85-4.83(m, 1H), 4.20 (s, 1H), 3.82(s, 3H), 3.48-3.46(m, 1H), 2.98-2.73 (m, 2H), 2.25 (s, 6H), 2.17-1.92(m, 2H), 1.38 (s, 3H).

### Example 181: Preparation of (2R,3S,5R)-3-(3,4-difluoro-2-methoxyphenyl)-N-(3-(1-(dimethylamino)ethyl)-1-hydroxy-1,3-dihydrobenzo[c][1,2]oxaborol-6-yl)-5-methyl-5-(trifluoromethyl)tetrahydrothiophene-2-carboxamide (compound a-77-A)

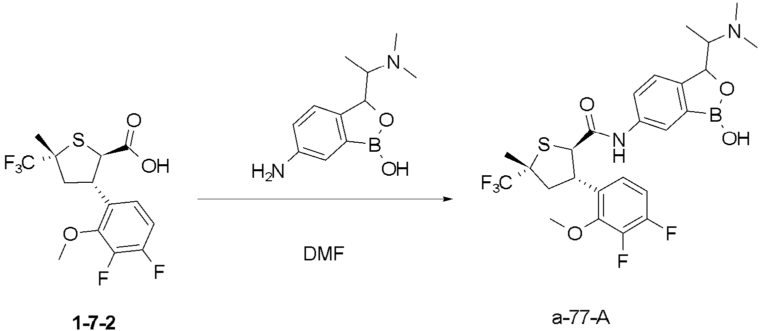

Compound 1-7-2 (50.0 mg, 0.14 mmol) was dissolved in anhydrous DMF (2 mL), 6-amino-3-(1-(dimethylamino)ethyl)benzo[c][1,2]oxaborol-1(3H)-ol (92.5 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (46.0 mg, 0.08 mmol, yield: 58.92%).

MS: m/z = 559.2, [M+H]⁺, 1H NMR (400 MHz, DMSO) δ 8.23 (s, 1H), 7.54 (d, J = 15.0 Hz, 1H), 7.42 (d, J = 15.0 Hz, 1H), 7.11 (dd, J = 15.0, 9.9 Hz, 1H), 6.83 (ddd, J = 15.7, 15.1, 10.1 Hz, 1H), 5.35 (dt, J = 21.2, 16.5 Hz, 1H), 4.72 (d, J = 14.5 Hz, 1H), 4.04 (d, J = 21.4 Hz, 1H), 3.91 (s, 3H), 2.87 (dd, J = 24.8, 16.6 Hz, 1H), 2.51 (dq, J = 14.2, 12.5 Hz, 1H), 2.25 (s, 6H), 2.13 (dd, J = 24.8, 16.6 Hz, 1H), 1.60 (s, 1H), 1.38 (s, 3H), 1.06 (d, J = 12.5 Hz, 3H).

### Example 182: Preparation of (2R,3S,5R)-3-(3,4-difluoro-2-methoxyphenyl)-5-methyl-N-(3-thiocyanatophenyl)-5-(trifluoromethyl)tetrahydrothiophene-2-carboxamide (compound a-78-A)

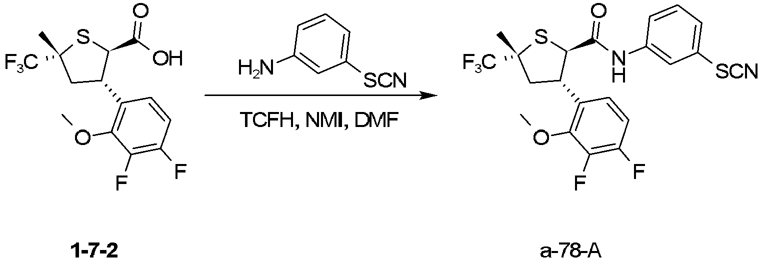

Compound 1-7-2 (50 mg, 0.14 mmol) was dissolved in anhydrous DMF (2 mL), 3-thiocyanatoaniline (63.0 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (48 mg, 0.10 mmol, yield: 70.11%).

MS: m/z = 489.1, [M+H]⁺,¹H NMR (400 MHz, DMSO) δ 10.02 (s, 1H), 8.21 (s, 1H), 7.66-7.21 (m, 3H), 7.07-7.02 (m, 1H), 6.92-6.86 (m, 1H), 4.25-4.23 (m, 1H), 3.83(s, 3H), 3.48-3.46(m, 1H), 2.21-1.96 (m, 2H), 1.38 (s, 3H).

### Example 183: Preparation of (2R,3S,5R)-3-(3,4-difluoro-2-methoxyphenyl)-N-(4-fluoro-3-sulfamoylphenyl)-5-methyl-5-(trifluoromethyl)tetrahydrothiophene-2-carboxamide (compound 233-A)

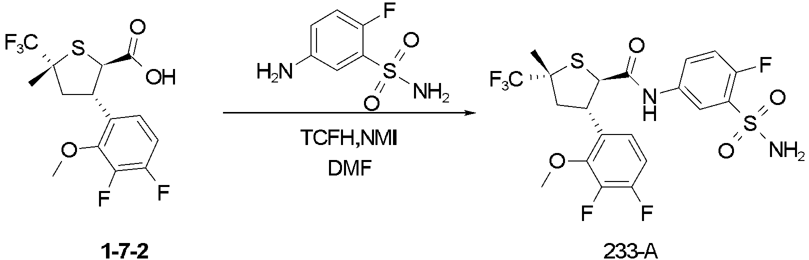

For the experimental procedures, reference may be made to Example 1.

MS: m/z = 529.3[M+H]+, ¹H NMR (400 MHz, CDCl₃) δ 8.19 (s, 1H), 8.03 - 7.93 (m, 1H), 7.76 - 7.68 (m, 1H), 7.20 - 7.10 (m, 1H), 7.05 - 6.91 (m, 1H), 6.92 - 6.78 (m, 1H), 5.22 (s, 2H), 4.45 (d, *J =* 10.8 Hz, 1H), 4.23 - 4.12 (m, 1H), 4.00 (d, *J* = 2.5 Hz, 3H), 2.67 - 2.51 (m, 1H), 2.30 - 2.16 (m, 1H), 1.80 (s, 3H).

### Example 184: Preparation of (2R,3S,5R)-3-(3,4-difluoro-2-methoxyphenyl)-5-methyl-5-(trifluoromethyl)-N-(3-(trimethoxysilyl)phenyl)tetrahydrothiophene-2-carboxamide (compound a-79-A)

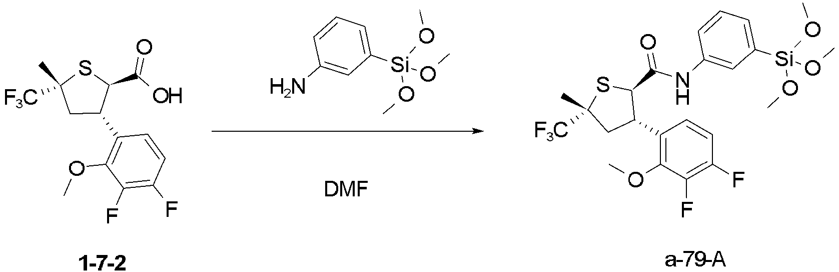

Compound 1-7-2 (50.0 mg, 0.14 mmol) was dissolved in anhydrous DMF (2 mL), 3-(trimethoxysilyl)aniline (89.5 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (38.5 mg, 0.07 mmol, yield: 48.33%).

MS: m/z = 552.1, [M+H]⁺, 1H NMR (400 MHz, DMSO) δ 10.30 (s, 1H), 7.72 - 7.63 (m, 2H), 7.33 (t, J = 7.6 Hz, 1H), 7.23 - 7.13 (m, 3H), 4.60 (d, J = 10.7 Hz, 1H), 4.27 (d, J = 4.5 Hz, 1H), 3.97 (s, 3H), 3.51 (s, 9H), 2.40 (d, J = 13.0 Hz, 1H), 2.33 - 2.28 (m, 1H), 1.73 (s, 3H).

### Example 185: Preparation of (2R,3S,5R)-3-(3,4-difluoro-2-methoxyphenyl)-5-methyl-N-(2-thiocyanatopyridin-4-yl)-5-(trifluoromethyl)tetrahydrothiophene-2-carboxamide (compound a-80-A)

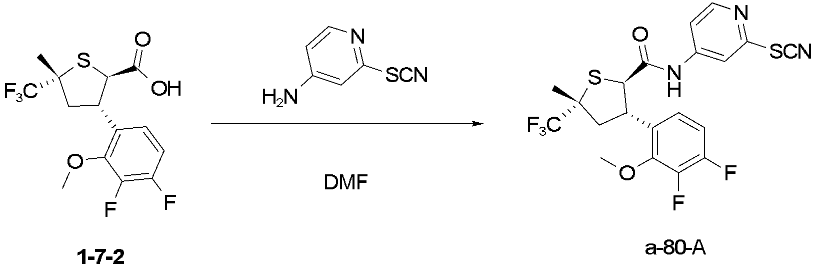

Compound 1-7-2 (50.0 mg, 0.14 mmol) was dissolved in anhydrous DMF (2 mL), 2-thiocyanatopyridin-4-amine (63.4 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred for 1 h. After the reaction was completed, the mixture was separated by Pre-HPLC to obtain the target compound (26.3 mg, 0.05 mmol, yield: 38.42%).

MS: m/z = 490.1, [M+H]⁺, ¹H NMR (400 MHz, DMSO) δ 10.94 (s, 1H), 8.38 (d, J = 5.6 Hz, 1H), 7.91 (d, J = 1.0 Hz, 1H), 7.44 (dd, J = 5.5, 1.4 Hz, 1H), 7.28 - 7.01 (m, 2H), 4.60 (d, J = 10.6 Hz, 1H), 4.27 (td, J = 12.9, 6.1 Hz, 1H), 3.97 (d, J = 1.4 Hz, 3H), 2.45 (d, J = 13.0 Hz, 1H), 2.32 (dd, J = 12.6, 6.0 Hz, 1H), 1.73 (s, 3H).

### Example 186: Preparation of tert-butyl 2-((2R,3S,5R)-3-(3,4-difluoro-2-methoxyphenyl)-5-methyl-5-(trifluoromethyl)tetrahydrothiophene-2-formyl)-2-(3-sulfamoylphenyl)hydrazinecarboxylate (compound a-81-A)

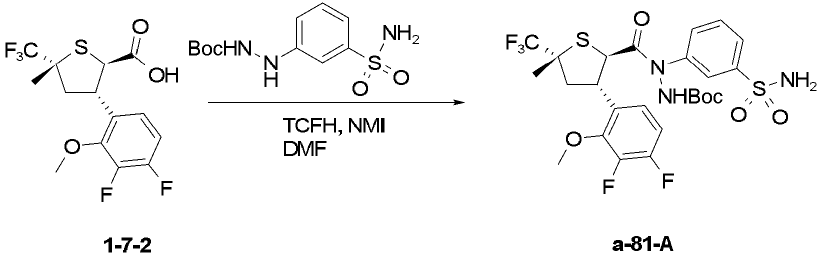

For the experimental procedures, reference may be made to Example 1.

MS: m/z = 625.1, [M+H]⁺, ¹H NMR (400 MHz, DMSO) δ 7.85 (s, 1H), 7.69 (d, *J =* 7.7 Hz, 1H), 7.59 (d, *J =* 6.5 Hz, 1H), 7.47 (s, 3H), 7.15 (t, *J =* 8.1 Hz, 1H), 4.27 (s, 1H), 4.00 (d, *J =* 13.2 Hz, 4H), 2.41 - 2.26 (m, 2H), 2.08 (s, 2H), 1.73 (s, 3H), 1.43 (d, *J =* 32.4 Hz, 9H).

### Example 187: Preparation of (2R,3S,5R)-3-(3,4-difluoro-2-methoxyphenyl)-5-methyl-5-(trifluoromethyl)-N-(3-(trihydroxysilyl)phenyl)tetrahydrothiophene-2-carboxamide (compound a-82-A)

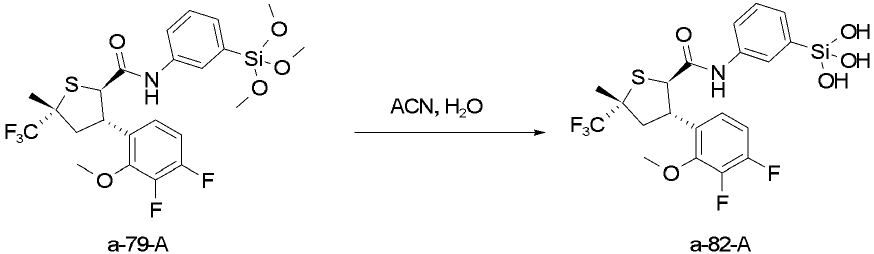

Compound a-79-A (20.0 mg, 0.04 mmol) was dissolved in ACN (3 mL) and H₂O (3 mL). The mixture was stirred at room temperature. After the reaction was completed, the mixture was separated by Pre-HPLC to obtain the target compound (15.9 mg, 0.03 mmol, yield: 78.23%).

MS: m/z = 510.1, [M+H]⁺, ¹H NMR (400 MHz, DMSO) δ 8.79 (s, 1H), 7.58 (dd, J = 14.9, 3.0 Hz, 1H), 7.42 (t, J = 14.9 Hz, 1H), 7.13 (dd, J = 15.0, 10.1 Hz, 1H), 7.02 (dd, J = 14.9, 3.0 Hz, 1H), 6.90 - 6.79 (m, 1H), 5.00 (d, J = 20.9 Hz, 1H), 4.89 (s, 3H), 3.92 (s, 3H), 3.69 (dt, J = 21.1, 16.8 Hz, 1H), 3.00 (dd, J = 24.8, 16.8 Hz, 1H), 2.50 (dd, J = 24.8, 16.8 Hz, 1H), 1.38 (s, 3H).

### Example 188: Preparation of 4-((2R,3S,5R)-3-(3,4-difluoro-2-methoxyphenyl)-5-methyl-5-(trifluoromethyl)tetrahydrothiophene-2-carboxamido)-N-methylpicolinamide (compound 253-A)

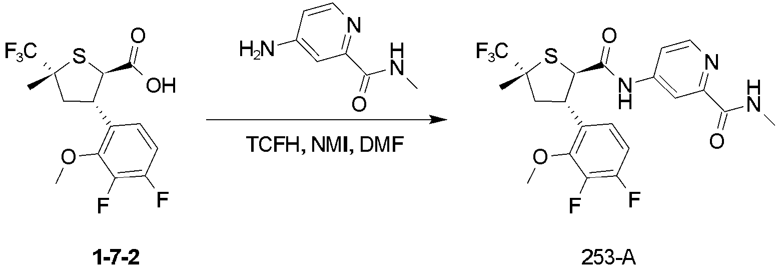

1-7-2 (50 mg, 0.14 mmol) was dissolved in anhydrous DMF (2 mL), 4-amino-N-methylpicolinamide (63.42 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (28 mg, 0.06 mmol, yield: 40.89%).

MS: m/z = 490.1, [M+H]⁺,¹H NMR (400 MHz, DMSO) δ 9.92 (s, 1H), 8.58 (s, 1H), 8.22-8.00 (m, 2H), 7.63-7.61 (m, 1H), 6.86-6.92 (m, 2H), 4.09-4.07 (m, 2H), 3.83(s, 3H), 3.48-3.46(m, 1H), 2.85 (s, 3H), 2.17-1.92(m, 2H), 2.21-1.96 (m, 2H), 1.38 (s, 3H).

### Example 189: Preparation of (2R,3S,5R)-3-(3,4-difluoro-2-methoxyphenyl)-N-(4-fluoro-3-(hydroxymethyl)phenyl)-5-methyl-5-(trifluoromethyl)tetrahydrothiophene-2-carboxamide (compound 254-A)

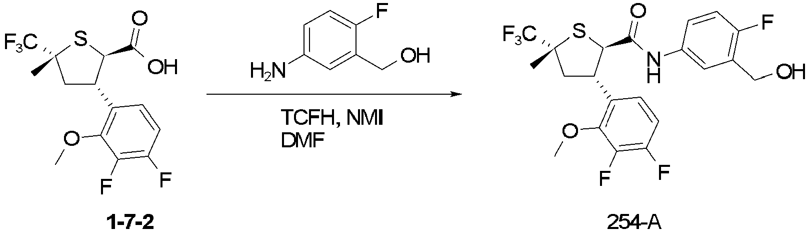

For the experimental procedures, reference may be made to Example 1.

MS: m/z = 479.1, [M+H]+, ¹H NMR (400 MHz, DMSO) δ 10.31 (s, 1H), 7.63 (dd, *J =* 6.7, 2.6 Hz, 1H), 7.48 - 7.41 (m, 1H), 7.24 - 7.13 (m, 2H), 7.10 - 7.03 (m, 1H), 4.61 (d, *J =* 10.7 Hz, 1H), 4.50 (d, *J =* 5.7 Hz, 2H), 4.28 (tt, *J =* 7.2, 5.1 Hz, 1H), 4.10 - 4.02 (m, 1H), 3.99 (d, *J =* 1.8 Hz, 3H), 2.49 - 2.29 (m, 2H), 1.76 (s, 3H).

### Example 190: Preparation of (2R,3S,5R)-3-(3,4-difluoro-2-methoxyphenyl)-N-(4-fluoro-3-formylphenyl)-5-methyl-5-(trifluoromethyl)tetrahydrothiophene-2-carboxamide (compound a-84-A)

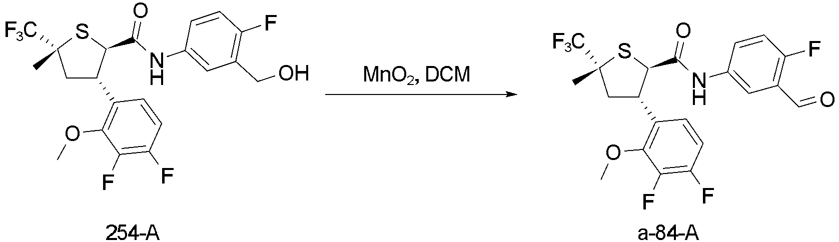

Compound 254-A (100 mg, 0.21 mmol) was dissolved in anhydrous DCM (5 mL), and active MnO₂ (55 mg, 0.63 mmol) was added. The mixture was stirred at room temperature. After the reaction was completed, the mixture was separated by Pre-HPLC to obtain the target compound (68 mg, 0.05 mmol, yield: 48.32%).

MS: m/z = 477.08, ¹H NMR (400 MHz, DMSO) δ 10.55 (s, 1H), 10.19 (s, 1H), 8.05 (dd, *J =* 6.2, 2.8 Hz, 1H), 7.78 (ddd, *J* = 8.8, 4.5, 2.9 Hz, 1H), 7.42 - 7.31 (m, 1H), 7.26 - 7.11 (m, 2H), 4.60 (d, *J =* 10.7 Hz, 1H), 4.29 (ddd, *J =* 17.0, 12.6, 6.1 Hz, 1H), 3.99 (d, *J =* 1.8 Hz, 3H), 2.50 - 2.29 (m, 2H), 1.76 (s, 3H).

### Example 191: Preparation of (2R,3S,5R)-3-(3,4-difluoro-2-methoxyphenyl)-N-(1-hydroxy-3,3-dimethyl-1,3-dihydrobenzo[c][1,2]oxaborol-6-yl)-5-methyl-5-(trifluoromethyl)tetrahydrothiophene-2-carboxamide (compound a-85-A)

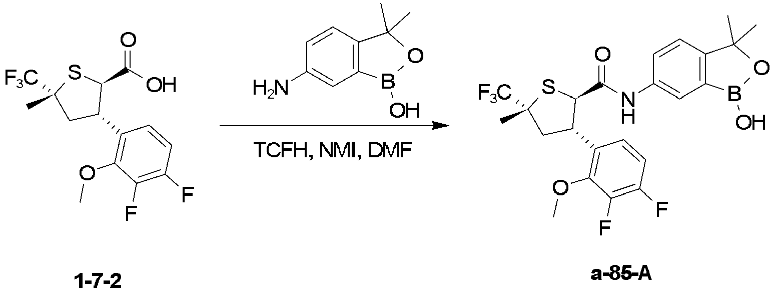

Compound 1-7-2 (50 mg, 0.14 mmol) was dissolved in anhydrous DMF (2 mL), 6-amino-3,3-dimethylbenzo[c][1,2]oxaborol-1(3H)-ol (74.6 mg, 0.42 mmol) was added, and TCFH (196.6 mg, 0.70 mmol) and NMI (92.1 mg, 1.12 mmol) were added in an ice bath. The mixture was allowed to naturally warm to room temperature and then stirred. After the reaction was completed, the mixture was separated by Pre-HPLC to obtain the target compound (28 mg, 0.05 mmol, yield: 38.83%).

MS: m/z = 516.2, [M+H]⁺,¹H NMR (400 MHz, DMSO) δ 10.02 (s,1H), 7.61-7.38 (m, 3H), 7.07-6.98 (m, 2H), 4.25-4.21 (m, 2H), 3.83(s, 3H), 3.48-3.46(m, 1H), 2.17-1.92(m, 2H), 1.38(s, 3H), 1.35(s, 6H).

### Example 192: Preparation of 4-((2R,3S,5R)-3-(3,4-difluoro-2-methoxyphenyl)-5-methyl-5-(trifluoromethyl)tetrahydrothiophene-2-carboxamido)-N-(N,N-dimethylsulfamoyl)picolinamide (compound a-86-A)

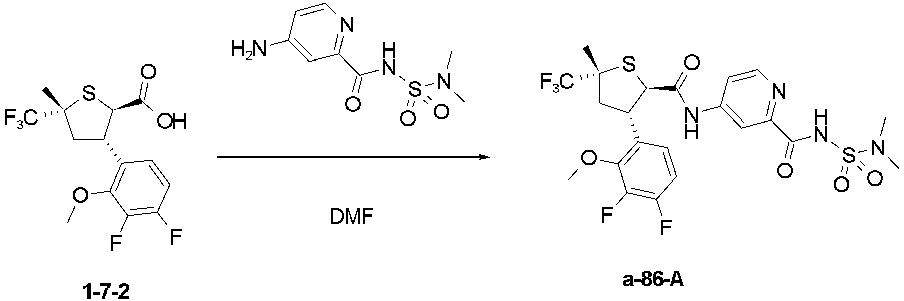

Compound 1-7-2 (50.0 mg, 0.14 mmol) was dissolved in anhydrous DMF (2 mL), 4-amino-N-(N,N-dimethylsulfamoyl)picolinamide (102.5 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (39.5 mg, 0.07 mmol, yield: 48.49%).

MS: m/z = 583.1, [M+H]⁺, ¹H NMR (400 MHz, DMSO) δ 10.94 (s, 1H), 8.53 (d, J = 5.5 Hz, 1H), 8.21 (d, J = 1.8 Hz, 1H), 7.73 (dd, J = 5.5, 2.0 Hz, 1H), 7.32 (dd, J = 12.4, 5.2 Hz, 2H), 7.15 (s, 1H), 4.29 (td, J = 13.0, 6.2 Hz, 1H), 4.19 - 4.10 (m, 1H), 3.97 (d, J = 1.6 Hz, 3H), 2.86 (s, 6H), 2.39 - 2.21 (m, 2H), 1.74 (s, 3H).

### Example 193: Preparation of 4-((2R,3S,5R)-3-(3,4-difluoro-2-methoxyphenyl)-5-methyl-5-(trifluoromethyl)tetrahydrothiophene-2-carboxamido)-3-methylpicolinamide (compound 255-A)

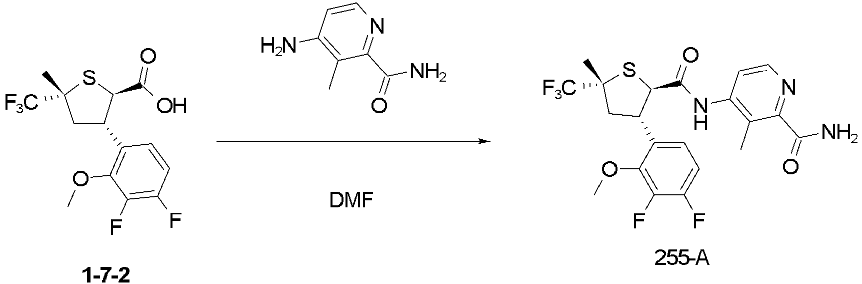

Compound 1-7-2 (50.0 mg, 0.14 mmol) was dissolved in anhydrous DMF (2 mL), 4-amino-3-methylpicolinamide (63.4 mg, 0.42 mmol) was added, and TCFH (196.0 mg, 0.70 mmol) and NMI (91.8 mg, 1.12 mmol) were added in an ice bath. The mixture was allowed to naturally warm to room temperature and then stirred. After the reaction was completed, the mixture was separated by Pre-HPLC to obtain the target compound (40.0 mg, 0.08 mmol, yield: 58.20%).

MS: m/z = 490.1, [M+H]⁺, ¹H NMR (400 MHz, DMSO) δ 9.83 (s, 1H), 8.28 (d, *J =* 5.3 Hz, 1H), 7.87 (s, 1H), 7.57 (d, *J =* 5.3 Hz, 1H), 7.48 (s, 1H), 7.22 - 7.14 (m, 2H), 4.81 (d, *J =* 10.7 Hz, 1H), 4.31 4.16 (m, 1H), 3.94 (d, *J =* 1.8 Hz, 3H), 2.42 - 2.29 (m, 2H), 2.13 (s, 3H), 1.73 (s, 3H).

### Example 194: Preparation of (2R,3S,5R)-N-(2-aminophenyl)-3-(3,4-difluoro-2-methoxyphenyl)-5-methyl-5-(trifluoromethyl)tetrahydrothiophene-2-carboxamide (compound 256-A)

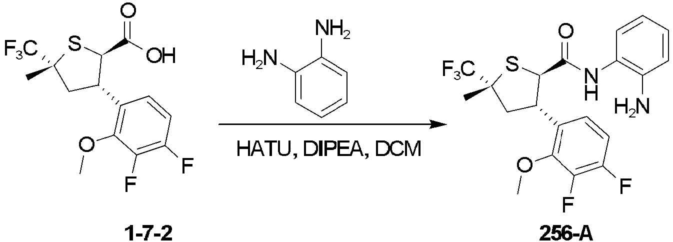

Compound 1-7-2 (500 mg, 1.40 mmol) was dissolved in anhydrous DCM (2 mL), benzene-1,2-diamine (910 mg, 8.42 mmol) was added, and HATU (1.60 g, 4.21 mmol) and DIPEA (543 mg, 4.21 mmol) were added in an ice bath. The mixture was allowed to naturally warm to room temperature and then stirred. After the reaction was completed, the mixture was separated by Pre-HPLC to obtain the target compound (325 mg, 0.728 mmol, yield: 52.03%).

MS: m/z = 447.1, [M+H]+, ¹H NMR (400 MHz, DMSO) δ 9.44 (s, 1H), 7.33 - 7.15 (m, 2H), 7.06 (dd, *J =* 7.9, 1.3 Hz, 1H), 6.97 - 6.86 (m, 1H), 6.69 (dd, *J =* 8.0, 1.3 Hz, 1H), 6.58 - 6.47 (m, 1H), 4.67 (d, *J =* 10.9 Hz, 3H), 4.33 - 4.20 (m, 1H), 3.98 (d, *J =* 1.8 Hz, 3H), 2.42 (t, *J =* 12.8 Hz, 1H), 2.33 (dd, *J* = 12.6, 6.2 Hz, 1H), 1.76 (s, 3H).

### Example 195: Preparation of 4-((2R,3S,5R)-3-(3,4-difluoro-2-methoxyphenyl)-5-methyl-5-(trifluoromethyl)tetrahydrothiophene-2-carboxamido)-N-hydroxypicolinamide (compound a-87-A)

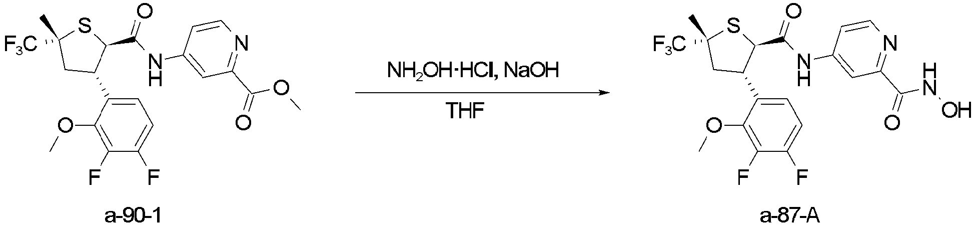

Hydroxylammonium chloride (354 mg, 5.10 mmol) was dissolved in anhydrous THF (5 mL), and solid sodium hydroxide (164 mg, 4.10 mmol) was slowly added in an ice bath. The mixture was allowed to react for 10 min in an ice bath, and compound a-90-1 (50 mg, 0.10 mmol) was then added. The mixture was allowed to naturally warm to room temperature and then stirred. After the reaction was completed, the mixture was separated by Pre-HPLC to obtain the target compound (12.7 mg, 0.025 mmol, yield: 25.84%).

MS: m/z = 492.1. ¹H NMR (400 MHz, DMSO) δ 11.35 (s, 1H), 10.81 (s, 1H), 9.06 (s, 1H), 8.42 (d, *J* = 5.5 Hz, 1H), 8.10 (d, *J =* 2.0 Hz, 1H), 7.64 (dd, *J =* 5.5, 2.2 Hz, 1H), 7.24 - 7.10 (m, 2H), 4.60 (d, *J =* 10.6 Hz, 1H), 4.33 - 4.22 (m, 1H), 3.97 (d, *J =* 1.9 Hz, 3H), 2.45 (d, *J =* 12.9 Hz, 1H), 2.33 (dd, *J =* 12.6, 6.1 Hz, 1H), 1.74 (s, 3H).

### Example 196: Preparation of N-(2-aminophenyl)-4-((2R,3S,5R)-3-(3,4-difluoro-2-methoxyphenyl)-5-methyl-5-(trifluoromethyl)tetrahydrothiophene-2-carboxamide)picolinamide (a-88-A)

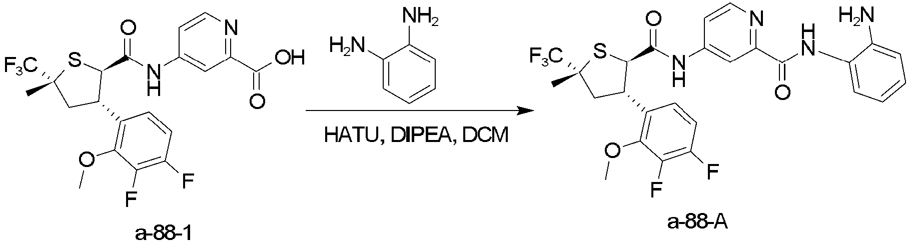

4-((2R,3S,5R)-3-(3,4-Difluoro-2-methoxyphenyl)-5-methyl-5-(trifluoromethyl)tetrahydrothiophene-2-carboxamido)pyridine-2-carboxylic acid (200 mg, 0.42 mmol) was dissolved in anhydrous DCM (2 mL), benzene-1,2-diamine (272 mg, 2.52 mmol) was added, and HATU (479 mg, 1.26 mmol) and DIPEA (163 mg, 1.26 mmol) were added in an ice bath. The mixture was allowed to naturally warm to room temperature and then stirred. After the reaction was completed, the mixture was separated by Pre-HPLC to obtain the target compound (196 mg, 0.346 mmol, yield: 82.70%).

MS: m/z = 567.1, [M+H]⁺. ¹H NMR (400 MHz, DMSO) δ 10.92 (s, 1H), 10.05 (s, 1H), 8.59 (d, *J =* 5.5 Hz, 1H), 8.27 (d, *J =* 2.0 Hz, 1H), 7.78 (dd, *J =* 5.5, 2.2 Hz, 1H), 7.52 (dd, *J =* 7.9, 1.3 Hz, 1H), 7.27 - 7.14 (m, 2H), 6.99 - 6.93 (m, 1H), 6.85 (dd, *J* = 7.9, 1.3 Hz, 1H), 6.75 - 6.62 (m, 1H), 4.65 (d, *J =* 10.6 Hz, 1H), 4.32 (ddd, *J* = 26.3, 17.4, 10.9 Hz, 1H), 4.01 (d, *J =* 2.0 Hz, 3H), 2.37 (dd, *J* = 12.6, 6.0 Hz, 1H), 1.78 (s, 3H).

### Example 197: Preparation of (2R,3S,5R)-N-(3-((E)-3-amino-2-cyano-3-oxopropen-1-yl)-4-fluorophenyl)-3-(3,4-difluoro-2-methoxyphenyl)-5-methyl-5-(trifluoromethyl)tetrahydrothiophene-2-carboxamide (compound a-89-A)

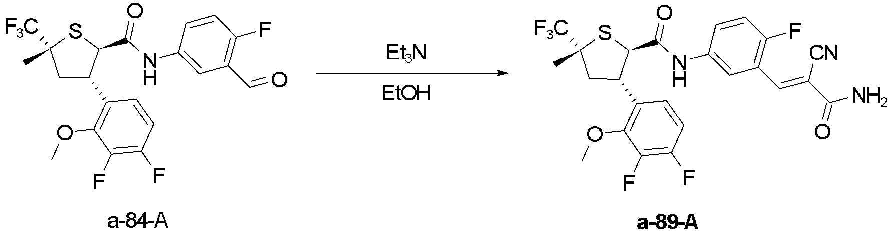

Compound a-84-A (100 mg, 0.21 mmol) was dissolved in anhydrous EtOH (5 mL), and Et₃N (64 mg, 0.63 mmol) was added. The mixture was warmed to 80 °C and stirred. After the reaction was completed, the mixture was separated by Pre-HPLC to obtain the target compound (73 mg, 0.13 mmol, yield: 64.13%).

MS: m/z = 543.11, ¹H NMR (400 MHz, DMSO) δ 10.58 (s, 1H), 8.28 (dd, *J* = 6.5, 2.5 Hz, 1H), 8.20 (s, 1H), 8.05 (s, 1H), 7.88 (s, 1H), 7.75 (ddd, *J* = 8.9, 4.6, 2.7 Hz, 1H), 7.36 (t, *J =* 9.5 Hz, 1H), 7.26 - 7.12 (m, 2H), 4.63 (d, *J =* 10.7 Hz, 1H), 4.33 (dq, *J* = 10.9, 6.2 Hz, 1H), 4.00 (d, *J =* 1.7 Hz, 3H), 2.46 (t, *J =* 12.9 Hz, 1H), 2.34 (dd, *J =* 12.6, 6.1 Hz, 1H), 1.76 (s, 3H).

### Example 198: Preparation of 4-(N-methyl(2R,3S,5R)-3-(3,4-difluoro-2-methoxyphenyl)-5-methyl-5-(trifluoromethyl)tetrahydrothiophene-2-carboxamido)pyridine-2-carboxamide (compound a-90-A)

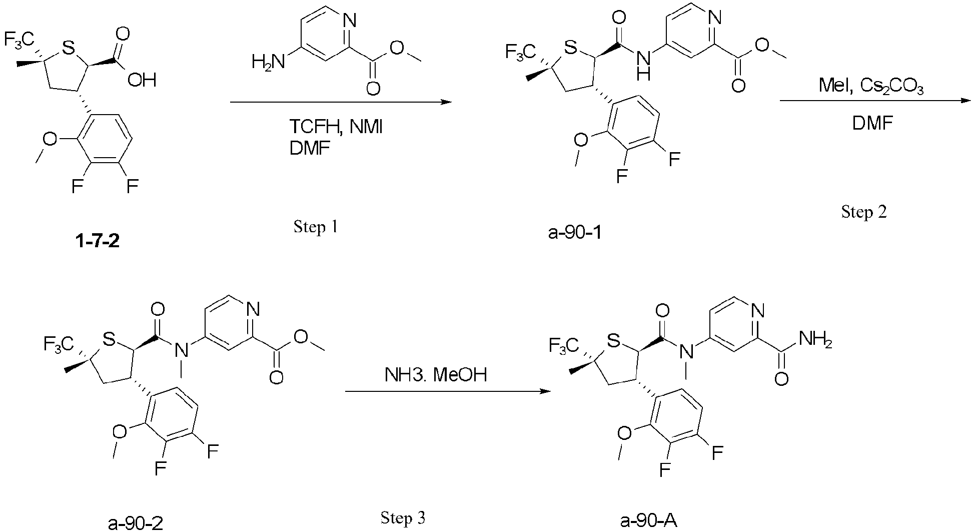

### Step 1: Synthesis of methyl 4-((2R,3S,5R)-3-(3,4-difluoro-2-methoxyphenyl)-5-methyl-5-(trifluoromethyl)tetrahydrothiophene-2-carboxamido)pyridine-2-carboxylate (compound a-90-1)

Compound 1-7-2 (50 mg, 0.14 mmol) was dissolved in anhydrous DMF (5 mL), methyl 4-aminopicolinate (64 mg, 0.42 mmol) was added, and TCFH (197 mg, 0.70 mmol) and NMI (92 mg, 1.12 mmol) were added in an ice bath. The mixture was allowed to naturally warm to room temperature and then stirred. After the reaction was completed, the mixture was separated by Pre-HPLC to obtain the target compound (65 mg, 0.133 mmol, yield: 94%). MS: m/z = 491.10, [M+H]⁺.

### Step 2: Synthesis of methyl 4-(N-methyl(2R,3S,5R)-3-(3,4-difluoro-2-methoxyphenyl)-5-methyl-5-(trifluoromethyl)tetrahydrothiophene-2-carboxamido)pyridine-2-carboxylate (a-90-2)

Compound a-90-1 (65 mg, 0.133 mmol) was dissolved in anhydrous DMF (5 mL), and Cs₂CO₃ (130 mg, 0.398 mmol) was added. The mixture was stirred at room temperature for 15 min. MeI (28 mg, 0.199 mmol) was added in an ice bath. The mixture was allowed to naturally warm to room temperature and then stirred. After the reaction was completed, the mixture was separated by Pre-HPLC to obtain the target compound (58 mg, 0.115 mmol, yield: 87%). MS: m/z = 505.11, [M+H]⁺.

### Step 3: Synthesis of 4-(N-methyl(2R,3S,5R)-3-(3,4-difluoro-2-methoxyphenyl)-5-methyl-5-(trifluoromethyl)tetrahydrothiophene-2-carboxamido)pyridine-2-carboxamide (a-90-A)

Compound a-90-2 (58 mg, 0.115 mmol) was dissolved in NH₃·MeOH (7 mol/L, 5 mL). The mixture was stirred at room temperature for 1 h. After the reaction was completed, the mixture was separated by Pre-HPLC to obtain the target compound (33 mg, 0.067 mmol, yield: 59%).

MS: m/z = 490.11, [M+H]⁺, ¹H NMR (400 MHz, DMSO) δ 8.56 - 8.32 (m, 3H), 7.25 - 7.12 (m, 2H), 6.23 (s, 2H), 4.62 (d, *J =* 10.7 Hz, 1H), 4.36 - 4.22 (m, 1H), 3.99 (d, *J =* 1.6 Hz, 3H), 3.41 (s, 3H), 2.44 (t, *J =* 12.9 Hz, 1H), 2.33 (dd, *J =* 12.6, 6.0 Hz, 1H), 1.76 (s, 3H).

### Experimental Example 1: Effects of test substances on current of stably overexpressed Nav1.8 channels monitored via manual patch-clamp technique

### Experimental method:

### 1. Reagent preparation

Test compounds were dissolved in dimethyl sulfoxide (DMSO).

The extracellular fluid was as follows: 140 mM NaCl, 3.5 mM KCl, 1 mM MgCl₂•6H₂O, 2 mM CaCl₂•2H₂O, 10 mM D-Glucose, 10 mM HEPES, 1.25 mM NaH₂PO₄•2H₂O; the pH was adjusted to 7.4 with NaOH. The intracellular fluid was as follows: 50 mM CsCl, 10 mM NaCl, 10 mM HEPES, 60 mM CsF, 20 mM EGTA; the pH was adjusted to 7.2 with CsOH.

### 2. Experimental materials and instruments

1) patch-clamp amplifier: EPC 10 (HEKA)
2) micromanipulator: MP225 (Sutter Instrument)
3) inverted microscope: MF53 (Mshot)
4) micropipette puller: P97 (Sutter Instrument)
5) capillary glass tube: BF150-86-10 (Sutter Instrument)

### 3. Experimental procedures

1) After the compounds were prepared into solutions with specified concentrations, the solutions were sequentially added to the pipeline of the drug delivery system and labeled.
2) The cell climbing coverslip was placed in the recording chamber, the appropriate cells were selected under the inverted microscope, and the position of the drug delivery tip was adjusted.
3) A capillary glass tube was pulled into a suitable recording electrode using the micropipette puller, and then the electrode filled with the intracellular fluid was mounted in the microelectrode holder. The micromanipulator was adjusted under the inverted microscope to allow the recording electrode to contact the cell, and negative pressure was applied to the electrode to form a high-resistance seal. At this time, fast capacitance compensation was performed, and then the application of negative pressure was continued to break the cell membrane, forming a whole-cell recording mode. Finally, slow capacitance compensation was performed, and related parameters were recorded.
4) After the cell current stabilized, administration was started. Each concentration of the drug was applied for five minutes or until the current stabilized before proceeding to monitor the next concentration. The drug solutions were administered via gravity-fed delivery, flowing through the recording chamber to act on the cells in an ascending order of concentration (from low to high). A peristaltic pump was employed for solution exchange in the recording process.

### 4 Test voltage program (resting state) and results

After the formation of the whole-cell seal, the cell voltage was clamped at -120 mV, followed by depolarization to 0 mV using a square-wave pulse with a duration of 50 ms to elicit Nav1.8 currents. This procedure was repeated every 20 s to monitor the maximum current induced by the square wave. After the current stabilized, the test compounds were administered. Once the response stabilized, the intensity of the current block was calculated.

### 5. The test results are shown in Tables 2-1, 2-2, and 3 below:

**Table 2-1. Determination results of inhibition rates of compounds of the present disclosure at concentration of 10 nM**

| Compound | Inhibition rate % | Compound | Inhibition rate % | Compound | Inhibition rate % |
|---|---|---|---|---|---|
| 9 | 84.93 | 134 | 76.51 | 240-A | 87.4 |
| 10-A | 87.5 | 134-A | 87.78 | 241-A | 95.4 |
| 20-A | 87.9 | 139 | 98.18 | 242-A | 97.7 |
| 31 | 65.03 | 139-A | 95.31 | a-3-A | 80.2 |
| 31-A | 84.7 | 170 | 97.92 | a-6-A | 76.9 |
| 48 | 79.75 | 172 | 86.52 | a-9 | 85.3 |
| 48-A | 91.5 | 178 | 85.96 | a-10 | 88 |
| 50-A | 80.9 | 191-A | 88.8 | a-16 | 92.3 |
| 82 | 81.13 | 192 | 96.4 | a-17-A | 65.5 |
| 88 | 95.6 | 197 | 91.1 | a-18-A | 86.3 |
| 88-A | 92.7 | 199 | 89.4 | a-25 | 96.5 |
| 93 | 89.7 | 219 | 88 | a-26-A | 95.6 |
| 101-A | 77.9 | 222 | 97 | a-28-A | 80.6 |
| 140-A | 96.2 | 227 | 97.7 | a-36-A | 83.7 |
| 146-A | 98.3 | 231-A | 83.7 | a-41-A | 80.6 |
| 178-A | 90.2 | 232 | 96.1 | | |

**Table 2-2. Determination results of inhibition rates of compounds of the present disclosure at concentration of 1 nM**

| Compound | Inhibition rate % | Compound | Inhibition rate % | Compound | Inhibition rate % |
|---|---|---|---|---|---|
| 136 | 61.9 | 248-A | 72.6 | a-42-A | 42 |
| 175 | 87.8 | 249-A | 81.6 | a-43-A | 40.1 |
| 202 | 81 | 252-A | 62.7 | a-44-A | 49.9 |
| 202-A | 86.9 | 253-A | 68.6 | a-46-A | 46.7 |
| 203 | 80.4 | 254-A | 76.8 | a-47-A | 92.1 |
| 225 | 91.2 | a-2 | 83.9 | a-48-A | 71.9 |
| 226-A | 32.2 | a-15 | 70.9 | a-51-A | 88.5 |
| 229-A | 48.6 | a-21-A | 84.6 | a-56-A | 66.4 |
| 232 | 78.4 | a-22 | 80.7 | a-73-A | 58.8 |
| 233-A | 94.1 | a-23-A | 56.5 | a-75-A | 55.2 |
| 238-A | 85.5 | a-30-A | 74.5 | a-84-A | 69.3 |
| 244-A | 87.8 | a-31-A | 81.9 | a-87-A | 47.7 |
| 246-A | 77.8 | a-39-A | 72 | a-89-A | 51.5 |

**Table 3. Determination results of inhibition rates (IC₅₀) of some compounds of the present disclosure**

| Compound | IC₅₀(nM) | Compound | IC₅₀(nM) | Compound | IC₅₀(nM) |
|---|---|---|---|---|---|
| 19 | 0.33 | a-7-A | 0.15 | a-35-A | 0.69 |
| 82-A | 0.38 | a-19-A | 0.34 | a-45-A | 0.024 |
| 119-A | 0.49 | a-20-A | 0.001 | a-57-A | 0.023 |
| 139 | 0.20 | a-29-A | 0.005 | a-65-A | 0.017 |
| 139-A | 0.06 | a-32-A | 0.1 | a-66-A | 0.26 |
| 223-A | 0.02 | a-34-A | 0.04 | a-80-A | 0.19 |

The compounds of the present disclosure, such as the example compounds, have a good Nav1.8 inhibitory effect.

### Experimental Example 2: Pharmacokinetic study of compounds in SD rats

Experimental animals: SD rats, male, 6-8 weeks old.

Compound preparation: 5% (final preparation volume) of DMSO was added to dissolve the test compound, and then 10% (final preparation volume) of Cremophor EL and 85% Saline were sequentially added.

Experimental design: The test compounds were administered to SD rats as a single dose via intravenous and oral administration, respectively (injection: 2 mg/kg; oral administration: 10 mg/kg; n = 3). The injection group was given free access to food and water. The oral group was fasted overnight (> 12 h) with free access to water, and was fed 4 h after administration. Blood samples were collected from the jugular vein of SD rats at the following time points: 5 min, 15 min, 0.5 h, 1 h, 2 h, 4 h, 8 h, and 24 h after injection administration; 15 min, 0.5 h, 1 h, 2 h, 4 h, 6 h, 8 h, and 24 h after oral administration. The collected blood samples were anticoagulated, placed in an ice bath, and then centrifuged at 6000 g for 5 min to separate plasma. The plasma samples were stored at -70 °C for analysis.

Sample monitoring: The concentrations of the designated compound drugs in plasma were determined by the LC-MS/MS method; the main pharmacokinetic parameters were calculated using the Winnolin 8.3 non-compartmental model.

The test results are shown in Table 4 below:

**Table 4. Pharmacokinetic test results of some compounds of the present disclosure in SD rats**

| Compound | Route of administration | Dose of administration | AUC_{0-inf} (h*ng/mL) | Cₘₐₓ (ng/mL) | Tₘₐₓ (h) | t_{1/2} (h) | F (%) |
|---|---|---|---|---|---|---|---|
| 139 | Injection | 2 mg/kg | 3844 | / | / | 4.86 | / |
| | Oral administration | 10 mg/kg | 9313 | 724 | 4 | 6.29 | 48.5 |
| VX-548(rac) | Injection | 2 mg/kg | 2647 | / | / | 2.64 | / |
| | Oral administration | 10 mg/kg | 4974 | 577 | 2 | 3.26 | 37.6 |

As can be seen from the data in Table 4, compound 139 of the present disclosure has better pharmacokinetic properties in rats, wherein the exposure level after oral administration was 2 times that of VX-548, and the t_{1/2} was also 2 times that of VX-548, indicating that compound 139 has stronger efficacy and longer analgesic time.

### Experimental Example 3: Pharmacokinetic study of compounds in ICR mice

### Experimental animals: ICR mice, male, 6-8 weeks old.

Compound preparation: 5% (final preparation volume) of DMSO was added to dissolve the test compound, and then 10% (final preparation volume) of Cremophor EL and 85% Saline were sequentially added.

Experimental design: The test compounds were administered to ICR mice as a single dose via intravenous and oral administration, respectively (injection: 2 mg/kg; oral administration: 10 mg/kg; n = 3). The injection group was given free access to food and water. The oral group was fasted overnight (> 12 h) with free access to water, and was fed 4 h after administration. Blood samples were collected from the jugular vein of ICR mice at the following time points: 5 min, 15 min, 0.5 h, 1 h, 2 h, 4 h, 8 h, and 24 h after injection administration; 15 min, 0.5 h, 1 h, 2 h, 4 h, 6 h, 8 h, and 24 h after oral administration. The collected blood samples were anticoagulated, placed in an ice bath, and then centrifuged for 5 min to separate plasma. The plasma was stored at -70 °C for analysis.

Sample test: The concentrations of the designated compound drugs in plasma were determined by the LC-MS/MS method; the main pharmacokinetic parameters were calculated using the Winnolin 8.3 non-compartmental model. The test results are shown in Table 5 below:

**Table 5. Pharmacokinetic test results of some compounds of the present disclosure in ICR mice**

| Compound | Route of administration | Dose of administration (mg/kg) | AUC_{0-inf}(h*ng/mL) |
|---|---|---|---|
| Compound 119-A | Injection | 2 | 842 |
| | Oral administration | 20 | 6736 |
| Compound 139-A | *i.v.* | 2 | 3083 |
| | *p.o.* | 20 | 24849 |
| Compound 223-A | Injection | 2 | 7203 |
| | Oral administration | 20 | 50574 |
| Compound a-7-A | Injection | 2 | 2086 |
| | Oral administration | 10 | 10228 |
| Compound 202-A | Injection | 2 | 2053 |
| | Oral administration | 10 | 12452 |
| Compound a-20-A | Injection | 2 | 1990 |
| | Oral administration | 10 | 15390 |
| Compound 227-A | Injection | 2 | 1860 |
| | Oral administration | 10 | 5738 |
| Compound 233-A | Injection | 2 | 9491 |
| | Oral administration | 10 | 34148 |
| Compound 242-A | Injection | 2 | 3481 |
| | Oral administration | 10 | 18632 |
| Compound a-32-A | Injection | 2 | 7386 |
| | Oral administration | 10 | 22949 |
| Compound 246-A | Injection | 2 | 1397 |
| | Oral administration | 10 | 2583 |
| Compound a-37-A | Injection | 2 | 2030 |
| | Oral administration | 10 | 8130 |

### Experimental Example 4: Tissue distribution study of compounds in ICR mice

### Experimental animals: ICR mice, male, 6-8 weeks old.

Compound preparation: 5% (final preparation volume) of DMSO was added to dissolve the test compound, and then 10% (final preparation volume) of Cremophor EL and 85% Saline were sequentially added.

Experimental design: The test compounds were administered to ICR mice as a single dose via oral administration (20 mg/kg; n = 3, 3 time points). Blood samples were collected at 0.25 h, 2 h, and 12 h after oral administration, followed by sacrifice of the animals. Dorsal root ganglia (DRG) were collected, rinsed with ice-cold normal saline to remove residual blood, blotted dry with absorbent paper, and stored at -70 °C for analysis.

Sample test: The concentrations of the compound drugs in dorsal root ganglia were determined by the LC-MS/MS method.

The test results are shown in Table 6 below:

**Table 6. Distribution test results of some compounds of the present disclosure in dorsal root ganglia of ICR mice**

| Compound | Time (h) | Concentration (ng/g) |
|---|---|---|
| Compound 119-A | 0.25 | 875 |
| | 2 | 2582 |
| | 12 | 199 |
| Compound 139-A | 0.25 | 1919 |
| | 2 | 9380 |
| | 12 | 2603 |
| Compound 223-A | 0.25 | 511 |
| | 2 | 2360 |
| | 12 | 635 |
| Compound 202-A | 0.25 | 710 |
| | 2 | 7200 |
| | 12 | 2720 |
| Compound a-20-A | 0.25 | 1045 |
| | 2 | 5898 |
| | 12 | 1912 |
| Compound 233-A | 0.25 | 400 |
| | 2 | 2850 |
| | 12 | 489 |
| Compound 242-A | 0.25 | 597 |
| | 2 | 6067 |
| | 12 | 2217 |

### Experimental Example 5: Tissue distribution study of compounds in SD rats

Experimental animals: SD rats, male, 6-8 weeks old.

Compound preparation: 5% (final preparation volume) of DMSO was added to dissolve the test compound, and then 10% (final preparation volume) of Cremophor EL and 85% Saline were sequentially added.

Experimental design: The test compounds were administered to SD rats as a single dose via oral administration (10 mg/kg; n = 3, 3 time points). Blood samples were collected at 0.5 h, 2 h, and 6 h after oral administration, followed by sacrifice of the animals. Dorsal root ganglia were collected, rinsed with ice-cold normal saline to remove residual blood, blotted dry with absorbent paper, and stored at -70 °C for analysis.

Sample test: The concentrations of the designated compound drugs in plasma and tissues were determined by the LC-MS/MS method.

The test results are shown in Table 7 below:

**Table 7. Distribution test results of some compounds of the present disclosure in dorsal root ganglia of SD rats**

| Compound | Time (h) | Concentration (ng/g) |
|---|---|---|
| Compound a-7-A | 0.5 | 115 |
| | 2 | 768 |
| | 6 | 557 |
| Compound 139-A | 0.5 | 1071 |
| | 2 | 973 |
| | 6 | 549 |

As can be seen from the data in Tables 6 and 7, the compounds of the present disclosure have a relatively high concentration distribution in the dorsal root ganglia for a long time.

Experimental Example 6: Pharmacodynamic effect study of compounds in incision pain model in ICR mice Experimental animals: ICR mice, male, 28-35 g.

Compound preparation: 5% (final preparation volume) of DMSO was added to dissolve the test compound, and then 10% (final preparation volume) of Cremophor EL and 85% Saline were sequentially added.

### Experimental design:

### 1. Model establishment

A plantar incision pain model was established in mice via surgery before the administration and test. Mice were placed in an anesthesia induction chamber and given 3%-4% isoflurane for anesthesia induction. After the anesthesia induction, a breathing mask was applied, and the isoflurane concentration was adjusted to 1%-2% to prevent the mice from suffering pain during the surgery. Each mouse was placed in a supine position, and the surgical site was disinfected with alcohol and iodophor. Under sterile conditions, a 0.5 cm longitudinal incision was made on the left hind paw surface, 0.2 cm away from the heel, extending toward the toes. The skin and fascia were incised; the plantar muscle was separated, slightly lifted, and longitudinally incised. The skin was then sutured with 5-0 sutures and disinfected.

### 2. Administration and test

The Von Frey test was performed once before the surgery. On the second day after animal modeling, the test was repeated, and animals meeting the baseline requirements were selected for enrollment. The animals were randomly grouped based on the 50% PWT values, with 10 animals per group. Administration was performed under a double-blind test protocol. The 50% PWT of the animals was measured separately before administration (baseline) and at 0.5 h, 2 h, and 4 h after administration, and a single dose was administered. The model control group was given normal saline, while the compound groups were given the compounds according to the administration doses in the table below.

The test results are shown in Table 8 below:

**Table 8. PWT 50% values of each group of mice at different time points with compounds of the present disclosure (g, Mean + SEM, n = 10)**

| Group | PWT 50% value (g) | | | | | |
|---|---|---|---|---|---|---|
| | Pre-surgery | Post-surgery, Preadministration | 0.5 h | 2 h | 4 h | AUC₀₋₈ₕ |
| Model control | 1.2±0.15 | 0.22±0.03 | 0.2±0.04 | 0.21±0.02 | 0.24±0.06 | 1.84±0.25 |
| Compound a-7-A 50 mg/kg | 1.43±0.43 | 0.27±0.13 | 0.47±0.16 | 0.72±0.35 | 0.47±0.25 | 3.62±1.28 |
| Compound a-19-A 50 mg/kg | 1.33±0.46 | 0.29±0.13 | 0.47±0.22 | 0.51±0.29 | 0.44±0.18 | 3.04±0.83 |
| Compound a-20-A 50 mg/kg | 1.14±0.21 | 0.29±0.09 | 0.39±0.14 | 0.49±0.18 | 0.57±0.22 | 3.48±0.82 |
| Compound a-34-A 50 mg/kg | 1.41±0.33 | 0.24±0.03 | 0.32±0.15 | 0.51±0.15 | 0.35±0.16 | 2.81±0.57 |
| Compound a-37-A 50 mg/kg | 1.43±0.33 | 0.24±0.03 | 0.33±0.12 | 0.55±0.18 | 0.32±0.1 | 2.71±0.39 |
| Compound a-57-A 50 mg/kg | 1.29±0.22 | 0.22±0.02 | 0.43±0.18 | 0.38±0.14 | 0.29±0.11 | 4.4±0.41 |
| Compound a-65-A 50 mg/kg | 1.13±0.08 | 0.22±0.02 | 0.38±0.16 | 0.43±0.17 | 0.51±0.2 | 4.87±0.97 |
| Compound 139-A 50 mg/kg | 1.39±0.31 | 0.22±0.03 | 0.74±0.3 | 1.05±0.38 | 0.74±0.29 | 5.45±1.62 |
| Compound 139-A 125 mg/kg | 1.25±0.31 | 0.22±0.02 | 0.82±0.33 | 1.09±0.32 | 0.87±0.16 | 6.08±1.2 |
| Compound 227-A 50 mg/kg | 1.5±0.22 | 0.22±0.02 | 0.4±0.19 | 0.52±0.15 | 0.34±0.15 | 2.74±0.56 |
| Compound 233-A 50 mg/kg | 1.54±0.4 | 0.22±0.02 | 0.42±0.34 | 0.62±0.32 | 0.41±0.21 | 3.21±1.35 |
| Compound 246-A 50 mg/kg | 1.4±0.17 | 0.24±0.03 | 0.32±0.12 | 0.33±0.16 | 0.4±0.27 | 2.53±0.82 |

As can be seen from the data in the table above, the compounds of the present disclosure have the properties of fast onset of action and long-lasting analgesic efficacy.

### Experimental Example 7: Pharmacodynamic effect study of compounds in incision pain model in SD rats

Experimental animals: SD rats, male, 180-220 g.

Compound preparation: 5% (final preparation volume) of DMSO was added to dissolve the test compound, and then 10% (final preparation volume) of Cremophor EL and 85% Saline were sequentially added.

### Experimental design:

### 1. Model establishment

A plantar incision pain model was established in rats via surgery before the administration and test. Rats were placed in an anesthesia induction chamber and given 3%-4% isoflurane for anesthesia induction. After the anesthesia induction, a breathing mask was applied, and the isoflurane concentration was adjusted to 1%-2% to prevent the rats from suffering pain during the surgery. Each rat was placed in a supine position, and the surgical site was disinfected with alcohol and iodophor. Under sterile conditions, a 0.5 cm longitudinal incision was made on the left hind paw surface, 0.2 cm away from the heel, extending toward the toes. The skin and fascia were incised; the plantar muscle was separated, slightly lifted, and longitudinally incised. The skin was then sutured with 5-0 sutures and disinfected.

### 2. Administration and test

The Von Frey test was performed once before the surgery. On the second day after animal modeling, the test was repeated, and animals meeting the baseline requirements were selected for enrollment. The animals were randomly divided into 6 groups based on the 50% PWT values, with 10 animals per group. Administration was performed under a double-blind test protocol. The 50% PWT of the animals was measured separately before administration (baseline) and at 0.5 h, 2 h, 4 h, 6 h, or 8 h after administration, and a single dose was administered. The model control group was given normal saline, while the compound groups were given the compounds according to the administration doses in the table below.

The test results are shown in Table 9 below:

**Table 9. PWT 50% values of each group of rats at different time points with compounds of the present disclosure (g, Mean + SEM, n = 10)**

| Group | PWT 50% value (g) | | | | | | |
|---|---|---|---|---|---|---|---|
| | Pre-surgery | Post-surgery, Preadministration | Time 0.5 h | Time 2 h | Time 6 h | Time 8 h | AUC₀₋₈ₕ |
| Model control | 14.74±1.8 | 4±1.19 | 4.7±1.06 | 3.87±1.41 | 4.49±1.86 | 4.71±1.21 | 33.26±4.27 |
| VX-548 125 mg/kg | 14.09±1.46 | 3.95±1.19 | 7.61±5.9 | 8.97±3.33 | 6.27±2.34 | 5.89±2.07 | 58.66±10.33 |
| Compound 139-A 125 mg/kg | 14.4±1.27 | 3.95±1.12 | 7.46±3.86 | 10.11±3.83 | 6.28±2.28 | 6.29±2.6 | 60.47±11.72 |

As can be seen from the data in the table above, compound 139-A shows a relatively good dose-effect relationship in the rat incision pain model. Compound 139-A has better analgesic activity than VX-548 at the same dose.

Experimental Example 8: Pharmacodynamic effect study of compounds in rat spinal nerve ligation model (SNL) Experimental animals: SD rats, male, 180-220 g.

Compound preparation: 5% (final preparation volume) of DMSO was added to dissolve the test compound, and then 10% (final preparation volume) of Cremophor EL and 85% Saline were sequentially added.

### Experimental design:

### 1. Model establishment

SD rats were anesthetized with isoflurane via inhalation. Each rat was placed in a prone position, and the hair at the surgical site was shaved locally. The surgical site was disinfected with alcohol and iodophor. Under sterile conditions, a 3-4 cm longitudinal incision was made at the level of the lower lumbar/upper sacrum to expose the left paravertebral muscles. Using small sharp-blunt scissors, the paravertebral muscles were bluntly dissected, and the transverse process of L6 was excised. The L4 and L5 spinal nerves were isolated and exposed, and the L5 spinal nerve was ligated with 6-0 silk sutures. The surgical wound was treated with ceftriaxone for anti-inflammation and infection prevention. The muscles and skin were sutured layer by layer, followed by disinfection with iodophor. After the surgery, the animals were placed on a warm electric blanket for heat preservation and returned to the cages for rearing after complete recovery from anesthesia. Postoperative behavioral observations were performed.

### 2. Administration and test

The Von Frey test was performed once before the surgery. On the 5th and 6th days after surgery, paw-lifting training was performed once each day. Animals with a baseline of 2-5 g were selected for enrollment and grouped based on the 50% PWT values, with 10 animals per group. The next day, a single dose was administered in a blinded manner. The 50% PWT of the animals was measured separately before administration (baseline) and at 0.5 h, 2 h, 4 h, 6 h, or 8 h after administration. The model control group was given normal saline, while the compound groups were given the compounds according to the administration doses in the table below.

The test results are shown in Table 10 below:

**Table 10. PTW 50% values of each group of rats at different time points with compounds of the present disclosure (g, Mean + SEM, n = 10)**

| Group | PWT 50% value (g) | | | | | | | |
|---|---|---|---|---|---|---|---|---|
| | Pre-surgery | Post-surgery, Preadministration | Time 0.5 h | Time 2 h | Time 4 h | Time 6 h | Time 8 h | AUC₀₋₈ₕ |
| Model control | 14.71±2.89 | 2.69±1.15 | 2.82±1.06 | 2.92±1.3 | 2.83±1.36 | 2.5±1.15 | 2.53±1.34 | 21.81±3.96 |
| Compound 139-A 50 mg/kg | 14.94±2.38 | 2.68±1.05 | 5.78±2.76 | 8.76±2.47 | 7.73±4.32 | 4.9±1.61 | 3.57±1.44 | 50.59±15.71 |
| Compound 139-A 125 mg/kg | 13.83±3.18 | 2.68±1 | 6.96±3.34 | 9.61±3.73 | 7.26±3.19 | 5.01±1.63 | 3.88±1.67 | 52.87±15.06 |

As can be seen from the data in Table 10, compound 139-A shows a relatively good dose-effect relationship in the rat SNL model. Compound 139-A showed significant differences compared with the model group at all time points.

Experimental Example 9: Pharmacodynamic effect study of compounds in CFA-induced rat inflammatory pain model Experimental animals: SD rats, male, 180-220 g.

Compound preparation: 5% (final preparation volume) of DMSO was added to dissolve the test compound, and then 10% (final preparation volume) of Cremophor EL and 85% Saline were sequentially added; an appropriate amount of Celecoxib was weighed, 5% (final preparation volume) of DMSO was added to dissolve the test compound, and then 5% (final preparation volume) of HS-15 and 90% Saline were sequentially added.

### Experimental design:

### 1. Model establishment

SD rats were anesthetized with isoflurane. The right hind paw was disinfected, a needle was inserted subcutaneously at the posterior plantar aspect, and 50 µL of complete Freund's adjuvant (CFA, 1 mg/mL) was administered via subcutaneous injection at the midplantar region, with the needle withdrawn slowly while rotating to prevent leakage. After modeling, local redness and swelling of the soles of the feet, difficulty in walking, and lifting of the affected feet were observed.

### 2. Administration and test

The Von Frey test was performed once before the surgery. On the second day after animal modeling, animals meeting the baseline requirements were selected for enrollment. The animals were randomly divided into 6 groups based on the 50% PWT values, with 10 animals per group. Administration was performed under a double-blind test protocol. The 50% PWT of the animals was measured separately before administration (baseline) and at 0.5 h, 2 h, and 8 h after administration. The model control group was given normal saline, while the compound groups were given the compounds according to the administration doses in the table below.

The test results are shown in Table 11 below:

**Table 11. PWT 50% values of each group of rats at different time points with compounds of the present disclosure (g, Mean + SEM, n = 10)**

| Group | PWT 50% value (g) | | | | | |
|---|---|---|---|---|---|---|
| | Pre-surgery | Post-surgery, Preadministration | Time 0.5 h | Time 2 h | Time 8 h | AUC₀₋₈ₕ |
| Model control | 13.44±3.71 | 3.13±1.2 | 3.32±1.39 | 3.21±1.25 | 3.11±1.19 | 25.08±5.52 |
| Celecoxib 80 mg/kg | 11.74±2.33 | 3.12±1.14 | 5.91±4.09 | 4.66±1.69 | 3.4±1.38 | 40.7±15.63 |
| Compound 139-A 125 mg/kg | 12.61±3.5 | 3.1±1.12 | 6.78±5.47 | 6.54±3.06 | 3.6±1.5 | 40.83±12.03 |

As can be seen from the data in Table 11, compound 139-A exhibits better analgesic activity in the CFA-induced rat inflammatory pain model, which is superior to the positive control Celecoxib.

### Experimental Example 10: Pharmacodynamic effect study of compounds in rat formalin pain model

Experimental animals: SD rats, male, 180-200 g.

Compound preparation: 5% (final preparation volume) of DMSO was added to dissolve the test compound, and then 10% (final preparation volume) of Cremophor EL and 85% Saline were sequentially added; an appropriate amount of Etoricoxib was weighed, 5% (final preparation volume) of DMSO was added to dissolve the test compound, and then 5% (final preparation volume) of HS-15 and 90% Saline were sequentially added; the tramadol injection was diluted with normal saline to the desired concentration.

### Experimental design:

### 1. Model establishment

Each group consisted of 10 animals, and 50 µL of a 5% formalin solution was injected using a microsyringe into the right plantar region.

### 2. Administration and test

Based on different pharmacokinetic properties of the drugs, the administration was performed at 0 min, 60 min, and 120 min before modeling. After injection of the formalin, the number of pain responses in the rats was observed. The rats exhibited behavioral responses such as foot raising, foot licking, and foot swinging, which were referred to as one pain response. Spontaneous pain responses were observed once every 5 min, and the number of pain responses in the rats within 1 min was counted, with a total observation duration of 60 min. Inhibition rate = (number of pain responses in control group - number of pain responses in administration group)/number of pain responses in control group × 100%.

Specific administration information is shown in Table 12 below.

**Table 12. Administration information of compounds of the present disclosure in rat formalin model**

| Group | Test substance | Dose of administration (mg/kg) | Route of administration | Time of administration |
|---|---|---|---|---|
| G1 | Model control | - | *p.o.* | 120 min before plantar injection of 5% formalin |
| G2 | Tramadol | 10 | *i.v.* | Immediate administration before plantar injection of 5% formalin |
| G3 | Etoricoxib | 30 | *p.o.* | 60 min before plantar injection of 5% formalin |
| G4 | VX-548 | 125 | *p.o.* | 120 min before plantar injection of 5% formalin |
| G5 | Compound 139-A | 50 | *p.o.* | |
| G6 | Compound 139-A | 125 | *p.o.* | |

The test results are shown in Table 13 below:

**Table 13. Number of pain responses of each group of rats with compounds of the present disclosure (Mean + SD, n = 10)**

| Group | 0 min | 10 min | 15 min | 30 min | 40 min | 50 min | 60 min |
|---|---|---|---|---|---|---|---|
| Model control | 42.1±6.5 | 2.9±3.4 | 13.5±5.2 | 21.3±4.6 | 18.7±3.8 | 14.8±9.4 | 12.5±5.9 |
| Tramadol 10 mg/kg | 31.9±15.0 | 1.9±3.0 | 4.1±4.4 | 12.8±6.7 | 13.2±8.1 | 8.6±3.6 | 9.3±5.1 |
| Etoricoxib 30 mg/kg | 37.5±9.2 | 2.0±2.4 | 10.1±4.0 | 14.3±5.3 | 10.8±6.2 | 5.1±8.5 | 3.3±3.7 |
| VX-548 125 mg/kg | 24.3±8.5 | 2.4±2.8 | 8.8±6.4 | 13.2±6.3 | 10.1±6.9 | 4.1±4.7 | 4.7±5.6 |
| Compound 139-A 50 mg/kg | 35.6±6.7 | 1.7±2.5 | 6.8±3.9 | 15.1±4.4 | 12.7±5.9 | 6.1±5.2 | 2.7±2.6 |
| Compound 139-A 125 mg/kg | 25.5±13.6 | 1.1±1.1 | 4.3±2.7 | 11.7±6.3 | 4.8±3.9 | 1.8±1.8 | 1.8±1.2 |

As can be seen from the data in Table 13, compound 139-A shows a relatively good dose-effect relationship in the rat formalin pain model. The inflammatory pain induced by formalin injection can be significantly manifested as 2 phases. The first phase is the pain response immediately after formalin subcutaneous injection, which is caused by the stimulation of nerve endings; the second phase is the pain response about 15-20 min after formalin subcutaneous injection, which is caused by the continuous afferent transmission of nociceptive information resulting from local chronic inflammation. Tramadol and etoricoxib exert superior analgesic effects in the first and second phases, respectively, in the model, and compound 139-A of the present disclosure shows significant analgesic activity in both the first and second phases, which is superior to that of VX-548.

### Experimental Example 11: Pharmacodynamic effect study of compounds in mouse acetic acid writhing model

Experimental animals: ICR mice, male, 20-24 g.

Compound preparation: 5% (final preparation volume) of DMSO was added to dissolve the test compound, and then 10% (final preparation volume) of Cremophor EL and 85% Saline were sequentially added; an appropriate amount of Etoricoxib was weighed, 5% (final preparation volume) of DMSO was added to dissolve the test compound, and then 5% (final preparation volume) of HS-15 and 90% Saline were sequentially added.

### Experimental design:

1. The mice were randomly grouped based on the body weight for model establishment, with 10 animals per group. A 0.8% (v/v) acetic acid solution was intraperitoneally injected at an injection volume of 10 mL/kg.
2. Administration and test

Administration was performed 60 min before modeling. After the acetic acid solution was injected, the number of writhing responses in mice was observed. The mice exhibited behavioral responses such as abdominal retraction, trunk and hindlimb extension, and hip elevation, which were referred to as one complete writhing response. The number of writhing responses was observed and recorded during two intervals: 0-15 min and 15-30 min, with a total observation duration of 30 min. Inhibition rate = (number of writhing responses in control group - number of writhing responses in administration group)/number of writhing responses in control group × 100%. The model control group was given normal saline, the positive control group 1 was given etoricoxib at 20 mg/kg, the positive control group 2 was given VX-548 at 50 mg/kg, and the remaining groups were given the compounds of the present disclosure according to the administration doses in the table below.

The test results are shown in Table 14 below:

**Table 14. Number of writhing responses of each group of mice with compounds of the present disclosure (Mean + SD, n = 10)**

| Group | Dose (mg/kg) | Number of writhing responses | Inhibition rate (%) |
|---|---|---|---|
| Model control group | / | 49 ± 16 | - |
| Etoricoxib | 20 | 29 ± 11 | - |
| VX-548 | 50 | 29±16 | 41.24 |
| Compound 119-A | 50 | 18 ± 15 | - |
| Compound 139-A | 50 | 27±18 | 45.36 |
| Compound 139-A | 125 | 18±12 | 62.89 |
| Compound a-20-A | 50 | 17 ± 11 | - |

As can be seen from the data in the table above, the compounds of the present disclosure, particularly compounds 119-A, 139-A, and a-20-A, are able to inhibit the writhing effect better than VX-548. Compound 139-A shows a relatively good dose-effect relationship in the mouse acetic acid writhing model; at the dose of 125 mg/kg, there is a very significant effect of inhibiting the number of writhing responses.

The above examples do not limit the solutions of the present application in any way. Various modifications of the present disclosure, in addition to those described herein, will be apparent to those skilled in the art on the basis of the aforementioned description. Such modifications are also intended to fall within the scope of the appended claims. References (including all patents, patent applications, journal articles, books, and any other publications) cited in the present application are all incorporated herein by reference in their entirety.

## Claims

1. A compound, or a pharmaceutically acceptable salt, a stereoisomer, a solvate, an isotopically labeled compound, or a polymorph thereof, wherein the compound has the following structure: 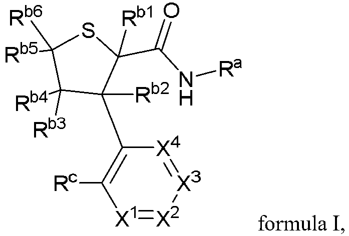 wherein:
R^{a} is selected from 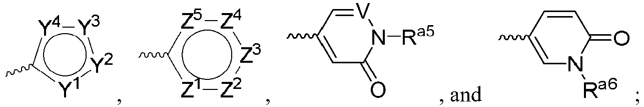
Y¹, Y², Y³, and Y⁴ are each independently selected from O, S, N, N-R^{a1}, and C-R^{a2};
each R^{a1} is independently selected from H, C₁₋₆ alkyl, C₁₋₆ haloalkyl, and -S(O)₂R¹;
each R^{a2} is independently selected from H, halogen, hydroxy, -CN, C₁₋₆ alkyl, C₁₋₆ haloalkyl, C₁₋₆ alkoxy, C₁₋₆ haloalkoxy, C₂₋₆ alkenyl, C₃₋₆ cycloalkyl, -NR²R³, -NHC(O)R⁴, -C(O)OR⁵, -C(O)NR⁶R⁷, -SR⁸, -S(O)R⁹, -S(O)₂R¹⁰, -S(O)₂NR¹¹R¹², -S(O)(NR¹³)R¹⁴, -P(O)R¹⁵R¹⁶, and 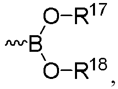 wherein the C₁₋₆ alkyl, C₁₋₆ haloalkyl, C₁₋₆ alkoxy, and C₁₋₆ haloalkoxy are optionally substituted with one or more substituents selected from hydroxy and -NR¹⁹R²⁰; or adjacent R^{a1} and R^{a2}, or two R^{a2}, together with the carbon atoms to which they are attached, form a 5-6 membered heteroaromatic ring;
R¹, R⁵, R⁶, R⁷, R⁸, R⁹, R¹⁰, R¹¹, R¹², R¹³, R¹⁴, R¹⁵, R¹⁶, R¹⁹, and R²⁰ are each independently selected from H and C₁₋₆ alkyl;
R² and R³ are each independently selected from H, C₁₋₆ alkyl, and C₁₋₆ alkyl substituted with carbonyl;
each R⁴ is independently selected from C₁₋₆ alkyl, C₁₋₆ haloalkyl, and C₂₋₆ alkenyl;
R¹⁷ and R¹⁸ are each independently selected from H and C₁₋₆ alkyl, or R¹⁷ and R¹⁸, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₆ alkyl;
Z¹, Z², Z³, Z⁴, and Z⁵ are each independently selected from N, N⁺-O⁻, and C-R^{a3};
each R^{a3} is independently selected from H, halogen, hydroxy, -CN, C₁₋₆ alkyl, C₁₋₆ haloalkyl, C₁₋₆ alkoxy, C₁₋₆ haloalkoxy, C₂₋₆ alkenyl, C₃₋₆ cycloalkyl, -NR²¹R²², -NHC(O)R²³, -C(O)OR²⁴, -C(O)NR²⁵R²⁶, -SR²⁷, -S(O)R²⁸, - S(O)₂R²⁹, -S(O)₂NR³⁰R³¹, -S(O)(NR³²)R³³, -P(O)R³⁴R³⁵, and 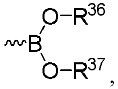 wherein the C₁₋₆ alkyl, C₁₋₆ haloalkyl, C₁₋₆ alkoxy, and C₁₋₆ haloalkoxy are optionally substituted with one or more substituents selected from hydroxy and - NR³⁸R³⁹;
or two adjacent R^{a3}, together with the carbon atoms to which they are attached, form 5-6 membered heterocyclyl or a 5-6 membered heteroaromatic ring, wherein the 5-6 membered heterocyclyl or 5-6 membered heteroaromatic ring is optionally substituted with one or more OH and C₁₋₆ alkyl;
R²⁴, R²⁵, R²⁶, R²⁷, R²⁸, R²⁹, R³⁰, R³¹, R³², R³³, R³⁴, R³⁵, R³⁸, and R³⁹ are each independently selected from H and C₁₋₆ alkyl;
R²¹ and R²² are each independently selected from H, C₁₋₆ alkyl, C₁₋₆ alkyl substituted with carbonyl, and -C(O)OC₁₋₆ alkyl;
each R²³ is independently selected from C₁₋₆ alkyl, C₁₋₆ haloalkyl, and C₂₋₆ alkenyl;
R³⁶ and R³⁷ are each independently selected from Hand C₁₋₆ alkyl, or R³⁶ and R³⁷, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₆ alkyl;
V is selected from N, N⁺-O⁻, and C-R^{a4};
R^{a4} is selected from H and C₁₋₆ alkyl;
R^{a5} is selected from H and C₁₋₆ alkyl;
R^{a6} is selected from H and C₁₋₆ alkyl;
R^{b1} and R^{b2} are each independently selected from H and deuterium;
R^{b3} and R^{b4} are each independently selected from H, deuterium, C₁₋₆ alkyl, C₁₋₆ haloalkyl, and C₃₋₆ cycloalkyl;
R^{b5} and R^{b6} are each independently selected from H, C₁₋₆ alkyl, C₁₋₆ haloalkyl, and C₃₋₆ cycloalkyl, or R^{b5} and R^{b6}, together with the carbon atom to which they are attached, form C₃₋₅ cycloalkyl and 4-6 membered heterocyclyl;
R^{c} is selected from H, hydroxy, halogen, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl, and -O-C₃₋₆ cycloalkyl, wherein the C₁₋₆ alkyl, C₁₋₆ haloalkyl, C₁₋₆ alkoxy, C₁₋₆ haloalkoxy, and C₃₋₆ cycloalkyl are optionally substituted with one or more substituents selected from hydroxy, carboxyl, C₁₋₆ alkoxy, C₁₋₆ haloalkoxy, C₂₋₆ alkenyl, -NR⁴⁰R⁴¹, C₃₋₆ cycloalkyl, 3-6 membered heterocyclyl, and 5-6 membered heteroaryl, wherein the 3-6 membered heterocyclyl and 5-6 membered heteroaryl are optionally substituted with one or more halogen and C₁₋₆ alkyl;
R⁴⁰ and R⁴¹ are each independently selected from H and C₁₋₆ alkyl;
X¹, X², X³, and X⁴ are each independently selected from N and C-R^{c1};
each R^{c1} is independently selected from H, halogen, C₁₋₆ alkyl, C₁₋₆ haloalkyl, C₁₋₆ alkoxy, and C₁₋₆ haloalkoxy.

2. The compound, or the pharmaceutically acceptable salt, the stereoisomer, the solvate, the isotopically labeled compound, or the polymorph thereof according to any one of claim 1, wherein:
R^{a} is selected from 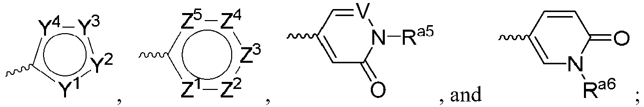
Y¹, Y², Y³, and Y⁴ are each independently selected from O, S, N, N-R^{a1}, and C-R^{a2};
each R^{a1} is independently selected from H, C₁₋₄ alkyl (e.g., methyl, ethyl, propyl, and butyl), C₁₋₄ haloalkyl (e.g., CF₃, CHF₂, and CH₂F), and -S(O)₂R¹ (e.g., -S(O)₂CH₃);
each R^{a2} is independently selected from H, halogen (e.g., fluorine, chlorine, bromine, and iodine), hydroxy, -CN, C₁₋₄ alkyl (e.g., methyl, ethyl, propyl, and butyl), C₁₋₄ haloalkyl (e.g., CF₃, CHF₂, and CH₂F), C₁₋₄ alkoxy, C₁₋₄ haloalkoxy, C₂₋₆ alkenyl, C₁₋₄ cycloalkyl, -NR²R³, -NHC(O)R⁴, -C(O)OR⁵, -C(O)NR⁶R⁷, SR⁸, -S(O)R⁹, -S(O)₂R¹⁰, -S(O)₂NR¹¹R¹², -S(O)(NR¹³)R¹⁴, -P(O)R¹⁵R¹⁶, and 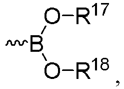 wherein the C₁₋₄ alkyl, C₁₋₄ haloalkyl, C₁₋₄ alkoxy, and C₁₋₄ haloalkoxy are optionally substituted with one or more substituents selected from hydroxy and -NR¹⁹R²⁰;
or adjacent R^{a1} and R^{a2}, or two R^{a2}, together with the carbon atoms to which they are attached, form a 5-6 membered heteroaromatic ring;
R¹, R⁵, R⁶, R⁷, R⁸, R⁹, R¹⁰, R¹¹, R¹², R¹³ , R¹⁴, R¹⁵, R¹⁶, R¹⁹, and R²⁰ are each independently selected from H and C₁₋₄ alkyl;
R² and R³ are each independently selected from H, C₁₋₄ alkyl, and C₁₋₄ alkyl substituted with carbonyl;
each R⁴ is independently selected from C₁₋₄ alkyl, C₁₋₄ haloalkyl, and C₂₋₆ alkenyl;
R¹⁷ and R¹⁸ are each independently selected from H and C₁₋₄ alkyl, or R¹⁷ and R¹⁸, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₄ alkyl;
Z¹, Z², Z³, Z⁴, and Z⁵ are each independently selected from N, N⁺-O⁻, and C-R^{a3};
each R^{a3} is independently selected from H, halogen (e.g., fluorine, chlorine, bromine, and iodine), hydroxy, -CN, C₁₋₄ alkyl (e.g., methyl, ethyl, propyl, and butyl), C₁₋₄ haloalkyl (e.g., CF₃, CHF₂, and CH₂F), C₁₋₄ alkoxy, C₁₋₄ haloalkoxy, C₂₋₆ alkenyl, C₁₋₄ cycloalkyl, -NR²¹R²², -NHC(O)R²³, -C(O)OR²⁴, -C(O)NR²⁵R²⁶, -SR²⁷, -S(O)R²⁸, -S(O)₂R²⁹, - S(O)₂NR³⁰R³¹, -S(O)(NR³²)R³³, -P(O)R³⁴R³⁵, and 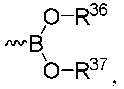 wherein the C₁₋₄ alkyl, C₁₋₄ haloalkyl, C₁₋₄ alkoxy, and C₁₋₄ haloalkoxy are optionally substituted with one or more substituents selected from hydroxy and -NR³⁸R³⁹; or two adjacent R^{a3}, together with the carbon atoms to which they are attached, form 5-6 membered heterocyclyl or a 5-6 membered heteroaromatic ring, wherein the 5-6 membered heterocyclyl or 5-6 membered heteroaromatic ring is optionally substituted with one or more OH and C₁₋₆ alkyl;
R²⁴, R²⁵, R²⁶, R²⁷, R²⁸, R²⁹, R³⁰, R³¹, R³², R³³, R³⁴, R³⁵, R³⁸, and R³⁹ are each independently selected from H and C₁₋₄ alkyl;
R²¹ and R²² are each independently selected from H, C₁₋₄ alkyl, C₁₋₄ alkyl substituted with carbonyl, and -C(O)OC₁₋₄ alkyl;
each R²³ is independently selected from C₁₋₄ alkyl, C₁₋₄ haloalkyl, and C₂₋₆ alkenyl;
R³⁶ and R³⁷ are each independently selected from H and C₁₋₄ alkyl, or R³⁶ and R³⁷, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₄ alkyl;
V is selected from N, N⁺-O⁻, and C-R^{a4};
R^{a4} is selected from H and C₁₋₄ alkyl (e.g., methyl, ethyl, propyl, and butyl);
R^{a5} is selected from H and C₁₋₄ alkyl (e.g., methyl, ethyl, propyl, and butyl);
R^{a6} is selected from H and C₁₋₄ alkyl (e.g., methyl, ethyl, propyl, and butyl).

3. The compound, or the pharmaceutically acceptable salt, the stereoisomer, the solvate, the isotopically labeled compound, or the polymorph thereof according to any one of claims 1-2, wherein:
R^{a} is selected from 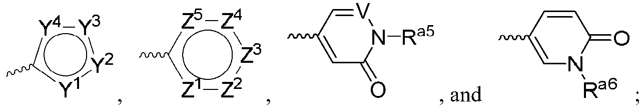
Y¹, Y², Y³, and Y⁴ are each independently selected from O, S, N, N-R^{a1}, and C-R^{a2};
each R^{a1} is independently selected from H, methyl, CF₃, CHF₂, and -S(O)₂CH₃;
each R^{a2} is independently selected from H, fluorine, chlorine, methyl, CF₃, CHF₂, -C(O)NH₂, -NH₂, and 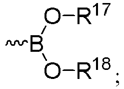 or adjacent R^{a1} and R^{a2}, or two R^{a2}, together with the carbon atoms to which they are attached, form a 5-6 membered heteroaromatic ring;
R¹⁷ and R¹⁸ are each independently selected from H and C₁₋₄ alkyl, or R¹⁷ and R¹⁸, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₄ alkyl;
Z¹, Z², Z³, Z⁴, and Z⁵ are each independently selected from N, N⁺-O⁻, and C-R^{a3};
each R^{a3} is independently selected from H, fluorine, chlorine, bromine, hydroxy, -CN, methyl, CF₃, CHF₂, methoxy, trifluoromethoxy, ethenyl, cyclopropyl, -CH₂OH, -C(CH₃)₂OH, -CH(OH)CH₂(OH), -CH(OH)CH₂F, - CH(NH₂)CH₂(OH), -NR²¹R²², -NC(O)R²³, -C(O)OR²⁴, -C(O)NR²⁵R²⁶, -SR²⁷, -S(O)R²⁸, -S(O)₂R²⁹, -S(O)₂NR³⁰R³¹, -S(O)(NR³²)R³³, -P(O)R³⁴R³⁵, and 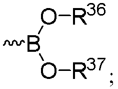
or two adjacent R^{a3}, together with the carbon atoms to which they are attached, form 5-6 membered heterocyclyl or a 5-6 membered heteroaromatic ring, wherein the heterocyclyl is optionally substituted with one or more hydroxy or methyl;
R²⁴, R²⁵, R²⁶, R²⁷, R²⁸, R²⁹, R³⁰, R³¹, R³², R³³, R³⁴, and R³⁵ are each independently selected from H and C₁₋₄ alkyl; R²¹ and R²² are each independently selected from H, C₁₋₄ alkyl, and C₁₋₄ alkyl substituted with carbonyl;
each R²³ is independently selected from C₁₋₄ alkyl, C₁₋₄ haloalkyl, and C₂₋₆ alkenyl;
R³⁶ and R³⁷ are each independently selected from H and C₁₋₄ alkyl, or R³⁶ and R³⁷, together with the boron and oxygen atoms to which they are attached, form 5-10 membered heterocyclyl (e.g., 5-6 membered heterocyclyl), wherein the heterocyclyl is optionally substituted with one or more substituents selected from H, halogen, and C₁₋₄ alkyl;
V is selected from N, N⁺-O⁻, and C-R^{a4};
R^{a4} is selected from H and methyl;
R^{a5} is selected from H and methyl;
R^{a6} is selected from H and methyl.

4. The compound, or the pharmaceutically acceptable salt, the stereoisomer, the solvate, the isotopically labeled compound, or the polymorph thereof according to any one of claims 1-3, wherein:
each R^{a3} is independently selected from H, fluorine, chlorine, bromine, hydroxy, -CN, methyl, CF₃, CHF₂, methoxy, trifluoromethoxy, ethenyl, cyclopropyl, -CH₂OH, -C(CH₃)₂OH, -CH(OH)CH₂(OH), -CH(OH)CH₂F, - CH(NH₂)CH₂(OH), -N(CH₃)-Boc, -NH(CH₃), -N(CH₃)₂, -C(O)OCH₃, -C(O)NH₂, -SH, -S(O)CH₃, -S(O)₂CH₃, - S(O)₂NH₂, -S(O)(NH)CH₃, -S(O)(NCH₃)CH₃, -P(O)(CH₃)₂, and 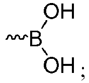
or two adjacent R^{a3}, together with the carbon atoms to which they are attached, form 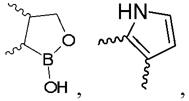 and

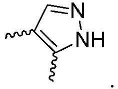

5. The compound, or the pharmaceutically acceptable salt, the stereoisomer, the solvate, the isotopically labeled compound, or the polymorph thereof according to any one of claims 1-4, wherein R^{b3} and R^{b4} are each independently selected from H, deuterium, C₁₋₄ alkyl (e.g., methyl, ethyl, propyl, and butyl), C₁₋₄ haloalkyl, and C₃₋₆ cycloalkyl.

6. The compound, or the pharmaceutically acceptable salt, the stereoisomer, the solvate, the isotopically labeled compound, or the polymorph thereof according to any one of claims 1-5, wherein R^{b5} and R^{b6} are each independently selected from H, C₁₋₄ alkyl, C₁₋₄ haloalkyl, and C₃₋₆ cycloalkyl, or R^{b5} and R^{b6}, together with the carbon atom to which they are attached, form C₃₋₅ cycloalkyl and 4-6 membered heterocyclyl (e.g., 4-6 membered oxygen-containing heterocyclyl).

7. The compound, or the pharmaceutically acceptable salt, the stereoisomer, the solvate, the isotopically labeled compound, or the polymorph thereof according to any one of claims 1-6, wherein R^{c} is selected from H, hydroxy, halogen, C₁₋₄ alkyl, C₁₋₄ deuterated alkyl, C₁₋₄ haloalkyl, C₁₋₄ alkoxy, C₁₋₄ deuterated alkoxy, C₁₋₄ haloalkoxy, C₂₋₆ alkenyl, and -O-C₃₋₆ cycloalkyl, wherein the C₁₋₄ alkyl, C₁₋₄ haloalkyl, C₁₋₄ alkoxy, C₁₋₄ haloalkoxy, and C₃₋₆ cycloalkyl are optionally substituted with one or more substituents selected from hydroxy, carboxyl, C₁₋₄ alkoxy, C₁₋₄ haloalkoxy, C₂₋₆ alkenyl, -NR⁴⁰R⁴¹, C₃₋₆ cycloalkyl, 3-6 membered heterocyclyl, and 5-6 membered heteroaryl, wherein the 3-6 membered heterocyclyl and 5-6 membered heteroaryl are optionally substituted with one or more halogen and C₁₋₄ alkyl; R⁴⁰ and R⁴¹ are each independently selected from H and C₁₋₄ alkyl.

8. The compound, or the pharmaceutically acceptable salt, the stereoisomer, the solvate, the isotopically labeled compound, or the polymorph thereof according to any one of claims 1-7, wherein:
X¹, X², X³, and X⁴ are each independently selected from N and C-R^{c1};
each R^{c1} is independently selected from H, halogen, C₁₋₄ alkyl, C₁₋₄ haloalkyl, C₁₋₄ alkoxy, and C₁₋₄ haloalkoxy.

9. The compound, or the pharmaceutically acceptable salt, the stereoisomer, the solvate, the isotopically labeled compound, or the polymorph thereof according to any one of claims 1-8, wherein R^{a} is selected from:

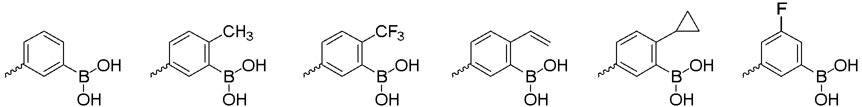

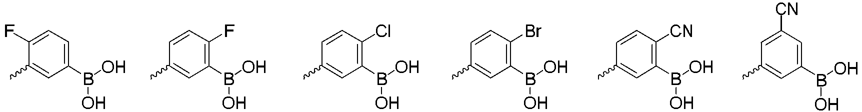

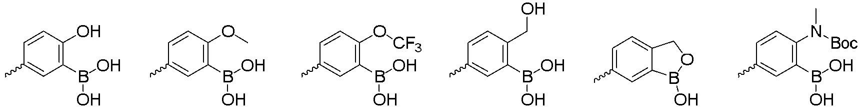

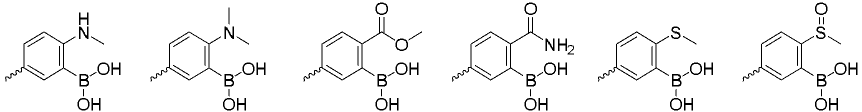

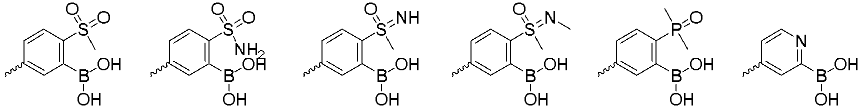

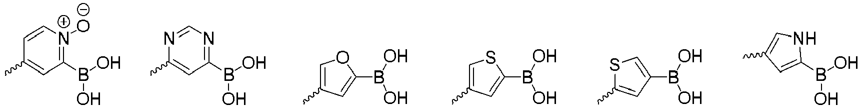

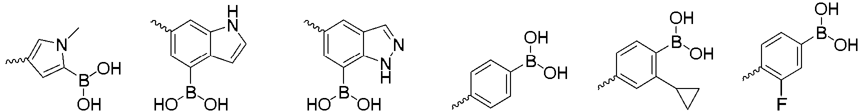

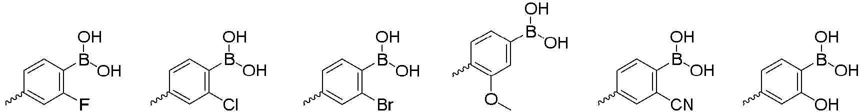

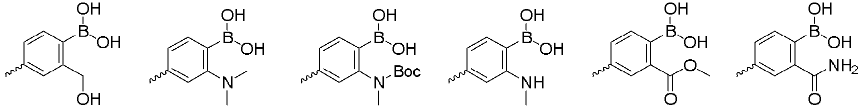

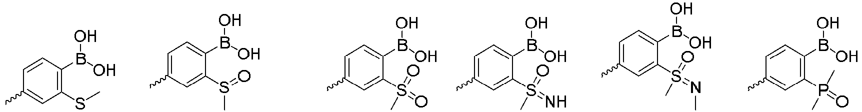

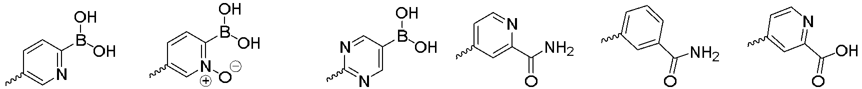

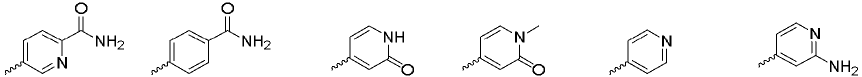

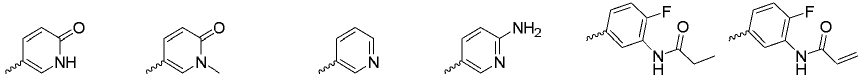

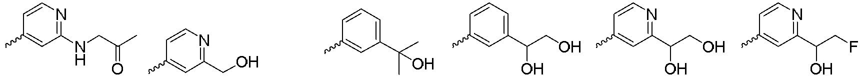

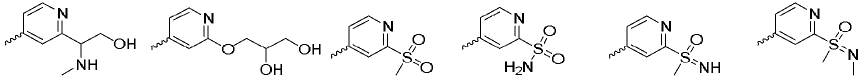

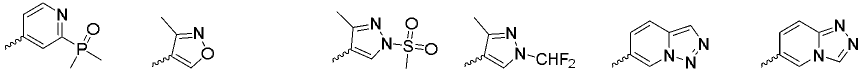

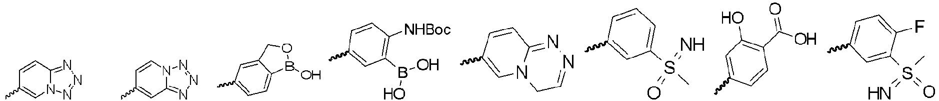

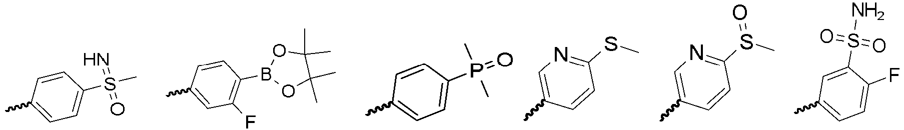

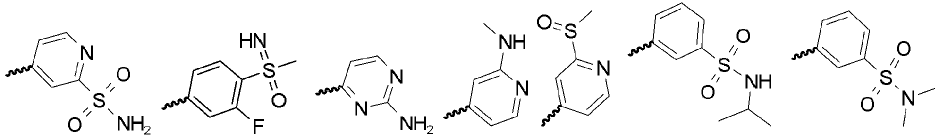

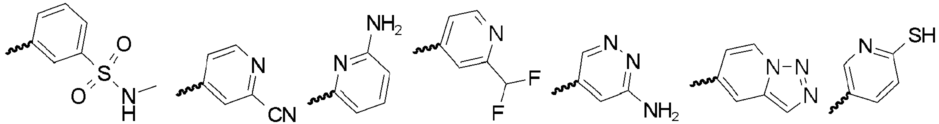

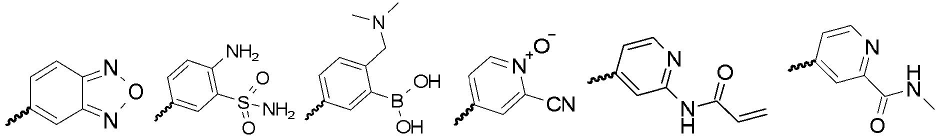

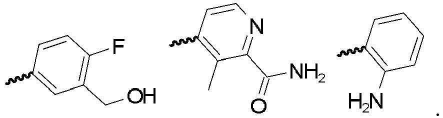

10. The compound, or the pharmaceutically acceptable salt, the stereoisomer, the solvate, the isotopically labeled compound, or the polymorph thereof according to any one of claims 1-9, wherein R^{c} is selected from: H, -OCH₃, - OCD₃, -OCH₂CH₃, -OCH(CH₃)₂, -OCH₂CF₃, -OCH₂CF₂CH₃, -OCH₂CHF₂, 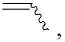 -OCHF₂, 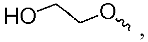 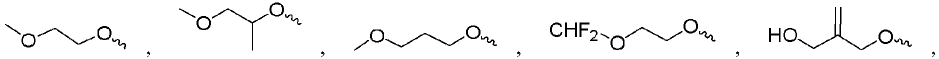 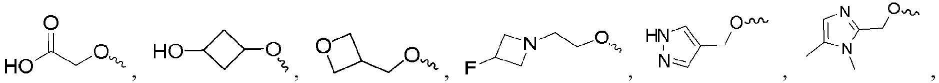 and

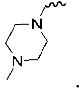

11. The compound, or the pharmaceutically acceptable salt, the stereoisomer, the solvate, the isotopically labeled compound, or the polymorph thereof according to any one of claims 1-10, wherein the compound is selected from:

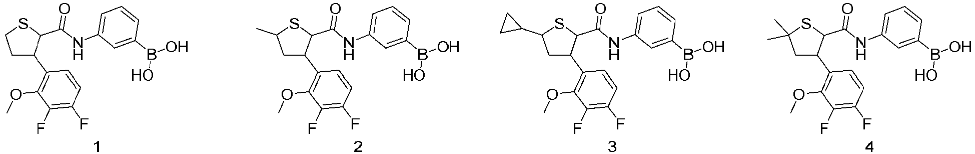

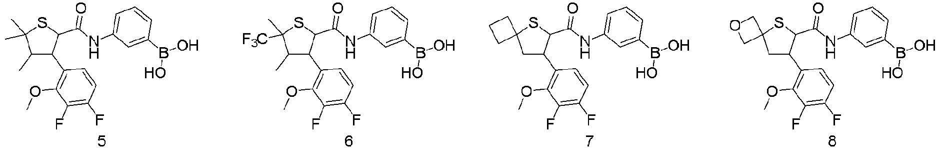

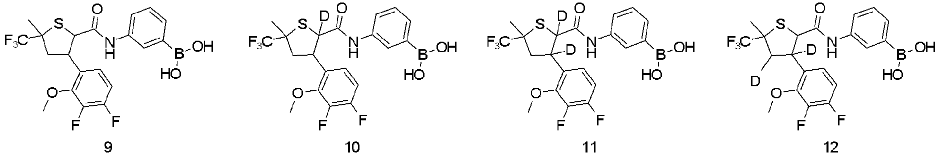

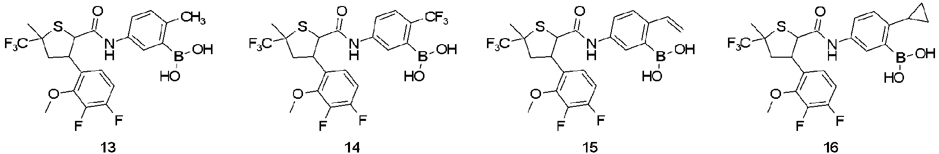

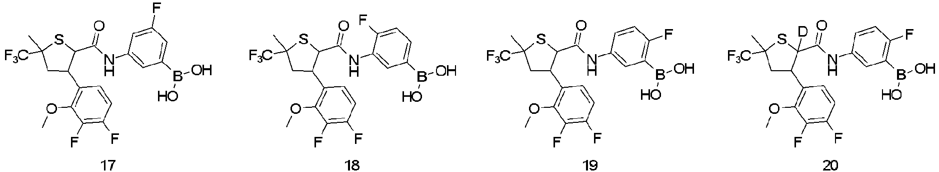

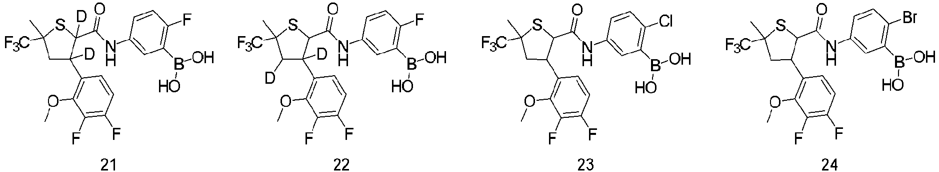

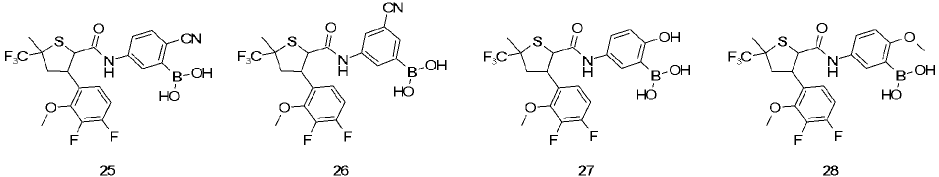

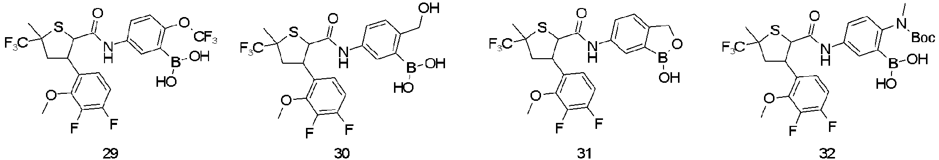

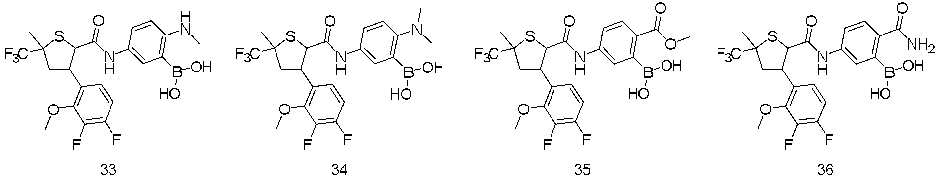

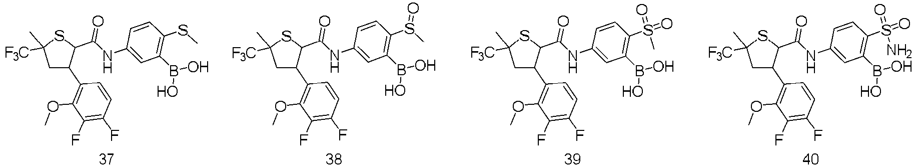

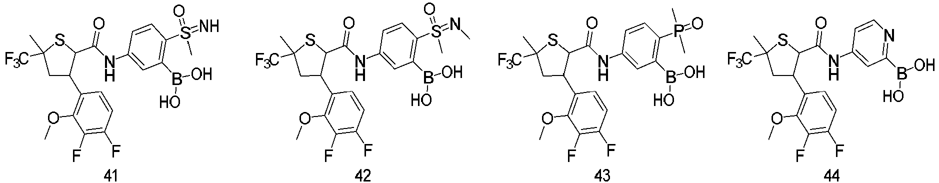

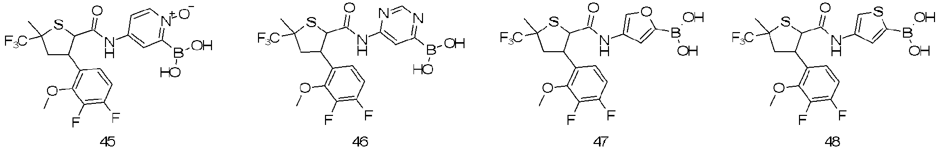

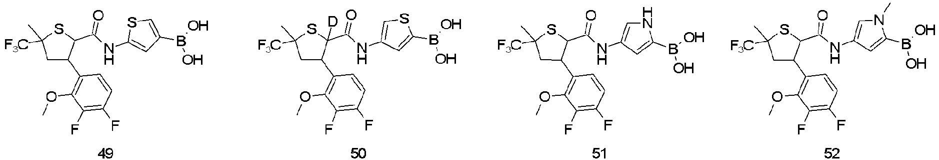

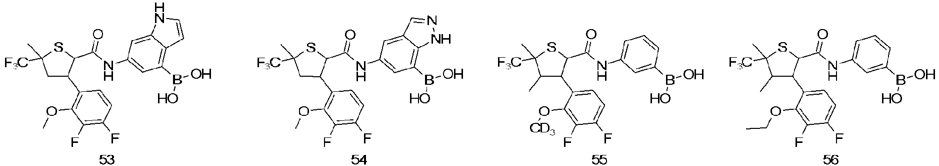

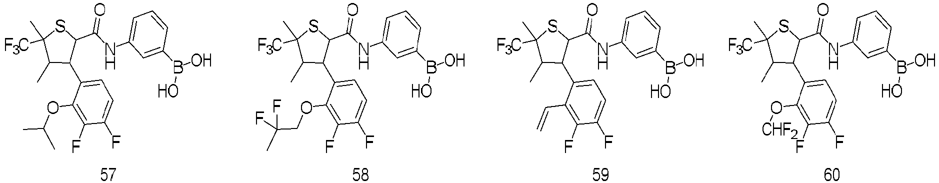

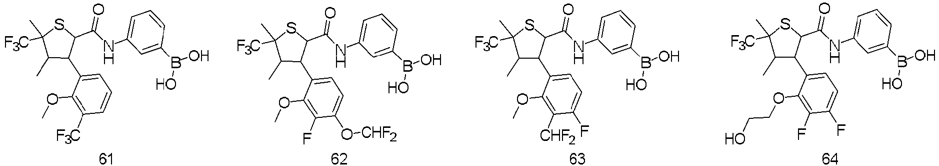

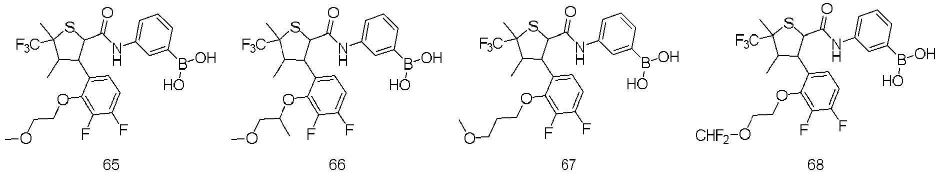

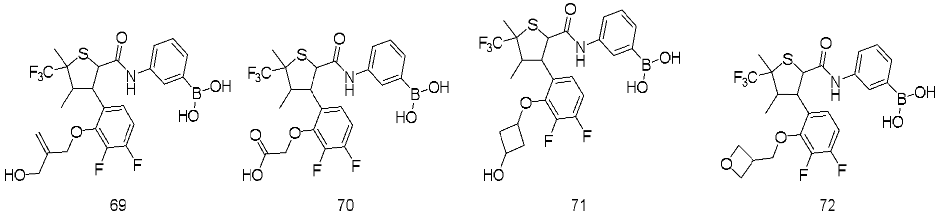

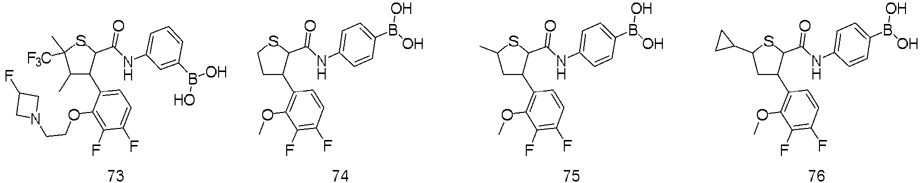

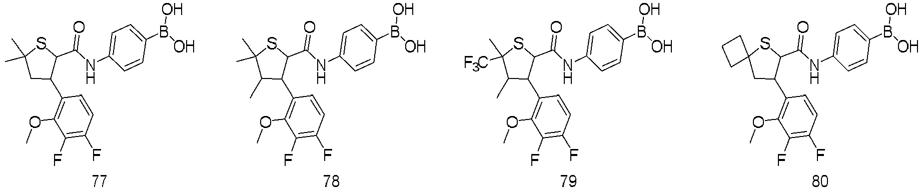

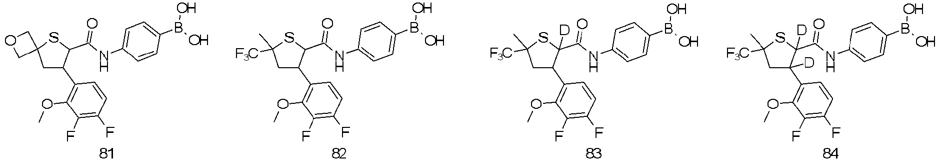

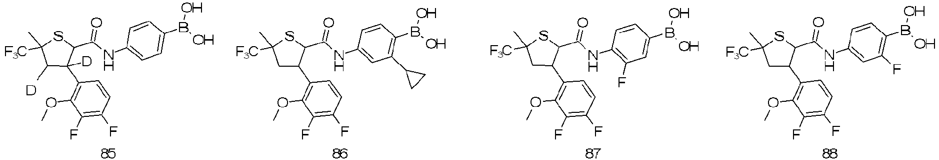

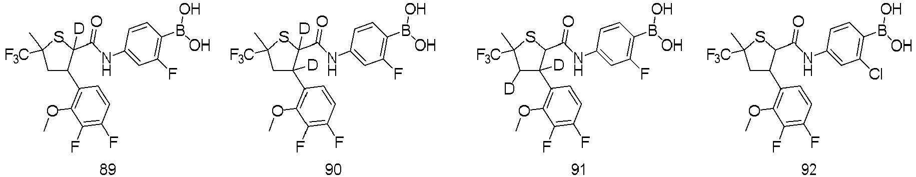

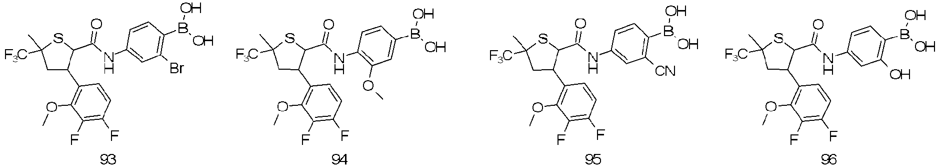

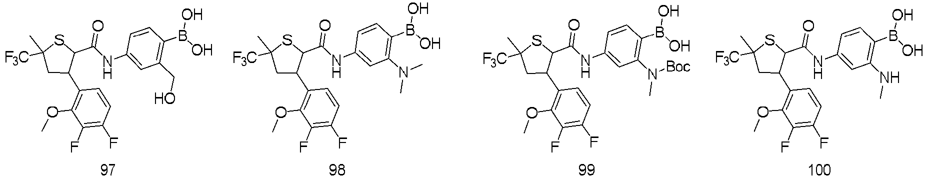

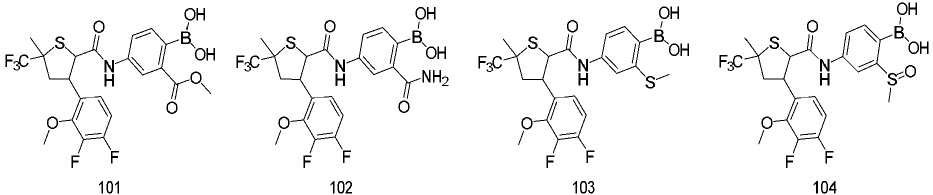

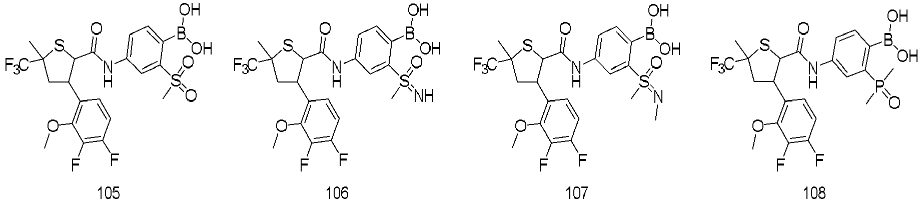

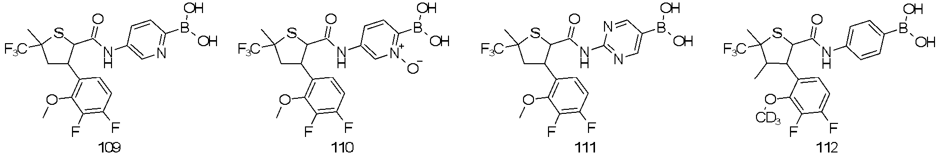

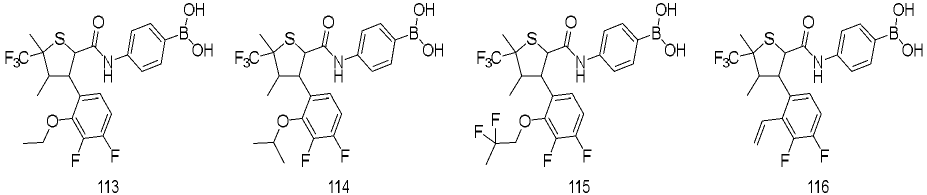

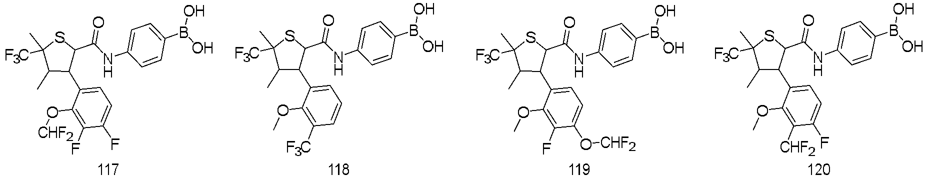

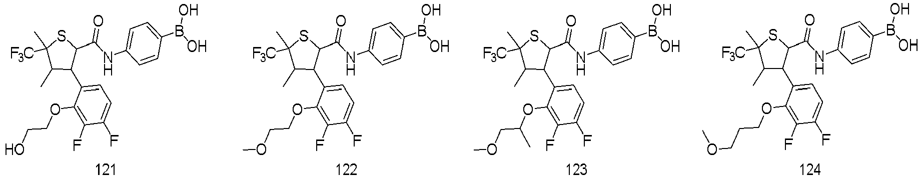

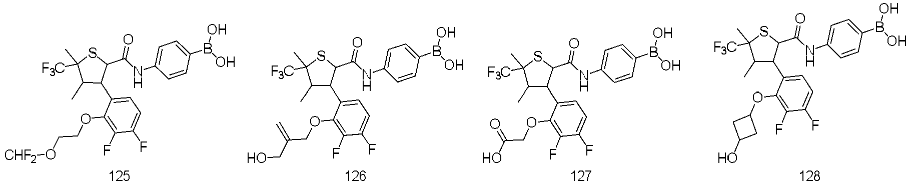

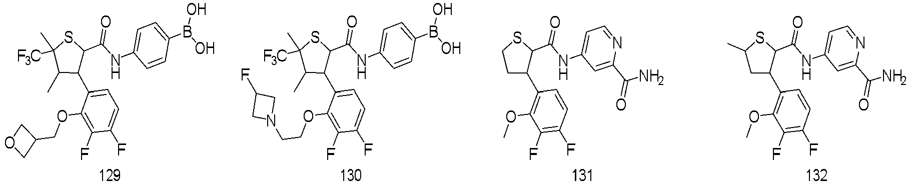

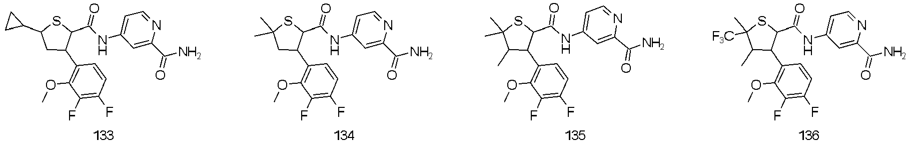

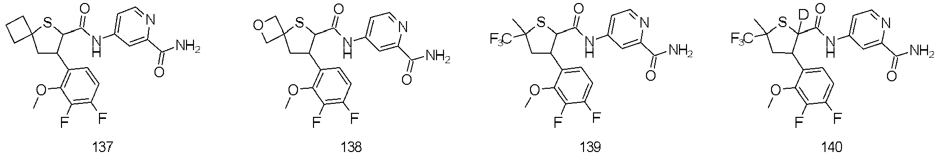

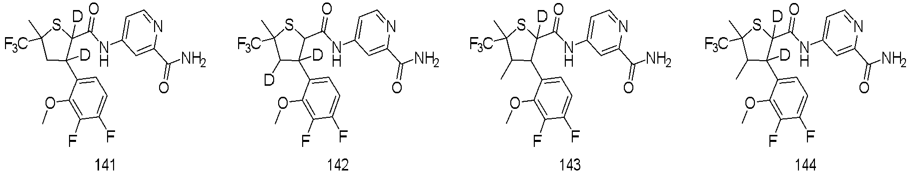

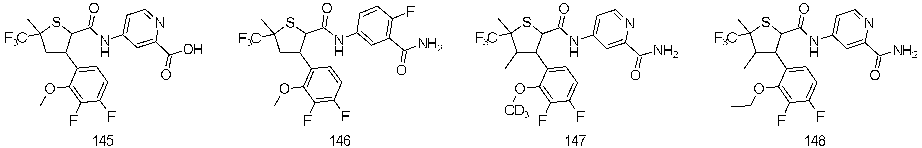

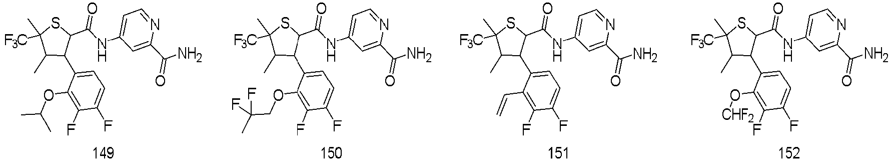

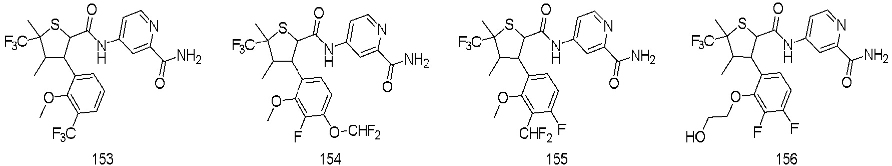

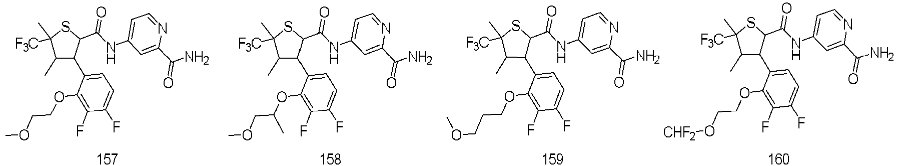

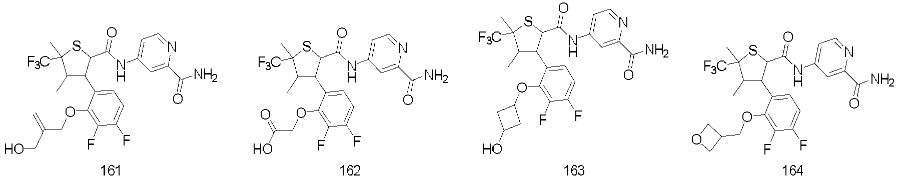

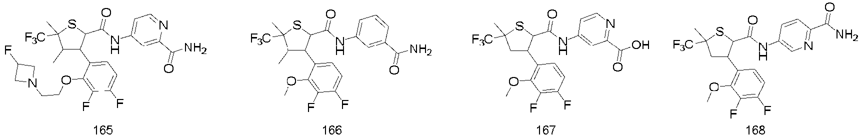

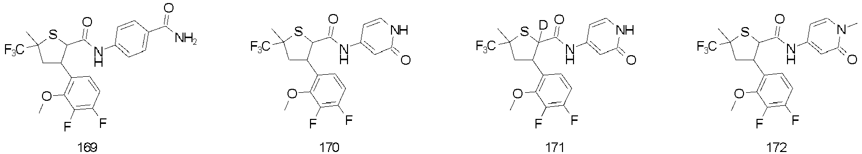

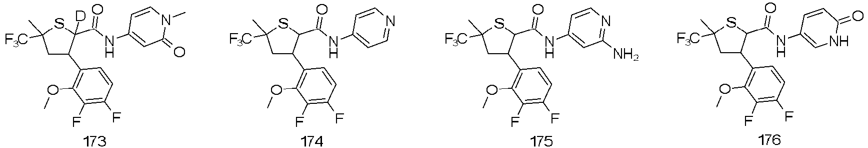

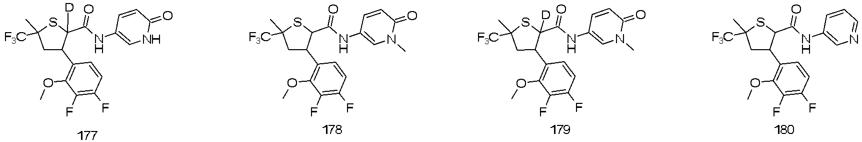

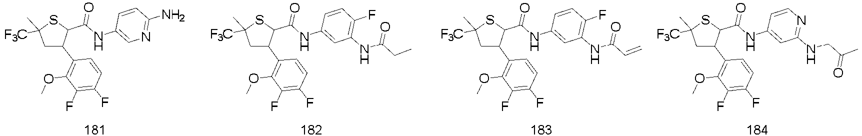

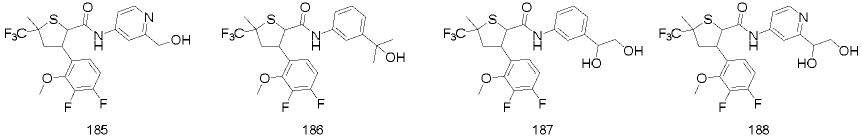

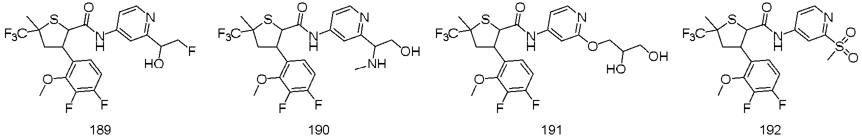

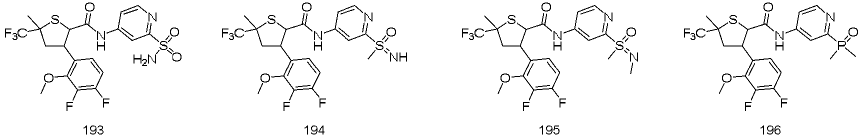

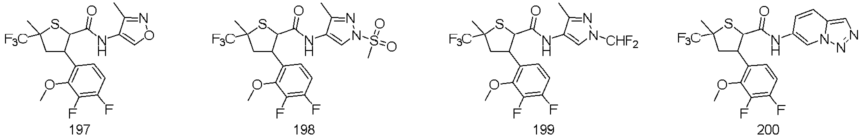

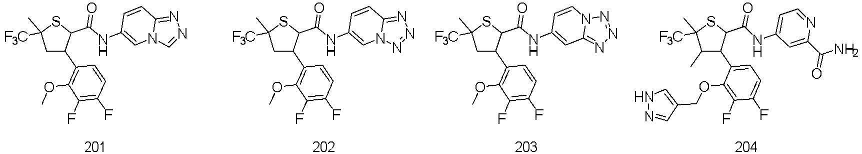

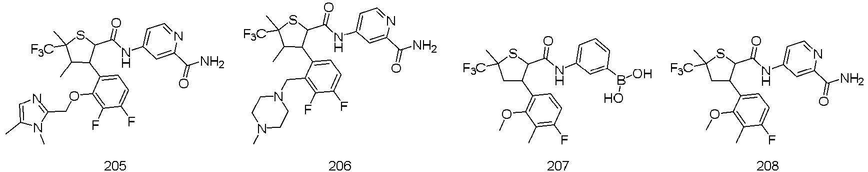

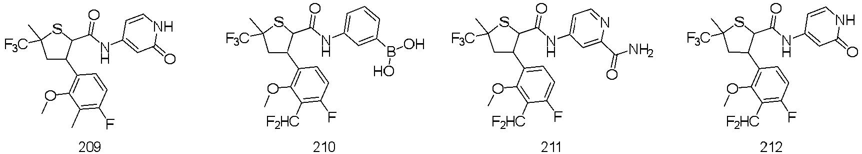

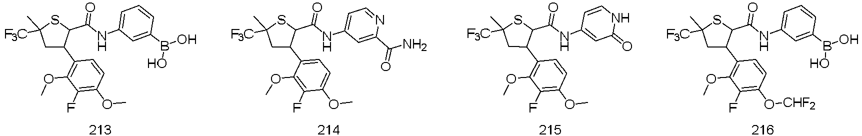

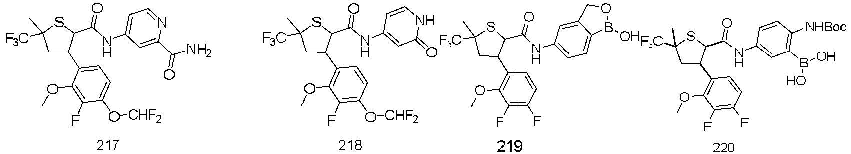

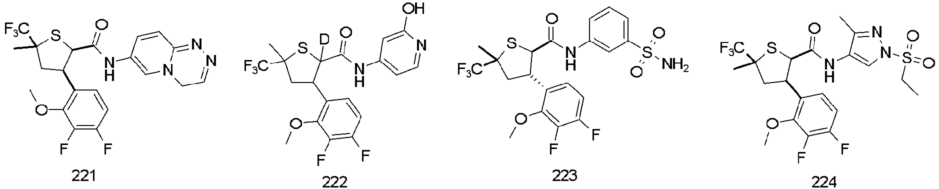

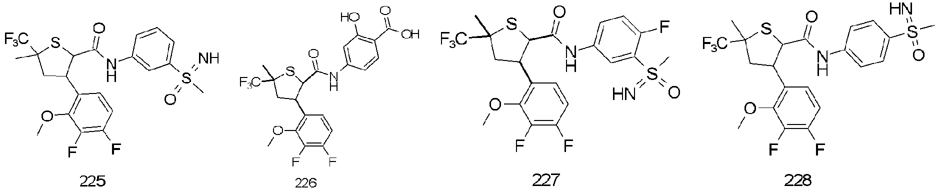

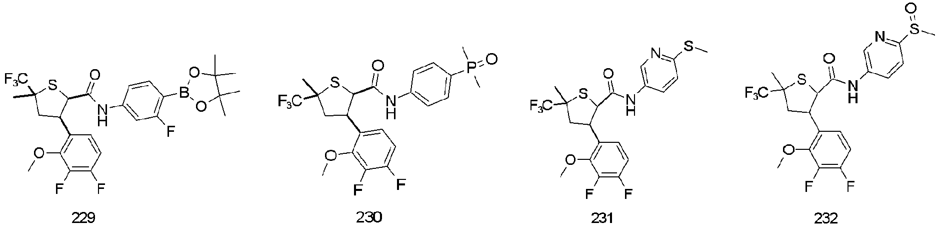

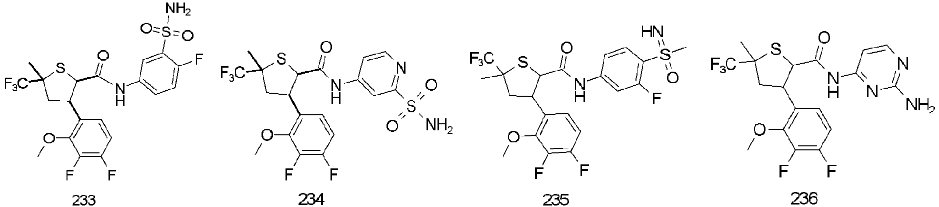

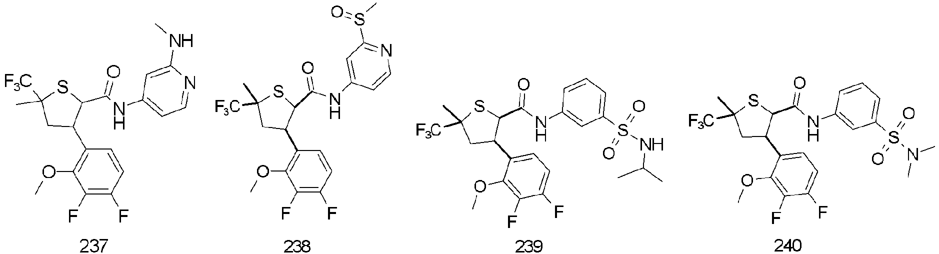

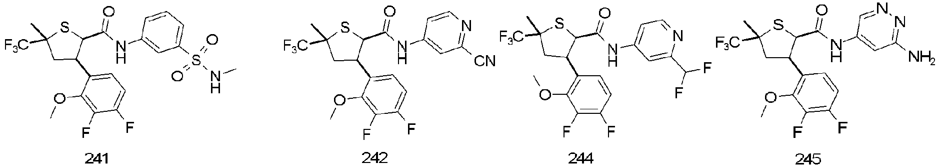

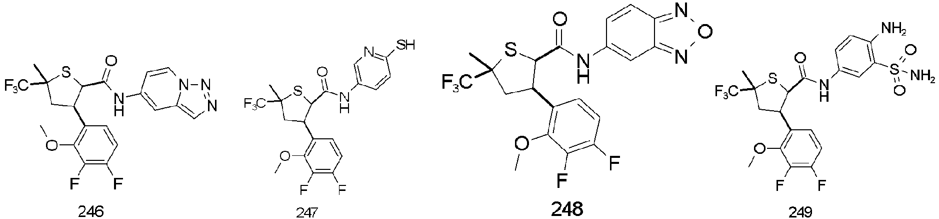

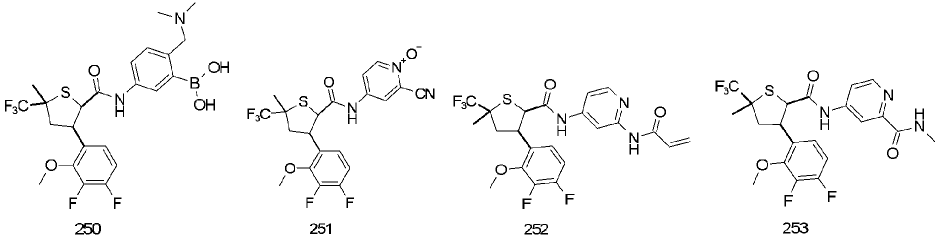

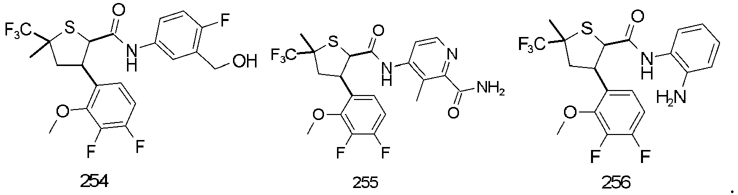

12. The compound, or the pharmaceutically acceptable salt, the stereoisomer, the solvate, the isotopically labeled compound, or the polymorph thereof according to any one of claims 1-11, wherein the compound is selected from:

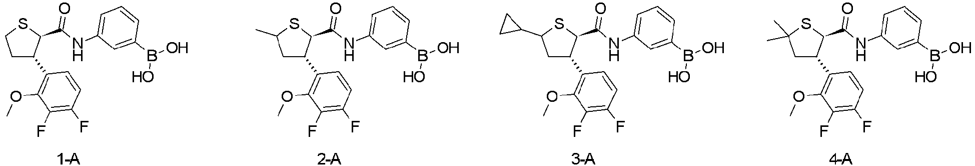

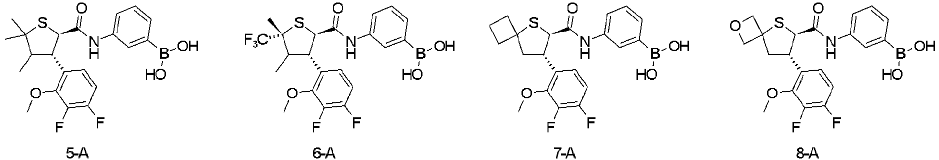

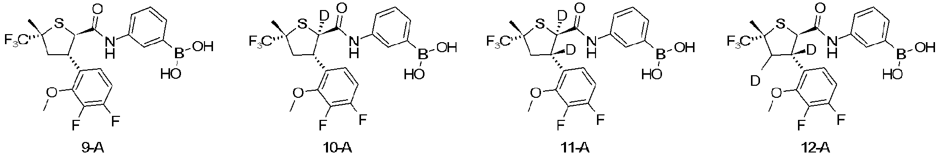

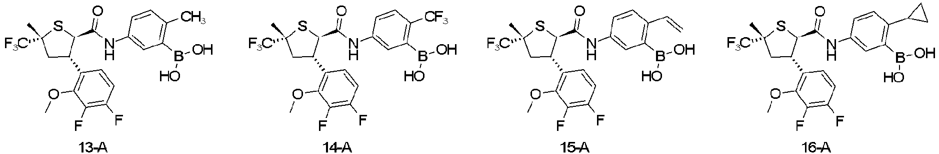

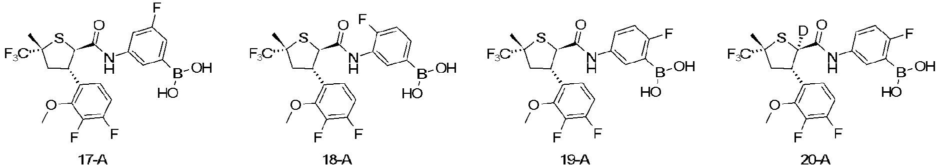

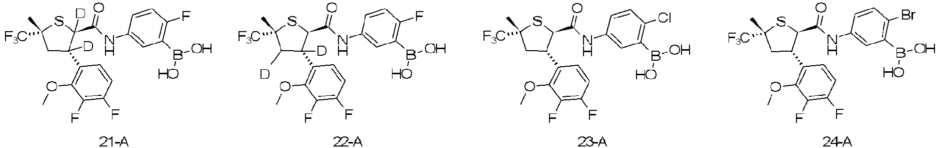

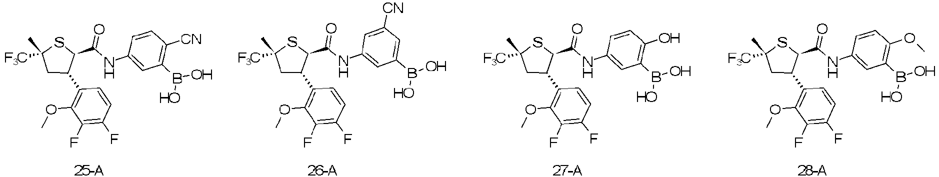

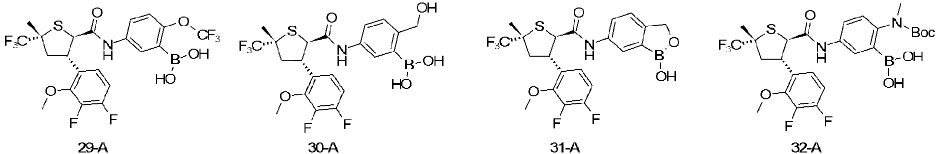

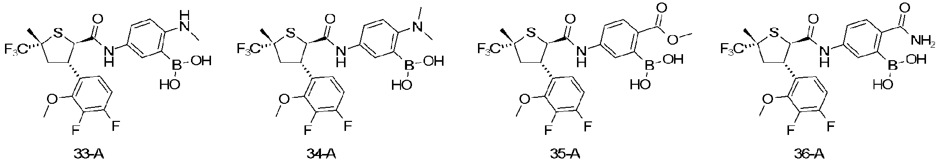

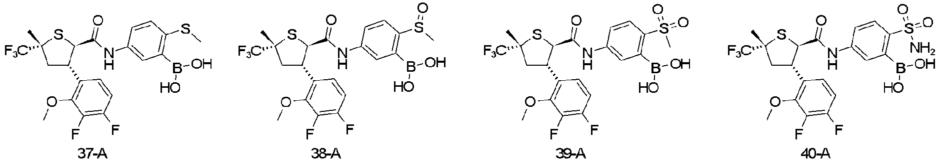

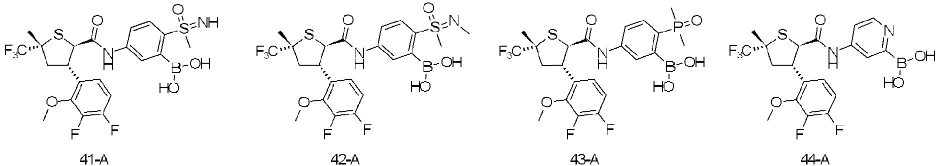

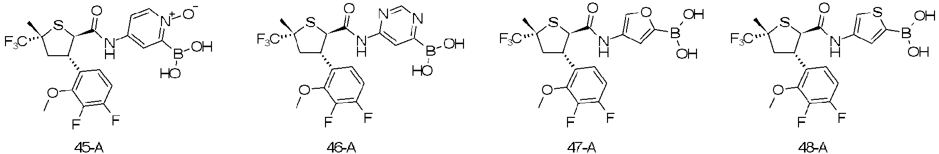

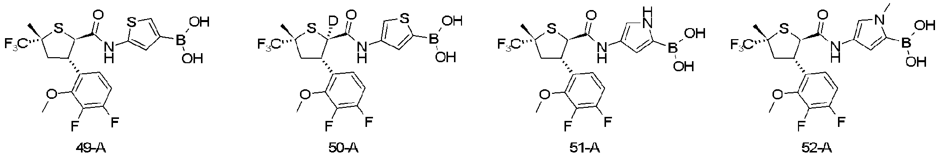

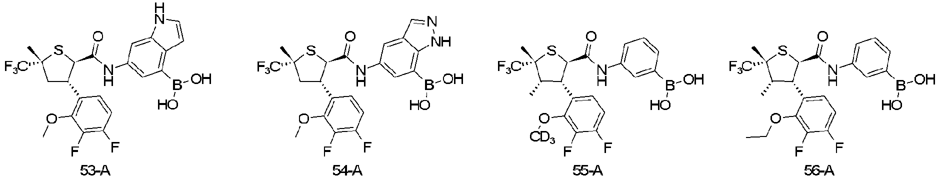

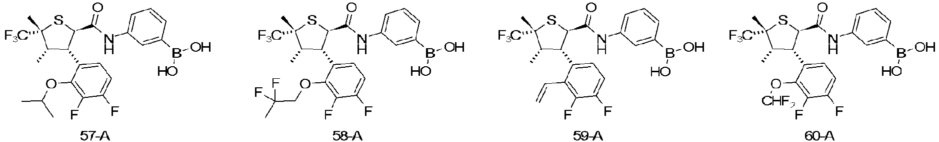

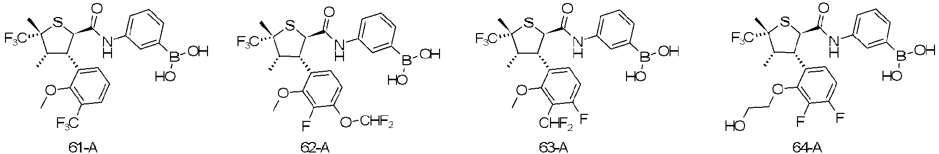

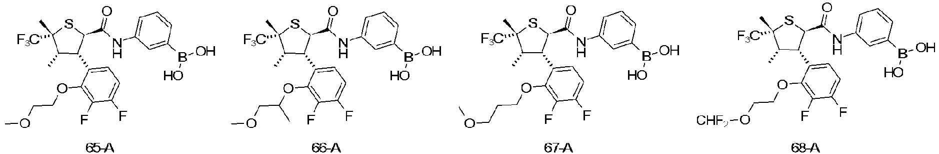

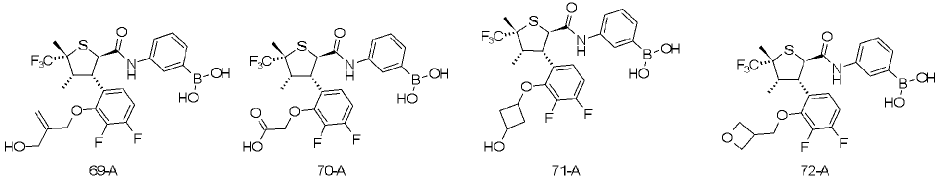

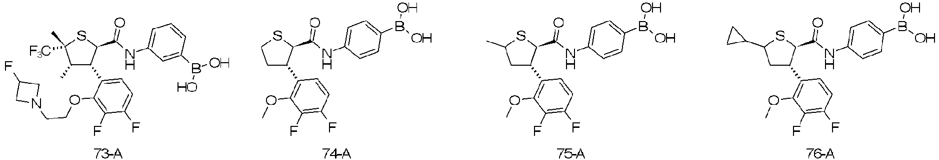

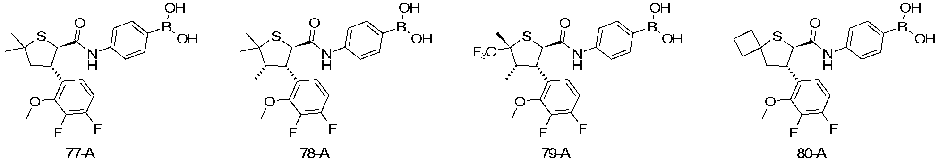

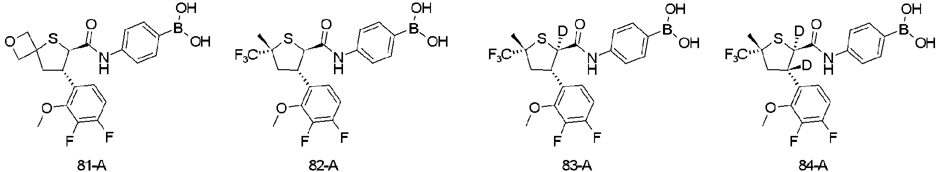

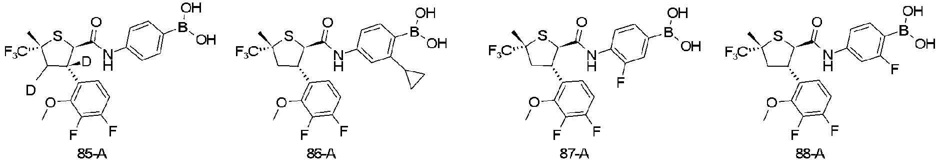

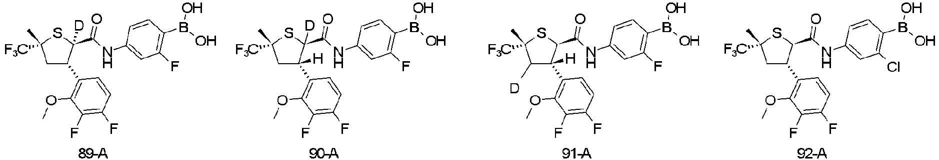

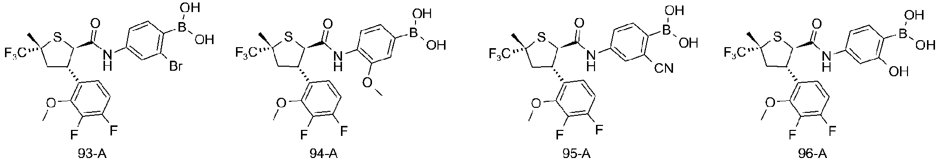

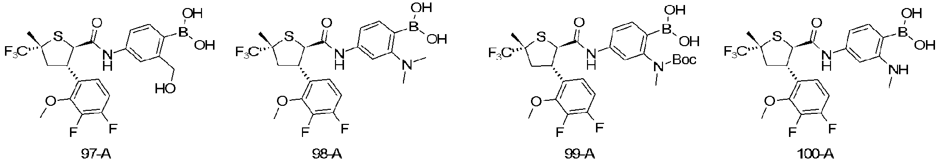

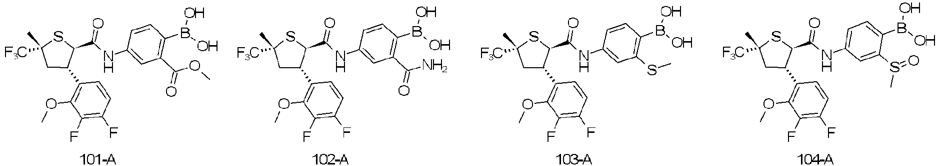

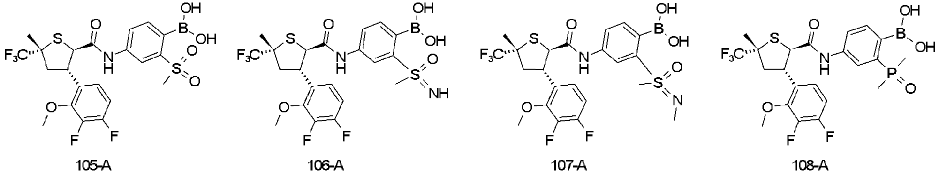

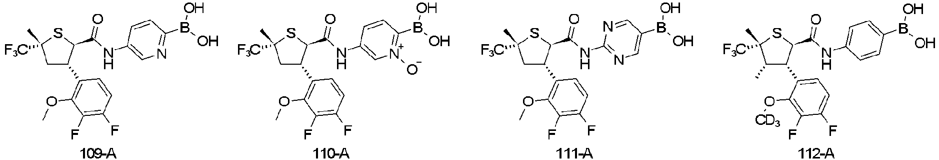

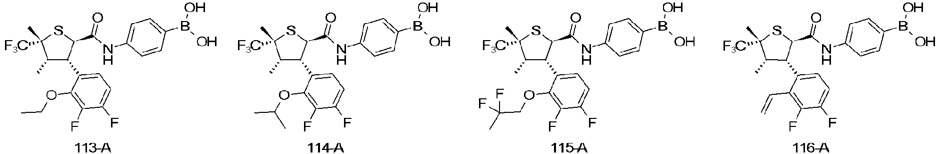

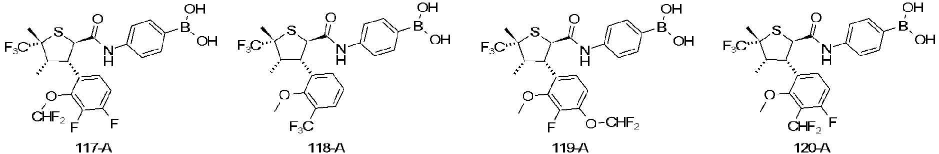

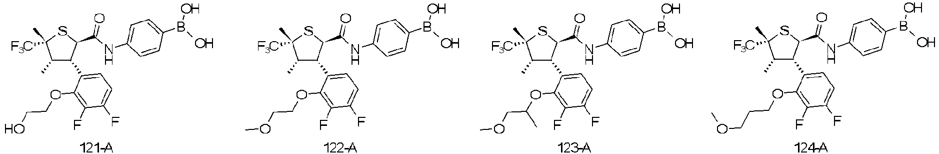

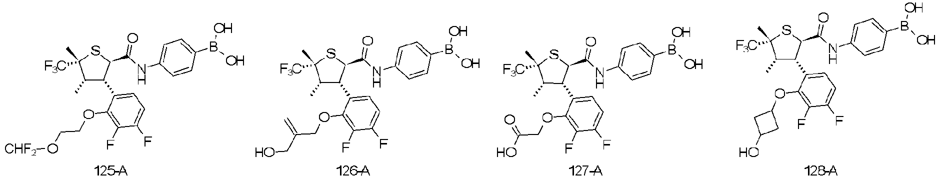

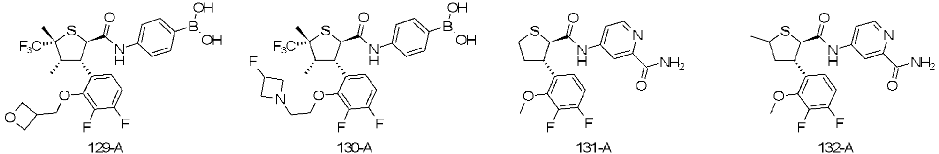

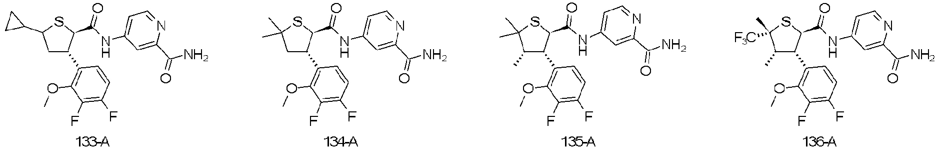

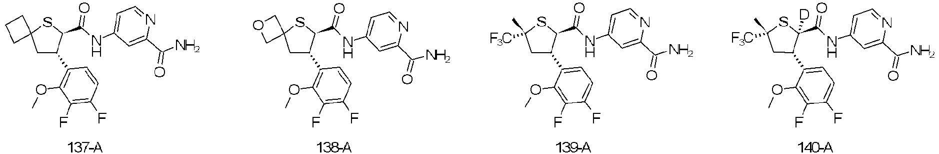

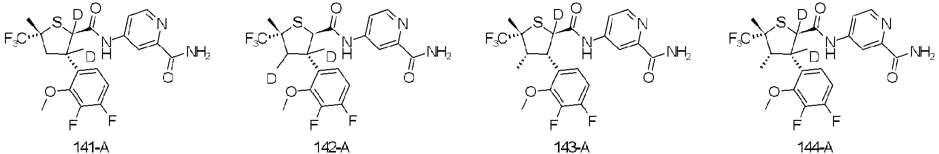

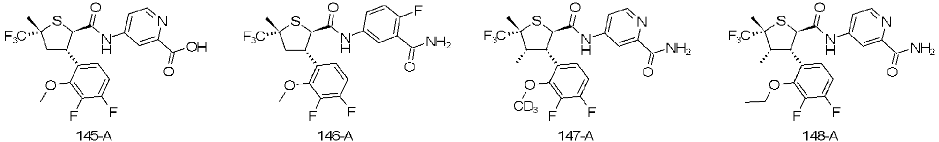

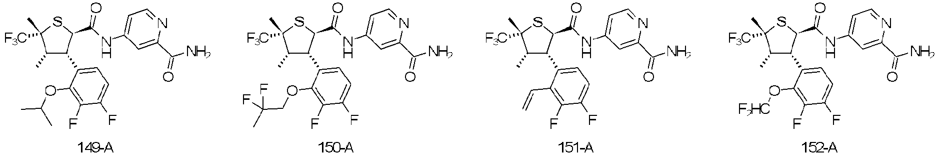

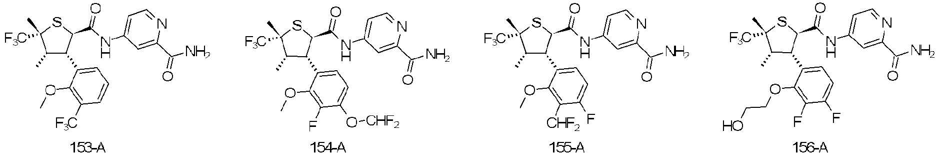

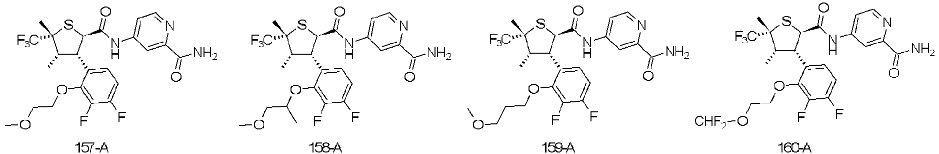

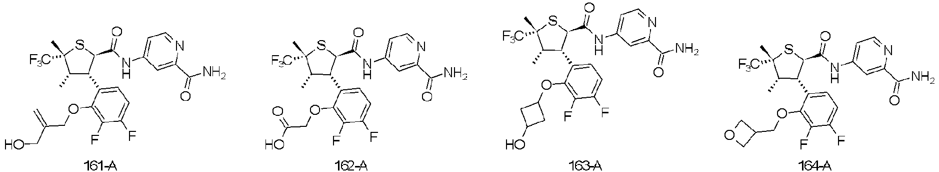

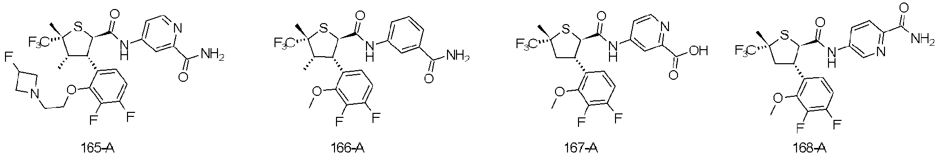

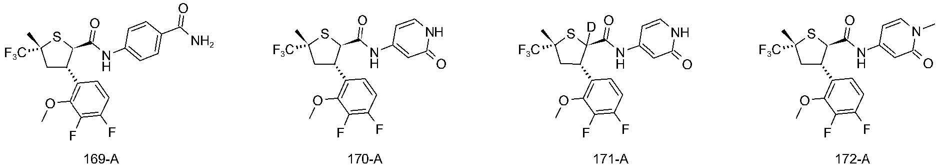

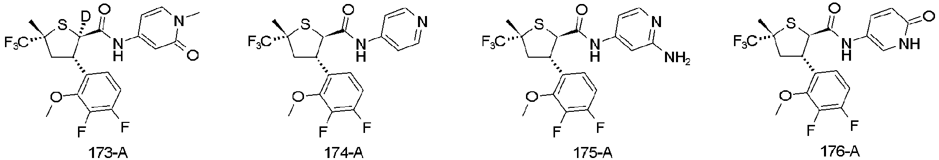

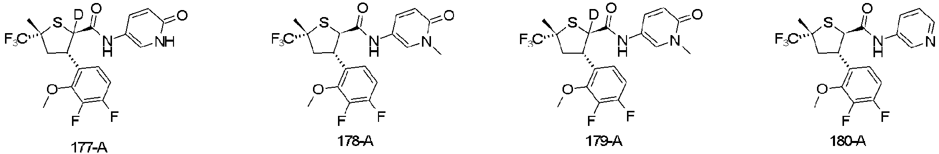

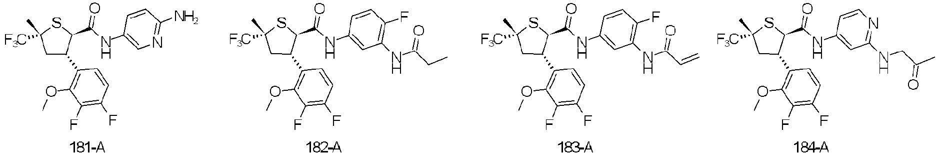

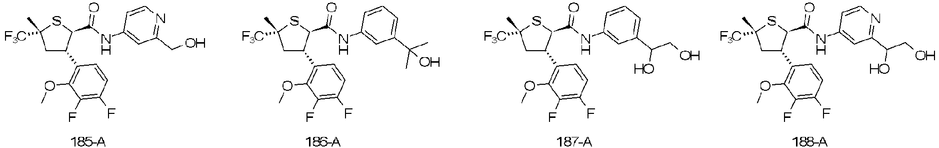

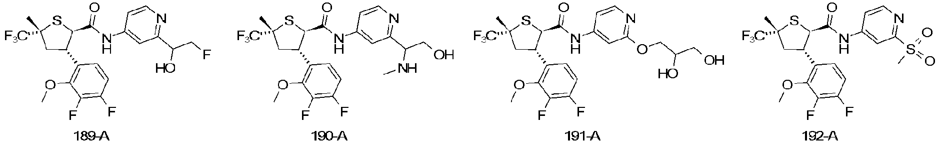

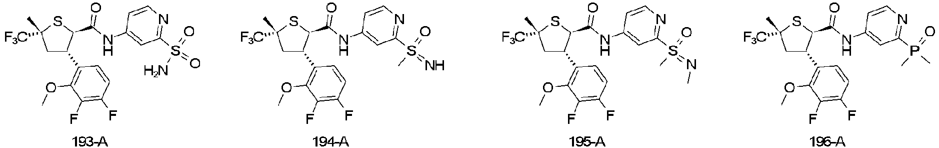

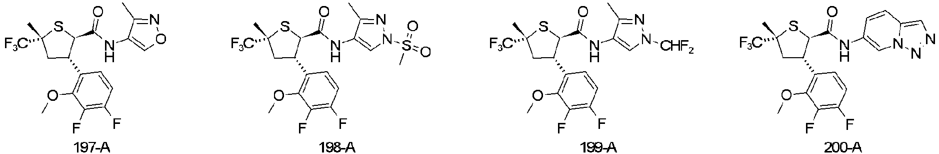

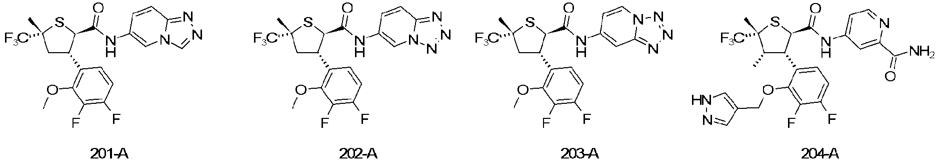

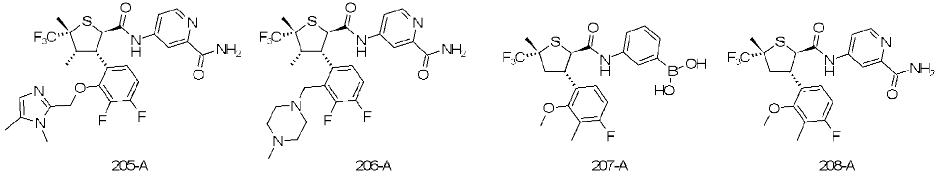

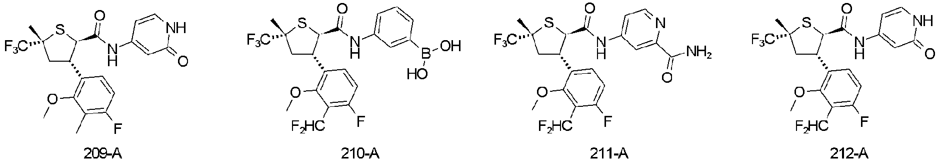

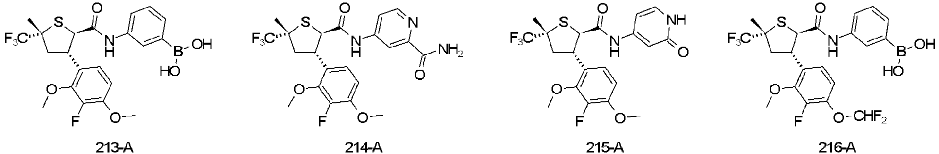

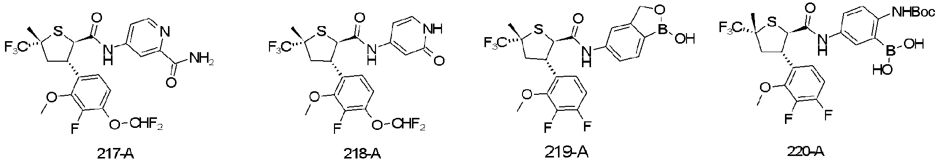

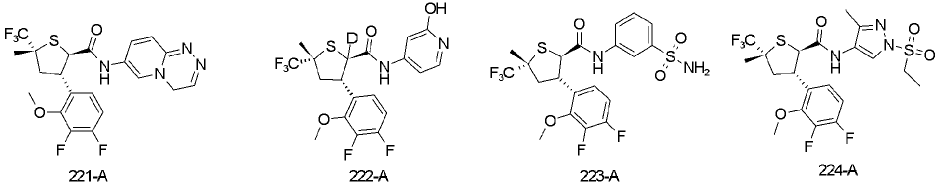

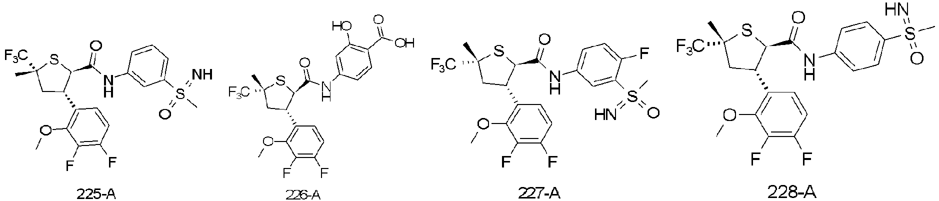

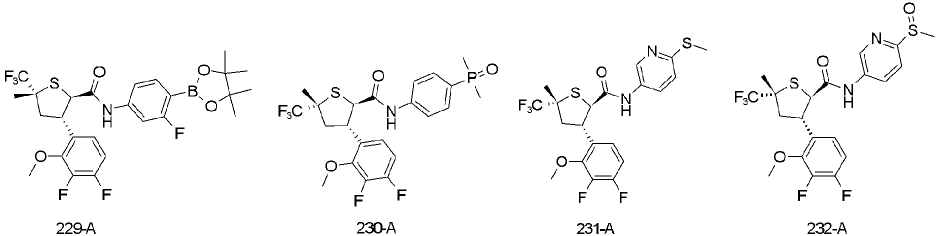

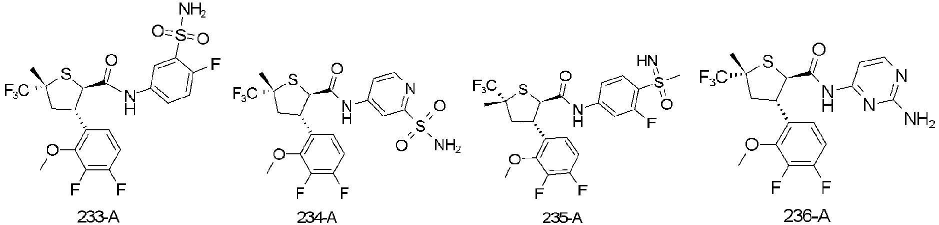

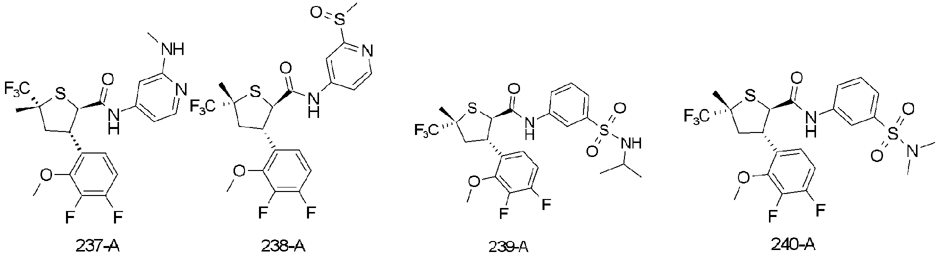

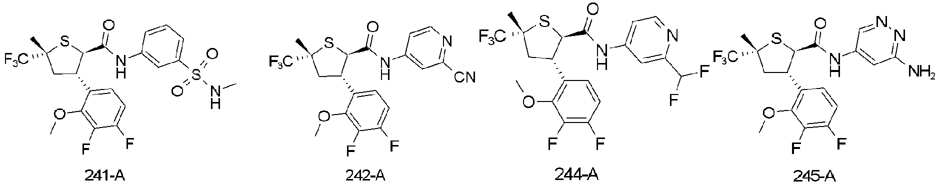

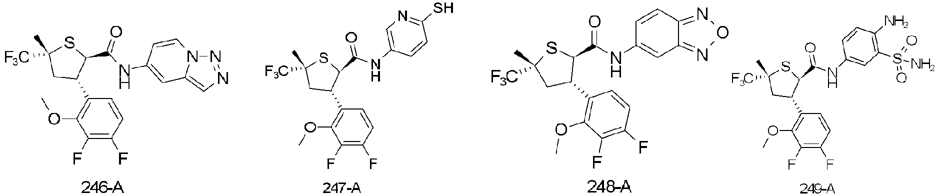

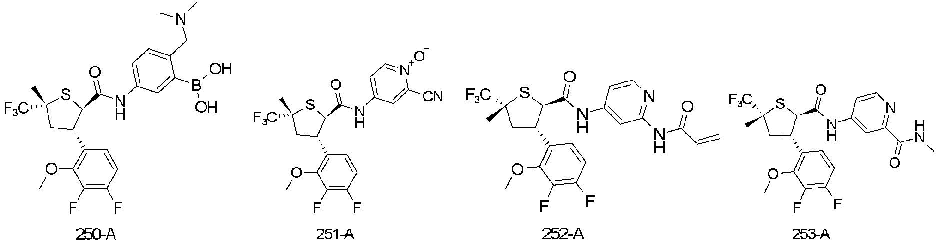

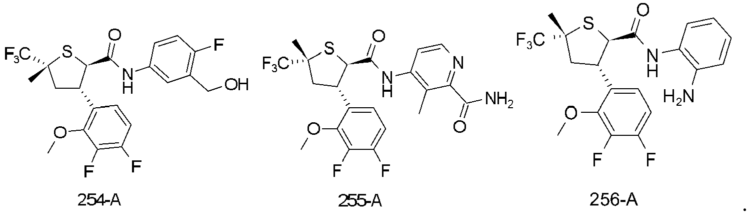

13. A compound of formula II, or a pharmaceutically acceptable salt, a stereoisomer, a solvate, an isotopically labeled compound, or a polymorph thereof, wherein the compound has the following structure: 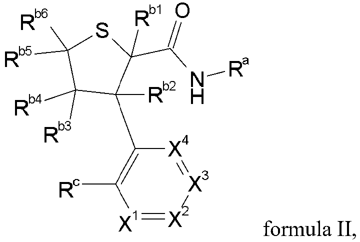
wherein, R^{a} is selected from 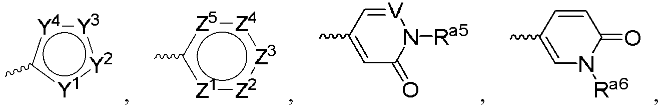 and 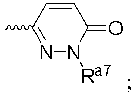
Y¹, Y², Y³, and Y⁴ are each independently selected from O, S, N, N-R^{a1}, and C-R^{a2};
each R^{a1} is independently selected from -S(O)₂-R⁴²;
each R^{a2} is independently selected from H and C₁₋₆ alkyl;
each R⁴² is independently selected from C₁₋₆ haloalkyl and C₃₋₆ cycloalkyl;
Z¹, Z², Z³, Z⁴, and Z⁵ are each independently selected from N, N⁺-O⁻, and C-R^{a3};
each R^{a3} is independently selected from H, halogen, -N₃, -NO₂, -SCN, -S(F)₅, -CH(O), C₂₋₆ alkenyl, C₂₋₆ alkynyl, C₃₋₆ cycloalkyl, 3-6 membered heterocyclyl, 5-6 membered heteroaryl, -C(O)NR⁴³R⁴⁴, -C(O)NR⁴⁵S(O)₂NR⁴⁶R⁴⁷, - C(S)NR⁴⁸R⁴⁹, -C(=NR⁵⁰)NR⁵¹R⁵², -C(=NR⁵³)R⁵⁴, -NR⁵⁵R⁵⁶, -NR⁵⁷S(O)₂R⁵⁸, -NR⁵⁹S(O)₂NR⁶⁰R⁶¹, -N=S(O)R⁶²R⁶³, - OS(O)₂R⁶⁴, -S(O)₂R⁶⁵, -S(O)₂NR⁶⁶R⁶⁷, -Si(OR⁶⁸)₃, and 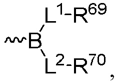 wherein the C₂₋₆ alkenyl, C₂₋₆ alkynyl, C₃₋₆ cycloalkyl, 3-6 membered heterocyclyl, and 5-6 membered heteroaryl are optionally substituted with one or more substituents selected from halogen, hydroxy, -CN, amino, C₁₋₆ alkyl, C₁₋₆ haloalkyl, C₁₋₆ alkoxy, C₁₋₆ haloalkoxy, - C(O)OR⁷¹, and -C(O)NR⁷²R⁷³;
or two adjacent R^{a3}, together with the carbon atoms to which they are attached, form 5-6 membered heterocyclyl or a 5-10 membered heteroaromatic ring, wherein the 5-6 membered heterocyclyl or 5-10 membered heteroaromatic ring is optionally substituted with one or more substituents selected from halogen, hydroxy, amino, -CN, =O, C₁₋₆ alkyl, and -C₁₋₆ alkyl NR⁷⁴R⁷⁵;
R⁴³, R⁴⁴, R⁵⁰, and R⁵³ are each independently selected from H, hydroxy, C₁₋₆ alkyl, and C₁₋₆ alkoxy;
R⁴⁵, R⁴⁶, R⁴⁷, R⁴⁸, R⁴⁹, R⁵¹, R⁵², R⁵⁴, R⁵⁷, R⁵⁹, R⁶², R⁶³, R⁶⁸, R⁷¹, R⁷², R⁷³, R⁷⁴, and R⁷⁵ are each independently selected from H, C₁₋₆ alkyl, and C₁₋₆ haloalkyl;
R⁵⁵, R⁵⁶, R⁶⁶, and R⁶⁷ are each independently selected from H, hydroxy, -CN, C₁₋₆ alkyl, -C(O)C₁₋₆ alkyl, -C(O)C₂₋₆ alkenyl, -C(O)C₂₋₆ alkynyl, C₃₋₆ membered cycloalkyl, and 3-6 membered heterocyclyl, wherein the C₁₋₆ alkyl, C₂₋₆ alkenyl, C₂₋₆ alkynyl, C₃₋₆ membered cycloalkyl, and 3-6 membered heterocyclyl are optionally substituted with halogen, hydroxy, amino, -CN, N₃, C₁₋₆ alkyl, C₁₋₆ alkoxy, and C₁₋₆ alkylamino;
R⁵⁸ is independently selected from halogen and C₁₋₆ alkyl, wherein the C₁₋₆ alkyl is optionally substituted with one or more substituents selected from halogen, hydroxy, amino, C₁₋₆ alkoxy, C₁₋₆ alkylamino, and N₃;
R⁶⁰ and R⁶¹ are each independently selected from C₁₋₆ alkyl, wherein the C₁₋₆ alkyl is optionally substituted with one or more substituents selected from halogen, hydroxy, amino, C₁₋₆ alkoxy, C₁₋₆ alkylamino, and N₃;
R⁶⁴ is independently selected from halogen, amino, and C₁₋₆ alkylamino;
R⁶⁵ is independently selected from halogen, N₃, C₁₋₆ alkyl, and C₁₋₆ haloalkyl;
R⁶⁹ and R⁷⁰ are each independently selected from H, halogen, -CN, C₁₋₆ alkyl, C₁₋₆ haloalkyl, -C(O)OC₁₋₆ alkyl, - C(O)NH₂, and -C(O)NHC₁₋₆ alkyl, wherein the C₁₋₆ alkyl and C₁₋₆ haloalkyl are optionally substituted with hydroxy and amino; or R⁶⁹ and R⁷⁰, together with the boron and oxygen atoms to which they are attached, form 5-10 membered heterocyclyl (e.g., 5-6 membered heterocyclyl), wherein the heterocyclyl is optionally substituted with one or more substituents selected from hydrogen, halogen, hydroxy, C₁₋₄ alkyl, and =O;
L¹ and L² are each independently selected from O and N;
V is selected from N, N⁺-O⁻, and C-R^{a4};
R^{a4} is selected from H and C₁₋₆ alkyl;
R^{a5} is selected from H and C₁₋₆ alkyl;
R^{a6} is selected from H and C₁₋₆ alkyl;
R^{a7} is selected from H and C₁₋₆ alkyl;
R^{b1} and R^{b2} are each independently selected from H and deuterium;
R^{b3} and R^{b4} are each independently selected from H, deuterium, C₁₋₆ alkyl, C₁₋₆ haloalkyl, and C₃₋₆ cycloalkyl;
R^{b5} and R^{b6} are each independently selected from H, C₁₋₆ alkyl, C₁₋₆ haloalkyl, and C₃₋₆ cycloalkyl, or R^{b5} and R^{b6}, together with the carbon atom to which they are attached, form C₃₋₅ cycloalkyl and 4-6 membered heterocyclyl;
R^{c} is selected from H, hydroxy, halogen, C₁₋₆ alkyl, C₁₋₆ deuterated alkyl, C₁₋₆ haloalkyl, C₁₋₆ alkoxy, C₁₋₆ deuterated alkoxy, C₁₋₆ haloalkoxy, C₂₋₆ alkenyl, and -O-C₃₋₆ cycloalkyl, wherein the C₁₋₆ alkyl, C₁₋₆ haloalkyl, C₁₋₆ alkoxy, C₁₋₆ haloalkoxy, and C₃₋₆ cycloalkyl are optionally substituted with one or more substituents selected from hydroxy, carboxyl, C₁₋₆ alkoxy, C₁₋₆ haloalkoxy, C₂₋₆ alkenyl, -NR⁴⁰R⁴¹, C₃₋₆ cycloalkyl, 3-6 membered heterocyclyl, and 5-6 membered heteroaryl, wherein the 3-6 membered heterocyclyl and 5-6 membered heteroaryl are optionally substituted with one or more halogen and C₁₋₆ alkyl;
R⁴⁰ and R⁴¹ are each independently selected from H and C₁₋₆ alkyl;
X¹, X², X³, and X⁴ are each independently selected from N and C-R^{c1};
each R^{c1} is independently selected from H, halogen, C₁₋₆ alkyl, C₁₋₆ haloalkyl, C₁₋₆ alkoxy, and C₁₋₆ haloalkoxy.

14. The compound, or the pharmaceutically acceptable salt, the stereoisomer, the solvate, the isotopically labeled compound, or the polymorph thereof according to claim 13, wherein R^{a} is selected from 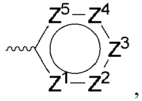 wherein Z¹, Z², Z³, Z⁴, and Z⁵ are each independently selected from N, N⁺-O⁻, and C-R^{a3}; each R^{a3} is independently selected from H, halogen, -N₃, -NO₂, -SCN, -S(F)₅, -CH(O), C₂₋₆ alkenyl, C₂₋₆ alkynyl, C₃₋₆ cycloalkyl, 3-6 membered heterocyclyl, 5-6 membered heteroaryl, -C(O)NR⁴³R⁴⁴, -C(O)NR⁴⁵S(O)₂NR⁴⁶R⁴⁷, -C(S)NR⁴⁸R⁴⁹, -C(=NR⁵⁰)NR⁵¹R⁵², - C(=NR⁵³)R⁵⁴, -NR⁵⁵R⁵⁶, -NR⁵⁷S(O)₂R⁵⁸, -NR⁵⁹S(O)₂NR⁶⁰R⁶¹, -N=S(O)R⁶²R⁶³, -OS(O)₂R⁶⁴, -S(O)₂R⁶⁵, - S(O)₂NR⁶⁶R⁶⁷, -Si(OR⁶⁸)₃, and 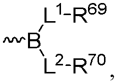 wherein the C₂₋₆ alkenyl, C₂₋₆ alkynyl, C₃₋₆ cycloalkyl, 3-6 membered heterocyclyl, and 5-6 membered heteroaryl are optionally substituted with one or more substituents selected from halogen, hydroxy, -CN, amino, C₁₋₆ alkyl, C₁₋₆ haloalkyl, C₁₋₆ alkoxy, C₁₋₆ haloalkoxy, -C(O)OR⁷¹, and -C(O)NR⁷²R⁷³; or two adjacent R^{a3}, together with the carbon atoms to which they are attached, form 5-6 membered heterocyclyl or a 5-10 membered heteroaromatic ring, wherein the 5-6 membered heterocyclyl or 5-10 membered heteroaromatic ring is optionally substituted with one or more substituents selected from halogen, hydroxy, amino, -CN, =O, C₁₋₆ alkyl, and -C₁₋₆ alkyl NR⁷⁴R⁷⁵;
R⁴³, R⁴⁴, R⁵⁰, and R⁵³ are each independently selected from H, hydroxy, C₁₋₆ alkyl, and C₁₋₆ alkoxy;
R⁴⁵, R⁴⁶, R⁴⁷, R⁴⁸, R⁴⁹, R⁵¹, R⁵², R⁵⁴, R⁵⁷, R⁵⁹, R⁶², R⁶³, R⁶⁸, R⁷¹, R⁷², R⁷³, R⁷⁴, and R⁷⁵ are each independently selected from H, C₁₋₆ alkyl, and C₁₋₆ haloalkyl;
R⁵⁵, R⁵⁶, R⁶⁶, and R⁶⁷ are each independently selected from H, hydroxy, -CN, C₁₋₆ alkyl, -C(O)C₁₋₆ alkyl, -C(O)C₂₋₆ alkenyl, -C(O)C₂₋₆ alkynyl, C₃₋₆ membered cycloalkyl, and 3-6 membered heterocyclyl, wherein the C₁₋₆ alkyl, C₂₋₆ alkenyl, C₂₋₆ alkynyl, C₃₋₆ membered cycloalkyl, and 3-6 membered heterocyclyl are optionally substituted with halogen, hydroxy, amino, -CN, N₃, C₁₋₆ alkyl, C₁₋₆ alkoxy, and C₁₋₆ alkylamino;
R⁵⁸ is independently selected from halogen and C₁₋₆ alkyl, wherein the C₁₋₆ alkyl is optionally substituted with one or more substituents selected from halogen, hydroxy, amino, C₁₋₆ alkoxy, C₁₋₆ alkylamino, and N₃;
R⁶⁰ and R⁶¹ are each independently selected from C₁₋₆ alkyl, wherein the C₁₋₆ alkyl is optionally substituted with one or more substituents selected from halogen, hydroxy, amino, C₁₋₆ alkoxy, C₁₋₆ alkylamino, and N₃;
R⁶⁴ is independently selected from halogen, amino, and C₁₋₆ alkylamino;
R⁶⁵ is independently selected from halogen, N₃, C₁₋₆ alkyl, and C₁₋₆ haloalkyl;
R⁶⁹ and R⁷⁰ are each independently selected from H, halogen, -CN, C₁₋₆ alkyl, C₁₋₆ haloalkyl, -C(O)OC₁₋₆ alkyl, - C(O)NH₂, and -C(O)NHC₁₋₆ alkyl, wherein the C₁₋₆ alkyl and C₁₋₆ haloalkyl are optionally substituted with hydroxy and amino; or R⁶⁹ and R⁷⁰, together with the boron and oxygen atoms to which they are attached, form 5-10 membered heterocyclyl (e.g., 5-6 membered heterocyclyl), wherein the heterocyclyl is optionally substituted with one or more substituents selected from hydrogen, halogen, hydroxy, C₁₋₄ alkyl, and =O;
L¹ and L² are each independently selected from O and N.

15. The compound, or the pharmaceutically acceptable salt, the stereoisomer, the solvate, the isotopically labeled compound, or the polymorph thereof according to any one of claims 13-14, wherein R^{a} is selected from phenyl, pyridine, and pyridazine substituted with one or more R^{3a}, wherein each R^{3a} is independently selected from H, halogen, -N₃, -NO₂, -SCN, -S(F)₅, -CH(O), C₂₋₆ alkenyl, C₂₋₆ alkynyl, C₃₋₆ cycloalkyl, 3-6 membered heterocyclyl, 5-6 membered heteroaryl, -C(O)NR⁴³R⁴⁴, -C(O)NR⁴⁵S(O)₂NR⁴⁶R⁴⁷, -C(S)NR⁴⁸R⁴⁹, -C(=NR⁵⁰)NR⁵¹R⁵², -C(=NR⁵³)R⁵⁴, - NR⁵⁵R⁵⁶, -NR⁵⁷S(O)₂R⁵⁸, -NR⁵⁹S(O)₂NR⁶⁰R⁶¹, -N=S(O)R⁶²R⁶³, -OS(O)₂R⁶⁴, -S(O)₂R⁶⁵, -S(O)₂NR⁶⁶R⁶⁷, -Si(OR⁶⁸)₃, and 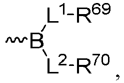 wherein the C₂₋₆ alkenyl, C₂₋₆ alkynyl, C₃₋₆ cycloalkyl, 3-6 membered heterocyclyl, and 5-6 membered heteroaryl are optionally substituted with one or more substituents selected from halogen, hydroxy, -CN, amino, C₁₋₆ alkyl, C₁₋₆ haloalkyl, C₁₋₆ alkoxy, C₁₋₆ haloalkoxy, -C(O)OR⁷¹, and -C(O)NR⁷²R⁷³; or two adjacent R^{a3}, together with the carbon atoms to which they are attached, form 5-6 membered heterocyclyl or a 5-10 membered heteroaromatic ring, wherein the 5-6 membered heterocyclyl or 5-10 membered heteroaromatic ring is optionally substituted with one or more substituents selected from halogen, hydroxy, amino, -CN, =O, C₁₋₆ alkyl, and -C₁₋₆ alkyl NR⁷⁴R⁷⁵.

16. The compound, or the pharmaceutically acceptable salt, the stereoisomer, the solvate, the isotopically labeled compound, or the polymorph thereof according to any one of claims 13-15, wherein R^{a} is selected from phenyl, pyridine, and pyridazine substituted with one or more R^{3a}, wherein each R^{3a} is independently selected from H, F, N₃, -SCN, N(H)CN, -S(O)₂CHF₂, -S(O)₂CF₃, SF₅, -NO₂, -S(O)₂N(H)CN, -N=S(O)(CH₃)₂, -OS(O)₂NH₂, -NH(OH), - NHS(O)₂NH₂, -NHS(O)₂CH₂CH₃, C(S)NH₂, -S(O)₂N₃, -OS(O)₂F, -NHS(O)₂F, -S(O)₂NH₂, -CH(O), -C(O)NH(OH), 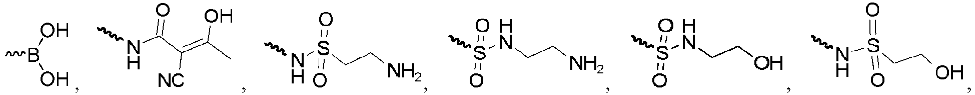 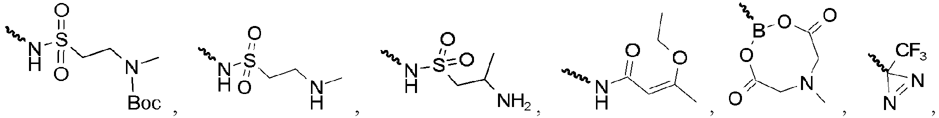 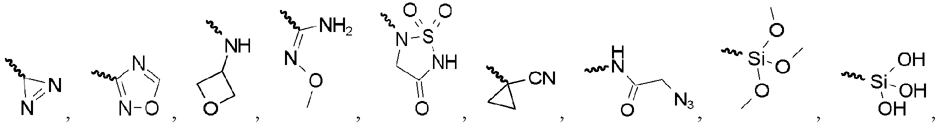 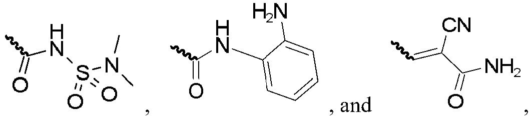 or two adjacent R^{a3}, together with the carbon atoms to which they are attached, form

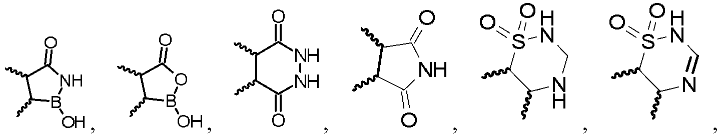

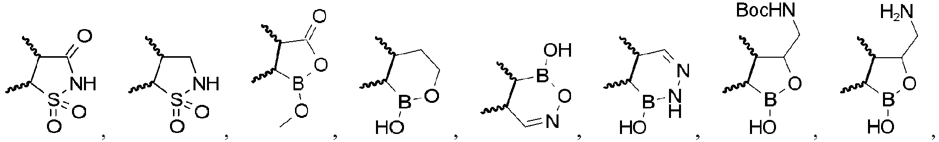

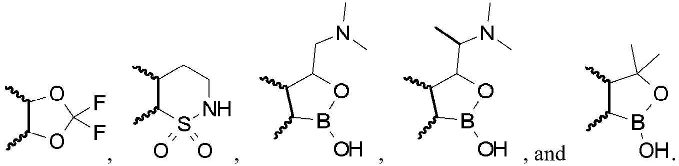

17. The compound, or the pharmaceutically acceptable salt, the stereoisomer, the solvate, the isotopically labeled compound, or the polymorph thereof according to any one of claims 13-16, wherein R^{a} is selected from 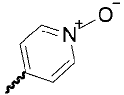 substituted with one or more R^{3a}, wherein each R^{3a} is independently selected from 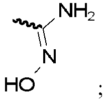
or, R^{a} is selected from 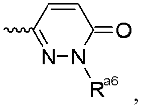 wherein each R^{a6} is selected from H;
or, R^{a} is selected from 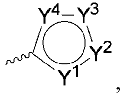 wherein Y¹, Y², Y³, and Y⁴ are each independently selected from O, S, N, N-R^{a1}, and C-R^{a2}; when present, each R^{a1} is independently selected from -S(O)₂-C₃₋₆ cycloalkyl and -S(O)₂-C₁₋₆ haloalkyl; each R^{a2} is independently selected from C₁₋₆ alkyl;
or, R^{a} is selected from 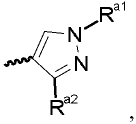 and each R^{a1} is independently selected from -S(O)₂-cyclopropyl; each R^{a2} is independently selected from methyl.

18. The compound, or the pharmaceutically acceptable salt, the stereoisomer, the solvate, the isotopically labeled compound, or the polymorph thereof according to any one of claims 13-17, wherein R^{a} is selected from:

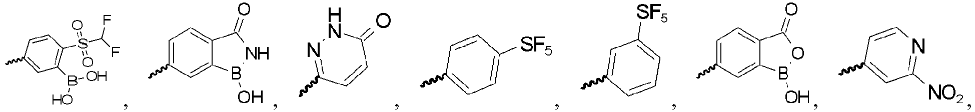

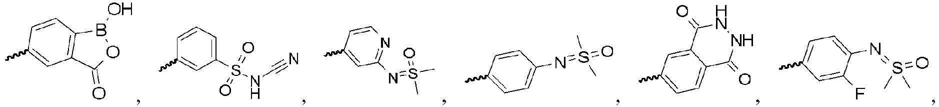

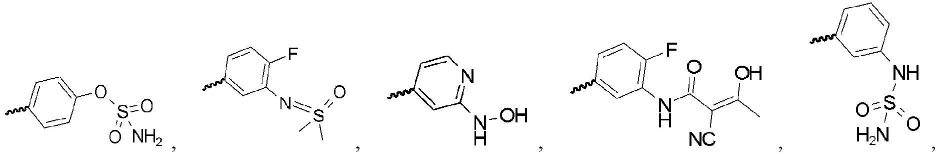

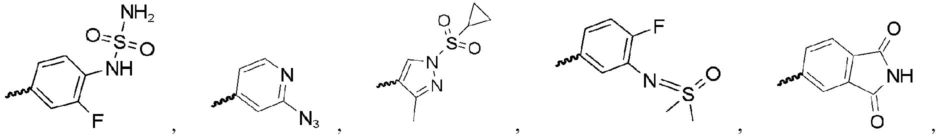

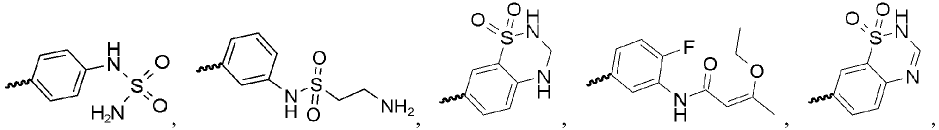

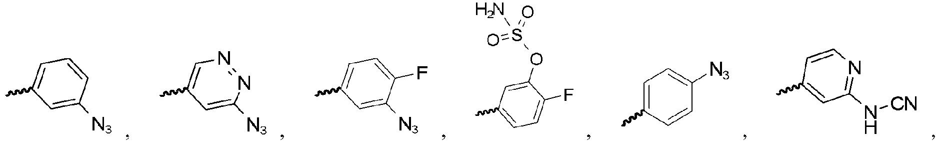

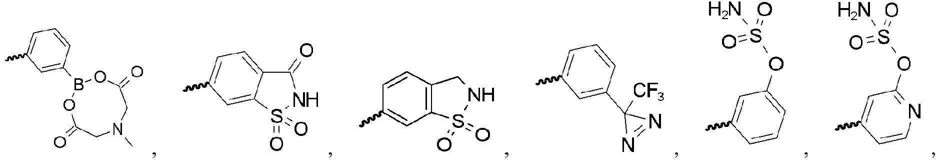

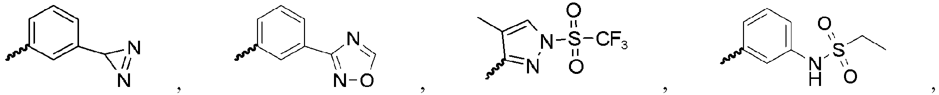

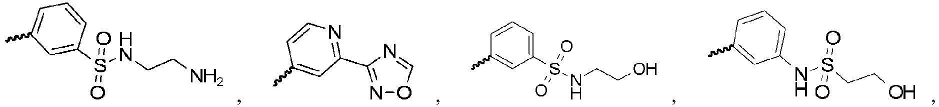

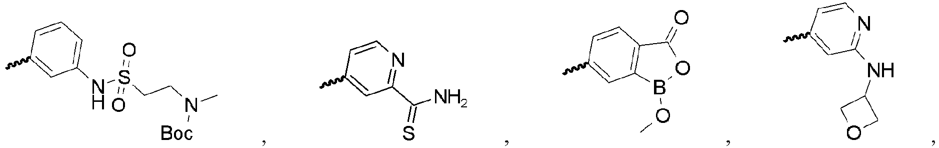

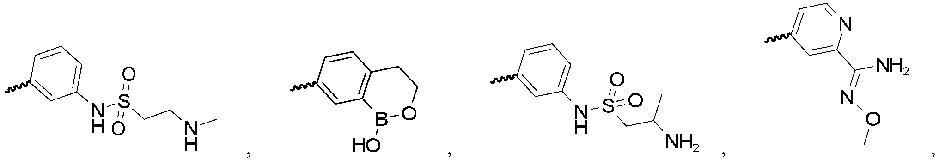

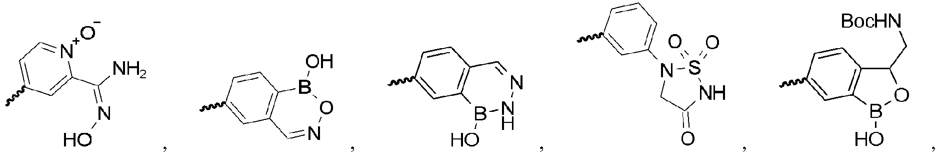

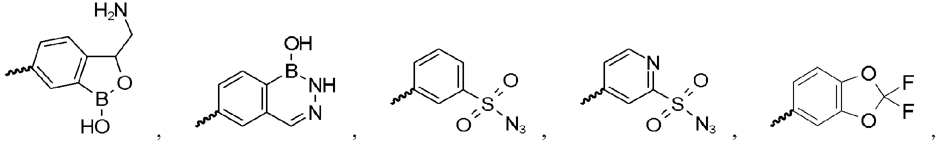

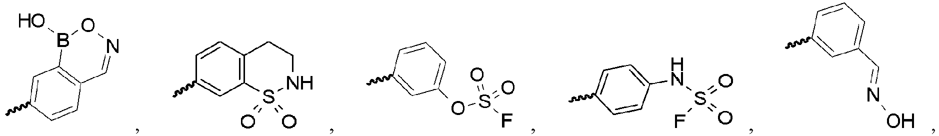

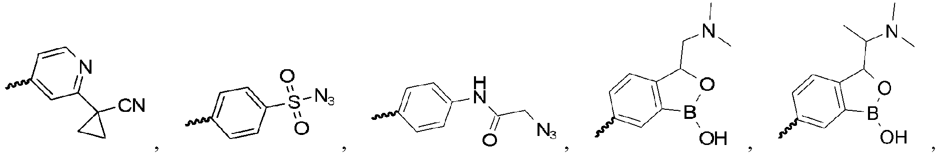

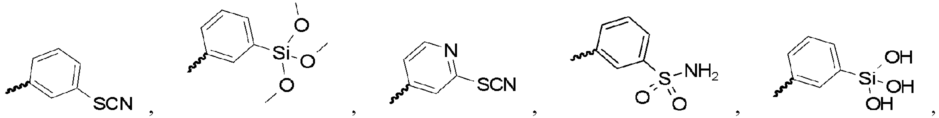

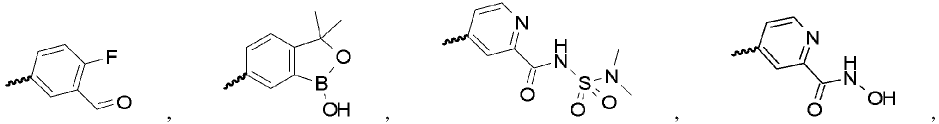

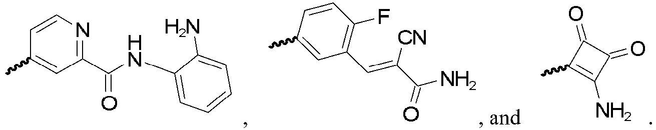

19. The compound, or the pharmaceutically acceptable salt, the stereoisomer, the solvate, the isotopically labeled compound, or the polymorph thereof according to any one of claims 13-18, wherein the compound is selected from:

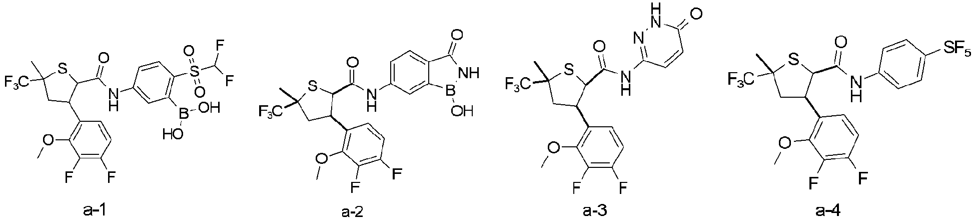

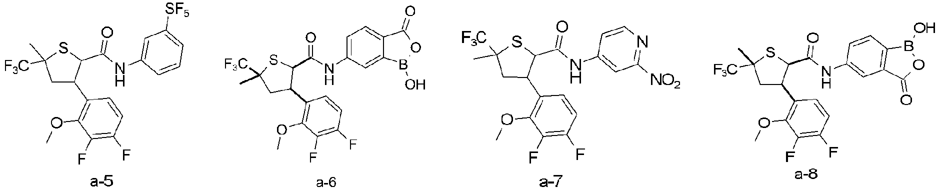

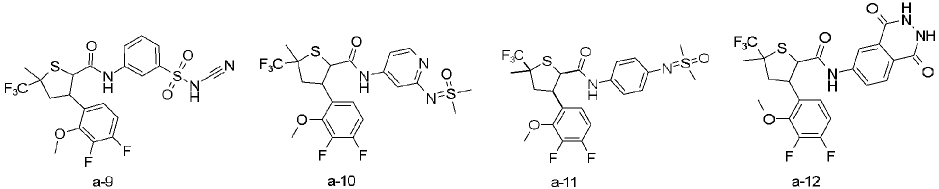

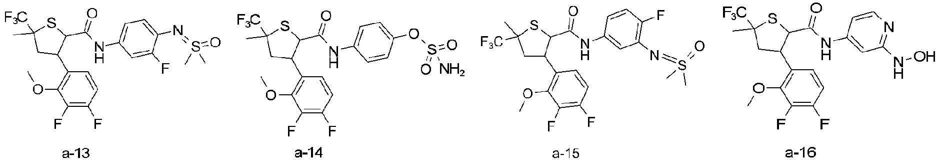

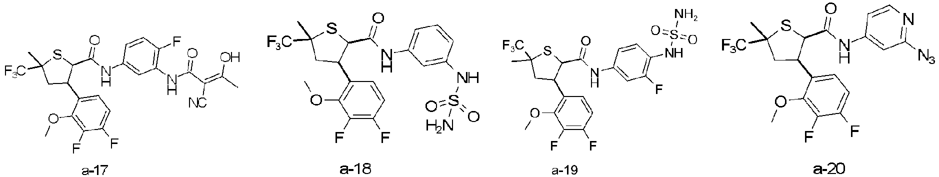

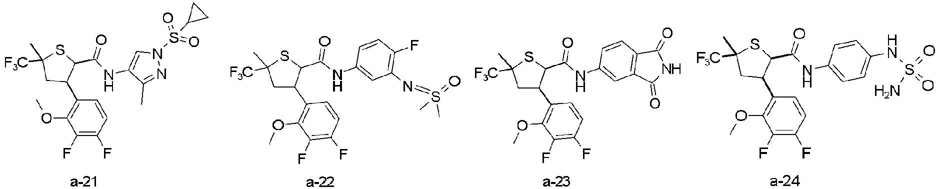

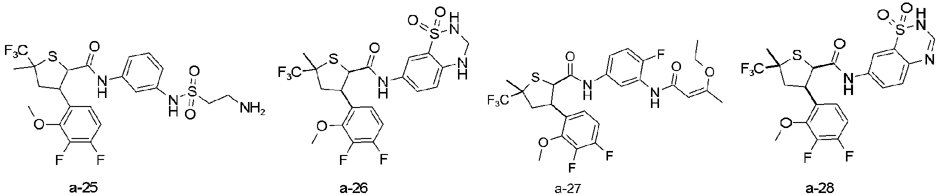

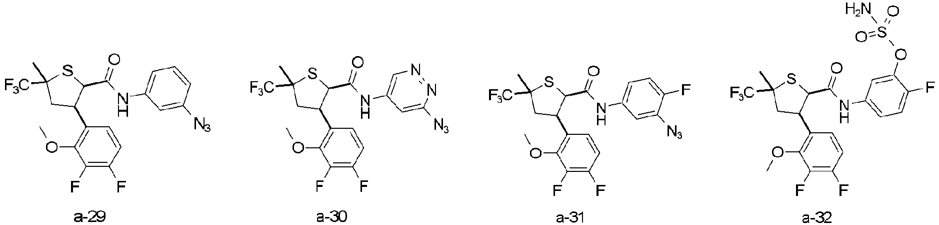

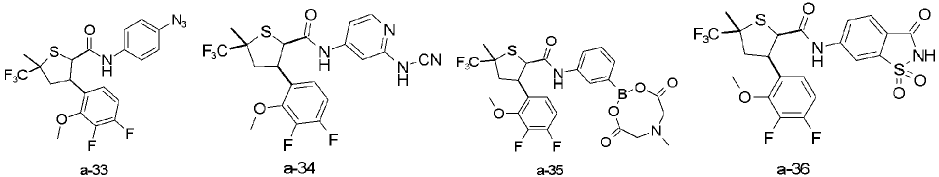

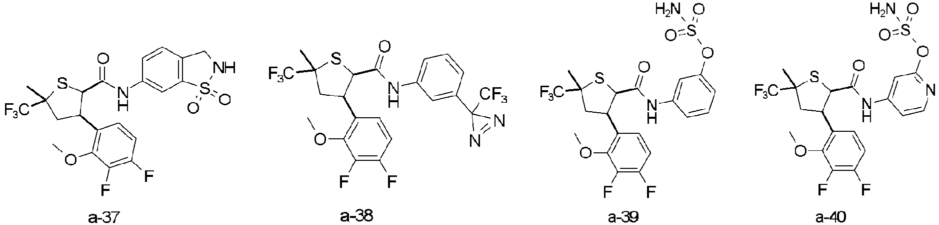

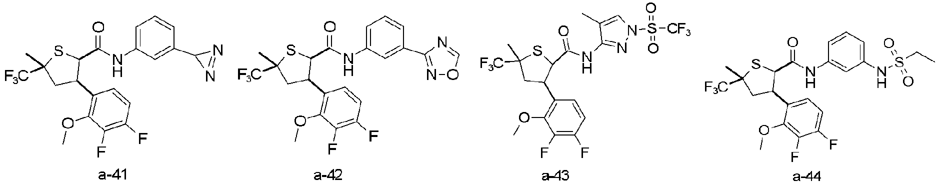

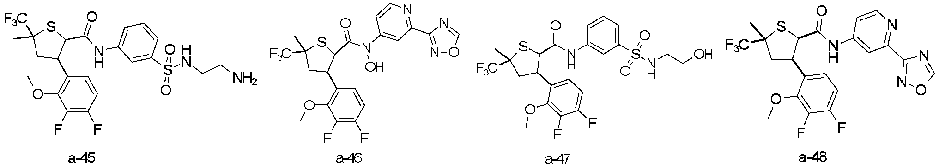

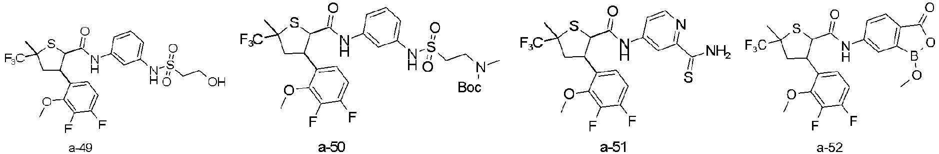

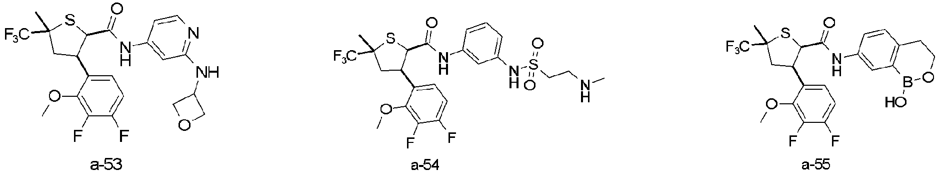

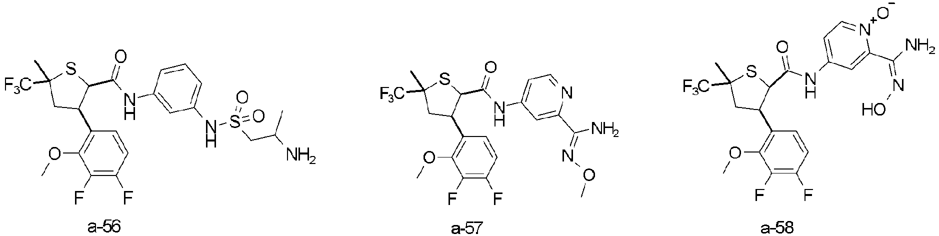

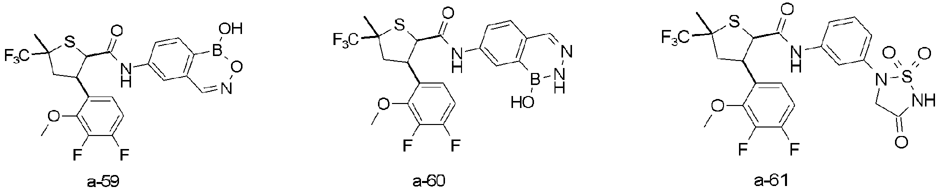

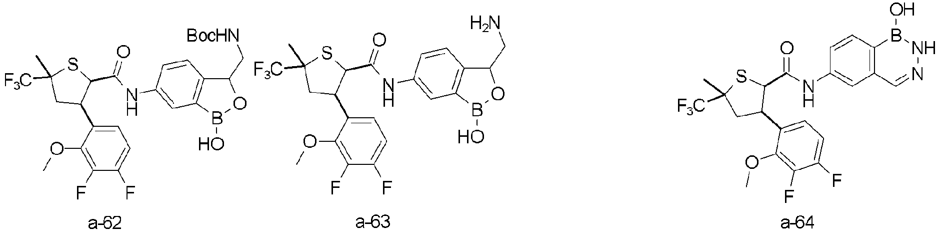

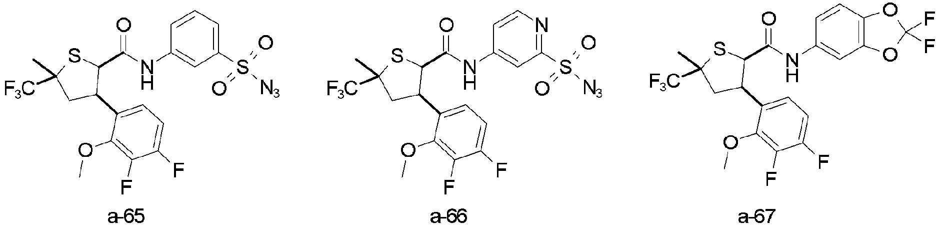

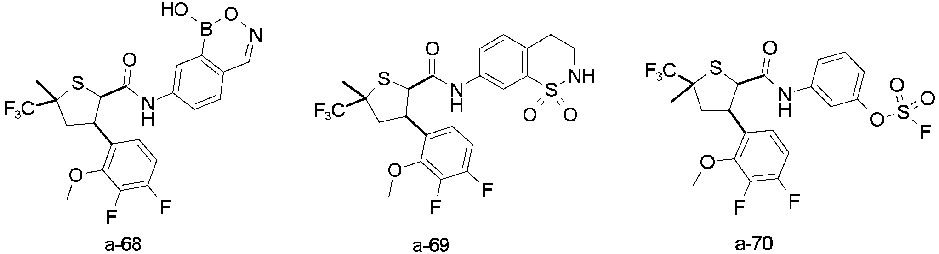

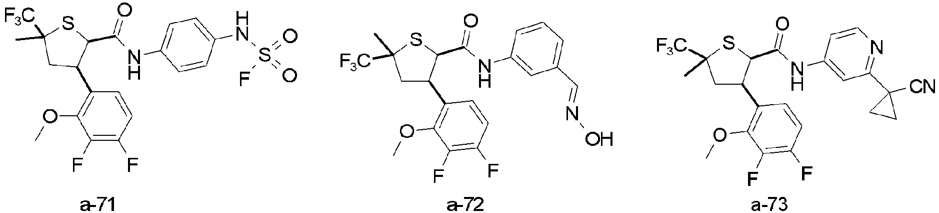

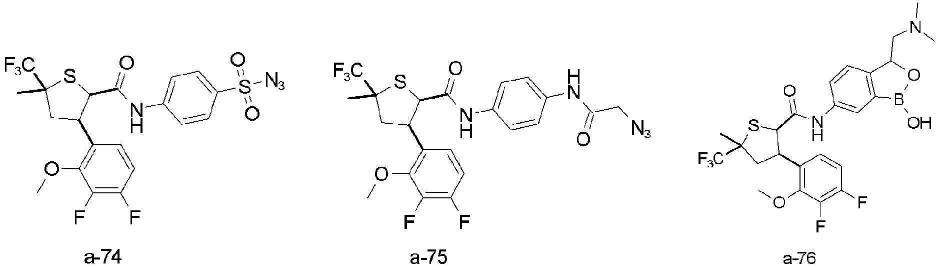

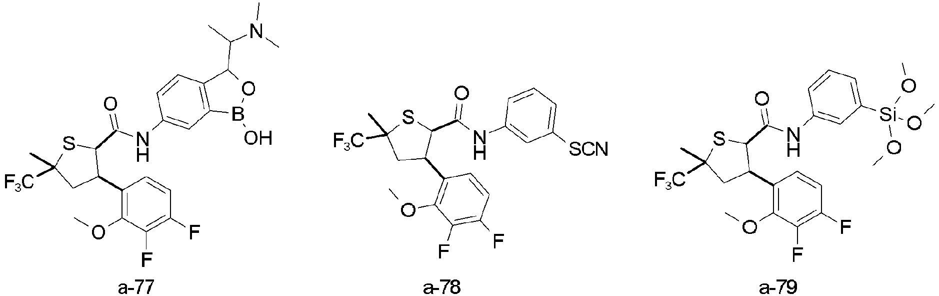

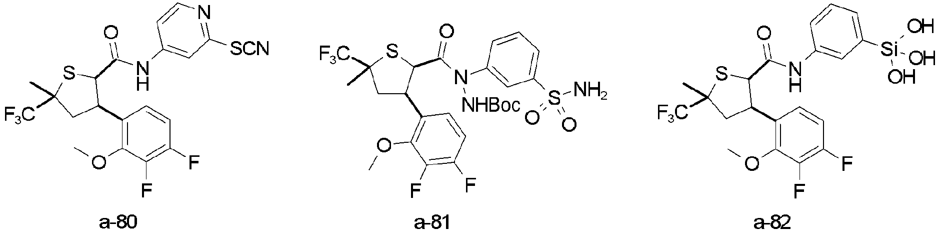

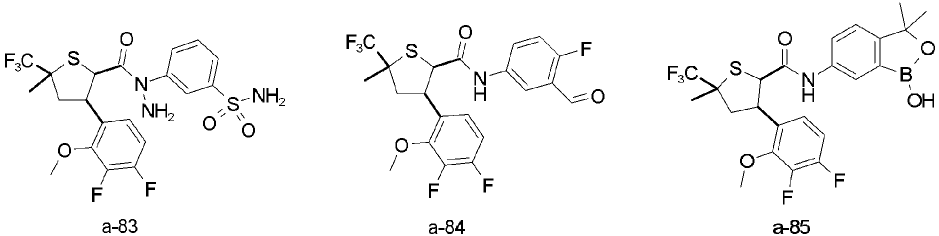

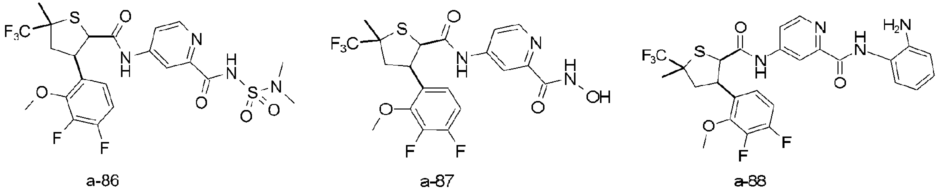

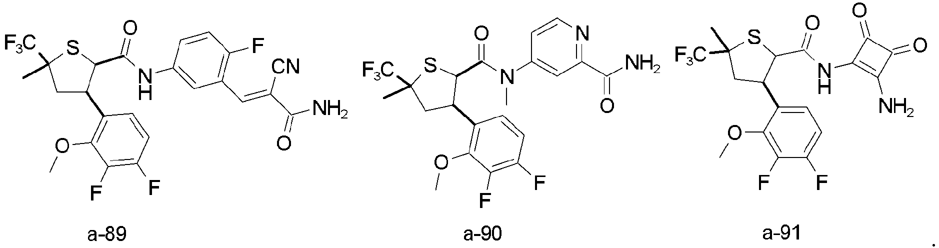

20. The compound, or the pharmaceutically acceptable salt, the stereoisomer, the solvate, the isotopically labeled compound, or the polymorph thereof according to any one of claims 13-19, wherein the compound is selected from:

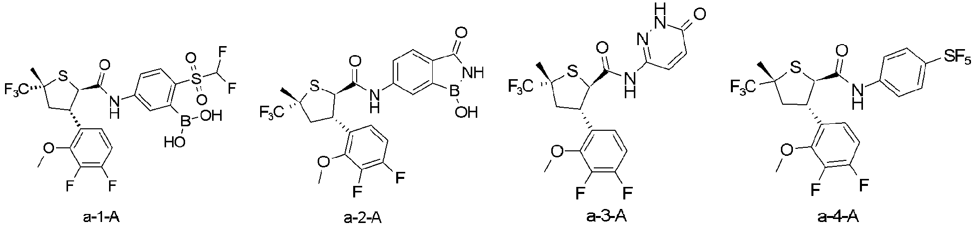

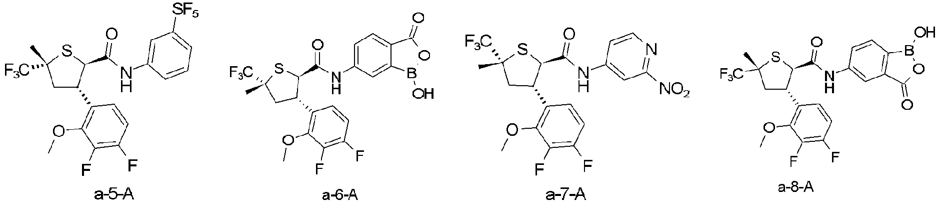

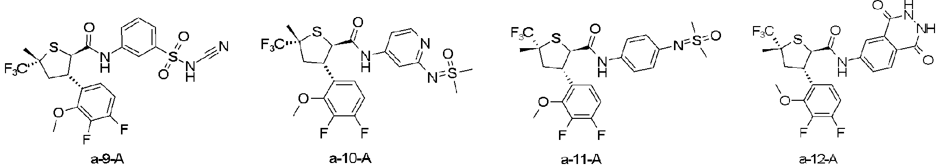

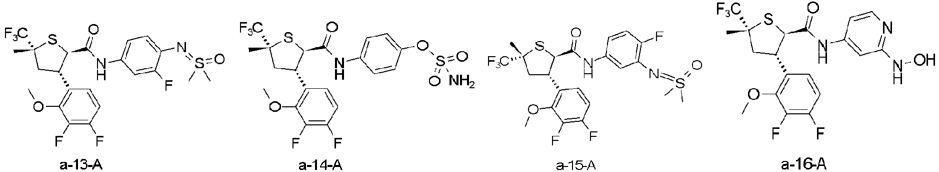

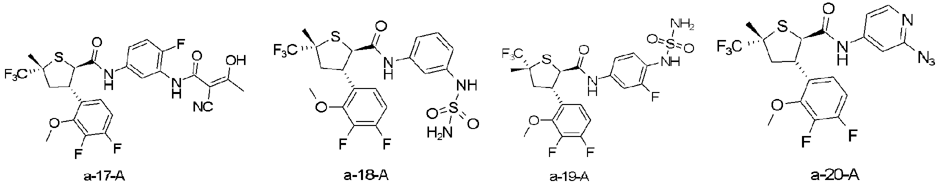

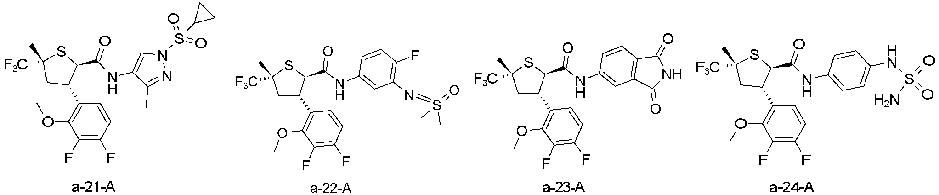

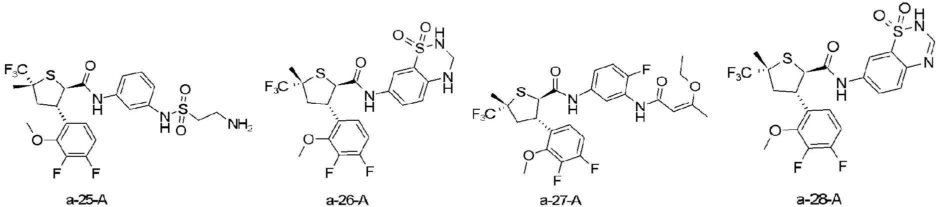

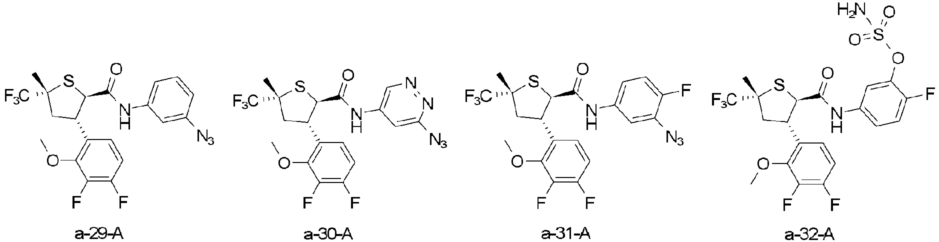

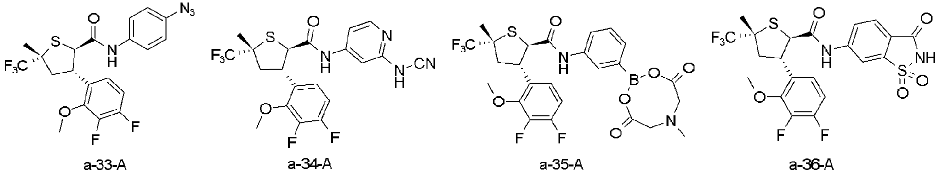

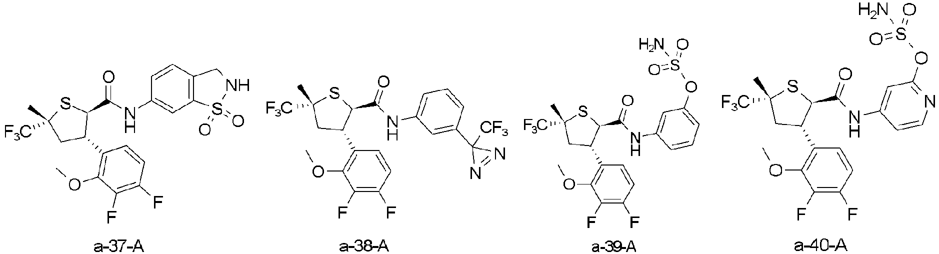

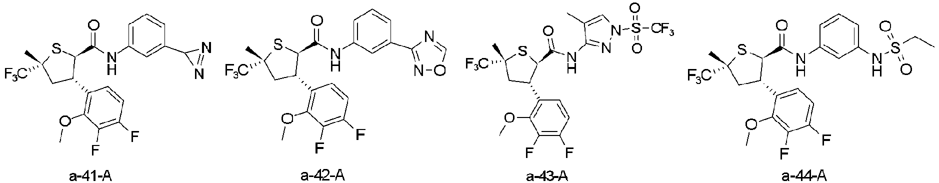

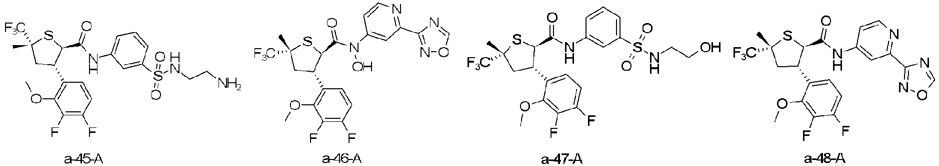

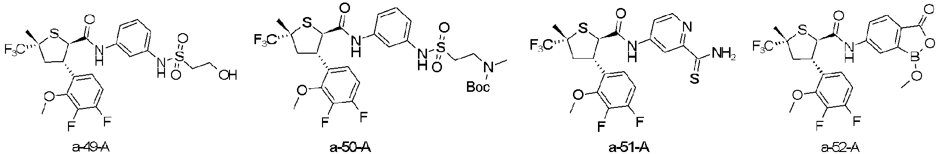

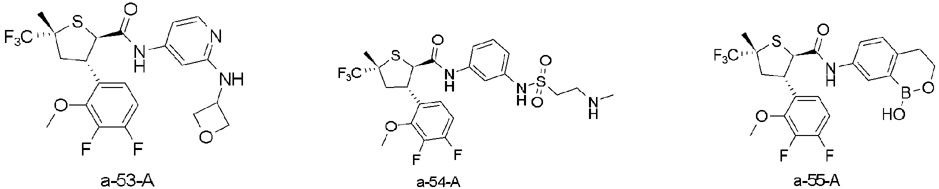

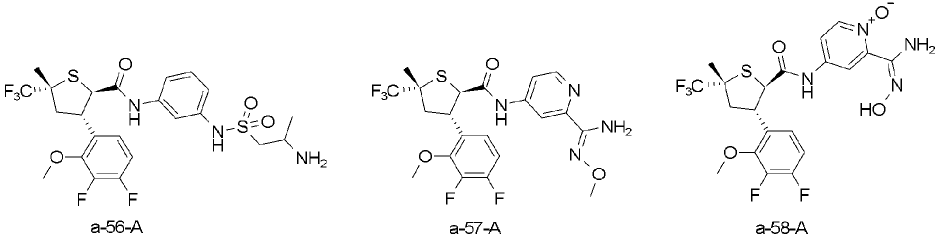

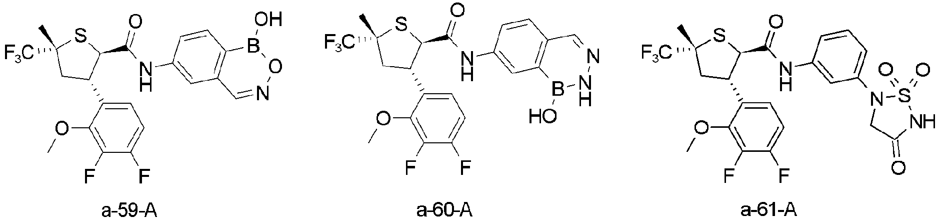

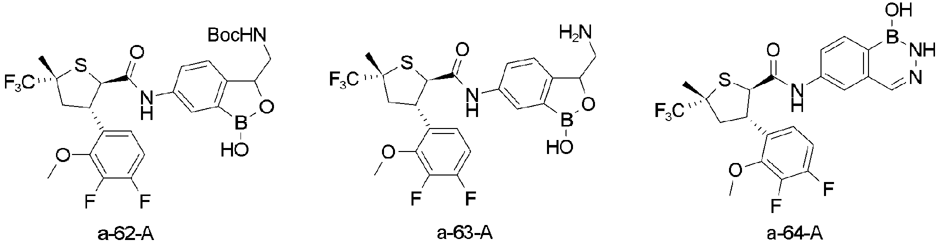

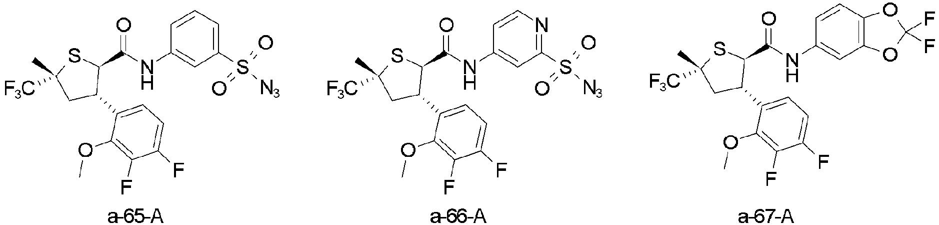

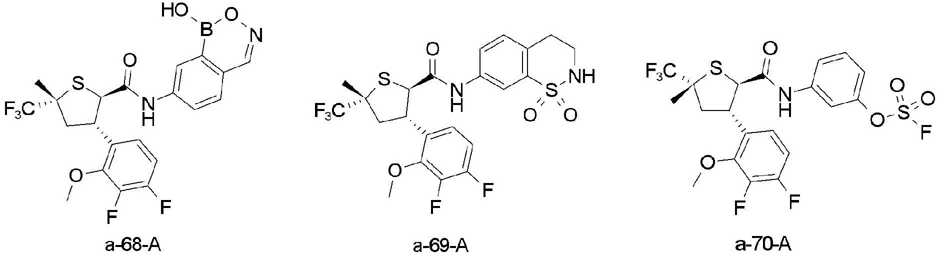

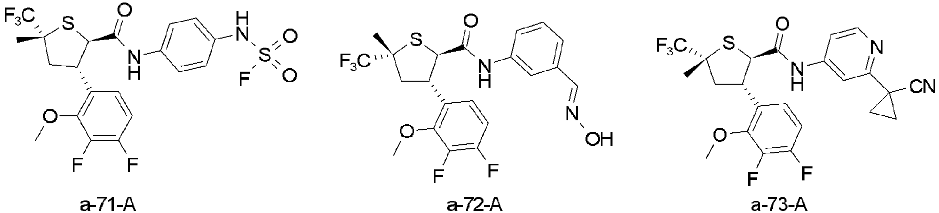

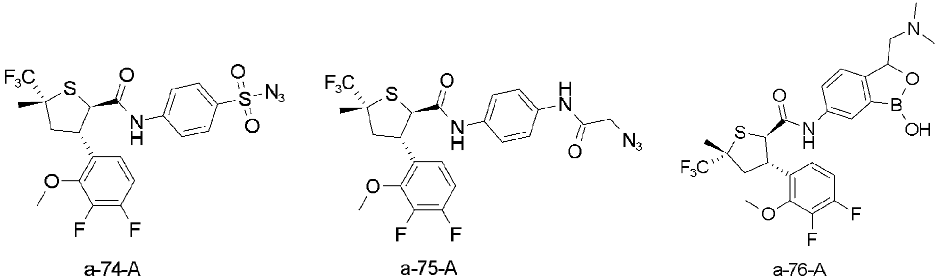

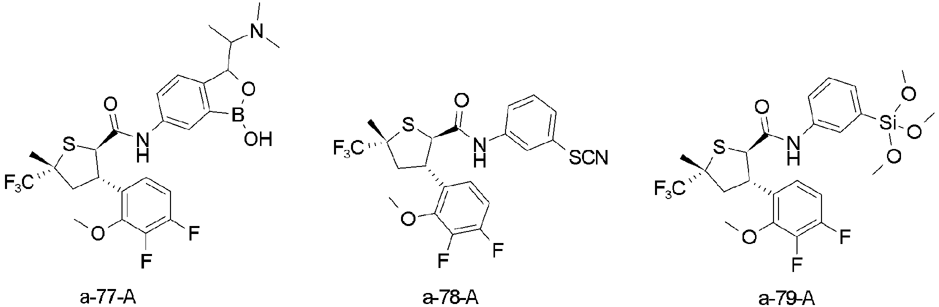

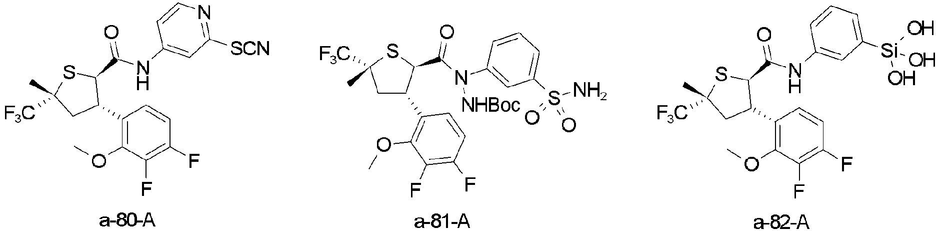

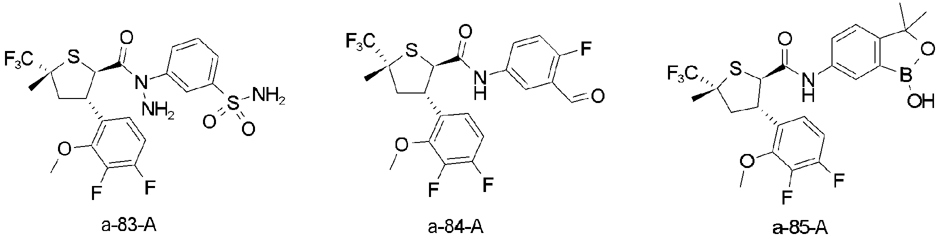

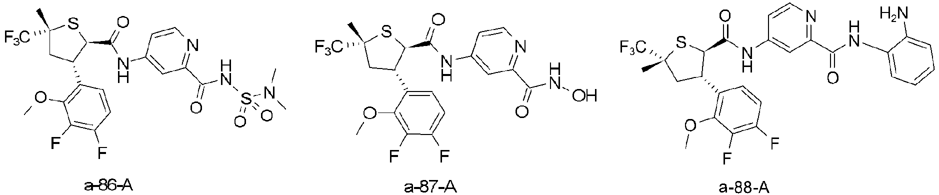

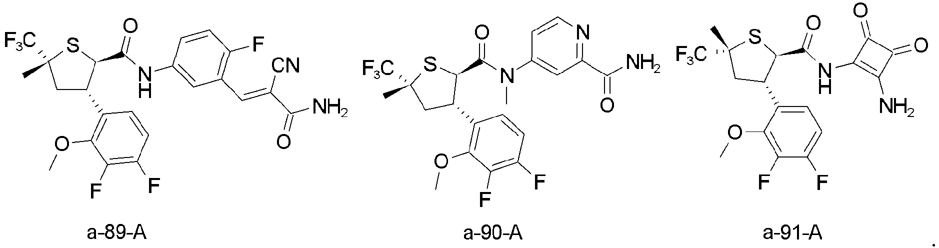

21. A pharmaceutical composition, comprising a prophylactically and/or therapeutically effective amount of the compound, or the pharmaceutically acceptable salt, the stereoisomer, the solvate, the isotopically labeled compound, or the polymorph thereof according to any one of claims 1-20, and one or more pharmaceutically acceptable carriers.

22. Use of the compound, or the pharmaceutically acceptable salt, the stereoisomer, the solvate, the isotopically labeled compound, or the polymorph thereof according to any one of claims 1-20, or the pharmaceutical composition according to claim 21 in the preparation of a medicament for treating and/or alleviating a NaV1.8-associated disease, wherein preferably, the NaV1.8-associated disease is pain, multiple sclerosis, Charcot-Marie-Tooth syndrome, incontinence, pathological cough, or arrhythmia.

23. The compound, or the pharmaceutically acceptable salt, the stereoisomer, the solvate, the isotopically labeled compound, or the polymorph thereof according to any one of claims 1-20, or the pharmaceutical composition according to claim 21, for use in the treatment and/or alleviation of a NaV1.8-associated disease, wherein preferably, the NaV1.8-associated disease is pain, multiple sclerosis, Charcot-Marie-Tooth syndrome, incontinence, pathological cough, or arrhythmia.

24. A method for treating and/or alleviating a NaV1.8-associated disease, comprising administering to an individual a therapeutically effective amount of the compound, or the pharmaceutically acceptable salt, the stereoisomer, the solvate, the isotopically labeled compound, or the polymorph thereof according to any one of claims 1-20, or the pharmaceutical composition according to claim 21, wherein preferably, the NaV1.8-associated disease is pain, multiple sclerosis, Charcot-Marie-Tooth syndrome, incontinence, pathological cough, or arrhythmia.

25. The use according to claim 22 or 23 or the method according to claim 24, wherein the pain is selected from chronic pain, acute pain, inflammatory pain, cancer pain, ICU analgesia, fracture or post-operative pain (e.g., bunionectomy pain, herniorrhaphy pain, and abdominoplasty pain), neuropathic pain (e.g., peripheral neuropathyrelated neuralgia, postherpetic neuralgia, peripheral neuralgia, small fiber neuropathy pain, trigeminal neuralgia, idiopathic small fiber neuralgia, or diabetic neuralgia), visceral pain (e.g., intestinal pain), musculoskeletal pain, primary pain, idiopathic pain, osteoarthritis pain, gouty arthritis pain, rheumatic or rheumatoid arthritis pain, odontalgia, arthralgia, labor pain, fibromyalgia, chronic low back pain, bladder pain syndrome, and sciatica; preferably, the pain is selected from post-operative pain, neuropathic pain (e.g., postherpetic neuralgia, small fiber neuropathy pain, and diabetic neuralgia), osteoarthritis pain, bladder pain syndrome, and cancer pain.
